# EUROPEAN PATENT APPLICATION

(11) **EP 3 831 841 A1**
(43) Date of publication of application: **09.06.2021**
(21) Application number: 20202443.6
(22) Date of filing: 16.05.2017
(51) Int. Cl.: C07K 7/64

(54) **NOVEL CYCLOSPORIN DERIVATIVES AND USES THEREOF**

(30) Priority: 17.05.2016 US 201662337377 P; 20.05.2016 US 201662339464 P; 08.09.2016 US 201662384822 P
(62) Divisional of application: 17799977.8
(71) Applicant: S&T Global Inc., Woburn, MA 01801 (US)
(72) Inventor: SU, Zhuang, Andover, MA 01810 (US); YANG, Suizhou, Dracut, MA 01826 (US); LONG, Zhengyu, Bolton, MA 01740 (US)
(74) Representative: HGF

(57) **Abstract**

A compound of the Formula (I) is disclosed: 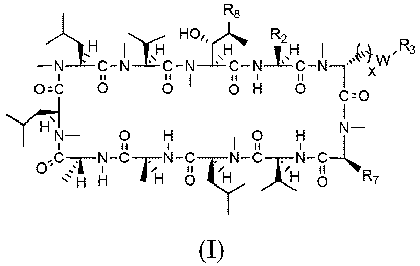 or pharmaceutically acceptable salt thereof, wherein the symbols are as defined in the specification. Also described are a pharmaceutical composition comprising the same and a method for treating or preventing viral infections, inflammation, dry eye, central nervous disorders, cardiovascular diseases, cancer, obesity, diabetes, muscular dystrophy, lung, and liver, and kindey diseases, and hair loss using the same.

## Description

### Related Application

This application is related to U.S. Application Serial No. 13/840,088, filed March 15, 2013. This application claims priority to U.S. Provisional Application No. 62/337,377, filed May 17, 2016, to U.S. Provisional Application No. 62/339,464, filed May 20, 2016, and to U.S. Provisional Application No. 62/384,822, filed September 8, 2016. The entire contents of these application are expressly incorporated by reference in its entirety.

### Field of the Invention

The invention relates to novel cyclosporine derivatives, their pharmaceutical compositions comprising the same, and methods for treating or preventing viral infections, inflammation, dry eye, central nervous disorders, liver, lung and kidney diseases, cardiovascular diseases, cancer, obesity, diabetes, muscular dystrophy, hair loss and so on.

### Background of the Invention

Cyclosporins in nature are poly-N-methyl, cyclic undecapeptides, isolated from fungi. Cyclosporin A has an immunosuppressive activity and has been used for almost 33 years to prevent rejection in kidney, heart and liver transplant recipients. It possesses anti-inflammatory properties and has been used for treating severe rheumatoid arthritis, severe psoriasis, Behget's uveitis, and dry eye disease. In addition, it is useful for treating severe ulcerative colitis, Crohn's disease, alopecia areata, aplastic anemia, HSV-1 stromal keratitis, systemic lupus erythematosus, and severe lupus nephritis. However, its strong immunosuppressive activity limits its applications in many diseases.

The anti-HIV activity of cyclosporin A was first discovered in 1986 and has been continually studied since then (Klatzmann, D., et al., 1986, C R Acad. Sci. III, 303(9):343-8; Wainberg, M. A., et al., 1988, Blood, 72, 1904-10; Luban, J., et al., 1993, Cell, 73, 1067-1078; each of which is incorporated herein by reference). Its non-immunosuppressive derivative, NIM-811, was reported to have potent anti HIV activity due to its ability to inhibit cyclophilin A (Franke, E. K., et al., 1994, Nature, 372, 359-362; Thali, M., et al., 1994, Nature, 372, 363-365; Gamble, T. R., et al., 1996, Cell, 87, 1157-1159; Rosenwirth B., et al., 1994, Antimicrob. Agents Chemother., 38, 1763-1772; each of which is incorporated herein by reference).

Cyclosporin A and its non-immunosuppressive derivatives, as such as NIM-811 (N-MeIle-4-Cyclosporin), Debio-025, SCY-635, EDP-494, DEP-546, NIM-258, CPI-431-32 (CRV431), and STG-175 and bind and inhibit cyclophilins, subsequent to prevent HCV RNA replication and protein synthesis. As a result, these compounds have an effective anti-HCV activity (Watashi, K., et al., 2007, Rev. Med. Virol., 17:245-252.37; Inoue, K., et al., 2001, Nippon Rinsho., 59, 1326-30; Inoue, K., et al., 2003, J. Gastroenterol., 38, 567-72; Watashi, K., et al., 2003, Hepatology, 38, 1282-8; Gaither, L. A., et al., 2010, Virology, 397, 43-55; Rhodin, M. H. J., et al, 2016, EASL, Poster 250, and the news of the 34th Annual L. P. Morgan Healthcare, Conference Presentation on Jan. 13, 2016 at 11:00AM by Enanta Pharmaceutical. Inc. (www.enanta.com) (EDP-494); Baugh, J. M., et al, 2013, Antiviral Res. 100(2): 555-561 (EDP-546 included); Fu, J., et al, 2014, J. Med. Chem., 57, 8503-8516 (NIM-258); Gallay, P. A., et al, 2015, PLoS One 10(8):e0134707.doi: 10.1371/journal.pone.0134707 (CPI-431-32(CRV431)); Gallay, P. A., et al, 2016, PLoS One 11(4):e0152036. doi: 10.1371/journal.pone.0152036 (STG-175); each of which is incorporated herein by reference). NIM-811, Debio-025, and SCY-635 had been evaluated in clinical trials phase II and III against HCV, and EDP-494 phase I study is beginning in the first quarter of 2016 for resistance-associated variants of HCV.

NIM-811 and Debio-025 have a chemical structure similar to cyclosporine A and possess a poor pharmacokinetic profile. In addition, they are metabolized by P450 for inducing drug interactions (Lill, J., et al., 2000, Am J Health-Syst Pharm 57, 1579; incorporated herein by reference).

SCY-635 has an improved pharmacokinetic profile and low blood serum binding. In addition, it has a low potential for drug-drug interactions. SCY-635's *in vitro* anti-HCV activity (EC₅₀) was reported to be 0.10 µM (Hopkins, S. et al., 2010, Antimicrob. Agents Chemother., 54, 660-672, incorporated herein by reference). However, SCY-635 is not chemically stable, as it is easily converted to its diastereoisomer by epimerization. Its diasteroisomer is expected to have poor binding activity with cyclophilins, and as a result, its anti-viral activity *in vivo* may be affected (See, e.g., WO2012/009715, WO2012/021796, and WO2012/075494, each of which incorporated herein by reference in its entirety).

Cyclosporin A and its non-immunosuppressive derivatives were also found to possess anti-HBV activity through the inhibition of cyclophilins (Chokshi, S., et al., 2012, Gut 61:A11; Chokshi, S., et al., 2012, Poster Presentations, 47th Annual Meeting of the European Association for the Study of the Liver (EASL 2012), Barcelona, Spain; Chokshi, S., et al., 2011, Abstract 190 (Poster Presentations), 46th Annual Meeting of the European Association for the Study of the Liver (EASL 2011), Berlin, March 30-April 3; Tian, X. C., et al., 2010, J. Virol., 84, 3373-3381; Xia, W. L., et al., 2004, Hepatobiliary Pancreat Dis Int., 4, 18-22; Michael, J., et al., 2003, J. Virol., 77, 7713-7719; each of which is incorporated herein by reference).

Furthermore, cyclophilins were reported to regulate the life cycle and pathogenesis of several viruses, including severe acute respiratory syndrome coronavirus, vaccinia virus, and herpes simplex virus (Castro, A. P., et al., 2003, J. Virol., 77, 9052-9068; Chen, Z., L., et al., 2005, J. Infect. Dis. 191(5):755-760; Arai, C., et al., Nihon RinshoMeneki Gakkai Kaishi., 35(1), 87-91; Labetoulle, M., 2012, J Fr Ophtalmol., 35(4), 292-307; De Clercq, E., 2008, Expert Opin Emerg Drugs., 13(3):393-416; Vahlne, A., 1992, Arch Virol., 122(1-2):61-75; each of which is incorporated herein by reference). Cyclosporin A and its non-immunosuppressive derivatives also possess such anti-viral activities.

N-MeVal-4-Cyclosporin (SDZ 220-384), another non-immunosuppressive cyclosporine derivative, was reported to have similar biological activities to that of NIM-811 (Fliri, H., et al., 1993, Ann. N YAcad Sci. 696, 47-53; Zenke, G., et al., 1993, Ann N YAcad Sci. 23;685:330-5).

Hepatitis C virus (HCV) is a small (55-65 nm in size), enveloped, positive sense single strand RNA virus in the Flaviviridae family. HCV has a high rate of replication and an exceptionally high mutation rate. About 80% of people infected with HCV develop chronic, persistent infection. More than 4 million Americans have been infected with HCV and more than 200 million people are estimated to be infected chronically worldwide. About 35,000 new cases of hepatitis C are estimated to occur in the United States each year. HCV infection is responsible for about 50% of all chronic liver disease, 30% of all liver transplants, and 30% of all cirrhosis, end-stage liver disease, and liver cancer in the U.S. The peg-interferon and ribavirin combination is the standard treatment for chronic hepatitis C, but it has low efficacy against HCV infection. Recently, the FDA has approved Vertex's Incivek (telaprevir) and Merck's Victrelis (boceprevir) as an add-on to the current interferon/ribavirin therapy for treating HCV. Both drugs are HCV protease inhibitors that target the virus to prevent its replication. However, due to HCV's fast mutation rate, drug resistance can be developed in a short period of time. Thus, there exists a need for an effective therapeutic for HCV treatment.

Hepatitis B virus (HBV) is a 42 nm partially double stranded DNA virus composed of a 27 nm nucleocapsid core (HBcAg) that is surrounded by an outer lipoprotein envelope containing the surface antigen (HBsAg). More than 2 billion people have been infected, and there are 350 million chronic carriers of the virus. The disease has caused epidemics in parts of Asia and Africa. Chronic hepatitis B will cause liver cirrhosis and liver cancer, a fatal disease with a very poor response to current chemotherapies. The infection is preventable by vaccination, and HBV load and replication can be reduced by current antiviral drugs, such as lamivudine (Epivir), adefovir (Hepsera), tenofovir (Viread), telbivudine (Tyzeka), entecavir (Baraclude), and the two immune system modulators interferon alpha-2a and PEGylated interferon alpha-2a (Pegasys). However, none of the available drugs can clear the infection. There remains a need for an effective therapeutic to treat HBV infection. Recently, NTCP has been identified as a HBV entry target (Yan, H., et al., 2012, eLife, 1:e00049; Yan, H., et al., J. Virol., 88(6):3273-84; Watashi, K., 2014, Int. J. Mol. Sci., 15(2):2892-905; Yan, H., et al., 2015, Antiviral. Res., 121:24-30.); Cyclosporin A and its analogs inhibit HBV entry with the NTCP receptor (Nkongolo, S., et al., 2014, J. Hepatol., 60(4):723-31; Watashi, K., 2014, Hepatology, 59(5):1726-37.) Cyclosporin analogs, Alisporivir and STG-175, also inhibit cyclophilin in hepatocyte cell and reduce replication of HBV RNA and HBsAg production and secretion (Phillips, S., et al., Gastroenterology, 148(2):403-14; Gallay, P. A., et al., PloS One, 11(4):e0152036. Doi: 10.1371/journal.pone.0152036.).

The non-immunosuppressive cyclosporin derivatives bind to cyclophilins, a family of host proteins that catalyze *cis-trans* peptidyl-prolyl isomerization in protein folding and regulation, which are crucial for the processing and maturation of the viral proteins for viral replication. HIV and HCV are viruses with a high mutation rate. All current anti-viral drugs target the virus itself; when the virus mutates, it leads to the development of drug resistance. Instead of directly targeting the virus, targeting host cofactors (cyclophilins) will be slow down the development of drug resistance due to a higher genetic barrier (Rosenwirth, B., et al., 1994, Antimicrob. Agents Chemother., 38, 1763-1772; Tang, H. L. et al., 2010, Viruses, 2, 1621-1634; Hopkins, S. et al., 2010, Oral Presentation, Scynexis's SCY-635 Demonstrates Impressive Barrier to Resistance in HCV Treatment, the 45th Annual Meeting of the European Association for the Study of the Liver (EASL 2010), Vienna, Austria, April 14-18; each of which is incorporated herein by reference). Cyclosporine derivatives affect a new target, cyclophilins, and therefore represent a new mechanism of action against viruses.

There are 19 cyclophilins in the human genome (Thapar, R., 2015, Biomolecules 5(2): 974-99.), but the functions of these cyclophilin isoforms are still unclear (Davis, T. L., et al., 2010, PLoSBiol. 8(7):e1000439; incorporated herein by reference). Cyclophilin A, B, C, D, and other such isoforms play an important role in the pathophysiology of a number of serious diseases, such as cancer (Campa, MJ., et al., 2003, Cancer Res., 63(7), 1652-6; Li, M., et al., 2006, Cancer, 106: 2284-94; Yang, H., et al., 2007, Biochem Biophys Res Commun., 361(3):763-7; Obchoei, S., et al., 2009, Med Sci Monit., 15(11), RA221-32; Andersson, Y., et al., 2009, Br J Cancer, 101, 1307-1315; Lee, J., 2010, Arch Pharm Res., 33(2), 181-7; Lee, J., et al., 2010, J Exp Clin Cancer Res., 29:97; Obchoei, S., 2011, Molecular Cancer, 10:102; Takahashi, M., et al., 2012, Oncol Rep., 27(1):198-203; Qian, Z., et al., 2010, BMC Cancer, 12:442; Lee, J., 2010, Arch. Pharm. Res., 33(9):1401-9; Hamilton, G., 2014, Curr. Cancer Drug Target, 14(1):46-58; Zhu, D., et al., 2015, Nat. Med., 21(6):572-80; Lavin, P. T., et al., 2015, Curr. Mol. Pharmacol., 9(2): 148-64; Seleh, T., et al., 2016, Nat. Chem. Biol., 12(2): 117-23; each of which is incorporated herein by reference), inflammations (the result of interactions between a secreted extracellular cyclophilin and CD-147, a surface protein; Yurchenko V., 2005, Immunology, 117(3):301-9; Yurchenko, V., 2010, Clin Exp Immunol., 160(3):305-17; Malesevic, M., 2010, Angew Chem Int Ed Engl., 49(1):213-5; each of which is incorporated herein by reference), cardiovascular diseases (including vascular stenosis, atherosclerosis, abdominal aortic aneurysms, aortic rupture, cardiac hypertrophy, pulmonary arterial hypertension, myocarditis and myocardial fibrosis, and ischaemic heart diseases, liver, kidney, and lung fibrosis, protection and regeneration; Jin, Z. G., et al., 2000, Circ Res., 87(9):789-96; Yurchenko, V., et al., 2005, Immunology, 117, 301-309; Suzuki, J., et al., 2006, Circ Res., 98(6):811-7; Satoh, K., et al., 2008, Circulation., 117(24):3088-98; Nishihara, M., et al., 2008, J Mol Cell Cardiol., 44(2):441-442; Satoh, K., et al., 2010, Circ J., 74(11):2249-56; Satoh, K., et al., 2010, Antioxid Redox Signal., 12(5):675-82; Hausenloy, D. J., et al., 2012, Br J Pharmacol. 165(5):1235-45; Coppinger, J. A., et al., 2004, Blood, 103(6):2096-104; Satoh, K., et al., 2010, Antioxid Redox Signal., 1:12(5), 675-682; Nigro, P., et al., 2010, J Exp Med., 208(1):53-66; Wang, W. L., et al., 2011, Med Hypotheses, 77(5):734-8; Hattori, F., 2012, J Mol Cell Cardiol., 53(1):1-2; Seizer P., 2012, J Mol Cell Cardiol., 53(1):6-14; Naoumov, N. V., 2014, L. Hepatol., 61(5):1166-74; each of which is incorporated herein by reference), rheumatoid arthritis (Wells, G., et al., 2000, Cochrane Database Syst Rev., (2):CD001083; Kim, H., et al., 2005, Clin Immunol., 116(3):217-24; Yang, Y., Rheumatology (Oxford), 47(9):1299-310; Yurchenko, V., et al., 2006, Immunology, 117(3):301-9; Damsker, J. M., 2009, Immunology, 126(1):55-62; Wang, L., et al., 2010, J Clin Immunol., 30(1):24-33; Billich A., et al., 1997, J Exp Med., 185:975-80; De Ceuninck F., et al., 2003, Arthritis Rheum., 48:2197-206; each of which is incorporated herein by reference), respiratory inflammation (Foda, H. D., et al., 2001, Am J Respir Cell Mol Biol., 25:717-24; Hasaneen, N. A., et al., FASEB J., 19:1507-9.Yurchenko, V., et al., 2006, Immunology, 117(3):301-9; Gwinn, W. M., 2006, J Immunol., 177(7):4870-9; Onoue, S., 2009, J Control Release., 138(1):16-23; Balsley, M. A., et al., 2010, J Immunol., 185(12):7663-70; Balsley, M., et al., 2010, Am. J. Respir. Crit. Care Med., 181(1): A6821; Stemmy, E. J., et al., 2011, J. Asthma, 48(10):986-993; Stemmy, E. J., et al., 2011, Am J Respir Cell Mol Biol., 45(5):991-8; Amin, K., 2012, Respir Med., 106(1):9-14; Onoue, S., 2012, Eur JPharm Biopharm., 80(1):54-60; each of which is incorporated herein by reference), lupus (Caccavo, D., et al., 1997, Arthritis & Rheumatism, 40(1):27-35; Dostál, C., et al., 1998, Lupus, 7(1):1 29-36; Tam, LS., et al., 1998, Q J Med., 91(8):573-580; Fu, LW., et al., 1998, Rheumatology 37 (2): 217-221; Hallegua, D., et al., 2009, Lupus, 9: 241-251; each of which is incorporated herein by reference), psoriasis (Ellis, C. N., 1991, N Engl J Med., 324, 277-284; Lebwohl, M., et al., 1998, J Am Acad Dermatol., 39(3):464-75; Rosmarin, DM., et al., 2010, J Am Acad Dermatol., 62(5):838-53; each of which is incorporated herein by reference), atopic dermatitis (Naeyaert, J. M., et al., 1999, Dermatology, 198:145-152; Pacor, ML., et al., 2001, Recenti ProgMed., 92(6):390-1; Ricci, G., et al., 2009, Drugs, 69(3):297-306; Simon, D., 2011, Curr Probl Dermatol., 41:156-64; each of which is incorporated herein by reference), dry eye disease (Pflugfelder, S. C., 2004, Am J Ophthalmol., 137(2), 337-42; Kymionis, G. D., et al 2008, Clin Ophthalmol., 2, 829-836; Kunert, K. S., et al., 2002, Arch Ophthalmol., 120, 330-7; Yavuz, B., et al., 2012, Scientific World Journal. 2012:194848.; each of which is incorporated herein by reference), severe Graves' ophthalmopathy (Prummel, M. F., 1989, N Engl JMed., 321(20), 1353-9; incorporated herein by reference), endogenous uveitis (Nussenblatt, R. B., et al., 1991, Am J Ophthalmol., 112(2), 138-46; which is incorporated herein by reference), Wegener's granulomatosis (Georganas, C., et al., 1996, Clin Rheumatol., 15(2), 189-92; incorporated herein by reference), vernal keratoconjutivitis (Pucci, N., et al., 2002, Ann Allergy Asthma Immunol., 89, 298-303; incorporated herein by reference), atopic keratoconjutivitis (Akpek, E. K., et al., 2004, Ophthalmology, 111, 476-82; incorporated herein by reference), ligneous conjutivitis (Rubin, B. I., et al., 1991, Am J Ophthalmol., 112, 95-96; incorporated herein by reference), conjuctival linchen planus (Levell, N. J., et al., 1992, Br J Dermatol., 127, 66-7; incorporated herein by reference), superior limbic keratoconjutivitis (Perry, H. D., et al., 2003, Ophthalmology, 110, 1578-81; incorporated herein by reference), inflammatory bowel disease-Crohn's Disease and Ulcerative Colitis (Sandborn, W. J., 1995, Inflamm Bowel Dis. 1:48-63; Shibolet, O., et al., 2005, Cochrane Database Syst Rev., (1):CD004277; Rufo, P. A., et al., 2006, Paediatr Drugs, 8(5):279-302; Reindl, W., et al., 2007, Gut., 56(7):1019; Hart, A. L., et al., 2010, Aliment Pharmacol Ther., 32(5):615-27; Cheifetz, A. S., et al., 2011, J Clin Gastroenterol., 45(2):107-12; Sharkey, L., 2011, J Crohns Colitis., 5(2):91-4; Fabro, M., et al., 2011, Curr Drug Targets., 12(10):1448-53; Van Assche, G., et al., 2011, Gut., 60(1):130-3; each of which is incorporated herein by reference), NSAID-induced enteropathy (LoGuidice, A., at al., 2010, Toxicol. Sci., 118, 276-285; which is incorporated herein by reference), and ischaemic brain diseases (Boulos, S., et al., 2007, Neurobiol Dis., 25:54-64; incorporated herein by reference).

Due to cyclophilin inhibition, cyclosporin derivatives also possess the following biological activities: anti-fungal (Kirkland, T. N., et al., 1983, Antimicrob Agents Chemother., 24(6): 921-924; Mody, C. H., et al., 1988, Infect Immun., 56(1): 7-12; Roilides, E., et al., 1994, Antimicrob Agents Chemother., 38(12): 2883-2888; Moussaïf, M., et al., 1997, Appl Environ Microbiol., 63(5): 1739-43; Cruz, M. C., et al., 2000, Antimicrob Agents Chemother., 44(1):143-9; each of which is incorporated herein by reference), anti-malarial (Nickell, S. P., et al., 1982, Infect Immun., 37(3):1093-100; Murphy, J. R., et al, 1988, Antimicrob Agents Chemother., 32(4):462-6; Marín-Menéndez, A., et al., 2012, Mol Biochem Parasitol., 184(1):44-7; each of which is incorporated herein by reference), and anti-parasitic (including Leishmania donovani, Cryptosporidium parvum, Hymenolepis nana, Toxoplasma, Trypanosoma cruzi, and Schistosome; Chappell, L. H., et al., 1992, Parasitology, 105 Suppl:S25-40; Bell, A., et al., 1996, Gen Pharmacol., 27(6):963-71; Yau, W. L., et al., 2010, PLoS Negl Trop Dis., 4(6):e729; Yurchenko, V., et al., 2008, Int JParasitol., 38(6):633-9; Perkins, M. E., et al., 1998, Antimicrob Agents Chemother., 42(4):843-8; Matsuzawa, K., et al., 1998, Int J Parasitol., 28(4):579-88; Silverman, J. A., et al., 1997, Antimicrob Agents Chemother., 41(9):1859-66; Búa, J., et al., 2008, Parasitology, 135(2):217-28; Búa, J., et al., 2004, Bioorg Med Chem Lett., 14(18):4633-7; Bout, D. T, et al., 1984, Am J Trop Med Hyg., 33(1):185-6; Bout, D., et al., 1986, Infect Immun., 52(3):823-7; Munro, G. H., et al., 1991, Parasitology, 102 Pt 1:57-63; each of which is incorporated herein by reference). In addition, cyclosporin derivatives can promote hair growth (Watanabe, S., et al., 1991, J Dermatol., (12):714-9; Paus R., et al., 1994, JInvest Dermatol., 103:2, 143-7; Hozumi, Y., et al., 1994, J Dermatol Sci., 7 Suppl:, S33-8; Takahashi, T., et al., 2001, J Invest Dermatol., 117(3):605-11; Taylor M., et al., 1993, JInvest Dermatol., 100:3, 237-9; Gafter-Gvili, A., et al., 2004, Arch Dermatol Res., 296(6):265-9; each of which is incorporated herein by reference).

The research for Alzheimer's disease indicated that Cyclophilin A is a key target for treating APOE4-mediated neurovascular injury and the resulting neuronal dysfunction and degeneration (Bell, R. D., et al., 2012, Nature, 485(7399):512-6; Bell, R. D., et al., 2009, Acta Neuropathol., 118(1):103-13; each of which is incorporated herein by reference).

Due to the function of extracellular cyclophilins, it is necessary to emphasize that the special target of a secreted extracellular cyclophilin using a cell-impermeable derivative of cyclosporine will be effective in reducing inflammation for diseases such as respiratory inflammation and cardiovascular diseases (Yurchenko V., 2005, Immunology, 117(3):301-9; Yurchenko, V., 2010, Clin Exp Immunol., 160(3):305-17; Malesevic, M., 2010, Angew Chem Int Ed Engl., 49(1):213-5; Balsley, M. A., et al., 2010, J Immunol., 185(12):7663-70; Balsley, M., et al., 2010, Am. J. Respir. Crit. Care Med., 181(1): A6821; Satoh, K., et al., 2010, Circ J., 74(11):2249-56; Bukrinsky, M., 2015, Biochim Biophys Acta, 1850(10): 2087-95; each of which is incorporated herein by reference). To target extracellular cyclophilin, MM284 had been discovered and tested for its anti-inflammatory property (Malesevic, M., et al., 2013, J. Med. Chem., 56, 7302-7311.). The further study for biliary atresia (BA) and other intrahepatic chronic disorders (Iordanskaia, T., et al., 2015, Mol. Med., 21(1): 657-664.), for myocardial inflammation and reduction of cardiac fibrosis (Heinzmann, D., et al., 2015, PLoS One 10(8):e0124606. doi: 10.1371/journal.pone. 0124606), study for reduction of TNF-alfa (Ditiatkovski, M., et al., 2015, J. Pharmacol. Exp. Ther., 353(3):490-5.) had been reported.

Cyclophilin D (CypD) is very important for mitochondrial related neuro and cardiovascular functions because it is an integral part of the mitochondrial permeability transition pore (mPTP). Unregulated opening of the mPTP can lead to mitochondrial swelling and cell death. Thus, the CypD-mediated mPTP is directly linked to a new pharmacologic treatment strategy for many neuro and cardiovascular diseases, such as Alzheimer's disease, Parkinson's disease, Huntington's disease, ALS, aging, heart failure, traumatic brain injury, spinal cord injury, epilepticus, stroke, ischemia-reperfusion injury in the brain, heart, liver, lung, kidney, and particularly in myocardial infarction. The CypD-mediated mPTP is also linked to a new treatment strategy for cancer, obesity, diabetes, and muscular dystrophy, liver fibrosis, liver protection and regeneration (Henry-Mowatt, J., 2004, Oncogene, 23, 2850-60; Galluzzi, L., 2006, Oncogene, 25, 4812-4830; Hirai, K., et al., 2001, J Neurosci., 21, 3017-3023; Friberg, H., et al., 2002, Biochimie, 84, 241-250; Waldmeier, P. C., et al., 2003, Curr Med Chem., 10, 1485-506; Hansson, M. J., et al., 2004, J Bioenerg Biomembr., 36, 407-13; Sullivan, P. G., et al., 2005, J Neurosci Res., 79, 231-9; Baines, C. P., et al, 2005, Nature 434, 658-662; Shanmuganathan, S., et al, 2005, Am J Physiol Heart Circ Physiol., 289, H237-H242; McBride, H. M., et al., 2006, Curr Biol., 16, R551-560; Mandemakers, W., et al., 2007, J Cell Sci., 120, 1707-1716; Kroemer, G., et al., 2007, Physiol Rev., 87, 99-163; Ibarra, A., et al., 2007, Brain Res., 1149, 200-209; Michelakis, E. D., et al, 2008, Circulation, 117, 2431-2434; Du, H., et al, 2008, Nature Medicine, 14, 1097-1105; Piot C., et al., 2008, N Engl J Med., 359, 473-81; Hatton, J., et al., 2008, J Neurosurg., 109, 699-707; Tatsuta, T., et al., 2008, EMBO J, 27, 306-314; Reutenauer, J., et al., 2008, Br J Pharmacol., 155, 574-84; Mazzeo, A. T., et al., 2009, Exp Neurol., 218, 363-370; Galluzzi, L., et al, 2009, Nature Rev Neurosci., 10, 481-494; Halestrap, A. P., et al., 2009, Biochim Biophys Acta., 1787, 1402-15; Arnett, A. L. H., et al., 2009, Curr. Opin. Genet. Dev., 19, 290-297; Tiepolo, T., et al., 2009, Br J Pharmacol., 157, 1045-1052; Wissing, E. R., et al., 2010, Neuromuscul Disord., 20, 753-60; Halestrap, A. P., et al., 2010, Biochem Soc Trans., 38, 841-860; Cernak, I., et al., 2010, J Cereb Blood Flow Metab., 30, 255-66; Elrod, J. W., et al., 2010, J Clin Invest., 120, 3680-3687; Duchen, M. R., et al., 2010, Essays Biochem., 47, 115-37; Schapira, A. H. V., et al., 2011, Parkinson's Disease, Volume 2011, 1-7 Article ID 159160; Osman, M. M., et al., 2011, Neuropeptides, 45, 359-368; Devalaraja-Narashimha K., et al., 2011, FEBS Lett., 585, 677-82; Fujimoto, K., et al., 2010, Proc Natl Acad Sci U S A. 107, 10214-9; Irwin, W. A., et al., 2003, Nat Genet., 35, 267-271; Angelin, A., et al., 2007, Proc Natl Acad Sci U S A, 104, 991-6; Merlini, L., et al., 2008, Proc Natl Acad Sci U S A, 105, 5225-9; Millay, D. P., 2008, Nat Med., 14, 442-7; Malouitre, S., et al., 2009 Biochem. J., 425(1): 137-48; Dear, J. W., et al., J. Immunol., 187(6):3347-52; Naoumov, N. V., 2014, L. Hepatol., 61(5): 1166-74; each of which is incorporated herein by reference). Cyclosporine A and its derivatives can block CypD to prevent mitochondrial swelling and cell death, and therefore could be useful for treatment of the aforementioned diseases, for example, as a neuro and cardiovascular and liver protective agent or as a novel mitochondrial medicine.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting.

### Summary of the Invention

In one aspect, the present invention provides a compound of Formula (I): 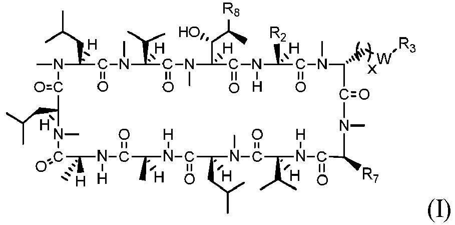 or pharmaceutically acceptable salt thereof, wherein:
x is 0 or 1;
R₈ is alkyl, alkenyl, alkynyl, cycloalkyl, cycloalkenyl, aryl, or heteroaryl; wherein R₈ is substituted by one or more R₁; provided that R₈-R₁ is not n-butyl or (E)-but-2-enyl;
R₂ is ethyl, 1-hydroxyethyl, isopropyl or n-propyl;
W is NR₁, O, S, or CH₂;
R₃ is H, alkyl or substituted alkyl, alkenyl or substituted alkenyl, alkynyl or substituted alkynyl, cycloalkyl or substituted cycloalkyl, cycloalkenyl or substituted cycloalkenyl, aryl or substituted aryl, or heteroaryl or substituted heteroaryl; wherein R₃ is optionally substituted by one or more R₁;
each occurrence of R₁ is independently H, halogen, aryl or substituted aryl, heteroaryl or substituted heteroaryl, OR_{A}, SR_{A}, NR_{A}R_{B}, -NR_{A}(CH₂)_{O}OR_{B}, -N((CH₂)_{O}OR_{A})((CH₂)_{O}OR_{B}) - C(=O)R_{A}, -C(=O)OR_{A}, -OC(=O)R_{A}, -OC(=O)(C₁-C₆ alkyl-R_{H}), -OC(=O)(CH₂)ₒOR_{A}, - C(=O)NR_{A}R_{B}, -NR_{A}C(=O)R_{B}, -N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B}, - N(C(=O)(CH₂)_{O}OR_{B})₂, -NR_{A}C(=O)(CH₂)_{O}NR_{A}R_{B}, -NR_{A}(CH₂)_{O}C(=O)OR_{B}, - N((CH₂)_{O}C(=O)OR_{A})((CH₂)_{O}C(=O)OR_{B}), -NR_{A}(CH₂)_{O}C(=O)NR_{A}R_{B}, - N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B}, - C(=O)N((CH₂)_{O}OR_{B})₂,-N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B}, - NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOP_{A}, O(CH₂)ₘO(CH₂)ₘOR_{A}, O(CH₂)ₘNR_{A}R_{B}, O(CH₂)ₘO(CH₂)ₘNR_{A}R_{B}, NRc(CH₂)ₘNR_{A}R_{B}, NR_{C}(CH₂)ₘNRc(CH₂)ₘNR_{A}R_{B}, 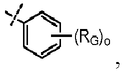 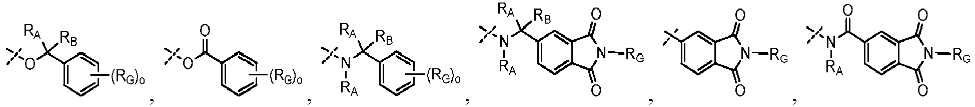 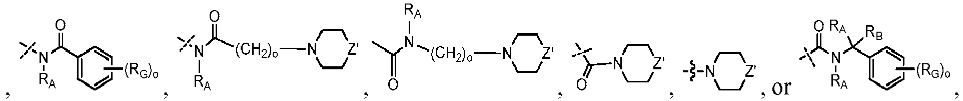 wherein said aryl or heteroaryl is optionally substituted by one or more groups which may be the same or different selected from the group consisting of halogen, hydroxy, (C₁-C₆)alkyl, (CH₂)ₚORA, (CH₂)ₚNR_{A}R_{B}, (CH₂)ₚC(=O)NR_{A}R_{B} and (CH₂)ₚC(=O)OR_{A};
R₇ is 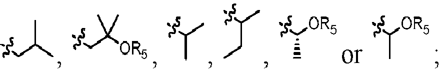
each R₅ is independently H, alkyl or substituted alkyl, alkenyl or substituted alkenyl, alkynyl or substituted alkynyl, cycloalkyl or substituted cycloalkyl, cycloalkenyl or substituted cycloalkenyl, or aryl or substituted aryl;
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl or 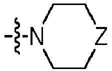 wherein Z is CH₂, O, S, NH, NCH₃, NEt, N-isopropyl, N-isopropyl, N-neoPentyl, N-CH₂CH₂OH, or N-CH₂CH₂OMe;
each occurrence of R_{G} is independently R_{A}, OR_{A}, SR_{A}, NR_{A}R_{B}, -(CH₂)ₒR_{A}, - (CH₂)ₒC(=O)OR_{A}, -(CH₂)ₒC(=O)NR_{A}R_{B}, C(=O)OR_{A}, OC(=O)R_{A}, NR_{A}C(=O)R_{B}, NR_{A}C(=O)(CH₂)_{O}OR_{A}, C(=O)O(CH₂)_{O}OR_{A}, C(=O)OR_{B}, C(=O)NR_{A}R_{B}, C(=O)NR_{A}(CH₂)_{O}OR_{B}, C(=O)N((CH₂)_{O}OR_{A})((CH₂)_{O}OR_{B}), C(=O)N((CH₂)_{O}C(=O)OR_{A})((CH₂)_{O}C(=O)OR_{B}), C(=O)N((CH₂)_{O}NR_{A}R_{B})((CH₂)_{O}NR_{A}R_{B}), C(=O)N((CH₂)_{O}OC(=O)(CH₂)_{O}OR_{A})((CH₂)_{O}OC(=O)(CH₂)_{O}OR_{B}), C(=O)N((CH₂)_{O}NR_{A}C(=O)(CH₂)_{O}OR_{B})((CH₂)_{O}NR_{A}C(=O)(CH₂)_{O}OR_{B}), C(=O)NR_{A}(CH₂)_{O}NR_{A}R_{B},, 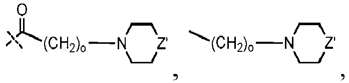 C(=O)NR_{A}(CH₂)_{O}OC(=O)R_{B}, C(=O)NR_{A}(CH₂)_{O}C(=O)OR_{B}, C(=O)NR_{A}(CH₂)_{O}C(=O)NR_{A}R_{B}, C(=O)NR_{A}(CH₂)_{O}OC(=O)(CH₂)_{O}OR_{B}, or 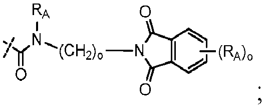
each occurrence of R_{H} is independently halogen;
each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNR_{A}C(=O)R_{B}, or N(CH₂)ₒOC(=O)(CH₂)ₒOR_{A};
each occurrence of o is independently 0, 1, 2, 3, 4, 5, or 6;
each occurrence of p is independently an integer of 0, 1, 2, 3, 4, or 5; and
each occurrence of m is independently an integer of 1, 2, 3, 4 or 5.

In certain specific embodiments, the compound disclosed herein has the structure of Formulae (II) or (III): 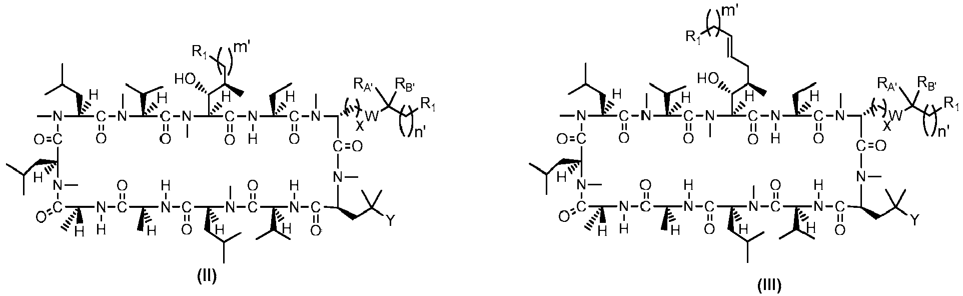 or pharmaceutically acceptable salt thereof, wherein:
x is 0 or 1;
Y is H or OR₅; wherein R₅ is H or methyl;
M' and n' are each independently 0, 1, 2, 3, 4, 5, 6, 7, 8, 9, 10, 11, or 12;
each occurrence of R_{A'} and R_{B'} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or R_{A'} and R_{B'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl; and
each occurrence of R_{D} is independently halogen, hydroxy, O(C₁-C₄)alkyl, C(=O)(C₁-C₄)alkyl, C(=O)O(C₁-C₄)alkyl or 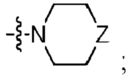 wherein Z is CH₂, O, S, NH, NCH₃, NEt, N-isopropyl, N-isopropyl, N-neoPentyl, N-CH₂CH₂OH, or N-CH₂CH₂OMe.

In certain specific embodiments, the compound disclosed herein has the structure of Formula (IV) or (V): 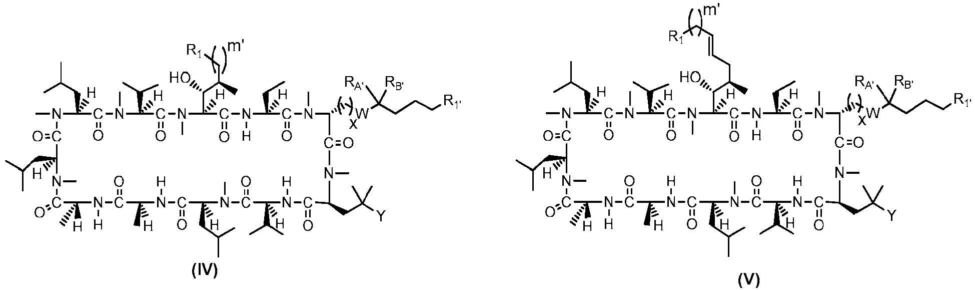 ; wherein x is 0 or 1; Y is H or OR₅; wherein R₅ is H or methyl; each occurrence of R_{1'} is independently H, halogen, aryl or substituted aryl, heteroaryl or substituted heteroaryl, OR_{A}, SR_{A}, NR_{A}R_{B}, -NR_{A}(CH₂)_{O}OR_{B}, -N((CH₂)_{O}OR_{A})((CH₂)_{O}OR_{B}) -C(=O)R_{A}, -C(=O)OR_{A}, - OC(=O)R_{A}, -OC(=O)(C₁-C₆ alkyl-R_{H}), -OC(=O)(CH₂)ₒOR_{A}, -C(=O)NR_{A}R_{B}, -NR_{A}C(=O)R_{B}, - N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B}, -N(C(=O)(CH₂)_{O}OR_{B})₂, - NR_{A}C(=O)(CH₂)_{O}NR_{A}R_{B}, -NR_{A}(CH₂)_{O}C(=O)OR_{B}, -N((CH₂)_{O}C(=O)OR_{A})((CH₂)_{O}C(=O)OR_{B}), - NR_{A}(CH₂)_{O}C(=O)NR_{A}R_{B}, -N((CH₂)_{O}C(=O)NR_{A}R_{B})((CH₂)_{O}C(=O)NR_{A}R_{B}), - NR_{A}(CH₂)_{O}C(=O)NR_{A}(CH₂)_{O}OR_{B}, -C(=O)N((CH₂)_{O}OR_{B})₂, - N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, - C(=O)NR_{A}(CH₂)_{O}OR_{B}, -C(=O)NR_{A}(CH₂)_{O}NR_{A}R_{B}, -N=CR_{A}-NR_{A}R_{B}, -NR_{B}-C(=NH)-NR_{A}R_{B}, O(CH₂)ₘOR_{A}, O(CH₂)ₘCOOR_{A}, O(CH₂)ₘCONR_{A}R_{B},O(CH₂)ₘCONR_{A}(CH₂)ₘOP_{A}, O(CH₂)ₘO(CH₂)ₘOP_{A}, O(CH₂)ₘNR_{A}R_{B}, O(CH₂)ₘO(CH₂)ₘNR_{A}R_{B}, NRc(CH₂)ₘNR_{A}R_{B}, NR_{C}(CH₂)ₘNR_{C}(CH₂)ₘNR_{A}R_{B}, 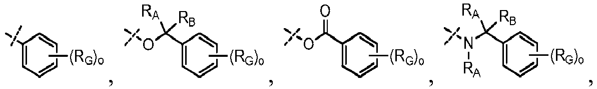 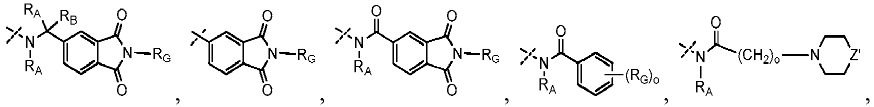 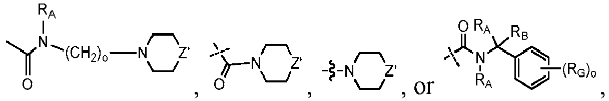 wherein said aryl or heteroaryl is optionally substituted by one or more groups which may be the same or different selected from the group consisting of halogen, hydroxy, (C₁-C₆)alkyl, (CH₂)ₚORA, (CH₂)ₚNR_{A}R_{B}, (CH₂)ₚC(=O)NR_{A}R_{B} and (CH₂)ₚC(=O)OR_{A}.

In certain specific embodiments, R₁ is -NR_{A}C(=O)R_{B} and R_{1'} is OR_{A}. Non-limiting examples of R_{A} include H, Me, Et, Pr, Bu, and Pentyl. Non-limiting examples of R_{B} include H, Me, Et, Pr, Bu, and Pentyl. In certain specific embodiments, wherein R₁ is -NHC(=O)R_{B} and R_{1'} is OH; and Y is OH or H.

In certain specific embodiments, the compound disclosed herein has the structure of Formula (VI): 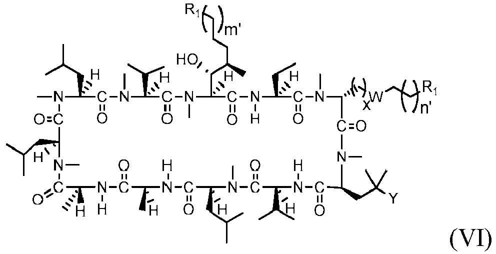 wherein
x is 0 or 1;
W is CH₂, O or S;
Y is H or OR₅; wherein R₅ is H or methyl;
m' and n' are each independently 0, 1, 2, 3, 4, 5, 6, 7, 8, 9, or 10; and
   each occurrence of R₁ is independently H, halogen, aryl or substituted aryl, heteroaryl or substituted heteroaryl, OR_{A}, SR_{A}, NR_{A}R_{B}, -C(=O)R_{A}, -C(=O)OR_{A}, - C(=O)NR_{A}R_{B}, -NR_{A}C(=O)R_{B}, -NR_{A}C(=O)(CH₂)_{O}R_{B}, -NR_{A}C(=O)(CH₂)_{O}OR_{B}, - NR_{A}C(=O)(CH₂)_{O}NR_{A}R_{B}, or -C(=O)NR_{A}(CH₂)_{O}R_{B}.

In yet another aspect, the present invention provides a pharmaceutical composition comprising at least one compound as described herein and a pharmaceutically-acceptable carrier.

In a further aspect, the present invention provides a method for treating or preventing a viral infection in a mammalian species in need thereof, the method comprising administering to the mammalian species a therapeutically effective amount of at least one compound as described herein.

In another aspect, the present invention provides a method for treating or preventing hepatitis C virus infection in a mammalian species in need thereof, the method comprising administering to the mammalian species a therapeutically effective amount of at least one compound as described herein.

In yet another aspect, the present invention provides a method for inhibiting a cyclophilin in a subject in need thereof, which comprises administrating to said subject an effective cyclophilin-inhibiting amount of at least one compound as described herein.

In yet another aspect, the present invention provides a method for treating or preventing diseases that are mediated by cyclophilins in a mammalian species in need thereof, the method comprising administering to the mammalian species a therapeutically effective amount of at least one compound as described herein.

In yet another aspect, the present invention provides a method for treating or preventing diseases in a mammalian species in need thereof, the method comprising administering to the mammalian species a therapeutically effective amount of at least one compound as described herein, wherein the diseases are selected from inflammation, respiratory inflammation, rheumatoid arthritis, and dry eye.

In yet another aspect, the present invention provides a method for treating or preventing diseases in a mammalian species in need thereof, the method comprising administering to the mammalian species a therapeutically effective amount of at least one compound as described herein, wherein the diseases are selected from neurodegenerative diseases such as Alzheimer's disease, Parkinson's disease, Huntington's Diseases, and ALS; traumatic brain injury; stroke; and ischemia-reperfusion injury in the brain, heart, and kindey.

In yet another aspect, the present invention provides a method for treating or preventing diseases in a mammalian species in need thereof, the method comprising administering to the mammalian species a therapeutically effective amount of at least one compound as described herein, wherein the diseases are selected from cardiovascular diseases, vascular stenosis, atherosclerosis, abdominal aortic aneurysms, cardiac hypertrophy, aortic rupture, pulmonary arterial hypertension, myocarditis and myocardial fibrosis, and ischaemic heart diseases.

In yet another aspect, the present invention provides a method for treating or preventing diseases or conditions in a mammalian species in need thereof, the method comprising administering to the mammalian species a therapeutically effective amount of at least one compound as described herein, wherein the diseases or conditions are selected from cancer; obesity; diabetes; muscular dystrophy; lung, and liver, and kindey diseases, and their protection; and hair loss.

In yet another aspect, the present invention provides a method for treating or preventing diseases or conditions in a mammalian species in need thereof, the method comprising administering to the mammalian species a therapeutically effective amount of at least one compound as described herein, wherein the diseases or conditions are selected from allergic conjunctivitis, atopic and vernal keratoconjunctivitis, atopic keratoconjunctivitis, anterior uveitis, Behcet's disease, blepharitis, chronic ocular surface inflammation caused by viral infection, corneal transplant rejection, corneal sensitivity impaired due to surgery on the cornea or other surface of the eye, meibomian gland disease, ptyregia, ocular symptoms of graft versus host disease, ocular allergy, ocular cicatricial pemphigoid, Steven Johnson syndrome, vernal keratoconjunctivitis, uveitis, herpes simplex keratitis, ocular rosacea, and Pinguecula.

### Detailed Description of the Invention

### Definitions

The following are definitions of terms used in the present specification. The initial definition provided for a group or term herein applies to that group or term throughout the present specification individually or as part of another group, unless otherwise indicated.

The terms "alkyl" and "alk" refer to a straight or branched chain alkane (hydrocarbon) radical containing from 1 to 12 carbon atoms, preferably 1 to 6 carbon atoms. Exemplary "alkyl" groups include methyl, ethyl, propyl, isopropyl, n-butyl, t-butyl, isobutyl pentyl, hexyl, isohexyl, heptyl, 4,4-dimethylpentyl, octyl, 2,2,4-trimethylpentyl, nonyl, decyl, undecyl, dodecyl, and the like. The term "(C₁-C₄)alkyl" refers to a straight or branched chain alkane (hydrocarbon) radical containing from 1 to 4 carbon atoms, such as methyl, ethyl, propyl, isopropyl, n-butyl, t-butyl, and isobutyl. The term "(C₁-C₆)alkyl" refers to a straight or branched chain alkane (hydrocarbon) radical containing from 1 to 6 carbon atoms, such as n-hexyl, 2-methylpentyl, 3-methylpentyl, 4-methylpentyl, 2,3-dimethylbutyl, 2,2-dimethylbutyl, in addition to those exemplified for "(C₁-C₄)alkyl." "Substituted alkyl" refers to an alkyl group substituted with one or more substituents, preferably 1 to 4 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. In the aforementioned exemplary substituents, groups such as alkyl, cycloalkyl, alkenyl, alkynyl, cycloalkenyl, heterocycle and aryl can themselves be optionally substituted.

The term "alkenyl" refers to a straight or branched chain hydrocarbon radical containing from 2 to 12 carbon atoms and at least one carbon-carbon double bond. Examples of such groups include ethenyl or allyl. The term "C₂-C₆ alkenyl" refers to a straight or branched chain hydrocarbon radical containing from 2 to 6 carbon atoms and at least one carbon-carbon double bond, such as ethylenyl, propenyl, 2-propenyl, (*E*)-but-2-enyl, (*Z*)-but-2-enyl, 2-methy(*E*)-but-2-enyl, 2-methy(*Z*)-but-2-enyl, 2,3-dimethy-but-2-enyl, (*Z*)-pent-2-enyl, (*E*)-pent-1-enyl, (*Z*)-hex-1-enyl, (*E*)-pent-2-enyl, (*Z*)-hex-2-enyl, (*E*)-hex-2-enyl, (*Z*)-hex-1-enyl, (*E*)-hex-1-enyl,, (*Z*)-hex-3-enyl, (*E*)-hex-3-enyl, and (*E*)-hex-1,3-dienyl. "Substituted alkenyl" refers to an alkenyl group substituted with one or more substituents, preferably 1 to 4 substituents, at any available point of attachment. Examples of substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "alkynyl" refers to a straight or branched chain hydrocarbon radical containing from 2 to 12 carbon atoms and at least one carbon to carbon triple bond. An exemplary of such groups includes ethynyl. The term "C₂-C₆ alkynyl" refers to a straight or branched chain hydrocarbon radical containing from 2 to 6 carbon atoms and at least one carbon-carbon triple bond, such as ethynyl, prop-1-ynyl, prop-2-ynyl, but-1-ynyl, but-2-ynyl, pent-1-ynyl, pent-2-ynyl, hex-1-ynyl, hex-2-ynyl, hex-3-ynyl. "Substituted alkynyl" refers to an alkynyl group substituted with one or more substituents, preferably 1 to 4 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "cycloalkyl" refers to a fully saturated cyclic hydrocarbon group containing from 1 to 4 rings and 3 to 8 carbons per ring. "C₃-C₇ cycloalkyl" refers to cyclopropyl, cyclobutyl, cyclopentyl, cyclohexyl, or cycloheptyl. "Substituted cycloalkyl" refers to a cycloalkyl group substituted with one or more substituents, preferably 1 to 4 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "cycloalkenyl" refers to a partially unsaturated cyclic hydrocarbon group containing 1 to 4 rings and 3 to 8 carbons per ring. Examples of such groups include cyclobutenyl, cyclopentenyl, cyclohexenyl, *etc.* "Substituted cycloalkenyl" refers to a cycloalkenyl group substituted with one more substituents, preferably 1 to 4 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "aryl" refers to cyclic, aromatic hydrocarbon groups that have 1 to 5 aromatic rings, especially monocyclic or bicyclic groups such as phenyl, biphenyl or naphthyl. When containing two or more aromatic rings (bicyclic, *etc*.), the aromatic rings of the aryl group may be joined at a single point *(e.g.,* biphenyl), or fused *(e.g.,* naphthyl, phenanthrenyl and the like). "Substituted aryl" refers to an aryl group substituted by one or more substituents, preferably 1 to 3 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include fused cylic groups, especially fused cycloalkyl, fused cycloalkenyl, fused heterocycle, or fused aryl, where the aforementioned cycloalkyl, cycloalkenyl, heterocycle and aryl substituents can themselves be optionally substituted.

The terms "heterocycle" and "heterocyclic" refer to fully saturated, or partially or fully unsaturated, including aromatic *(i.e.,* "heteroaryl") cyclic groups (for example, 4 to 7 membered monocyclic, 7 to 11 membered bicyclic, or 8 to 16 membered tricyclic ring systems) which have at least one heteroatom in at least one carbon atom-containing ring. Each ring of the heterocyclic group containing a heteroatom may have 1, 2, 3, or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. Exemplary monocyclic heterocyclic groups include azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, isoindolyl, benzothiazolyl, benzoxazolyl, benzoxadiazolyl, benzothienyl, benzo[d][1,3]dioxolyl, 2,3-dihydrobenzo[b][1,4]dioxinyl, quinuclidinyl, quinolinyl, tetrahydroisoquinolinyl, isoquinolinyl, benzimidazolyl, benzopyranyl, indolizinyl, benzofuryl, benzofurazanyl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl] or furo[2,3-b]pyridinyl), dihydroisoindolyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, acridinyl, phenanthridinyl, xanthenyl and the like.

"Substituted heterocycle" and "substituted heterocyclic" (such as "substituted heteroaryl") refer to heterocycle or heterocyclic groups substituted with one or more substituents, preferably 1 to 4 substituents, at any available point of attachment. Exemplary substituents include, but are not limited to, one or more of the following groups: hydrogen, halogen *(e.g.,* a single halogen substituent or multiple halo substituents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted. Exemplary substituents also include spiro-attached or fused cylic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "alkylamino" refers to a group having the structure -NHR', wherein R' is hydrogen, alkyl or substituted alkyl, cycloalkyl or substituted cyclolakyl, as defined herein. Examples of alkylamino groups include, but are not limited to, methylamino, ethylamino, n-propylamino, iso-propylamino, cyclopropylamino, n-butylamino, tert-butylamino, neopentylamino, n-pentylamino, hexylamino, cyclohexylamino, and the like.

The term "dialkylamino" refers to a group having the structure -NRR', wherein R and R' are each independently alkyl or substituted alkyl, cycloalkyl or substituted cycloalkyl, cycloalkenyl or substituted cyclolalkenyl, aryl or substituted aryl, heterocylyl or substituted heterocyclyl, as defined herein. R and R' may be the same or different in a dialkyamino moiety. Examples of dialkylamino groups include, but are not limited to, dimethylamino, methyl ethylamino, diethylamino, methylpropylamino, di(n-propyl)amino, di(isopropyl)amino, di(cyclopropyl)amino, di(n-butyl)amino, di(tert-butyl)amino, di(neopentyl)amino, di(n-pentyl)amino, di(hexyl)amino, di(cyclohexyl)amino, and the like. In certain embodiments, R and R' are linked to form a cyclic structure. The resulting cyclic structure may be aromatic or non-aromatic. Examples of cyclic diaminoalkyl groups include, but are not limited to, aziridinyl, pyrrolidinyl, piperidinyl, morpholinyl, pyrrolyl, imidazolyl, 1,3,4-trianolyl, and tetrazolyl.

The terms "halogen" or "halo" refer to chlorine, bromine, fluorine or iodine.

Unless otherwise indicated, any heteroatom with unsatisfied valences is assumed to have hydrogen atoms sufficient to satisfy the valences.

The compounds of the present invention may form salts which are also within the scope of this invention. Reference to a compound of the present invention is understood to include reference to salts thereof, unless otherwise indicated. The term "salt(s)", as employed herein, denotes acidic and/or basic salts formed with inorganic and/or organic acids and bases. In addition, when a compound of the present invention contains both a basic moiety such as, but is not limited to, a pyridine or imidazole, and an acidic moiety such as, but is not limited to, a carboxylic acid, zwitterions ("inner salts") may be formed and are included within the term "salt(s)" as used herein. Pharmaceutically acceptable (*i.e.,* non-toxic, physiologically acceptable) salts are preferred, although other salts are also useful, *e.g.,* in isolation or purification steps which may be employed during preparation. Salts of a compound of the present invention may be formed, for example, by reacting a compound of Formula I with an amount of acid or base, such as an equivalent amount, in a medium such as one in which the salt precipitates or in an aqueous medium followed by lyophilization.

The compounds of the present invention which contain a basic moiety such as, but is not limited to, an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (*e.g*., 2-hydroxyethanesulfonates), lactates, maleates, methanesulfonates, naphthalenesulfonates (*e.g*., 2-naphthalenesulfonates), nicotinates, nitrates, oxalates, pectinates, persulfates, phenylpropionates (*e.g*., 3-phenylpropionates), phosphates, picrates, pivalates, propionates, salicylates, succinates, sulfates (such as those formed with sulfuric acid), sulfonates, tartrates, thiocyanates, toluenesulfonates such as tosylates, undecanoates, and the like.

Compounds of the present invention which contain an acidic moiety, such but not limited to a carboxylic acid, may form salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N,N-bis(dehydroabietyl) ethylenediamine), N-methyl-D-glucamines, N-methyl-D-glycamides, t-butyl amines, and salts with amino acids such as arginine, lysine and the like. Basic nitrogen-containing groups may be quaternized with agents such as lower alkyl halides (*e.g*., methyl, ethyl, propyl, and butyl chlorides, bromides and iodides), dialkyl sulfates (*e.g*., dimethyl, diethyl, dibutyl, and diamyl sulfates), long chain halides (e.g., decyl, lauryl, myristyl and stearyl chlorides, bromides and iodides), aralkyl halides (e.g., benzyl and phenethyl bromides), and others.

Prodrugs and solvates of the compounds of the invention are also contemplated herein. The term "prodrug" as employed herein denotes a compound that, upon administration to a subject, undergoes chemical conversion by metabolic or chemical processes to yield a compound of the present invention, or a salt and/or solvate thereof. Solvates of the compounds of the present invention include, for example, hydrates.

Compounds of the present invention, and salts or solvates thereof, may exist in their tautomeric form (for example, as an amide or imino ether). All such tautomeric forms are contemplated herein as part of the present invention.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (*e.g.,* as a pure or substantially pure optical isomer having a specified activity), or may be admixed, for example, as racemates or with all other, or other selected, stereoisomers. The chiral centers of the present invention may have the S or R configuration as defined by the International Union of Pure and Applied Chemistry (IUPAC) 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation, conventional methods, such as, for example, salt formation with an optically active acid followed by crystallization.

Compounds of the present invention are, subsequent to their preparation, preferably isolated and purified to obtain a composition containing an amount by weight equal to or greater than 90%, for example, equal to or greater than 95%, equal to or greater than 99% pure ("substantially pure" compound I), which is then used or formulated as described herein. Such "substantially pure" compounds of the present invention are also contemplated herein as part of the present invention.

All configurational isomers of the compounds of the present invention are contemplated, either in admixture or in pure or substantially pure form. The definition of compounds of the present invention embraces both *cis* (Z) and *trans* (*E*) alkene isomers, as well as *cis* and *trans* isomers of cyclic hydrocarbon or heterocyclic rings.

Throughout the specifications, groups and substituents thereof may be chosen to provide stable moieties and compounds.

Definitions of specific functional groups and chemical terms are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito: 1999, the entire contents of which are incorporated herein by reference.

Certain compounds of the present invention may exist in particular geometric or stereoisomeric forms. The present invention contemplates all such compounds, including *cis-* and *trans*-isomers, R- and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are all contemplated by the present invention. Those of ordinary skill in the art will readily appreciate that analogous ratios are contemplated for more complex isomer mixtures.

The present invention also includes isotopically labeled compounds, which are identical to the compounds disclosed herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, or an enantiomer, diastereomer, tautomer, or pharmaceutically acceptable salt or solvate thereof, which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds can generally be prepared by carrying out the procedures disclosed in the Schemes and/or in the Examples below, by substituting a readily available isotopically labeled reagent for a non-isotopically labeled reagent.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example, of infectious diseases or proliferative disorders. The term "stable", as used herein, preferably refers to compounds which possess stability sufficient to allow manufacture and which maintain the integrity of the compound for a sufficient period of time to be detected and preferably for a sufficient period of time to be useful for the purposes detailed herein.

### Compounds

The novel cyclosporin derivatives of the present invention are potent inhibitors of cyclophilins and are useful for inhibiting viruses such as HCV, HBV, and HIV.

In one aspect, the present invention provides a compound of Formula (I): 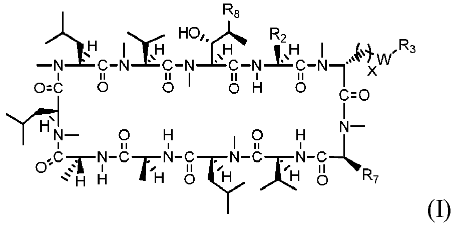 or pharmaceutically acceptable salt thereof, wherein:
x is 0 or 1;
R₈ is alkyl, alkenyl, alkynyl, cycloalkyl, cycloalkenyl, aryl, or heteroaryl; wherein R₈ is substituted by one or more R₁; provided that R₈-R₁ is not n-butyl or (*E*)-but-2-enyl;
R₂ is ethyl, 1-hydroxyethyl, isopropyl or n-propyl;
W is O, S, or CH₂;
R₃ is H, alkyl or substituted alkyl, alkenyl or substituted alkenyl, alkynyl or substituted alkynyl, cycloalkyl or substituted cycloalkyl, cycloalkenyl or substituted cycloalkenyl, aryl or substituted aryl, or heteroaryl or substituted heteroaryl; wherein R₃ is optionally substituted by one or more R₁;
each occurrence of R₁ is independently H, halogen, aryl or substituted aryl, heteroaryl or substituted heteroaryl, CN, OR_{A}, SR_{A}, NR_{A}R_{B}, -NR_{A}(CH₂)_{O}OR_{B}, -N((CH₂)_{O}OR_{A})((CH₂)_{O}OR_{B}) -C(=O)R_{A}, -C(=O)OR_{A}, -OC(=O)R_{A}, -OC(=O)(C₁-C₆ alkyl-R_{H}), -OC(=O)(CH₂)ₒOR_{A}, - C(=O)NR_{A}R_{B}, -NR_{A}C(=O)R_{B}, -N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B}, - N(C(=O)(CH₂)_{O}OR_{B})₂, -NR_{A}C(=O)(CH₂)_{O}NR_{A}R_{B}, -NR_{A}(CH₂)_{O}C(=O)OR_{B}, - N((CH₂)_{O}C(=O)OR_{A})((CH₂)_{O}C(=O)OR_{B}), -NR_{A}(CH₂)_{O}C(=O)NR_{A}R_{B}, - N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B}, - C(=O)N((CH₂)_{O}OR_{B})₂,-N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B}, - NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOP_{A}, O(CH₂)ₘO(CH₂)ₘOR_{A}, O(CH₂)ₘNR_{A}R_{B}, O(CH₂)ₘO(CH₂)ₘNR_{A}R_{B}, NRc(CH₂)ₘNR_{A}R_{B}, NR_{C}(CH₂)ₘNR_{C}(CH₂)ₘNR_{A}R_{B}, 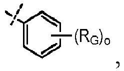 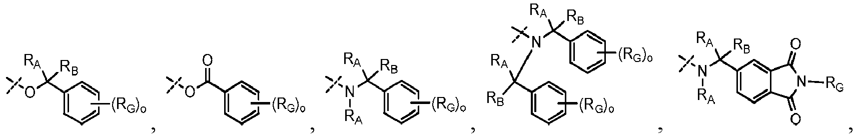 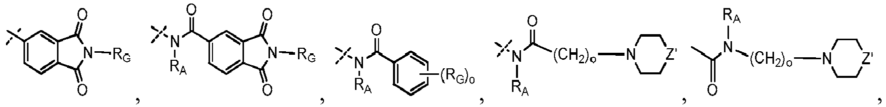 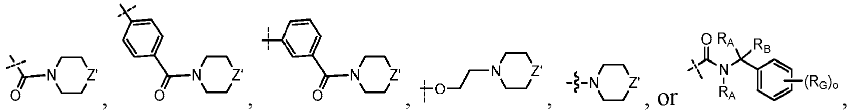 wherein said aryl or heteroaryl is optionally substituted by one or more groups which may be the same or different selected from the group consisting of halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
R₇ is 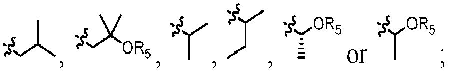
each R₅ is independently H, alkyl or substituted alkyl, alkenyl or substituted alkenyl, alkynyl or substituted alkynyl, cycloalkyl or substituted cycloalkyl, cycloalkenyl or substituted cycloalkenyl, or aryl or substituted aryl;
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl or 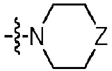 wherein Z is CH₂, O, S, NH, NCH₃, NEt, N-isopropyl, N-isopropyl, N-neoPentyl, N-CH₂CH₂OH, or N-CH₂CH₂OMe;
each occurrence of R_{G} is independently R_{A}, OR_{A}, SR_{A}, NR_{A}R_{B}, -(CH₂)ₒR_{A}, - (CH₂)ₒC(=O)OR_{A}, -(CH₂)ₒC(=O)NR_{A}R_{B}, C(=O)OR_{A}, OC(=O)R_{A}, NR_{A}C(=O)R_{B}, NR_{A}C(=O)(CH₂)_{O}OR_{A}, C(=O)O(CH₂)_{O}OR_{A}, C(=O)OR_{B}, C(=O)NR_{A}R_{B}, C(=O)NR_{A}(CH₂)_{O}OR_{B}, C(=O)N((CH₂)_{O}OR_{A})((CH₂)_{O}OR_{B}), C(=O)N((CH₂)_{O}C(=O)OR_{A})((CH₂)_{O}C(=O)OR_{B}), C(=O)N((CH₂)_{O}NR_{A}R_{B})((CH₂)_{O}NR_{A}R_{B}), C(=O)N((CH₂)_{O}OC(=O)(CH₂)_{O}OR_{A})((CH₂)_{O}OC(=O)(CH₂)_{O}OR_{B}), C(=O)N((CH₂)_{O}NR_{A}C(=O)(CH₂)_{O}OR_{B})((CH₂)_{O}NR_{A}C(=O)(CH₂)_{O}OR_{B}), C(=O)NR_{A}(CH₂)_{O}NR_{A}R_{B},, 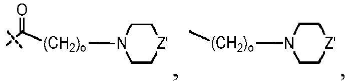 C(=O)NR_{A}(CH₂)_{O}OC(=O)R_{B}, C(=O)NR_{A}(CH₂)_{O}C(=O)OR_{B}, C(=O)NR_{A}(CH₂)_{O}C(=O)NR_{A}R_{B}, C(=O)NR_{A}(CH₂)_{O}OC(=O)(CH₂)_{O}OR_{B}, or 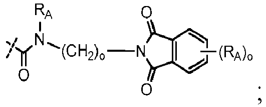
each occurrence of R_{H} is independently halogen;
each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNP_{A}C(=O)R_{B}, or N(CH₂)ₒOC(=O)(CH₂)ₒOR_{A};
each occurrence of ο is independently 0, 1, 2, 3, 4, 5, or 6;
each occurrence of p is independently an integer of 0, 1, 2, 3, 4, or 5; and
each occurrence of m is independently an integer of 1, 2, 3, 4 or 5.

In some embodiments, x is 0. In other embodimetns, x is 1. In some embodiments, R₈ is (C₁-C₁₂)alkyl, (C₂-C₁₂)alkenyl, (C₂-C₁₂)alkynyl, (C₃-C₁₂)cycloalkyl, or phenyl or CH₂-phenyl, optionally substituted by one or more groups which may be the same or different selected from halogen, hydroxy, (C₁-C₆)alkyl. In some embodiments, R₈ is (C₁-C₁₂)alkyl. In yet other embodiments, R₈ is (C₁-C₆) linear alkyl. In yet other embodiments, R₈ is (C₇-C₁₂) linear alkyl. In yet other embodiments, R₈ is (C₄-C₆) linear alkyl. In yet other embodiments, R₈ is (C₆-C₈) linear alkyl. In some specific embodiments, R₈ is a -(CH₂)₃₋₁₁-alkyl chain.

In some embodiments, R₈ is (C₂-C₁₂)alkenyl. In yet other embodiments, R₈ is (C₂-C₆) linear alkenyl. In yet other embodiments, R₈ is (C₇-C₁₂) linear alkenyl. In yet other embodiments, R₈ is (C₄-C₆) linear alkenyl. In yet other embodiments, R₈ is (C₆-C₈) linear alkenyl.

In some embodiments, R₈ is (C₂-C₁₂)alkynyl. In yet other embodiments, R₈ is (C₂-C₆) linear alkynyl. In yet other embodiments, R₈ is (C₇-C₁₂) linear alkynyl. In yet other embodiments, R₈ is (C₄-C₆) linear alkynyl. In yet other embodiments, R₈ is (C₆-C₈) linear alkynyl.

In some embodiments, R₁ is H. In yet other embodiments, R₁ is halogen. In yet other embodiments, R₁ is selected from the group consisting of H, OR_{A}, SR_{A}, NR_{A}R_{B}, - C(=O)R_{A}, -C(=O)OR_{A}, -C(=O)NR_{A}R_{B}, -NR_{A}C(=O)R_{B}, -NR_{A}C(=O)(CH₂)_{O}R_{B}, - NR_{A}C(=O)(CH₂)_{O}OR_{B}, -NR_{A}C(=O)(CH₂)_{O}NR_{A}R_{B}, -C(=O)NR_{A}(CH₂)_{O}R_{B}, - C(=O)NR_{A}(CH₂)_{O}OR_{B}, and -C(=O)NR_{A}(CH₂)_{O}NR_{A}R_{B}. In other embodiments, R₁ is selected from the group consisting of O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NRc(CH₂)ₘNR_{A}R_{B}, and NR_{C}(CH₂)ₘNR_{C}(CH₂)ₘNR_{A}R_{B}.

In certain specific embodiments, the compound disclosed herein has the structure of Formulae (II) or (III): 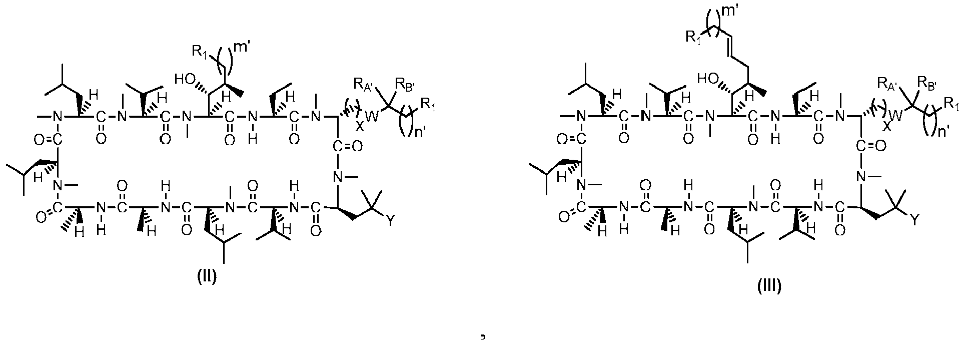 or pharmaceutically acceptable salt thereof, wherein:
x is 0 or 1;
Y is H or OR₅; wherein R₅ is H or methyl;
m' and n' are each independently 0, 1, 2, 3, 4, 5, 6, 7, 8, 9, 10, 11, or 12;
each occurrence of R_{A'} and R_{B'} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or R_{A'} and R_{B'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl; and
each occurrence of R_{D} is independently halogen, hydroxy, O(C₁-C₄)alkyl, C(=O)(C₁-C₄)alkyl, C(=O)O(C₁-C₄)alkyl or 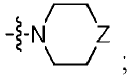 wherein Z is CH₂, O, S, NH, NCH₃, NEt, N-isopropyl, N-isopropyl, N-neoPentyl, N-CH₂CH₂OH, or N-CH₂CH₂OMe.

In certain specific embodiments, the compound disclosed herein has the structure of Formula (IV) or (V): 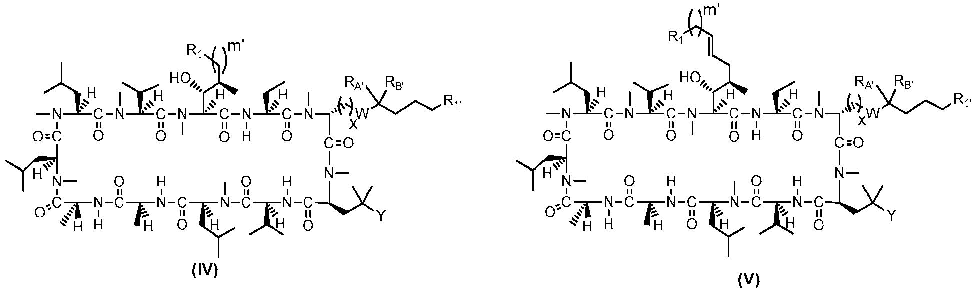 ; wherein x is 0 or 1; Y is H or OR₅; wherein R₅ is H or methyl; each occurrence of R_{1'} is independently H, halogen, aryl or substituted aryl, heteroaryl or substituted heteroaryl, OR_{A}, SR_{A}, NR_{A}R_{B}, -NR_{A}(CH₂)_{O}OR_{B}, -N((CH₂)_{O}OR_{A})((CH₂)_{O}OR_{B}) -C(=O)R_{A}, -C(=O)OR_{A}, - OC(=O)R_{A}, -OC(=O)(C₁-C₆ alkyl-R_{H}), -OC(=O)(CH₂)ₒOR_{A}, -C(=O)NR_{A}R_{B}, -NR_{A}C(=O)R_{B}, - N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B}, -N(C(=O)(CH₂)_{O}OR_{B})₂, - NR_{A}C(=O)(CH₂)_{O}NR_{A}R_{B}, -NR_{A}(CH₂)_{O}C(=O)OR_{B}, -N((CH₂)_{O}C(=O)OR_{A})((CH₂)_{O}C(=O)OR_{B}), - NR_{A}(CH₂)_{O}C(=O)NR_{A}R_{B}, -N((CH₂)_{O}C(=O)NR_{A}R_{B})((CH₂)_{O}C(=O)NR_{A}R_{B}), - NR_{A}(CH₂)_{O}C(=O)NR_{A}(CH₂)_{O}OR_{B}, -C(=O)N((CH₂)_{O}OR_{B})₂, - N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, - C(=O)NR_{A}(CH₂)_{O}OR_{B}, -C(=O)NR_{A}(CH₂)_{O}NR_{A}R_{B}, -N=CR_{A}-NR_{A}R_{B}, -NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOP_{A}, O(CH₂)ₘO(CH₂)ₘOR_{A}, O(CH₂)ₘNR_{A}R_{B}, O(CH₂)ₘO(CH₂)ₘNR_{A}R_{B}, NRc(CH₂)ₘNR_{A}R_{B}, NR_{C}(CH₂)ₘNR_{C}(CH₂)ₘNR_{A}R_{B}, 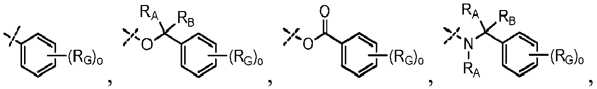 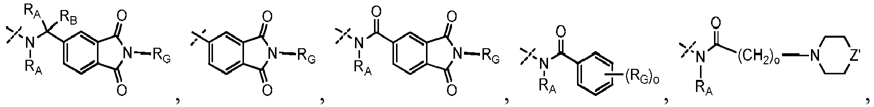 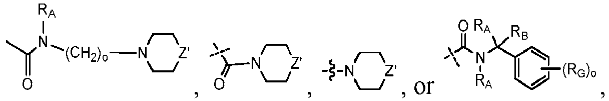 wherein said aryl or heteroaryl is optionally substituted by one or more groups which may be the same or different selected from the group consisting of halogen, hydroxy, (C₁-C₆)alkyl, (CH₂)ₚORA, (CH₂)ₚNR_{A}R_{B}, (CH₂)ₚC(=O)NR_{A}R_{B} and (CH₂)ₚC(=O)OR_{A}.

In certain specific embodiments, R_{1'} is OR_{A}, OCOCH₂OR_{A}, SR_{A}, NHR_{A}, N(R_{A})₂, or NHCOCH₂OR_{A}. In certain specific embodiments, R_{1'} is OAc, OCOCH₂Cl, OCOCH₂CH₃, OCOCHMe₂, OCOCMe₃, OCOCH=CH₂, NHCH₂CH₂OH, NHCH₂CH₂OMe, N(CH₂CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂.

In certain specific embodiments, R_{A'} and R_{B'} are each independently H, Me, Et, n-Propyl, isoProyl, isoButyl, neoPentyl, cyclopentyl, cyclohexyl, CH₂CH₂OH, CH₂CH₂OMe, 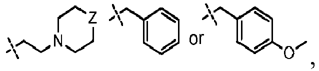 wherein Z is CH₂, O, S, NH, NCH₃, NEt, N-isopropyl, N-isopropyl, N-neoPentyl, N-CH₂CH₂OH, or N-CH₂CH₂OMe. In certain specific embodiments, R_{A'} and R_{B'} are each Me. In certain specific embodiments, R_{A'} and R_{B'} are each Et.

In certain specific embodiments, the compound disclosed herein has the structure of Formula (VI): 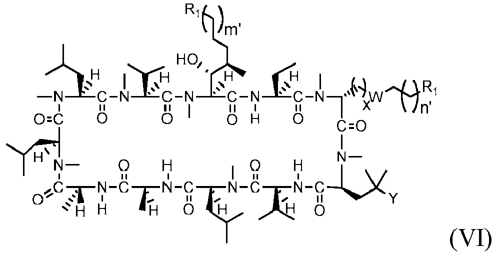 wherein
x is 0 or 1;
W is CH₂, O or S;
Y is H or OR₅; wherein R₅ is H or methyl;
m' and n' are each independently 0, 1, 2, 3, 4, 5, 6, 7, 8, 9, or 10; and
   each occurrence of R₁ is independently H, halogen, aryl or substituted aryl, heteroaryl or substituted heteroaryl, OR_{A}, SR_{A}, NR_{A}R_{B}, -C(=O)R_{A}, -C(=O)OR_{A}, - C(=O)NR_{A}R_{B}, -NR_{A}C(=O)R_{B}, -NR_{A}C(=O)(CH₂)_{O}R_{B}, -NR_{A}C(=O)(CH₂)_{O}OR_{B}, - NR_{A}C(=O)(CH₂)_{O}NR_{A}R_{B}, or -C(=O)NR_{A}(CH₂)_{O}R_{B}.

In certain specific embodiments, R₁ is selected from the group consisting of H, OR_{A}, SR_{A}, NR_{A}R_{B}, -C(=O)R_{A}, -C(=O)OR_{A}, -C(=O)NR_{A}R_{B}, -NR_{A}C(=O)R_{B}, - NR_{A}C(=O)(CH₂)_{O}R_{B}, -NR_{A}C(=O)(CH₂)_{O}OR_{B}, -NR_{A}C(=O)(CH₂)_{O}NR_{A}R_{B}, - C(=O)NR_{A}(CH₂)_{O}R_{B}, -C(=O)NR_{A}(CH₂)_{O}OR_{B}, and -C(=O)NR_{A}(CH₂)_{O}NR_{A}R_{B}.

In certain specific embodiments, R₁ is selected from the group consisting of O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, and NR_{C}(CH₂)ₘNR_{C}(CH₂)ₘNR_{A}R_{B}.

In certain specific embodiments, R₁ is selected from the group consisting of OR_{A}, SR_{A}, NR_{A}R_{B}, -C(=O)R_{A}, -C(=O)OR_{A}, and -C(=O)NR_{A}R_{B}. In some specific embodiments, R₁ is OH, OMe, OEt, O-isopropyl, O-isoButyl, O-neoPentyl, O-cyclopentyl, O-cyclohexyl, SH, SMe, S-isopropyl, S-isoButyl, S-neoPentyl, S-cyclopentyl, S-cyclohexyl, 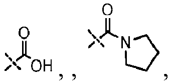 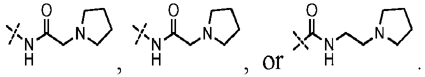 In certain specific embodiments, R₁ is 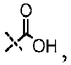 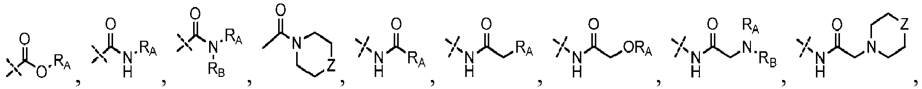 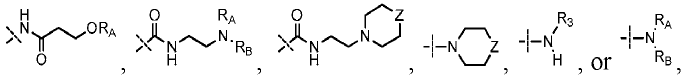 wherein Z is CH₂, O, S, NH, NCH₃, NEt, N-isopropyl, N-isopropyl, N-neoPentyl, N-CH₂CH₂OH, or N-CH₂CH₂OMe. In some embodiments, R_{A} and R_{B} are described herein. In other embodiments, R_{A} and R_{B} are each H, Me, Et, isoProyl, isoButyl, cyclopentyl, or cyclohexyl. In certain embodiments, R₁ is hydroxyl. In certain other embodiments, R₁ is C(=O)OR_{A}.

In yet other embodiments, R₁ is selected from the group consisting of H, OH, OMe, OEt, O-isoProyl, O-isoButyl, O-neoPentyl, O-cyclopentyl, O-cyclohexyl, OCH₂CH₂OH, OCH₂CH₂OCH₃, OCH₂COOH, OCH₂COOCH₃, OCH₂CONH₂, OCH₂CONHMe, OCH₂CONMe₂, OCH₂CONHCH₂CH₂OH, OCH₂CONHCH₂CH₂OMe, OAc, OOCCH₂Cl, OOCCH₂OR_{A}, OOCCH₂CH₃, OOCCHMe₂, OOCCMe₃, and OC(=O)CCH=CH₂.

In yet other embodiments, R₁ is selected from the group consisting of:

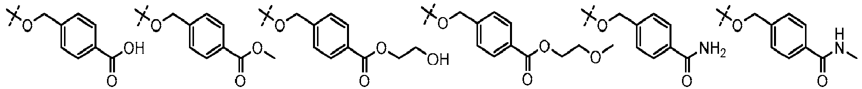

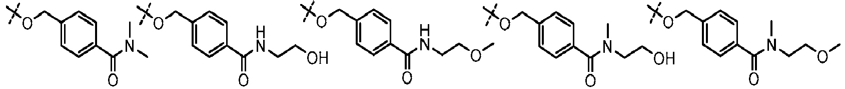

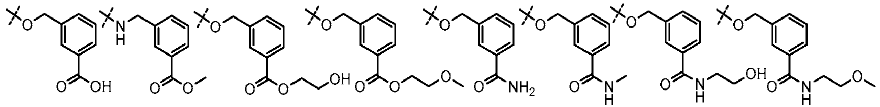

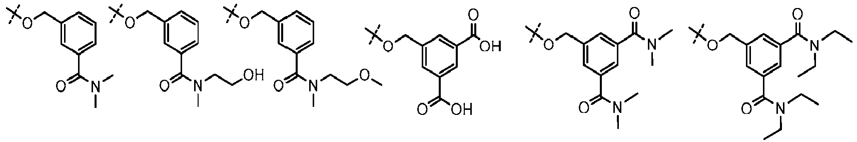

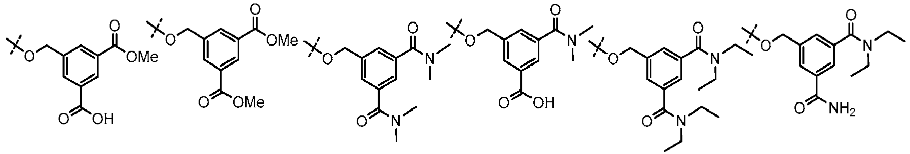

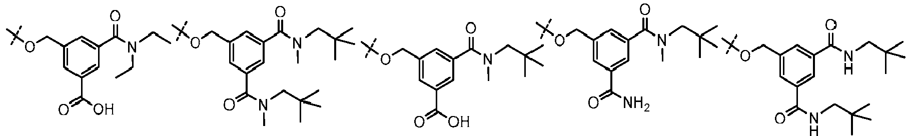

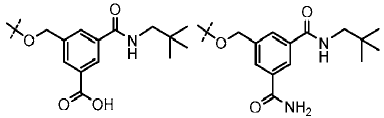

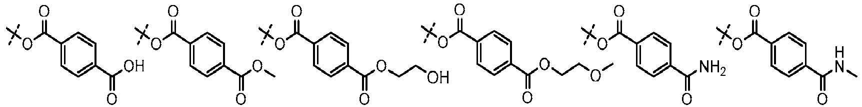

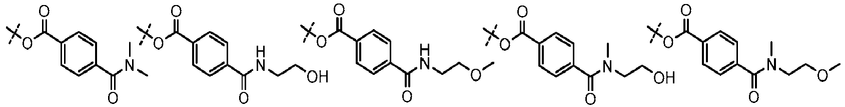

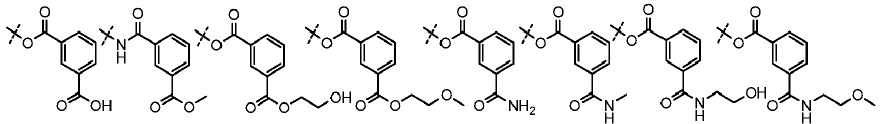

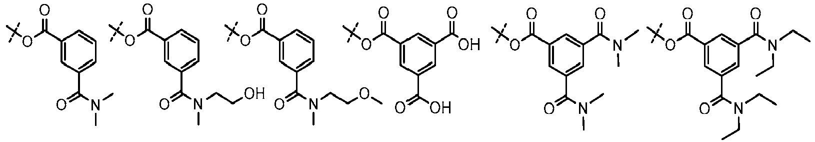

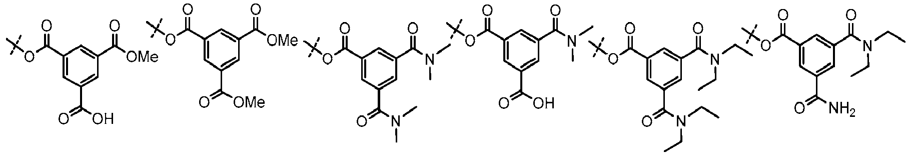

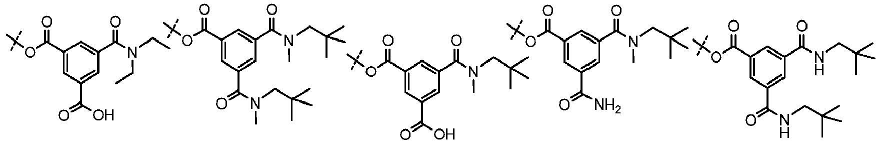

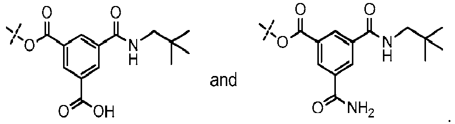

In yet other embodiments, R₁ is SH, SMe, SEt, S-isoProyl, S-isoButyl, S-neoPentyl, O-cyclopentyl, or S-cyclohexyl.

In yet other embodiments, R₁ is selected from the group consisting of NH₂, NHCH₃, NHCH₂CH₃, NHCH₂CHOH, NHCH₂CH₂OMe, NMe₂, NEt₂, NHCH₂CHMe₂, NHCH₂CMe₃, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂OR_{A}, NHCOCH₂CH₃, NHCOCHMe₂, NHCOCMe₃, NHCOCH=CH₂, N(COCH₂Cl)₂, N(COCH₂OR_{A})₂, N(COCH₂CH₃)₂, N(COCHMe₂)₂, N(COCMe₃)₂, and N(COCH=CH₂)₂.

In yet other embodiments, R₁ is 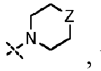 wherein Z is CH₂, O, S, NH, NMe, NEt, N-isopropyl, N-neoPentyl, N-CH₂CH₂OH, NCH₂CH₂OCH₃, NCH₂CH₂OCH₃, NCH₂COOH, NCH₂COOMe, N-CH₂CONH₂, NCH₂CONHMe, or NCH₂CONMe₂.

In yet other embodiments, R₁ is selected from the group consisting of:

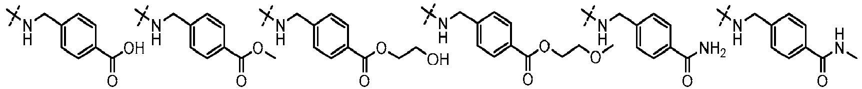

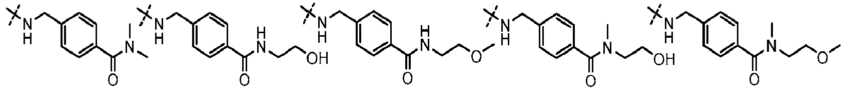

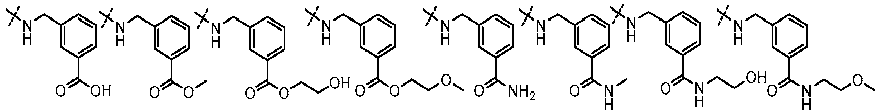

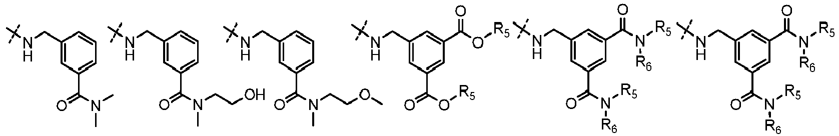

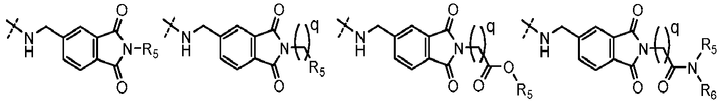

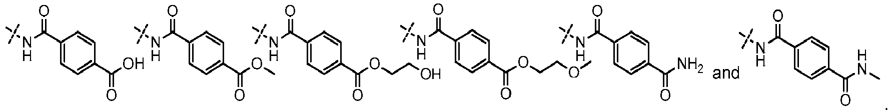

In yet other embodiments, R₁ is selected from the group consisting of: 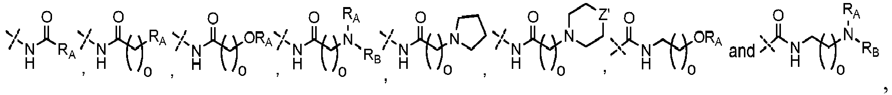 wherein each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNR_{A}C(=O)R_{B}, or N(CH₂)ₒOC(=O)(CH₂)ₒOR_{A}.

In yet other embodiments, R₁ is -COOH, -COOMe, -COOEt, -CONH₂, -CONHMe,-CONMe₂, -CONHEt, -CONEt₂, -CONHCH₂CH₂OH, -CONHCH₂CH₂OMe,-CON(CH₂CH₂OH)₂, -CON(CH₂CH₂OMe)₂, or -CONMe₂.

In yet other embodiments, R₁ is selected from the group consisting of: 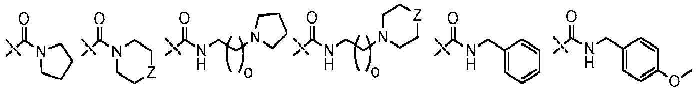 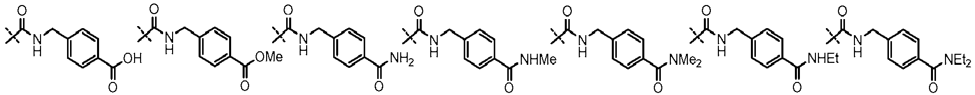 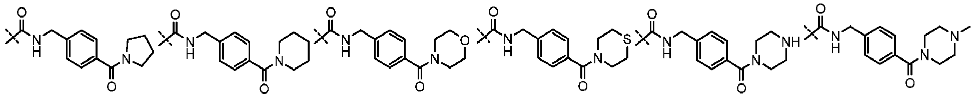 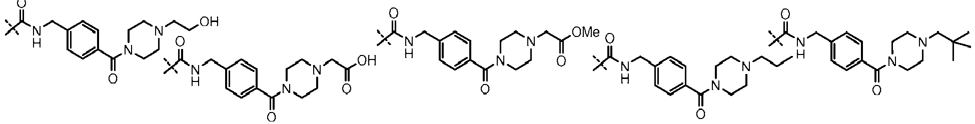 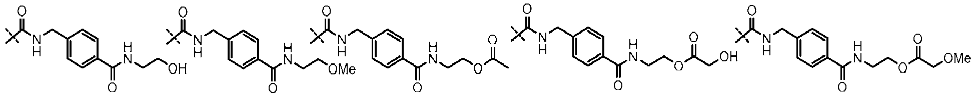 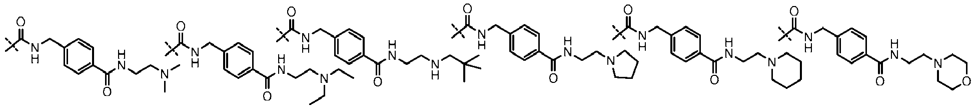 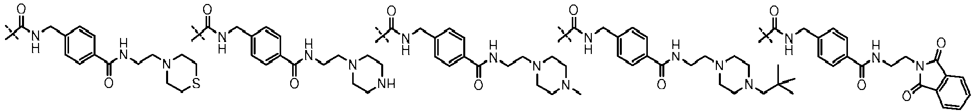 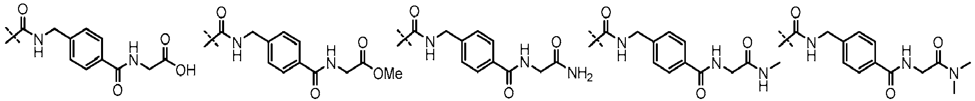 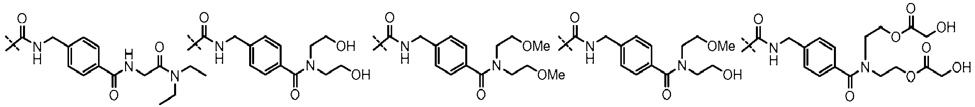 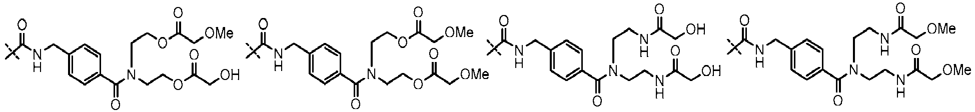 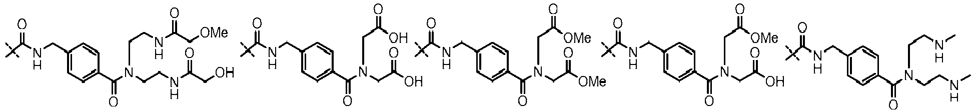 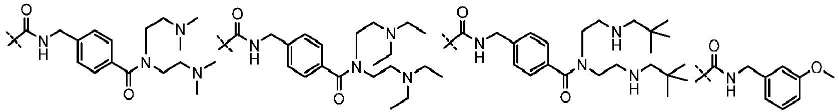 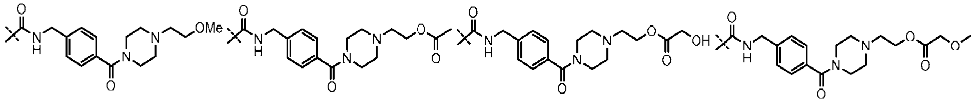 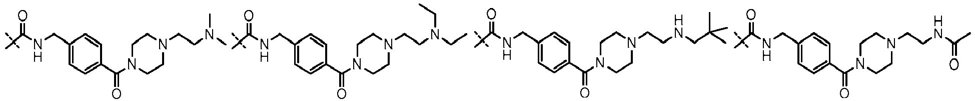 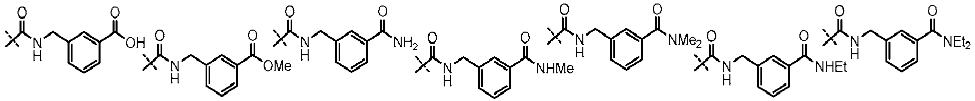 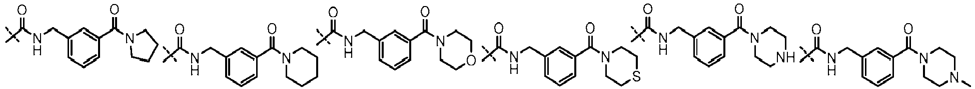 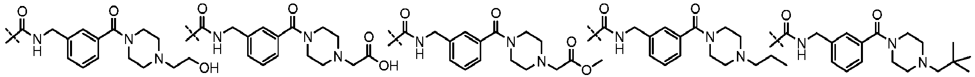 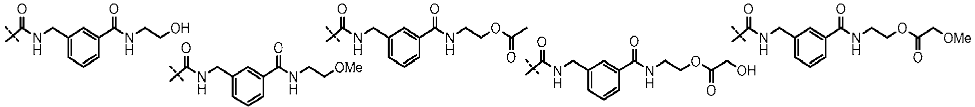 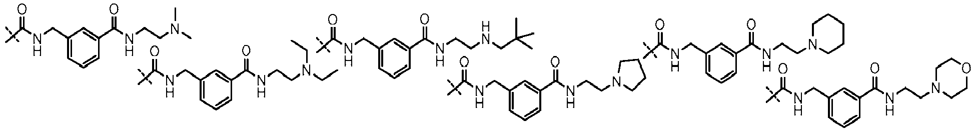 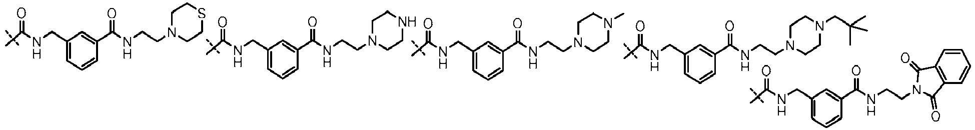 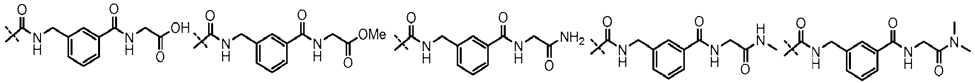 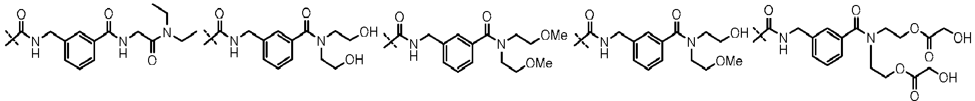 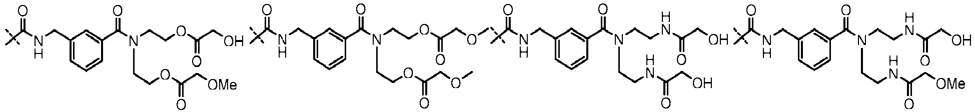 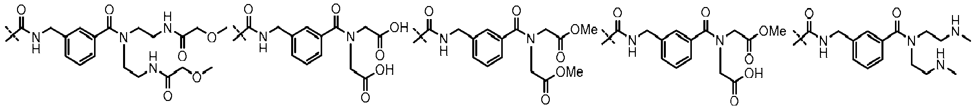 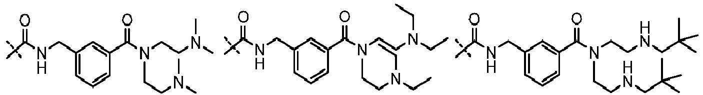 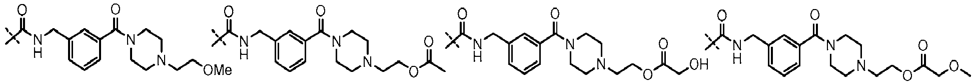 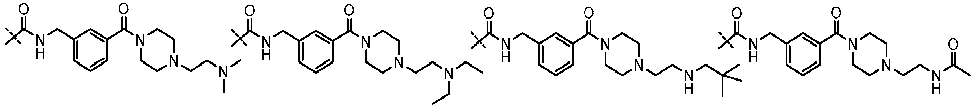 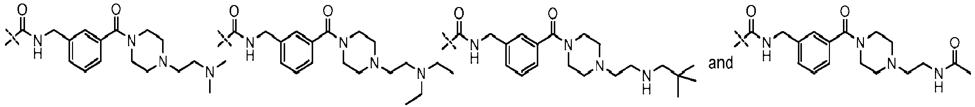 ; wherein Z is CH₂, O, S, NH, NCH₃, NEt, N-isopropyl, N-isopropyl, N-neoPentyl, N-CH₂CH₂OH, or N-CH₂CH₂OMe.

In yet other embodiments, R₁ is 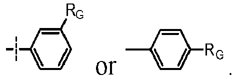 In some embodiments, R_{G} is OH, OMe, OAc, NH₂, NHMe, NHAc, NMe₂, NEt₂, NHCH₂CMe₃.

In yet other embodiments, R₁ is selected from the group consisting of:

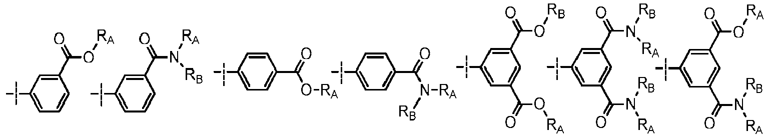

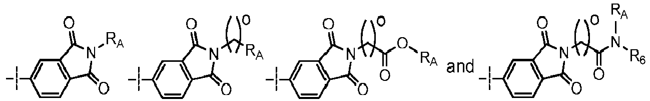

In yet other embodiments, R₁ is OR_{A}, OCOCH₂OR_{A}, SR_{A}, NHR_{A}, N(R_{A})₂, or NHCOCH₂OR_{A}.

In yet other embodiments, R₁ is OAc, OCOCH₂Cl, OCOCH₂CH₃, OCOCHMe₂, OCOCMe₃, OCOCH=CH₂, NHCH₂CH₂OH, NHCH₂CH₂OMe, N(CH₂CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂.

In yet other embodiments, R₁ is aryl or heteroaryl optionally substituted by one or more groups which may be the same or different selected from halogen, hydroxy, (C₁-C₆)alkyl, (C₃-C₇)cycloalkyl, SR_{A}, (CH₂)ₚOR_{A}, (CH₂)ₚNR_{A}R_{B}, (CH₂)ₚC(=O)R_{A}, (CH₂)ₚC(=O)NR_{A}R_{B} and (CH₂)ₚC(=O)OR_{A}; wherein p is 0, 1, 2, 3, 4, 5, 6; and each occurrence of R_{A} and R_{B} are defined herein. In some embodiments, R₁ is 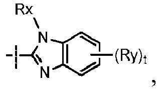 wherein Rx is H, (C₁-C₆)alkyl, or (C₃-C₇)cycloalkyl; Ry is H, (C₁-C₆)alkyl, (C₃-C₇)cycloalkyl, OR_{A}, SR_{A}, NR_{A}R_{B}, -C(=O)R_{A}, -C(=O)OR_{A}, or -C(=O)NR_{A}R_{B}; and t is 1, 2, 3, or 4. In some embodiments, Rx is H or Me; and Ry is -C(=O)OR_{A} or -C(=O)NR_{A}R_{B}. In some specific embodiments, R₁ is 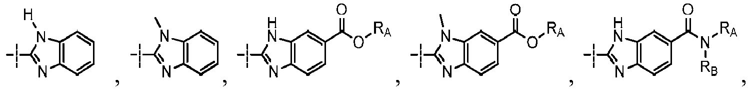 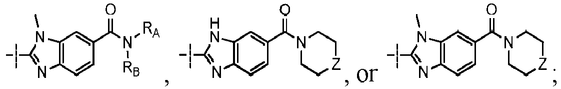 wherein Z is CH₂, O, S, NH, NCH₃, NEt, N-isopropyl, N-isopropyl, N-neoPentyl, N-CH₂CH₂OH, or N-CH₂CH₂OMe.

In yet other embodiments, R_{A} and R_{B} are each independently H, Me, Et, isoProyl, isoButyl, neoPentyl, cyclopentyl, cyclohexyl, CH₂CH₂OH, CH₂CH₂OMe, or 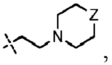 wherein Z is CH₂, O, S, NH, NCH₃, NEt, N-isopropyl, N-isopropyl, N-neoPentyl, N-CH₂CH₂OH, or N-CH₂CH₂OMe. In some embodiments, R_{A} and R_{B} are each independently H, Me, Et, isopropyl, isobutyl, cyclopentyl, or cyclohexyl. In some embodiments, each occurrence R_{A} and R_{B} is independently H or (C₁-C₆)alkyl.

In some embodiments, R₃ is H, (C₁-C₁₂)alkyl, (C₂-C₁₂)alkenyl, (C₂-C₁₂)alkynyl, (C₃-C₁₂)cycloalkyl, or phenyl or CH₂-phenyl, optionally substituted by one or more groups which may be the same or different selected from halogen, hydroxy, (C₁-C₆)alkyl. In some embodiments, R₃ is (C₁-C₁₂)alkyl. In yet other embodiments, R₃ is (C₁-C₆) linear alkyl. In yet other embodiments, R₃ is (C₇-C₁₂) linear alkyl. In yet other embodiments, R₃ is (C₄-C₆) linear alkyl. In yet other embodiments, R₃ is (C₆-C₈) linear alkyl. In certain embodiments, R₃ is (C₇-C₁₀)alkyl. In certain other embodiments, R₃ is (C₇-C₈)alkyl. In yet other embodiments, R₃ is (C₇-C₁₀) linear alkyl. In yet other embodiments, R₃ is (C₇-C₈) linear alkyl. In some specific embodiments, R₃ is a -(CH₂)₃₋₁₁-alkyl chain.

In some embodiments, R₃ is (C₂-C₁₂)alkenyl. In yet other embodiments, R₃ is (C₂-C₆) linear alkenyl. In yet other embodiments, R₃ is (C₇-C₁₂) linear alkenyl. In yet other embodiments, R₃ is (C₄-C₆) linear alkenyl. In yet other embodiments, R₃ is (C₆-C₈) linear alkenyl.

In some embodiments, R₃ is (C₂-C₁₂)alkynyl. In yet other embodiments, R₃ is (C₂-C₆) linear alkynyl. In yet other embodiments, R₃ is (C₇-C₁₂) linear alkynyl. In yet other embodiments, R₃ is (C₄-C₆) linear alkynyl. In yet other embodiments, R₃ is (C₆-C₈) linear alkynyl. In any of the embodiments described herin, R₃ may be 4-hydroxybutyl.

In certain embodiments, R₂ is ethyl. In certain embodiments, R₇ is 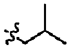 or 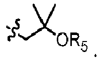 In certain embodiments, W is O or S. In certain embodiments described herin, W is S; x = 1; and R₃ is 4-hydroxybutyl.

In certain embodiments, the compound of Formula I has the structure of Formulae (II') through (VI'): 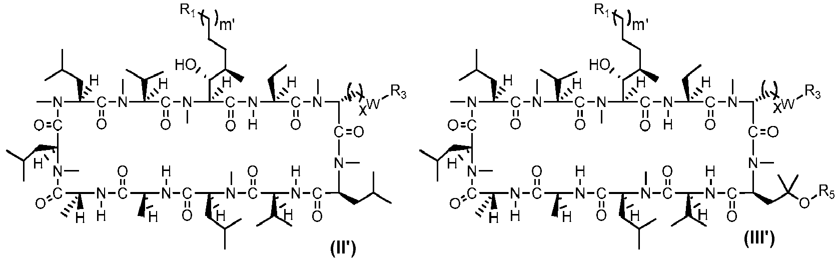 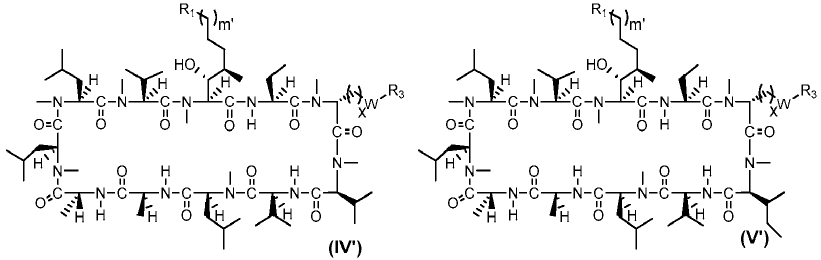 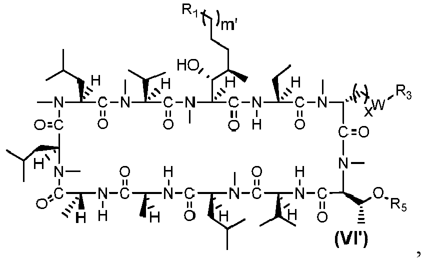 or pharmaceutically acceptable salt thereof, wherein:
each x is 0 or 1; each W is independently O, S, or CH₂,
each occurrence of R₁ is independently H, halogen, aryl, heteroaryl, OR_{A}, SR_{A}, NR_{A}R_{B}, -C(=O)R_{A}, -C(=O)OR_{A}, -C(=O)NR_{A}R_{B}, -NR_{A}C(=O)R_{B}, -NR_{A}C(=O)(CH₂)_{O}R_{B}, -NR_{A}C(=O)(CH₂)_{O}OR_{B}, -NR_{A}C(=O)(CH₂)_{O}NR_{A}R_{B}, -C(=O)NRₐ(CH₂)_{O}R_{B}, -N=CR_{A}-NR_{A}R_{B}, -NR_{B}-C(=NH)-NR_{A}R_{B}, -C(=O)NR_{A}(CH₂)_{O}OR_{B}, -C(=O)NR_{A}(CH₂)_{O}NR_{A}R_{B}, O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or heteroaryl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each R₃ is independently (C₁-C₆)alkyl, (C₇-C₁₂)alkyl, (C₂-C₆)alkenyl, (C₇-C₁₂)alkenyl, (C₂-C₆)alkynyl, or (C₇-C₁₂)alkynyl, aryl, or heteroaryl all of which may be optionally substituted by one or more groups R₁ which may be the same or different;
each R₅ is independently:
   H;
   (C₁-C₆)alkyl, optionally substituted by one or more groups R₆, which may be the same or different;
   (C₂-C₆)alkenyl, optionally substituted by one or more groups which may be the same or different and each selected from halogen, hydroxy, (C₁-C₆)alkyl, aryl (e.g., phenyl), (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different and each selected from halogen, hydroxy, amino, monoalkylamino and dialkylamino;
   (C₃-C₇)cycloalkyl, optionally substituted by one or more groups which may be the same or different and each selected from halogen, hydroxy, amino, monoalkylamino and dialkylamino;
   phenyl or CH₂-phenyl, optionally substituted by one or more groups which may be the same or different and each selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different and each selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different and each selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different and each selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different and each selected from the group consisting of alkyl, phenyl and benzyl;
   or R_{A} and R_{B}, together with the nitrogen atom to which they are attached, form -N=CH-NR_{F}R_{F'}, -N=CMe-NR_{F}R_{F'}, or -NR_{F}C(=NH)NR_{F}R_{F'};
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each occurrence of R_{F} and R_{F}, is independently hydrogen, (C₁-C₆)alkyl, phenyl, benzyl, or R_{F} and R_{F'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different and each selected from the group consisting of alkyl, phenyl and benzyl;
p is an integer of 0, 1, 2, 3, 4, 5, or 6;
m' is 0, 1, 2, 3, 4, 5, 6, 7, 8 or 9;
m is an integer of 1, 2, 3, 4, 5, or 6; and
n is an integer of 1, 2, 3, 4, 5 or 6.

In any of the embodiments described herein, x is 0 or 1. In any of the embodiments described herein, x may be 0. In any of the embodfiemtns described herein, x may be 1. In certain embodiments, W is O. In certain other embodiments, W is S. In yet other embodiments, W is CH₂.

In certain embodiments, m' is 1. In certain other embodiments, m' is 2. In yet other embodiments, m' is 3. In yet other embodiments, m' is 4 or 5. In yet other embodiments, m' is 6, 7, 8 or 9.

In certain embodiments, m is 1. In certain other embodiments, m is 2. In yet other embodiments, m is 3. In yet other embodiments, m is 4 or 5.

In certain embodiments, p is 0. In certain other embodiments, p is 1. In yet other embodiments, m is 2. In yet other embodiments, m is 3, 4 or 5.

In certain embodiments, R₃ is 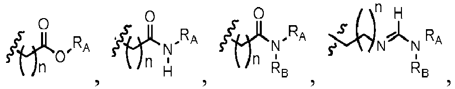 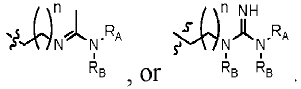 In certain embodiments, R₃ is H, methyl, ethyl, n-propyl, i-propyl, n-butyl, i-butyl, t-butyl, CH₂CMe₃, phenyl, CH₂-phenyl,

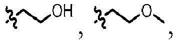

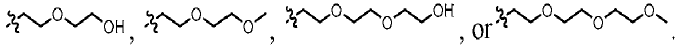

In certain embodiments, R₃ is -(CH₂)ₙNR_{A}R_{B}, wherein n is an integer of 2, 3, 4, 5 or 6, or integer of 7, 8, 9, 10, 11 or 12; and wherein each occurrence of R_{A} and R_{B} is independently hydrogen; (C₁-C₄)alkyl, optionally substituted by one or more groups R_{D} which may be the same or different, in which each occurrence of R_{D} is independently halogen, hydroxy, O(C₁-C₄)alkyl, C(=O)(C₁-C₄)alkyl, C(=O)O(C₁-C₄)alkyl; or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from (C₁-C₄)alkyl, phenyl and benzyl.

In certain embodiments, R₃ is -(CH₂)ₙNR_{A}R_{B}, wherein n is an integer of 2, 3, 4, 5 or 6, or integer of 7, 8, 9, 10, 11 or 12; and wherein R_{A} and R_{B}, together with the nitrogen atom to which they are attached, form a saturated or unsaturated heterocyclic ring containing from three to seven ring atoms, which ring may optionally contain another heteroatom selected from nitrogen, oxygen and sulfur and may be optionally substituted by from one to four groups which may be the same or different selected from (C₁-C₄)alkyl, phenyl and benzyl.

In certain embodiments, n is 2. In certain other embodiments, n is 3. In yet other embodiments, n is 4, 5, or 6. In yet other embodiments, n is 7 or 8. In yet other embodiments, n is 9 or 10. In yet other embodiments, n is 11 or 12.

In certain embodiments, R₃ is -(CH₂)ₙNR_{A}R_{B}, wherein n is an integer of 7, 8, 9, 10, 11 or 12; and wherein each occurrence of R_{A} and R_{B} is independently hydrogen; (C₁-C₄)alkyl, optionally substituted by one or more groups R_{D} which may be the same or different, in which each occurrence of R_{D} is independently halogen, hydroxy, O(C₁-C₄)alkyl, C(=O)(C₁-C₄)alkyl, C(=O)O(C₁-C₄)alkyl; or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from (C₁-C₄)alkyl, phenyl and benzyl.

In certain embodiments, n is 7. In certain other embodiments, n is 8. In yet other embodiments, n is 9, 10, 11 or 12.

In certain embodiments, R₃ is 2-aminoethyl, 2-aminopropyl, 3-aminopropyl, 2-monoalkylaminoethyl, 2-monoalkylaminopropyl, 3-monoalkylaminopropyl, 2-dialkylaminoethyl, 2-dialkylaminopropyl, or 3-dialkylaminopropyl, wherein said alkyl is (C₁-C₄)alkyl.

In certain embodiments, R₃ is 2-aminoethyl, 2-aminopropyl, 3-aminopropyl, 2-monoalkylaminoethyl, 2-monoalkylaminopropyl, 3-monoalkylaminopropyl, 2-dialkylaminoethyl, 2-dialkylaminopropyl, or 3-dialkylaminopropyl, wherein said alkyl is (C₁-C₄)alkyl, wherein R₃ is dimethylaminoethyl, diethylaminoethyl, methylethylaminoethyl, methyl-iso-butylaminoethyl, ethyl-iso-butylaminoethyl, methyl-tert-butylaminoethyl, or ethyl-tert-butylaminoethyl.

In certain embodiments, R₃ is 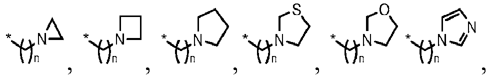 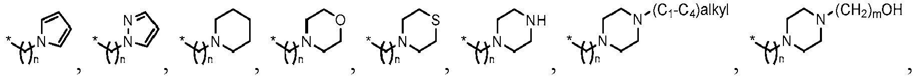 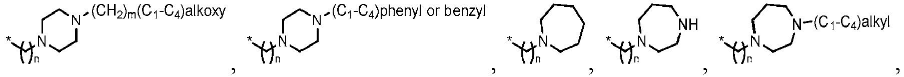 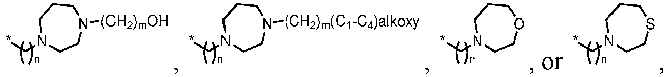 in which n is an integer of 2, 3, 4, 5, or 6, and m is an integer of 2, 3, or 4. In certain embodiments, n is 2. In certain other embodiments, n is 3. In yet other embodiments, n is 4, or 5, or 6. In certain embodiments, m is 2. In certain other embodiments, m is 3. In certain other embodiments, m is 4. In certain embodiments, n is 7. In certain other embodiments, n is 8. In yet other embodiments, n is 9, 10, 11 or 12.

In certain embodiments, R₅ is H, (C₁-C₆)alkyl, (C₂-C₆)alkenyl, phenyl, benzyl, CH₂-S-(C₁-C₆)alky, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substitutents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5.

In certain embodiments, R₅ is H. In certain other embodiments, R₅ is methyl. In yet other embodiments, R₅ is CH₂-S-(C₁-C₆)alky, e.g., CH₂-S-CH₃. In yet other embodiments, R₅ is CH₂-O-(C₁-C₆)alkyl, e.g., CH₂-O-CH₂-CH₃. In yet other embodiments, R₅ is (C₂-C₆)alkenyl, e.g., CH₂-CH=CH₂. In yet other embodiments, R₅ is benzyl. In yet other embodiments, R₅ is (C₂-C₆)OH. In yet other embodiments, R₅ is (C₁-C₆)-monoalkyl amine, e.g., CH₂-NH-Me. In yet other embodiments, R₅ is (C₁-C₆)-dialkyl amine, e.g., CH₂-CH₂-N(Et)₂. In yet other embodiments, R₅ is (C₁-C₆)-cyclic amine, e.g., CH₂-CH₂-morpholine.

In certain embodiments, each occurrence R_{A} and R_{B} is independently H, (C₁-C₆)alkyl, phenyl, CH₂-phenyl, (C₁-C₆)alkylOH, (CH₂)ₚO(CH₂)ₘOH, or (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl. In certain other embodiments, R_{A} and R_{B}, together with the nitrogen atom to which they are attached, form a heterocycle selected from 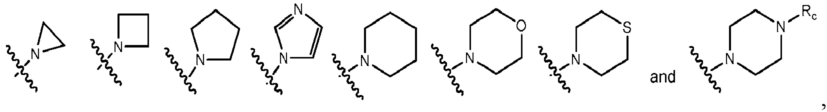 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.

In another aspect, the present invention provides a compound of Formulae (IIa)-(VIa): 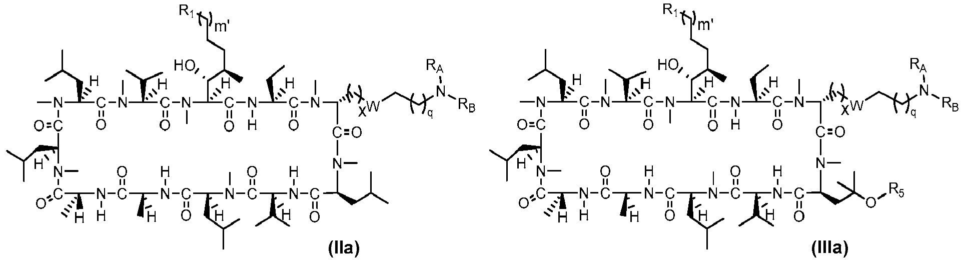 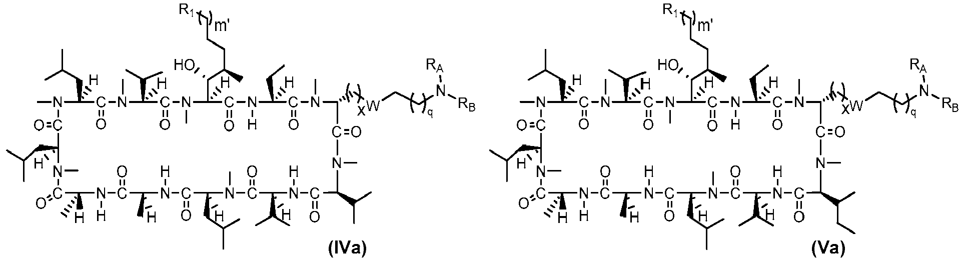 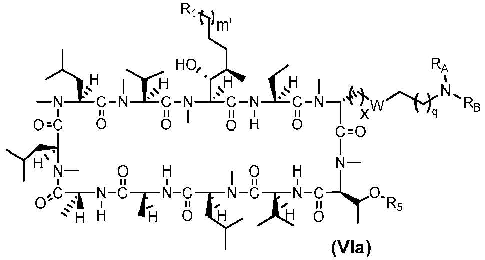 or a pharmaceutically acceptable salt thereof, wherein:
each x is 0 or 1; each W is independently O, S, or CH₂;
each occurrence of R₁ is independently H, halogen, aryl, heteroaryl, OR_{A}, SR_{A}, NR_{A}R_{B}, -C(=O)R_{A}, -C(=O)OR_{A}, -C(=O)NR_{A}R_{B}, -NR_{A}C(=O)R_{B}, -NR_{A}C(=O)(CH₂)_{O}R_{B}, -NR_{A}C(=O)(CH₂)_{O}OR_{B}, -NR_{A}C(=O)(CH₂)_{O}NR_{A}R_{B}, -C(=O)NR_{A}(CH₂)_{O}R_{B}, -C(=O)NR_{A}(CH₂)_{O}OR_{B}, -C(=O)NR_{A}(CH₂)_{O}NR_{A}R_{B}, O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or heteroaryl is optionally substituted by one or more groups which may be the same or different and each selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each R₅ is independently:
   H;
   (C₁-C₆)alkyl, optionally substituted by one or more groups R₆ which may be the same or different;
   (C₂-C₆)alkenyl, optionally substituted by one or more groups which may be the same or different and each selected from halogen, hydroxy, (C₁-C₆)alkyl, aryl (e.g., phenyl), (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different and each selected from halogen, hydroxy, amino, monoalkylamino and dialkylamino;
   (C₃-C₇)cycloalkyl, optionally substituted by one or more groups which may be the same or different and each selected from halogen, hydroxy, amino, monoalkylamino and dialkylamino;
   phenyl or CH₂-phenyl, optionally substituted by one or more groups which may be the same or different and each selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different and each selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different and each selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different and each selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different and each selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each p is independently an integer of 0, 1, 2, 3, 4, or 5;
m' is 0, 1, 2, 3, 4, 5, 6, 7, 8, or 9;
each of m and n is independently an integer of 1, 2, 3, 4 or 5;
q is an integer of 1, 2, 3, 4, 5, 6, 7, 8, 9, 10 or 11.

In certain embodiments, q is 1. In certain other embodiments, q is 2. In certain other embodiments, n is independently an integer of 6, 7, 8, 9, 10 or 11.

In certain embodiments, W is S. In certain other embodiments, W is O.

In certain embodiments, R₁ is hydrogen. In certain other embodiments, R₁ is (C₁-C₆)alkyl. In certain embodiments, R₃ is (C₁-C₆)alkyl. In certain other embodiments, R₃ is NR_{C}CH₂(CH₂)ₚNR_{A}R_{B}.

In certain embodiments, R₅ is H, (C₁-C₆)alkyl, (C₂-C₆)alkenyl, phenyl, benzyl, CH₂-S-(C₁-C₆)alkyl, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substituents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5.

In certain other embodiments, R₅ is H, (C₁-C₄)alkyl, (C₂-C₄)alkenyl, phenyl, benzyl, CH₂-S-(C₁-C₄)alkyl, CH₂-O-(C₁-C₄)alkyl, (CH₂)₂OH, or (CH₂)₂O(C₁-C₄)alkyl. In certain embodiments, R₅ is H. In certain other embodiments, R₅ is methyl.

In certain embodiments, each occurrence R_{A} and R_{B} is independently H, (C₁-C₆)alkyl, phenyl, CH₂-phenyl, (C₁-C₆)alkylOH, (CH₂)ₚO(CH₂)ₘOH, or (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl. In certain other embodiments, each occurrence R_{A} and R_{B} is independently H or (C₁-C₆)alkyl. In yet other embodiments, R_{A} and R_{B}, together with the nitrogen atom to which they are attached, form a heterocycle selected from 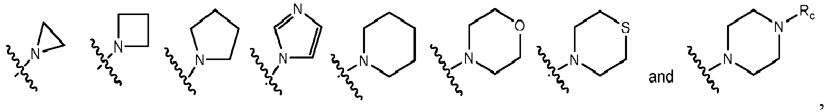 in which R_{C} is H, Me, Et, n-Pr, i-Pr, n-Bu, i-Bu, t-Bu, CH₂CMe₃, Ph, CH₂Ph, or CH₂CH₂OH and CH₂CH₂OR_{d}.

In certain embodiments, the compounds are selected from the group consisting of: 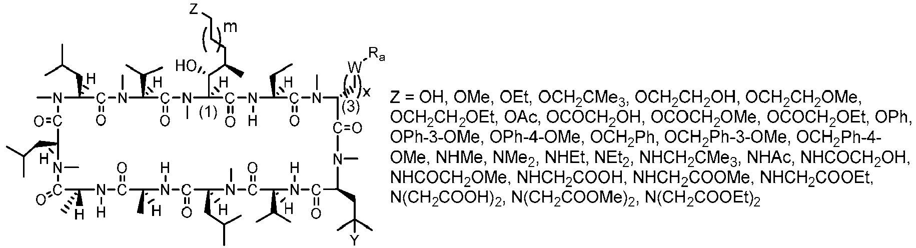 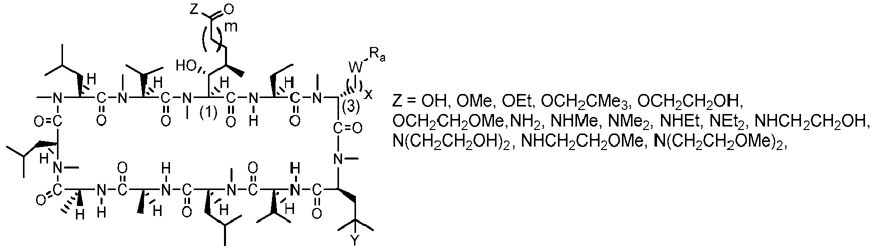 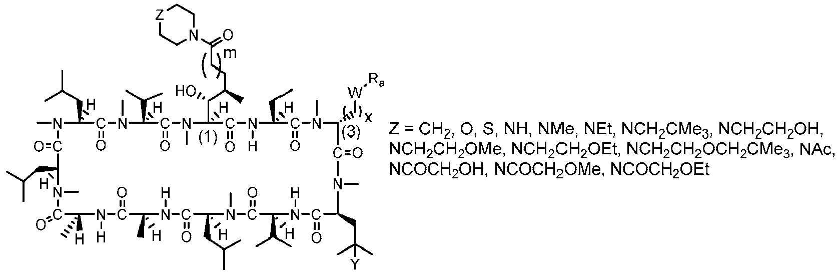 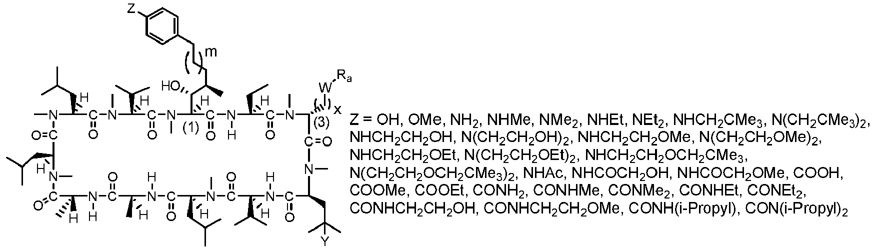 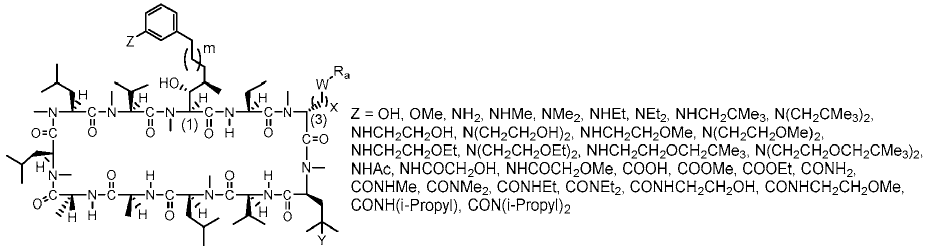 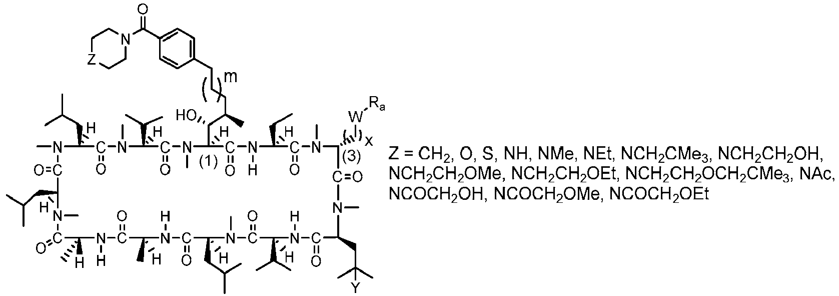 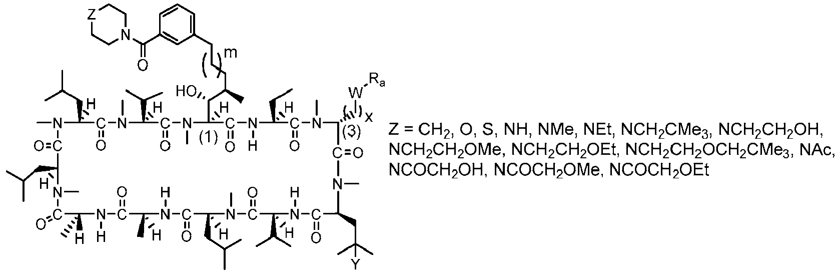 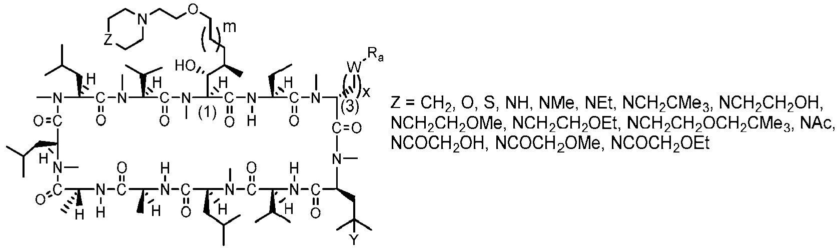 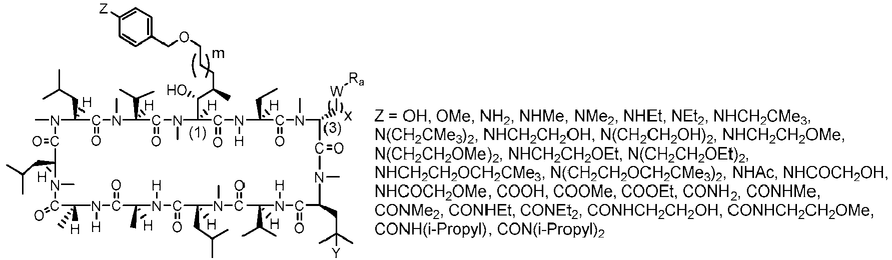 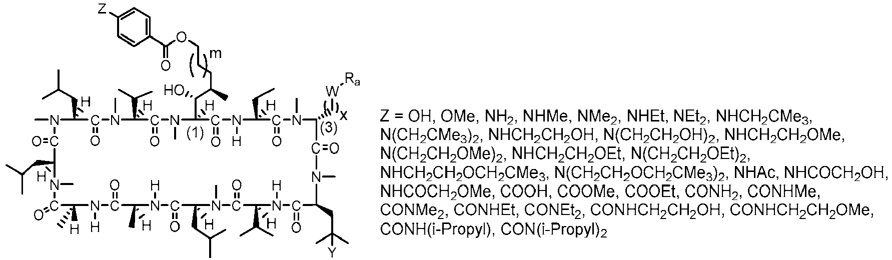 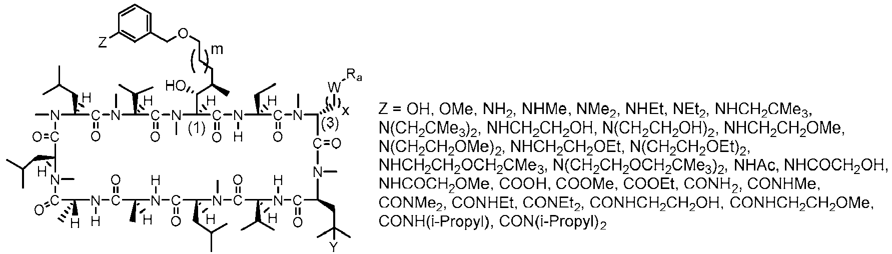 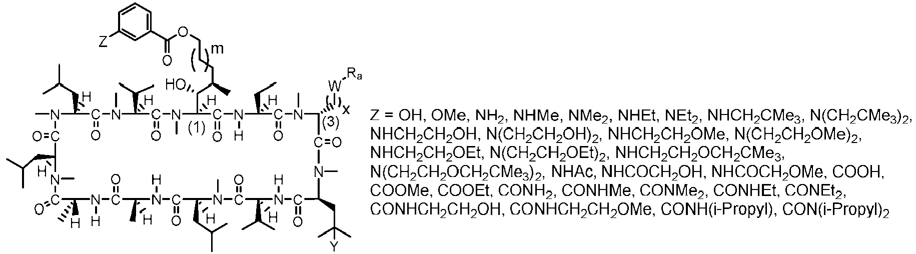 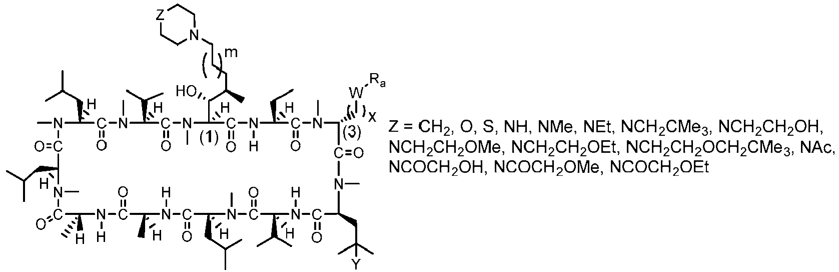 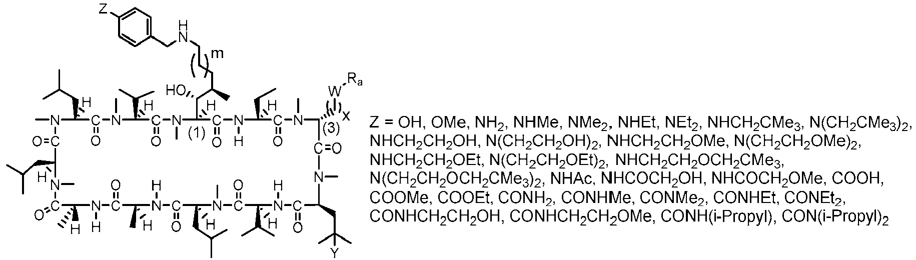 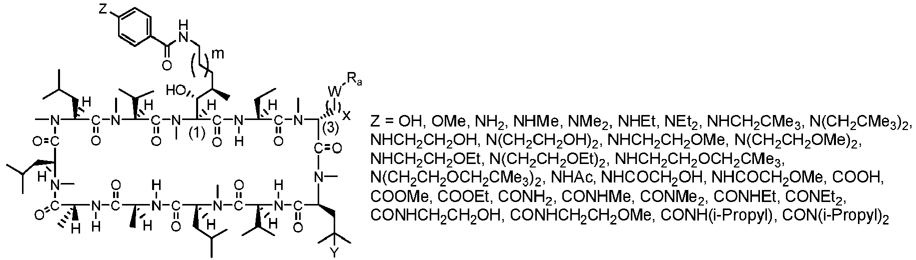 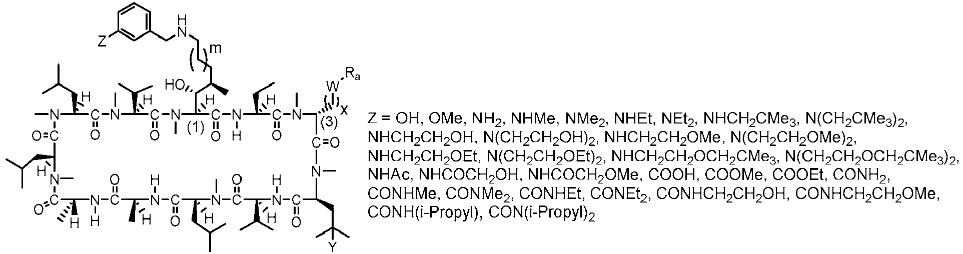 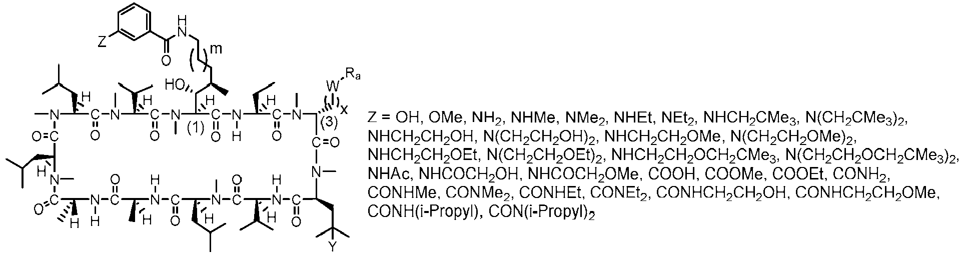 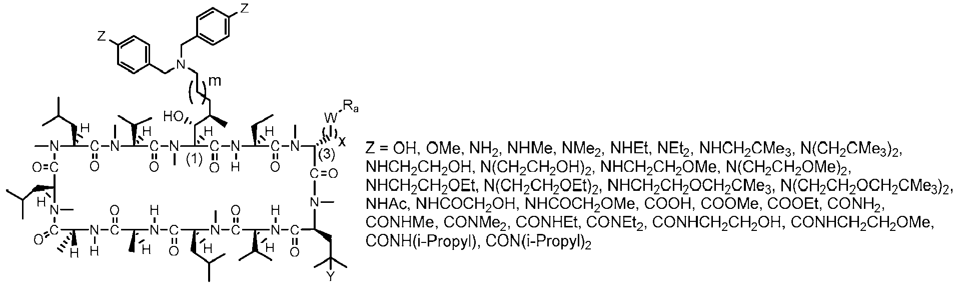 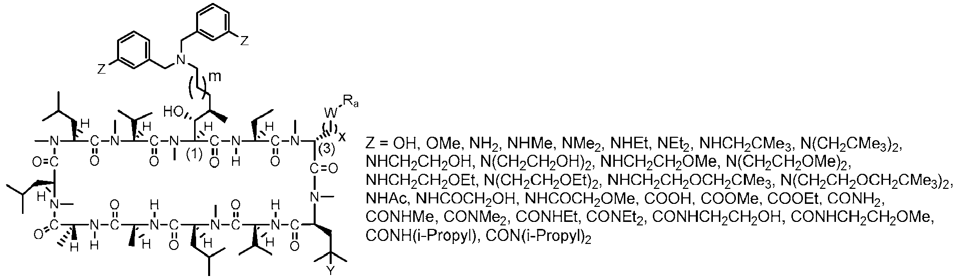 and, wherein x is 0 or 1; m is 2, 3, 4, 5, 6, 7, or 8; Y is H, OH or OMe; W is O or S; and each Rₐ is independently selected from the group consisting of the moieties shown in Table 1;

**Table 1**

| Rₐ | | |
|---|---|---|
| 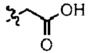 | 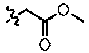 | 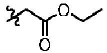 |
| 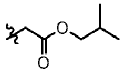 | 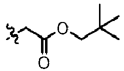 | 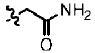 |
| 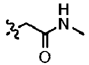 | 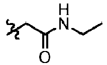 | 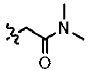 |
| 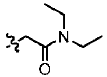 | 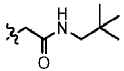 | 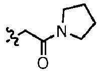 |
| 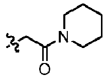 | 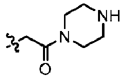 | 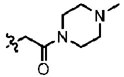 |
| 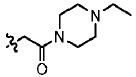 | 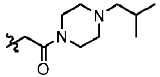 | 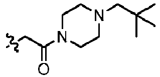 |
| 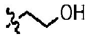 | 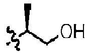 | 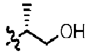 |
| 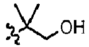 | 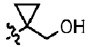 | 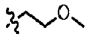 |
| 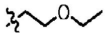 | 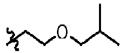 | 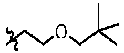 |
| 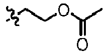 | 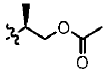 | 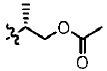 |
| 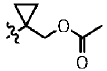 | 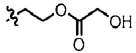 | 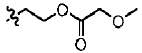 |
| 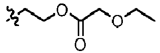 | 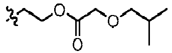 | 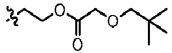 |
| 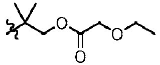 | 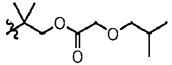 | 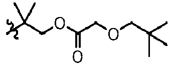 |
| 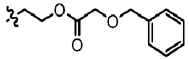 | 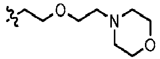 | 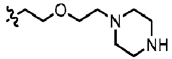 |
| 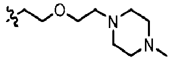 | 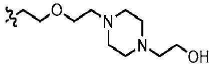 | 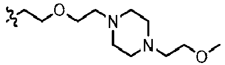 |
| 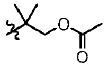 | 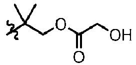 | 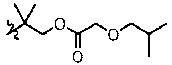 |
| 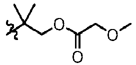 | 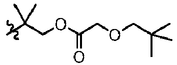 | 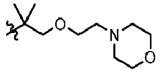 |
| 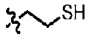 | 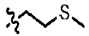 | 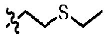 |
| 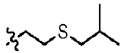 | 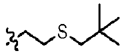 | 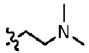 |
| 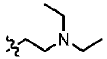 | 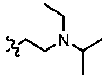 | 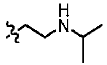 |
| 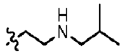 | 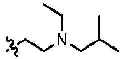 | 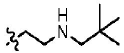 |
| 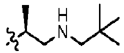 | 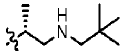 | 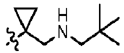 |
| 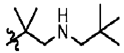 | 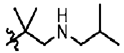 | 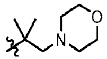 |
| 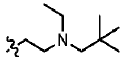 | 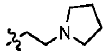 | 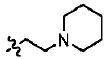 |
| 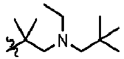 | 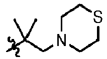 | 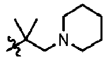 |
| 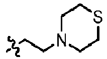 | 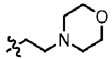 | 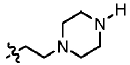 |
| 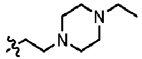 | 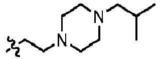 | 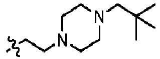 |
| 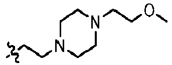 | 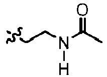 | 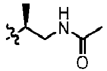 |
| 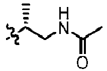 | 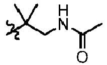 | 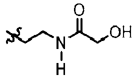 |
| 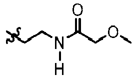 | 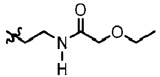 | 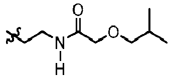 |
| 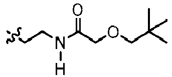 | 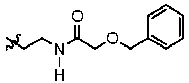 | 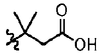 |
| 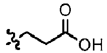 | 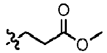 | 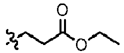 |
| 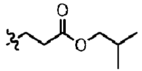 | 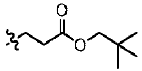 | 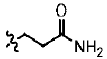 |
| 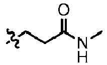 | 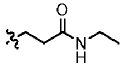 | 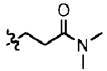 |
| 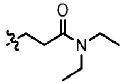 | 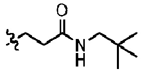 | 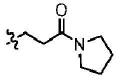 |
| 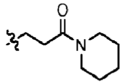 | 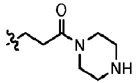 | 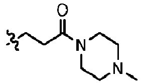 |
| 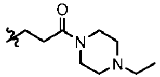 | 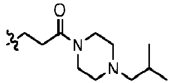 | 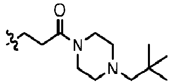 |
| 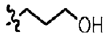 | 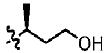 | 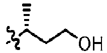 |
| 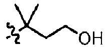 | 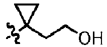 | 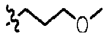 |
| 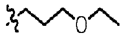 | 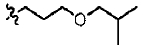 | 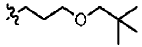 |
| 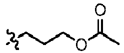 | 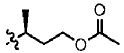 | 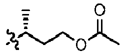 |
| 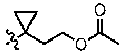 | 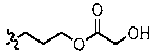 | 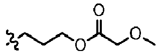 |
| 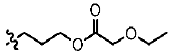 | 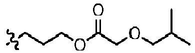 | 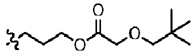 |
| 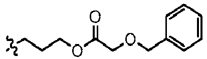 | 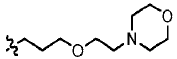 | 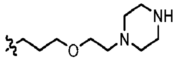 |
| 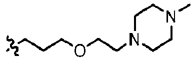 | 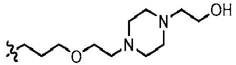 | 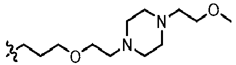 |
| 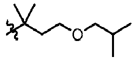 | 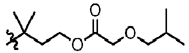 | 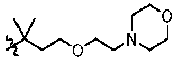 |
| 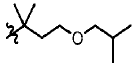 | 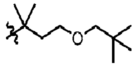 | 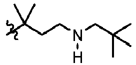 |
| 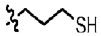 | 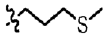 | 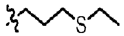 |
| 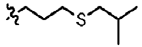 | 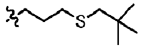 | 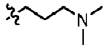 |
| 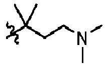 | 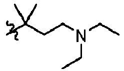 | 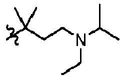 |
| 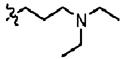 | 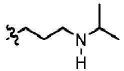 | 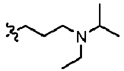 |
| 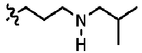 | 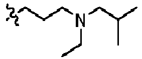 | 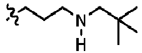 |
| 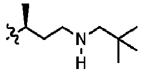 | 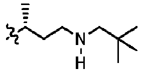 | 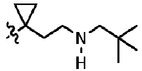 |
| 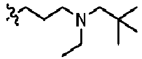 | 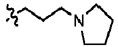 | 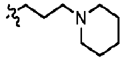 |
| 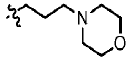 | 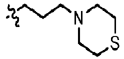 | 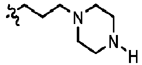 |
| 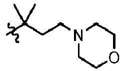 | 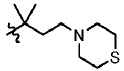 | 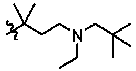 |
| 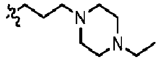 | 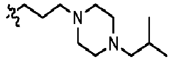 | 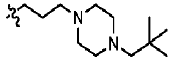 |
| 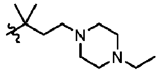 | 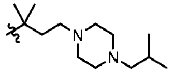 | 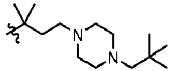 |
| 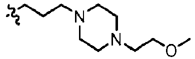 | 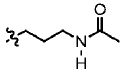 | 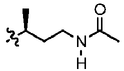 |
| 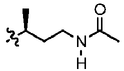 | 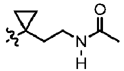 | 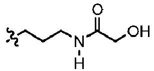 |
| 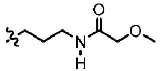 | 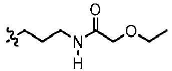 | 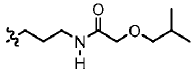 |
| 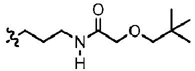 | 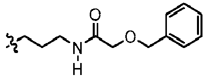 | 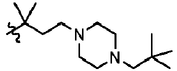 |
| 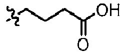 | 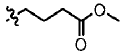 | 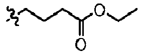 |
| 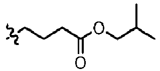 | 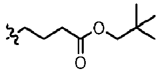 | 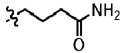 |
| 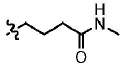 | 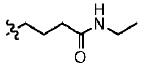 | 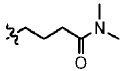 |
| 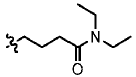 | 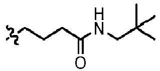 | 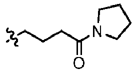 |
| 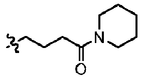 | 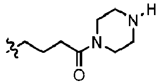 | 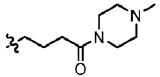 |
| 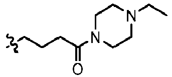 | 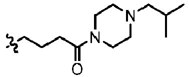 | 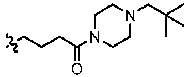 |
| 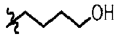 | 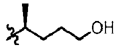 | 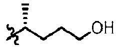 |
| 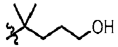 | 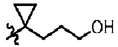 | 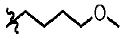 |
| 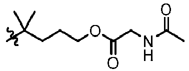 | 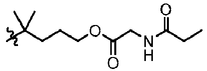 | 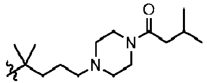 |
| 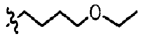 | 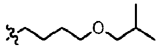 | 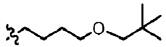 |
| 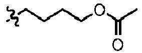 | 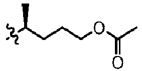 | 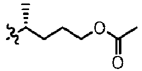 |
| 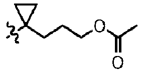 | 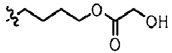 | 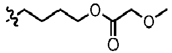 |
| 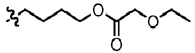 | 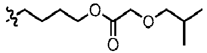 | 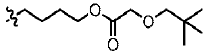 |
| 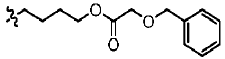 | 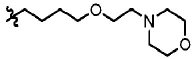 | 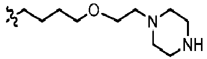 |
| 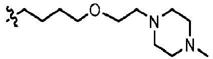 | 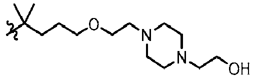 | 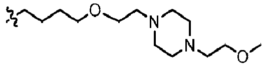 |
| 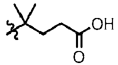 | 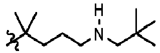 | 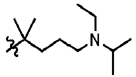 |
| 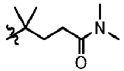 | 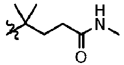 | 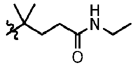 |
| 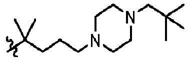 | 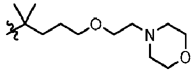 | 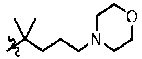 |
| 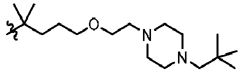 | 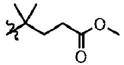 | 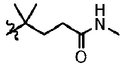 |
| 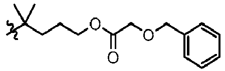 | 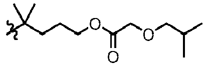 | 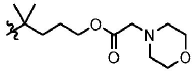 |
| 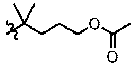 | 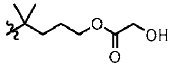 | 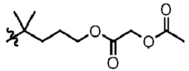 |
| 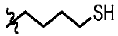 | 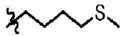 | 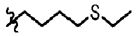 |
| 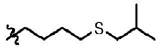 | 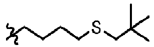 | 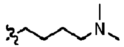 |
| 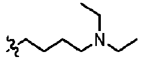 | 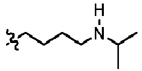 | 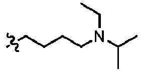 |
| 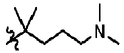 | 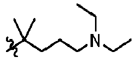 | 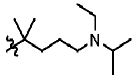 |
| 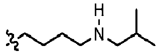 | 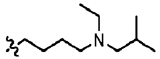 | 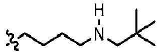 |
| 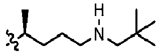 | 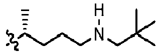 | 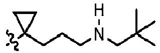 |
| 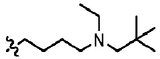 | 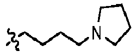 | 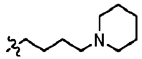 |
| 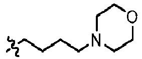 | 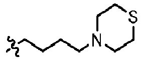 | 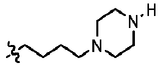 |
| 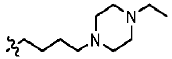 | 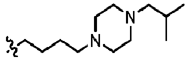 | 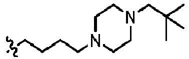 |
| 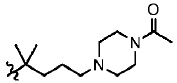 | 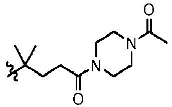 | 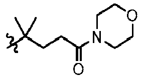 |
| 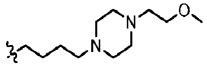 | 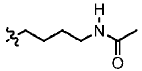 | 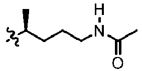 |
| 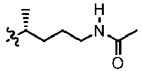 | 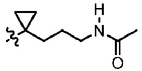 | 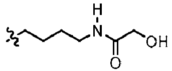 |
| 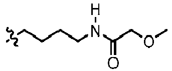 | 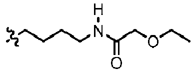 | 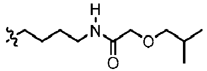 |
| 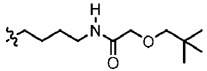 | 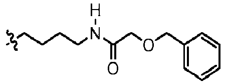 | 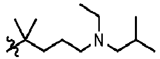 |
| 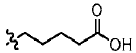 | 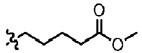 | 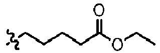 |
| 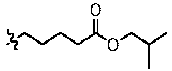 | 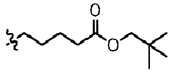 | 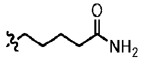 |
| 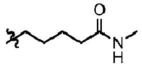 | 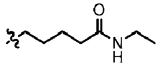 | 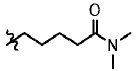 |
| 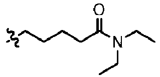 | 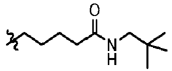 | 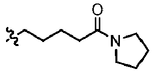 |
| 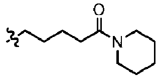 | 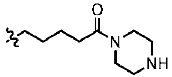 | 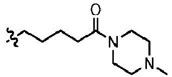 |
| 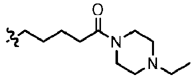 | 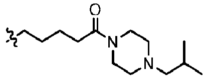 | 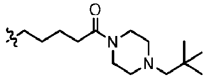 |
| 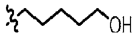 | 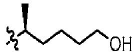 | 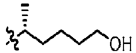 |
| 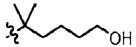 | 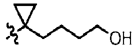 | 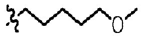 |
| 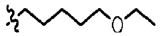 | 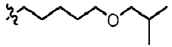 | 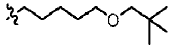 |
| 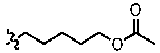 | 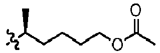 | 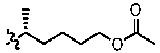 |
| 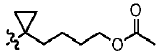 | 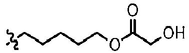 | 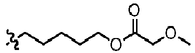 |
| 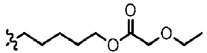 | 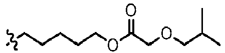 | 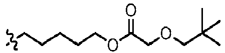 |
| 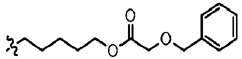 | 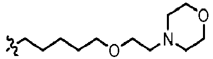 | 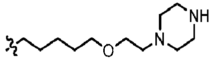 |
| 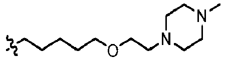 | 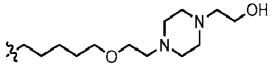 | 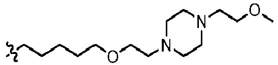 |
| 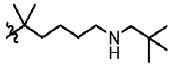 | 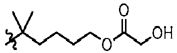 | 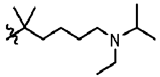 |
| 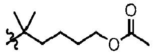 | 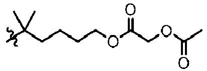 | 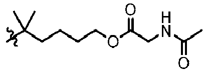 |
| 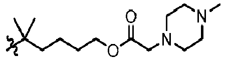 | 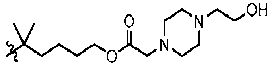 | 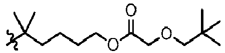 |
| 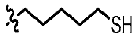 | 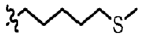 | 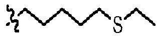 |
| 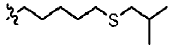 | 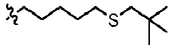 | 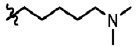 |
| 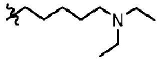 | 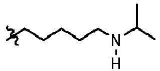 | 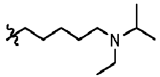 |
| 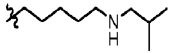 | 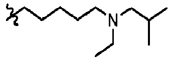 | 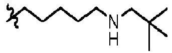 |
| 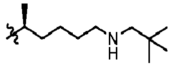 | 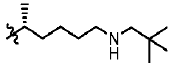 | 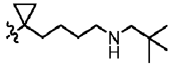 |
| 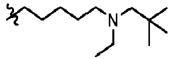 | 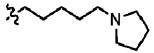 | 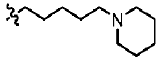 |
| 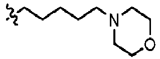 | 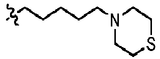 | 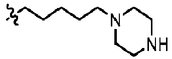 |
| 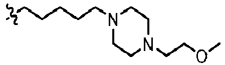 | 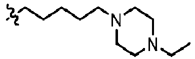 | 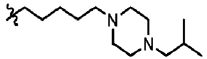 |
| 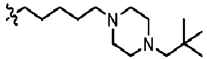 | 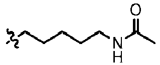 | 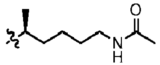 |
| 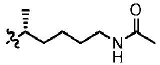 | 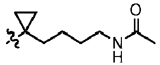 | 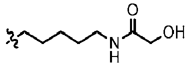 |
| 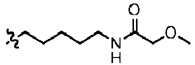 | 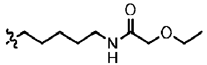 | 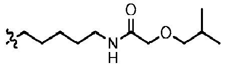 |
| 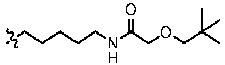 | 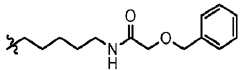 | 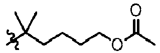 |
| 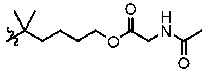 | 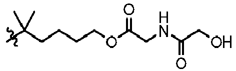 | 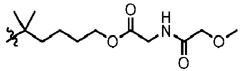 |
| 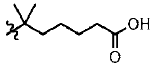 | 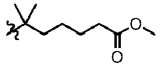 | 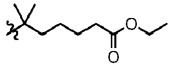 |
| 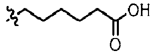 | 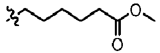 | 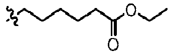 |
| 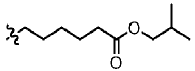 | 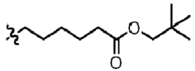 | 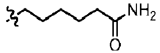 |
| 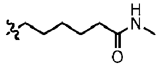 | 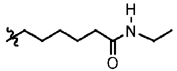 | 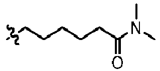 |
| 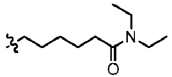 | 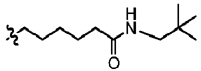 | 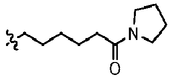 |
| 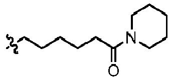 | 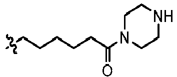 | 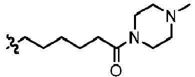 |
| 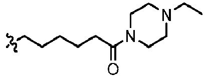 | 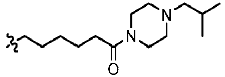 | 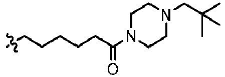 |
| 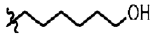 | 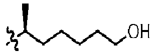 | 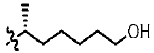 |
| 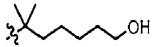 | 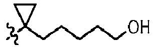 | 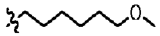 |
| 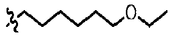 | 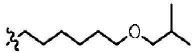 | 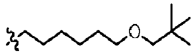 |
| 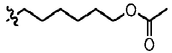 | 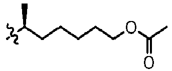 | 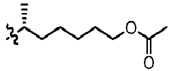 |
| 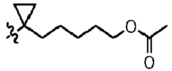 | 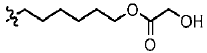 | 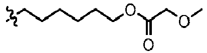 |
| 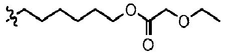 | 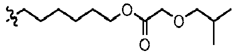 | 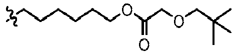 |
| 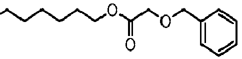 | 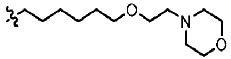 | 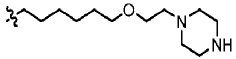 |
| 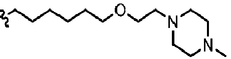 | 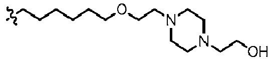 | 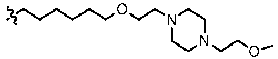 |
| 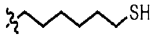 | 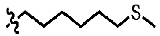 | 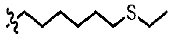 |
| 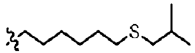 | 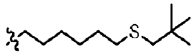 | 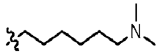 |
| 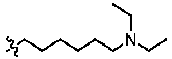 | 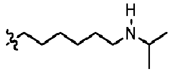 | 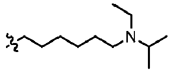 |
| 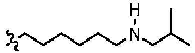 | 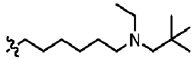 | 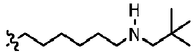 |
| 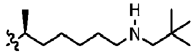 | 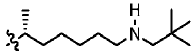 | 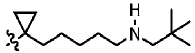 |
| 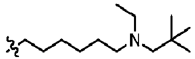 | 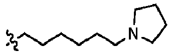 | 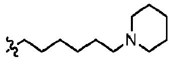 |
| 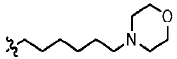 | 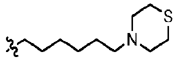 | 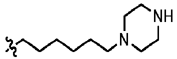 |
| 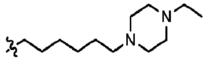 | 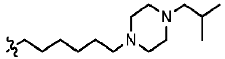 | 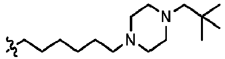 |
| 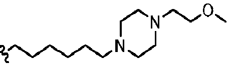 | 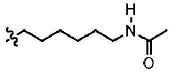 | 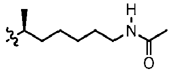 |
| 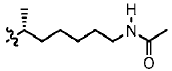 | 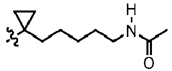 | 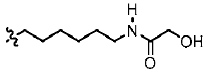 |
| 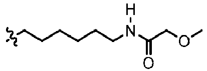 | 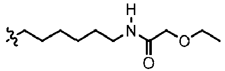 | 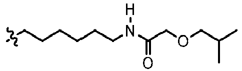 |
| 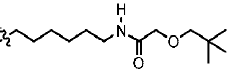 | 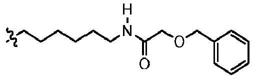 | 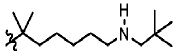 |
| 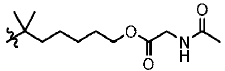 | 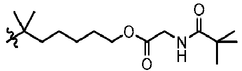 | 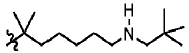 |

, or a pharmaceutically acceptable salt thereof.

In another aspect, a compound having the following formula is described, 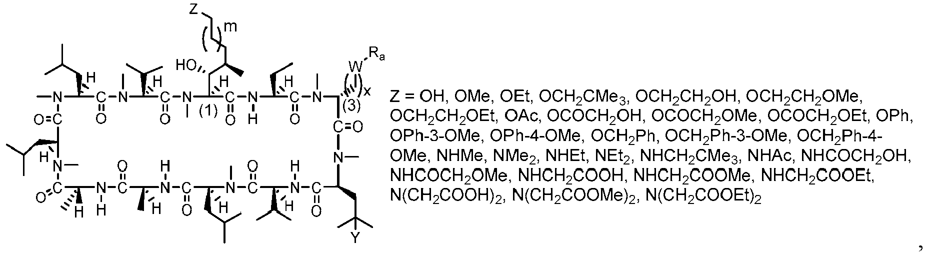 wherein x is 1; m is 2, 3, 4, 5, 6, 7, or 8; Y is H, OH or OMe; W is O or S; and each Rₐ is 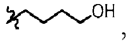 or a pharmaceutically acceptable salt thereof.

In another aspect, a compound having the following formula is described, 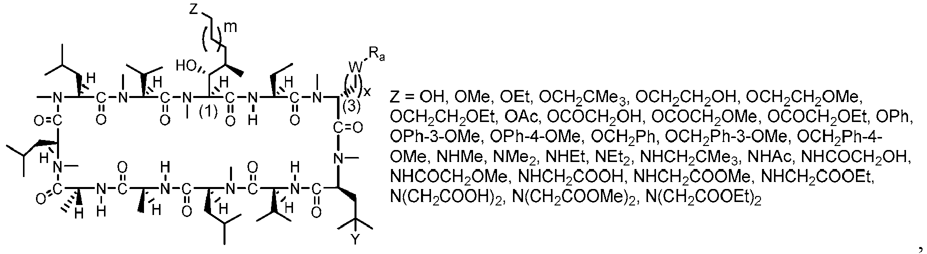 wherein x is 1; m is 2, 3, 4, 5, 6, 7, or 8; Y is H, OH or OMe; W is O or S; and each Rₐ is 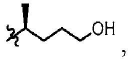 or a pharmaceutically acceptable salt thereof.

In another aspect, a compound having the following formula is described, 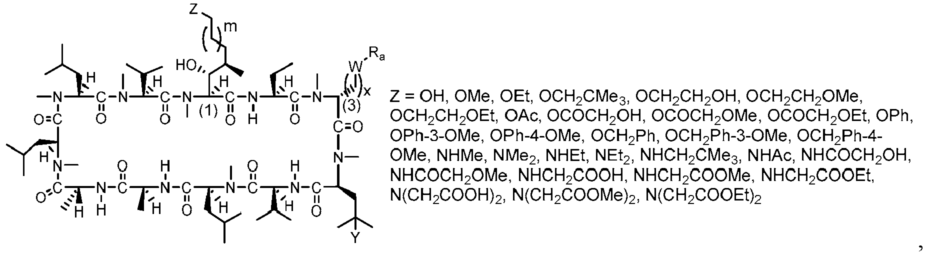 wherein x is 1; m is 2, 3, 4, 5, 6, 7, or 8; Y is H, OH or OMe; W is O or S; and each Rₐ is 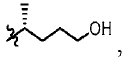 or a pharmaceutically acceptable salt thereof.

In another aspect, a compound having the following formula is described, 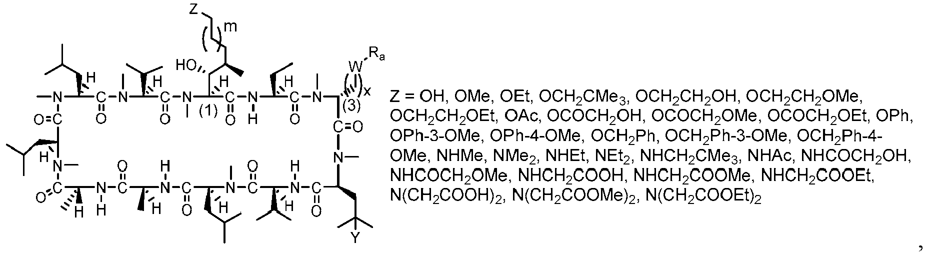 wherein x is 1; m is 2, 3, 4, 5, 6, 7, or 8; Y is H, OH or OMe; W is O or S; and each Rₐ is 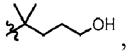 or a pharmaceutically acceptable salt thereof.

In another aspect, a compound having the following formula is described, 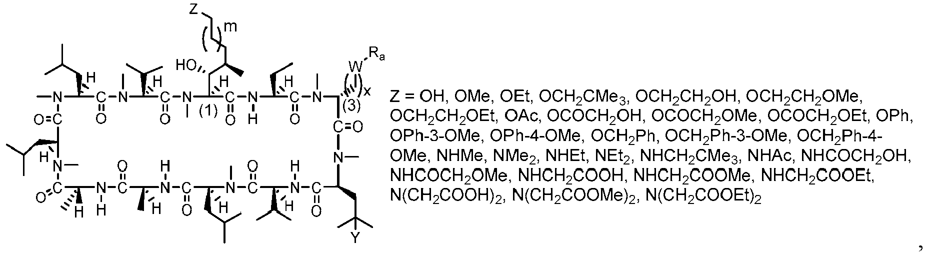 wherein x is 1; m is 2, 3, 4, 5, 6, 7, or 8; Y is H, OH or OMe; W is O or S; and each Rₐ is 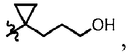 or a pharmaceutically acceptable salt thereof.

In another aspect, the present invention provides a pharmaceutical composition comprising at least one compound described herein and a pharmaceutically-acceptable carrier or diluent.

In a further aspect, the present invention provides a method for treating or preventing a viral infection in a mammalian species in need thereof, the method comprising administering to the mammalian species a therapeutically effective amount of at least one compound described herein. In certain embodiments, the viral infection is HIV infection. In certain other embodiments, the viral infection is HBV infection. In yet other embodiments, the viral infection is HCV infection. In yet other embodiments, the viral infection is influenza A virus infection, severe acute respiratory syndrome coronavirus infection or vaccinia virus infection.

In another aspect, the present invention provides a method for treating or preventing hepatitis C virus infection in a mammalian species in need thereof, the method comprising administering to the mammalian species a therapeutically effective amount of at least one compound described herein.

In yet another aspect, the present invention provides a method for inhibiting a cyclophilin in a subject in need thereof, which comprises administrating to said subject an effective cyclophilin-inhibiting amount of at least one compound as described herein.

In yet another aspect, the present invention provides a method for treating or preventing diseases that are mediated by cyclophilins in a mammalian species in need thereof, the method comprising administering to the mammalian species a therapeutically effective amount of at least one compound as described herein.

In yet another aspect, the present invention provides a method for treating or preventing diseases in a mammalian species in need thereof, the method comprising administering to the mammalian species a therapeutically effective amount of at least one compound as described herein, wherein the diseases are selected from inflammation, respiratory inflammation, rheumatoid arthritis, and dry eye.

In yet another aspect, the present invention provides a method for treating or preventing diseases in a mammalian species in need thereof, the method comprising administering to the mammalian species a therapeutically effective amount of at least one compound as described herein, wherein the diseases are selected from neurodegenerative diseases such as Alzheimer's disease, Parkinson's disease, Huntington's Diseases, and ALS; traumatic brain injury; stroke; and ischemia-reperfusion injury in the brain, heart, and kidney.

In yet another aspect, the present invention provides a method for treating or preventing diseases in a mammalian species in need thereof, the method comprising administering to the mammalian species a therapeutically effective amount of at least one compound as described herein, wherein the diseases are selected from cardiovascular diseases, vascular stenosis, atherosclerosis, abdominal aortic aneurysms, cardiac hypertrophy, aortic rupture, pulmonary arterial hypertension, myocarditis and myocardial fibrosis, and ischaemic heart diseases.

In yet another aspect, the present invention provides a method for treating or preventing diseases or conditions in a mammalian species in need thereof, the method comprising administering to the mammalian species a therapeutically effective amount of at least one compound as described herein, wherein the diseases or conditions are selected from cancer; obesity; diabetes; muscular dystrophy; lung, and liver, and kidney diseases, and their protection; and hair loss.

In yet another aspect, the present invention provides a method for treating or preventing diseases or conditions in a mammalian species in need thereof, the method comprising administering to the mammalian species a therapeutically effective amount of at least one compound as described herein, wherein the diseases or conditions are selected from allergic conjunctivitis, atopic and vernal keratoconjunctivitis, atopic keratoconjunctivitis, anterior uveitis, Behcet's disease, blepharitis, chronic ocular surface inflammation caused by viral infection, corneal transplant rejection, corneal sensitivity impaired due to surgery on the cornea or other surface of the eye, meibomian gland disease, ptyregia, ocular symptoms of graft versus host disease, ocular allergy, ocular cicatricial pemphigoid, Steven Johnson syndrome, vernal keratoconjunctivitis, uveitis, herpes simplex keratitis, ocular rosacea, and Pinguecula.

### Methods of Preparation

In certain embodiments, the compound of formulae (I) can be prepared by the modification of cyclosporine A at position 1 of MeBmt, a similar method was used as described by US Patent No. 9,200,038 B2 (which is incorporated herein by reference) and US Patent Application No. 2013/0190223 A1 (which is incorporated herein by reference), for the synthesis of the intermediate 3, 4, and 5. The cyclosporin A is treated with ClAc₂O and DMAP in Pyridine, subsequent cleavage the double bond by OsO₄ and NaIO₄ in dioxane give the corresponding aldehyde **2,** and through the wittig olefination reaction and NaBH₄ reduction the amine **4** are produced, and then coupling with AcOH give amide **5,** following its hydrolysis yield its free hydroxyl of amide **6.** The α-Methylene group on the Sarcosine at the position 3 of cyclosporine is introduced, by a similar method, described by WO2012/051194A1 (which is incorporated herein by reference) and US Patent Application No. 2013/0210704 A1 (which is incorporated herein by reference), to have its important intermediate **7,** when sulfur nucleophile is used for 1, 4-Michael Addition on the methylene group, the methylene sulfur side chain with S-conformation can be formed on the sarcosine of position 3 as novel cyclosporine derivatives. For example:

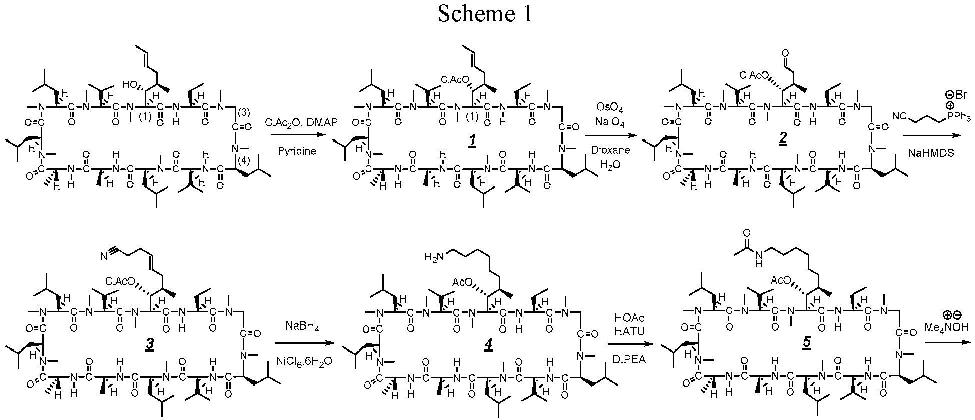

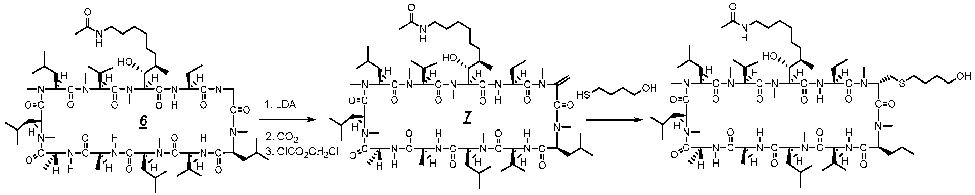

Other compounds disclosed herein may be prepared by using method similar to the method disclosed in Scheme 1, and/or method including any modification or variant thereof known to one of ordinary skill in the art.

Recently, we published our research on STG-175 (Gallay, P. A., et al, 2016, PLoS One 11(4):e0152036. doi: 10.1371/journal.pone.0152036, which is incorporated herein by reference). The side of CH₂-S-CH₂CH₂CH₂CH₂OH on positon 3 of Sarcosine of cyclosporine has been proved for high cyclophilin binding (EC₅₀ 0.6 nM for cyclophilin A) and with high anti-HCV activity (EC₅₀ 11.5-38.9 nM for multi-genotype HCV GTla to 4a). By comparison with Sofosbuvir, when its viral replication rebound, STG-175 still clears cells from HCV since no viral replication rebound is observed after cessation of drug treatment. Even at the drug dose is less than 2.5 times to Sofosbuvir. In addition, it presents a higher barrier to resistance than other cyclophilin inhibitors or direct anti-viral agents (DAAs), No cross-resistance is observed with DAAs. Therefore, by this discovery we would believe that could be possible to make even better derivatives by the modification position 1 of MeBmt and with the side of CH₂-S-CH₂CH₂CH₂CH₂OH on positon 3 of Sarcosine of cyclosporine as novel cyclosporine derivatives.

### Pharmaceutical Compositions

This invention also provides a pharmaceutical composition comprising at least one of the compounds as described herein or a pharmaceutically-acceptable salt or solvate thereof, and a pharmaceutically-acceptable carrier.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as butylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

As set out above, certain embodiments of the present pharmaceutical agents may be provided in the form of pharmaceutically-acceptable salts. The term "pharmaceutically-acceptable salt", in this respect, refers to the relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al., (1977) "Pharmaceutical Salts", J. Pharm. Sci. 66:1-19).

The pharmaceutically acceptable salts of the subject compounds include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, butionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge *et al.,* supra)

Wetting agents, emulsifiers and lubricants, such as sodium lauryl sulfate, magnesium stearate, and polyethylene oxide-polybutylene oxide copolymer as well as coloring agents, release agents, coating agents, sweetening, flavoring and perfuming agents, preservatives and antioxidants can also be present in the compositions.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient, which can be combined with a carrier material to produce a single dosage form, will vary depending upon the host being treated and the particular mode of administration. The amount of active ingredient, which can be combined with a carrier material to produce a single dosage form will generally be that amount of the compound which produces a therapeutic effect. Generally, out of 100%, this amount will range from about 1% to about 99% of active ingredient, preferably from about 5% to about 70%, most preferably from about 10% to about 30%.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethyl cellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, sodium carbonate, and sodium starch glycolate; solution retarding agents, such as paraffin; absorption accelerators, such as quaternary ammonium compounds; wetting agents, such as, for example, cetyl alcohol, glycerol monostearate, and polyethylene oxide-polybutylene oxide copolymer; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxybutylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxybutylmethyl cellulose in varying butortions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples are embedding compositions, which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isobutyl alcohol, ethyl carbonate, ethyl acetate, benzyl alcohol, benzyl benzoate, butylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Additionally, cyclodextrins, e.g., hydroxybutyl-.beta.-cyclodextrin, may be used to solubilize compounds.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active pharmaceutical agents of the invention.

Formulations of the present invention which are suitable for vaginal administration also include pessaries, tampons, creams, gels, pastes, foams or spray formulations containing such carriers as are known in the art to be apbutriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or butellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary butellants, such as chlorofluorohydrocarbons and volatile unsubstituted hydrocarbons, such as butane and butane.

Transdermal patches have the added advantage of providing controlled delivery of a compound of the present invention to the body. Such dosage forms can be made by dissolving, or dispersing the pharmaceutical agents in the buffer medium. Absorption enhancers can also be used to increase the flux of the pharmaceutical agents of the invention across the skin. The rate of such flux can be controlled, by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle. One strategy for depot injections includes the use of polyethylene oxide-polybutylene oxide copolymers wherein the vehicle is fluid at room temperature and solidifies at body temperature.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly (anhydrides). Depot injectable formulations are also prepared by entrapping the drug in liposomes or microemulsions, which are compatible with body tissue.

When the compounds of the present invention are administered as pharmaceuticals, to humans and animals, they can be given per se or as a pharmaceutical composition containing, for example, 0.1% to 99.5% (more preferably, 0.5% to 90%) of active ingredient in combination with a pharmaceutically acceptable carrier.

The compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, the compound of the present invention may be administered concurrently with another anti-HCV agent), or they may achieve different effects (e.g., control of any adverse effects).

The compounds of the invention may be administered intravenously, intramuscularly, intraperitoneally, subcutaneously, topically, orally, or by other acceptable means. The compounds may be used to treat arthritic conditions in mammals (i.e., humans, livestock, and domestic animals), birds, lizards, and any other organism, which can tolerate the compounds.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### Equivalents

The representative examples which follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art. The following examples contain important additional information, exemplification and guidance which can be adapted to the practice of this invention in its various embodiments and equivalents thereof.

### Examples

### Example 1

### [α-Methylene-Sar]-3-cyclosporin

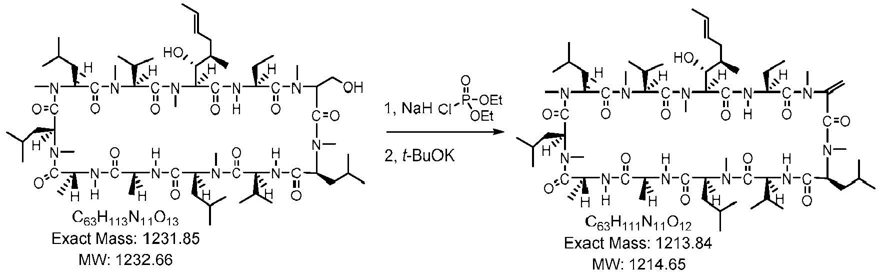

To a solution of [α-hydroxymethyl-Sar]-3-cyclosporin (246 mg, 0.20 mmol) in tetrahydrofuran (15 ml) were added sodium hydride (120 mg 60% in mineral oil, 3 mmol) and diethyl chlorophosphate (412 mg, 2.40 mmol). The resulting mixture was stirred at room temperature overnight. After the starting material disappeared, methanol (10 ml) and potassium tert-butoxide (33 mg, 26.00 mmol) were added. The mixture was stirred at room temperature for three hours and the solvents were removed under reduced pressure. The residue was dissolved in dichloromethane (50 ml). The dichloromethane layer was washed with brine, dried over magnesium sulfate and evaporated under reduced pressure. The residue was purified by flash chromatography using dichloromethane/methanol as eluent to give 205 mg of product [Molecular formula: C₆₃H₁₁₁N₁₁O₁₂; Exact Mass: 1213.84; MS (m/z): 1214.59 (M+1)⁺; HPLC RT: 17.47 min. (C8 reverse phase column: 250mm; acetonitrile/water (0.05% trifluoroacetic acid), operation temperature: 64 °C; Detector: 210 nm].

### Examples 2

### [(3R,4R)-3-Hydroxy-4-methyl-6-(4-methoxycarbonylbenzyl)-N-MeNle]-1-[(S)-(4-hydroxybutylthio)methyl-Sar]-3-cyclosporin

### [3-Acetyt-MeBmt]-1-cyctosporin

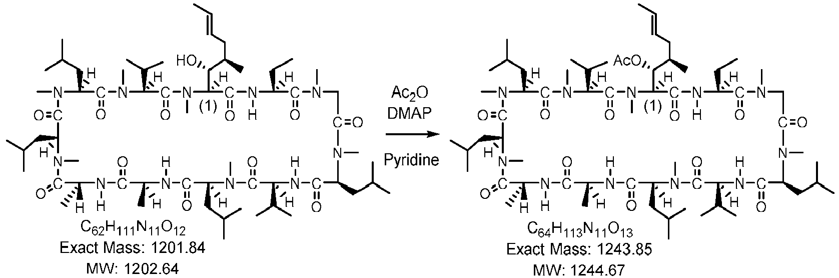

To a dried flask under nitrogen were added cyclosporine (12.00 g, 9.98 mmol), N,N-dimethylaminopyridine (0.12 g, 0.10 mmol), anhydrous pyridine (120 ml) and acetic anhydride (54 ml, 0.54 mol). After stirred at room temperature overnight, the mixture was poured into ice-water (600 ml) and stirred until the ice was melted. Ethyl acetate (100 ml) was added and the mixture was separated. The ethyl acetate layer was washed with 1.0 N hydrochloric acid solution (100 ml x 2), saturated sodium bicarbonate solution (100 ml), brine (100 ml), dried over magnesium sulfate and evaporated under reduced pressure. The residue was purified by chromatography (hexane/acetone) to give 11.40 g of pure [3-acetyl-MeBmt]-1-cyclosporin [Molecular Formula: C₆₄H₁₁₃N₁₁O₁₃; Exact Mass: 1243.85; MS (m/z): 1244.53 (M+1)⁺, 1266.70 (M+Na)⁺; HPLC RT: 18.02 min. (C8 reverse phase column: 250 mm; acetonitrile/water (0.05% trifluoroacetic acid); operation temperature: 64 °C; detector: 210 nm)].

### [(3R,4R)-3-Acetyloxy-4-methyl-6-oxo-N-MeNle]-1-cyclosporin

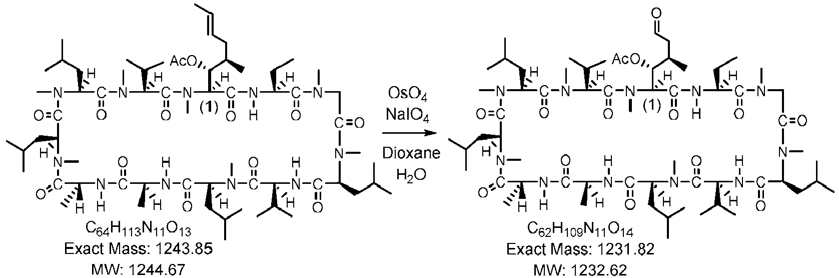

To a solution of [3-acetyl-MeBmt]-1-cyclosporin (10.00 g, 8.04 mmol) in dioxane (200 ml) were added water (20 ml), osmium(VIII) oxide solution (15.74 mM, 51 ml, 0.80 mmol) and sodium metaperiodate (6.88 g, 32.16 mmol). The reaction mixture was stirred at room temperature for five hours. Ethyl acetate (100 ml) and brine (100 ml) were added and the mixture was separated. The organic layer was washed with saturated sodium bicarbonate solution, brine, dried over magnesium sulfate and evaporated under reduced pressure. The residue was purified by chromatography (hexane/acetone) to give 5.10 g of pure [(3R, 4R)-3-acetyloxy-4-methyl-6-oxoN-MeNle]-1-cyclosporin [Molecular Formula: C₆zH₁₀₉N₁₁O₁₄; Exact Mass: 1231.82; MS (m/z): 1232.75 (M+1)⁺, 1254.77 (M+Na)⁺; HPLC RT: 15.74 min. (C8 reverse phase column: 250 mm; acetonitrile/water (0.05% trifluoroacetic acid); operation temperature: 64 °C; detector: 210 nm)].

### [(3R, 4R)-3-Acetyloxy-4-methyl-6-(4-methoxycarbonylphenyl)methylene-N-MeNle]-1-cyclosporin

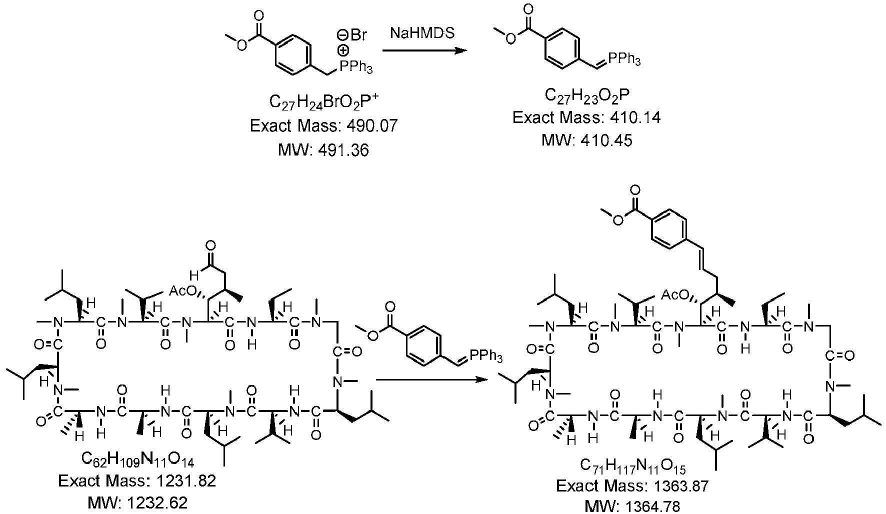

To a solution of (4-methoxycarbonylbenzyl)triphenylphosphonium bromide (4.00 g, 8.14 mmol) in anhydrous tetrahydrofuran (120 ml) under nitrogen were added sodium bis(trimethylsilyl)amide (1.0 M in THF, 10 ml, 10.00 mmol). The reaction mixture was stirred at room temperature for one hour and cooled to -40 °C. A solution of [(3R,4R)-3-acetyloxy-4-methyl-6-oxo-N-MeNle]-1-cyclosporin (5.00 g, 4.05 mmol) in anhydrous tetrahydrofuran (25 ml) was added. The mixture was stirred for another two hours at -30 °C and saturated ammonium chloride solution (20 ml) was added to quench the reaction. Most of tetrahydrofuran was evaporated under reduced pressure. Ethyl acetate (150 ml) and brine (50 ml) were added and the mixture was separated. The organic layer was dried over magnesium sulfate and evaporated under reduced pressure. The residue was purified by chromatography (dichloromethane/methanol) to give 2.00 g of pure [(3R,4R)-3-acetyloxy-4-methyl-6-(4-methoxycarbonylphenyl)methylene-N-MeNle]-1-cyclosporin [Molecular Formula: C₇₁H₁₁₇N₁₁O₁₅; Exact Mass: 1363.87; MS (m/z): 1364.61 (M+1)⁺, HPLC RT: 17.89 min. (C8 reverse phase column: 250 mm; acetonitrile/water (0.05% trifluoroacetic acid); operation temperature: 64 °C; detector: 210 nm)].

### [(3R, 4R)-3-Acetyloxy-4-methyl-6-(4-methoxycarbonylbenzyl)-N-MeNle]-1-cyclosporin

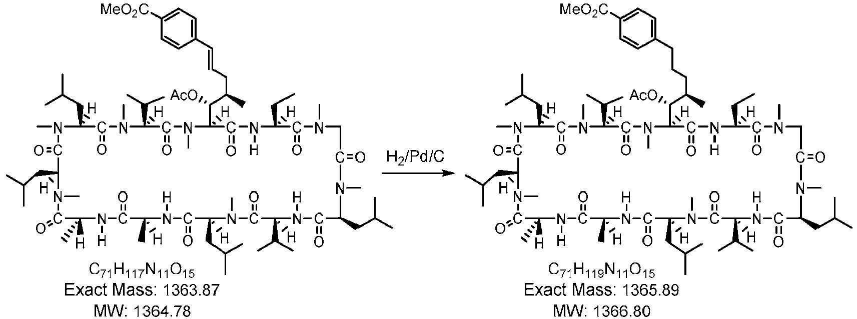

To a solution of [(3R,4R)-3-acetyloxy-4-methyl-6-(4-methoxycarbonylphenyl)methylene-N-MeNle]-1-cyclosporin (2.00 g, 1.46 mmol) in methanol (50 ml), palladium (10 wt% on carbon, 20 mg) and acetic acid (5 drops) were added. The mixture was stirred at room temperature under hydrogen for two hours. The mixture was filtered and the filtrate was evaporated under reduced pressure to give crude [(3R,4R)-3-acetyloxy-4-methyl-6-(4-methoxycarbonybenzyl)-N-MeNle]-1-cyclosporin [Molecular Formula: C₇₁H₁₁₉N₁₁O₁₅; Exact Mass: 1365.89; MS (m/z): 1366.73 (M+1)⁺; HPLC RT: 18.02 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(4-carboxybenzyl)-N-MeNle]-1-cyclosporin

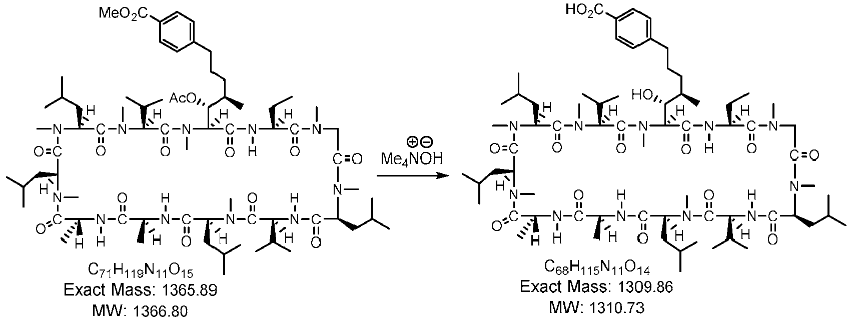

[(3R,4R)-3-Acetyloxy-4-methyl-6-(4-methoxycarbonylbenzyl)-N-MeNle]-1-cyclosporin (0.25 g, 0.18 mmol) was dissolved in methanol (4 ml). Water (2 ml) and tetramethylammonium hydroxide pentahydrate (70 mg) were added. The mixture was stirred at room temperature overnight. Then most of the methanol was evaporated. The PH of mixture was adjusted to 5 with acetic acid. Ethyl acetate (50 ml) and brine (10 ml) were added and the mixture was separated. The organic layer was dried over magnesium sulfate and evaporated under reduced pressure to give crude product [(3R, 4R)-3-hydroxy-4-methyl-6-(4-carboxybenzyl)-N-MeNle]-1-cyclosporin [Molecular Formula: C₆₈H₁₁₅N₁₁O₁₄; Exact Mass: 1309.86; MS (m/z): 1310.61 (M+1)⁺; HPLC RT: 14.36 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(4-carboxybenzyl)-N-MeNle]-1-[α-methylene-Sar]-3-cyclosporin

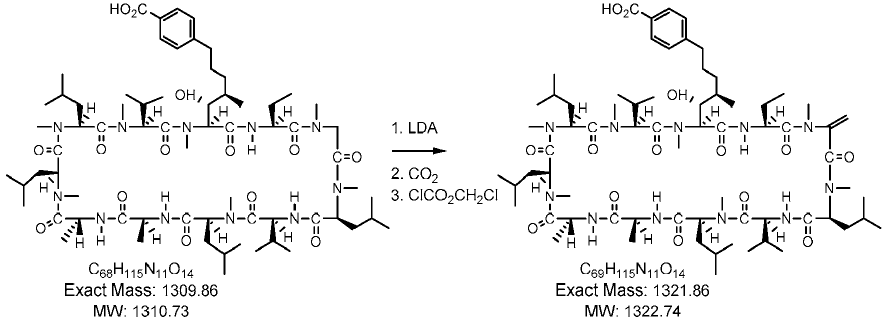

n-Butyllithium (2.65 M, 7.7 ml, 20.41 mmol) was added to a solution of diisopropylamine (2.05 g, 20.30 mmol) in tetrahydrofuran (50 ml) at -78 °C under nitrogen. After the reaction mixture was stirred for one hour, a solution of [(3R,4R)-3-hydroxy-4-methyl-6-(4-carboxybenzyl)-N-MeNle]-1-cyclosporin (2.40 g, 1.20 mmol) in tetrahydrofuran (10 ml) was added. The mixture was stirred at -78 °C for three hours. After carbon dioxide gas was bubbled into the reaction mixture for 15 minutes, the mixture was stirred at -78 °C for another hour. Then the cooling bath was removed and the reaction mixture was allowed to warm up to room temperature to let unreacted carbon dioxide come out. The mixture was cooled to -78 °C again and chloromethyl chloroformate (3.00 ml) was added. The mixture was stirred and allowed to warm to room temperature overnight. Brine (5 ml) was added to quench the reaction. Most of tetrahydrofuran was removed under reduced pressure. Ethyl acetate (80 ml) and brine (50 ml) were added and the mixture was separated. The organic layer was dried over magnesium sulfate and evaporated under reduced pressure to give 1.50 g of crude product [(3R,4R)-3-hydroxy-4-methyl-6-(4-carboxybenzyl)-N-MeNle]-1-[α-methylene-Sar]-3-cyclosporin [Molecular Formula: C₆₉H₁₁₅N₁₁O₁₄; Exact Mass: 1321.86; MS (m/z): 1322.55 (M+1)⁺; HPLC RT: 16.13 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(4-methoxycarbonylbenzyl)-N-MeNle]-1-[α-methylene-Sar]-3-cyclosporin

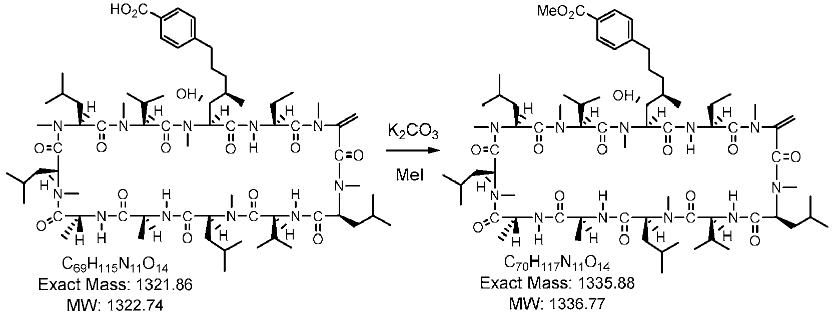

To a solution of [(3R,4R)-3-hydroxy-4-methyl-6-(4-carboxybenzyl)-N-MeNle]-1-[α-methylene-Sar]-3-cyclosporin(1.32 g, 1.00 mmol) in N,N-dimethylformamide (15 ml) were added potassium carbonate (0.38 g, 2.75 mmol) and iodomethane (0.50 g, 3.52 mmol). The mixture was stirred at room temperature for three hours. Ethyl acetate (100 ml) and water (50 ml) were added and the mixture was separated. The organic layer was dried over magnesium sulfate and evaporated under reduced pressure. The residue was purified by chromatography (dichloromethane/methanol) to give 1.60 g of pure [(3R,4R)-3-hydroxy-4-methyl-6-(4-methoxycarbonylbenzyl)-N-MeNle]-1-[α-methylene-Sar]-3-cyclosporin [Molecular Formula: C₇₀H₁₁₇N₁₁O₁₄; Exact Mass: 1335.88; MS (m/z): 1336.64 (M+1)⁺; HPLC RT: 18.03 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(4-methoxycarbonylbenzyl)-N-MeNle]-1-[(S)-(4-hydroxybutylthio)methyl-Sar]-3-cyclosporin

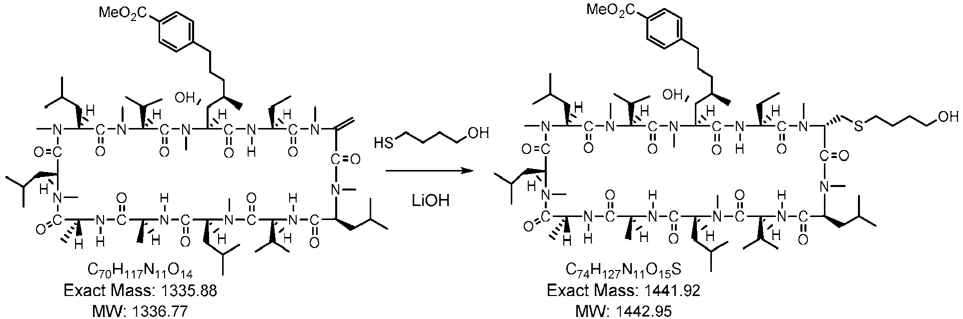

To a solution of [(3R, 4R)-3-hydroxy-4-methyl-6-(4-methoxycarbonylbenzyl)-N-MeNle]-1-[a-methylene-Sar]-3-cyclosporin (0.60 g, 0.45 mmol) in methanol (25 ml) were added 4-mercapto-1-butanol (0.45 g, 4.25 mmol) and lithium hydroxide (0.10 g, 4.25 mmol). The reaction mixture was stirred at room temperature overnight. Most of the methanol was evaporated under reduced pressure. Ethyl acetate (60 ml) and brine (30 ml) were added and the mixture was separated. The organic layer was dried over magnesium sulfate and evaporated under reduced pressure. The residue was purified by chromatography (dichloromethane/methanol) to give pure [(3R,4R)-3-hydroxy-4-methyl-6-(4-methoxycarbonylbenzyl)-N-MeNle]-1-[(S)-(4-hydroxybutylthio)methyl-Sar]-3-cyclosporin [Molecular Formula: C₇₄H₁₂₇N₁₁O₁₅S; Exact Mass: 1441.92; MS (m/z): 1422.65 (M+1)⁺; HPLC RT: 16.79 min. (C8 reverse phase column: 250 mm; acetonitrile/water (0.05% trifluoroacetic acid); operation temperature: 64 °C; detector: 210 nm)].

### Examples 3

### [(3R,4R)-3-Hydroxy-4-methyl-6-(4-carboxybenzyl)-N-MeNle]-1-[(S)-(4-hydroxybutylthio)methyl-Sar]-3-cyclosporin

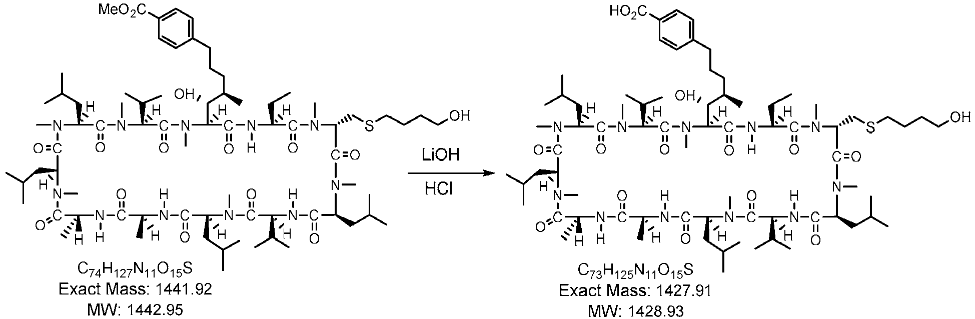

To a solution of [(3R,4R)-3-hydroxy-4-methyl-6-(4-methoxycarbonylbenzyl)-N-MeNle]-1-[(S)-(4-hydroxybutylthio)methyl-Sar]-3-cyclosporin (85 mg, 0.06 mmol) in methanol (5 ml) was added a solution of lithium hydroxide (15 mg) in water (5 ml). The reaction mixture was stirred at room temperature overnight. Most of the methanol was evaporated under reduced pressure. The PH of the mixture was adjusted to 6 with 1.0 N hydrochloric acid. Ethyl acetate (60 ml) and brine (10 ml) were added and the mixture was separated. The organic layer was dried over magnesium sulfate and evaporated under reduced pressure. The residue was purified to give pure [(3R,4R)-3-hydroxy-4-methyl-6-(4-carboxybenzyl)-N-MeNle]-1-[(S)-(4-hydroxybutylthio) methyl-Sar]-3-cyclosporin [Molecular Formula: C₇₃H₁₂₅N₁₁O₁₅S; Exact Mass: 1427.91; MS (m/z): 1428.63 (M+1)⁺; HPLC RT: 14.37 min. (C8 reverse phase column: 250 mm; acetonitrile/water (0.05% trifluoroacetic acid); operation temperature: 64 °C; detector: 210 nm)].

### Example 4

### [(3R,4R)-3-Hydroxy-4-methyl-6-(3-amino)benzyl-N-MeNle]-1-[(S)-(4-hydroxybutylthio)methyl-Sar]-3-cyclosporin

### (3-Nitrobenzyl)triphenylphosphonium bromide

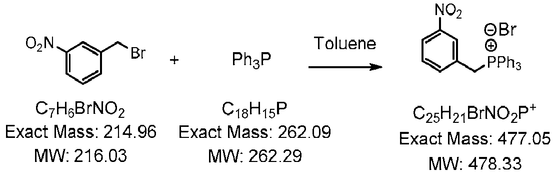

3-Nitrobenzyl bromide (5.00 g, 23.15 mmol) and triphenylphosphine (6.06 g, 23.15 mmol) were added to toluene (60 ml). The mixture was stirred and heated to reflux overnight. After cooled to room temperature, the precipitate was filtered off, washed with toluene and hexane and dried in vacuum to give 10.50 g product [Molecular Formula: C₂₅H₂₁BrNO₂P; Exact Mass: 477.05; MS (m/z): 398.45 (M+1)⁺-Br].

### [(3R,4R)-3-Acetyloxy-4-methyl-6-(3-nitro-phenylmethylene)-N-MeNle]-1-cyclosporin

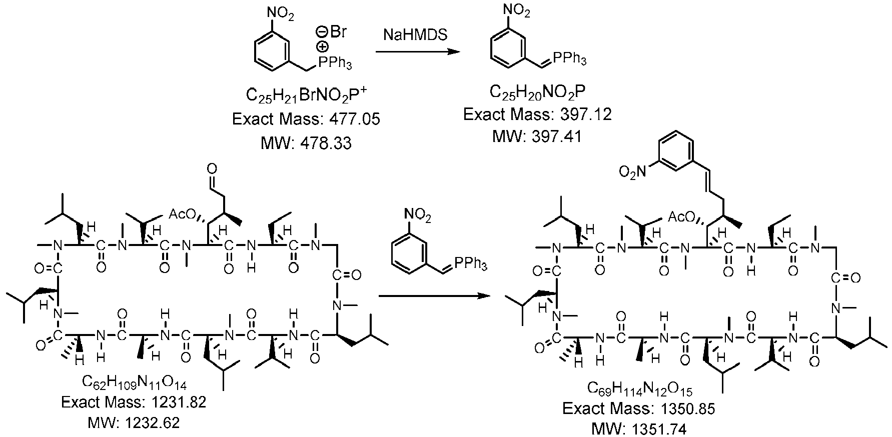

To a solution of (3-nitrobenzyl)triphenylphosphonium bromide (2.40 g, 5.03 mmol) in anhydrous tetrahydrofuran (90 ml) under nitrogen was added sodium bis(trimethylsilyl)amide (1.0 M in THF, 6 ml, 6.00 mmol). The reaction mixture was stirred at room temperature for one hour and cooled to -40 °C. A solution of [(3R, 4R)-3-aceyloxy-4-methyl-6-oxo-N-MeNle]-1-cyclosporin (3.69 g, 3.00 mmol) in anhydrous tetrahydrofuran (25 ml) was added. The mixture was stirred another two hours at -30 °C. Then saturated ammonium chloride solution (20 ml) was added to quench the reaction. Most of tetrahydrofuran was evaporated under reduced pressure. Ethyl acetate (150 ml) and brine (50 ml) were added and the mixture was separated. The organic layer was dried over magnesium sulfate and evaporated under reduced pressure. The residue was purified by chromatography (dichloromethane/methanol) to give 1.60 g of pure [(3R,4R)-3-aceyloxy-4-methyl-6-(3-nitro-phenylmethylene)N-MeNle]-1-cyclosporin [Molecular Formula: C₆₉H₁₁₄N₁₂O₁₅; Exact Mass: 1350.85; MS (m/z): 1351.52 (M+1)⁺].

### [(3R, 4R)-3-Acetyloxy-4-methyl-6-(3-amino)benzyl-N-MeNle]-1-cyclosporin

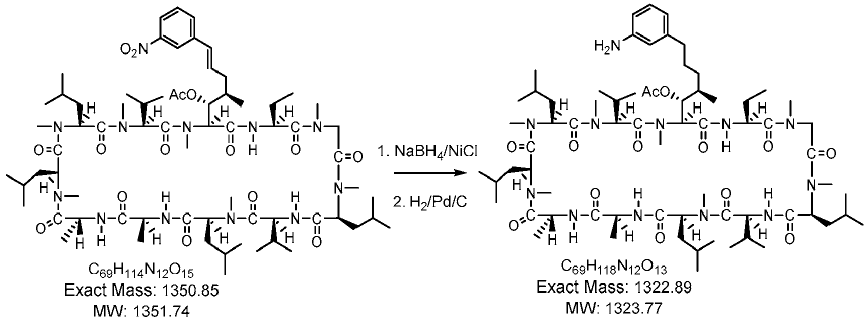

To a solution of [(3R,4R)-3-acetyloxy-4-methyl-6-(3-nitro-phenylmethylene)-N-MeNle]-1-cyclosporin (3.00 g, 2.22 mmol) in methanol (50 ml) under nitrogen was added nickel (II) chloride hexahydrate (0.19 g, 0.81 mmol). The reaction mixture was put into ice-water bath. Sodium borohydride (1.80 g, 47.33 mmol) was added in four batches in two hours. After the mixture was stirred for another two hours at 0°C, water (10 ml) was added. Most of the methanol was evaporated under reduced pressure. Ethyl acetate (50 ml) and saturated sodium bicarbonate solution (50 ml) were added and the mixture was separated. The organic layer was washed with brine, dried over magnesium sulfate and evaporated under reduced pressure. The residue was dissolved in methanol (30 ml). Palladium (10 wt% on carbon, 20 mg) and acetic acid (5 drops) were added. The mixture was stirred at room temperature under hydrogen for two hours. The mixture was filtered and the filtrate was evaporated under reduced pressure to give crude [(3R,4R)-3-acetyloxy-4-methyl-6-(3-amino)benzyl-N-MeNle]-1-cyclosporin [Molecular Formula: C₆₉H₁₁₈N₁₂O₁₃; Exact Mass: 1322.89; MS (m/z): 1323.73 (M+1)⁺].

### [(3R, 4R)-3-Hydroxy-4-methyl-6-(3-amino)benzyl-N-MeNle]-1-cyclosporin

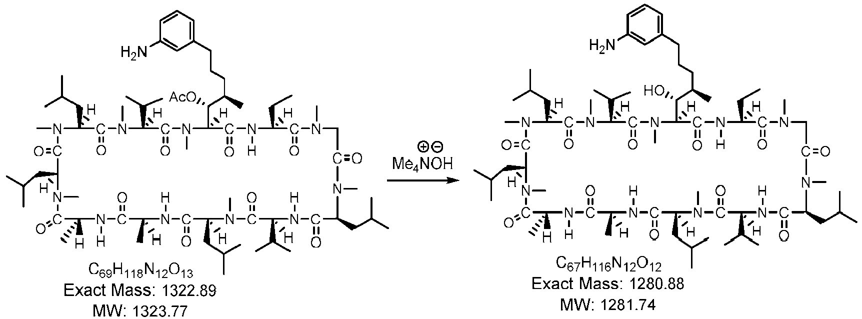

[(3R,4R)-3-Acetyloxy-4-methyl-6-(3-amino)benzyl-N-MeNle]-l-cyclosporin (2.60 g, 1.56 mmol) was dissolved in methanol (70 ml). Water (35 ml) and tetramethylammonium hydroxide pentahydrate (2.00 g, 11.01 mmol) were added. The mixture was stirred at room temperature for eight hours. Then most of the methanol was evaporated. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 1.90 g of pure [(3R,4R)-3-hydroxy-4-methyl-6-(3-aminophenyl)methyl-N-MeNle]-1-cyclosporin [Molecular Formula: C₆₇H₁₁₆N₁₂O₁₂; Exact Mass: 1280.88; MS (m/z): 1281.64 (M+1)⁺].

### [(3R, 4R)-3-Hydroxy-4-methyl-6-(3-(tert-butoxycarbonyl)amino)benzyl-N-MeNle]-1-cyclosporin

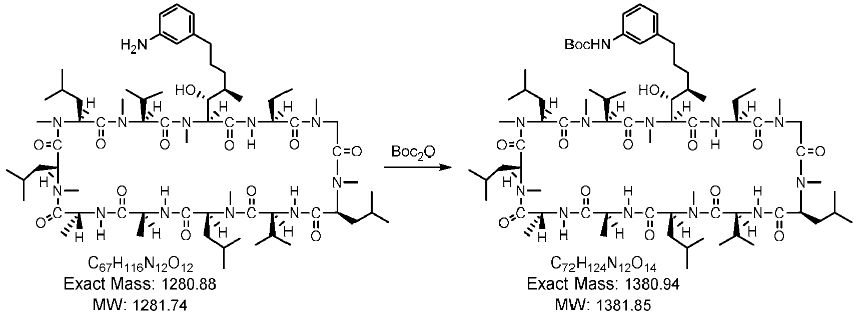

[(3R,4R)-3-Hydroxy-4-methyl-6-(3-amino)benzyl-N-MeNle]-1-cyclosporin(0.80 g, 0.62 mmol) was dissolved in tetrahydrofuran (30 ml). Di-tert-butyldicarbonate (0.37 g, 1.70 mmol) was added. The mixture was stirred at room temperature for two days. Most of tetrahydrofuran was evaporated under reduced pressure. Ethyl acetate (30 ml) and brine (30 ml) were added and the mixture was separated. The organic layer was dried over magnesium sulfate and evaporated under reduced pressure. The residue was purified by chromatography to give 750 mg of pure [(3R,4R)-3-hydroxy-4-methyl-6-(3-(tert-butoxycarbonyl)amino)benzyl-N-MeNle]-1-cyclosporin [Molecular Formula: C₇₂H₁₂₄N₁₂O₁₄; Exact Mass: 1380.94; MS (m/z): 1381.65 (M+1)⁺].

### [(3R, 4R)-3-Hydroxy-4-methyl-6-(3-(tert-butoxycarbonyl)amino)benzyl-N-MeNle]-1-[α-methylene-Sar]-3-cyclosporin

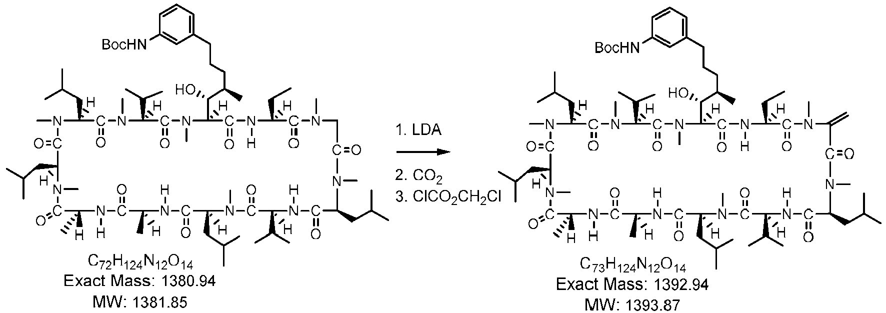

n-Butyllithium (2.6.5M, 2.70 ml, 7.16 mmol) was added to a solution of diisopropylamine (0.71 g, 7.15 mmol) in tetrahydrofuran (50 ml) at -78 °C under nitrogen. After the reaction mixture was stirred for an hour, a solution of [(3R,4R)-3-hydroxy-4-methyl-6-(3-(tert-butoxycarbonyl)amino)benzyl-N-MeNle]-1-cyclosporin (0.90 g, 0.65 mmol) in tetrahydrofuran (2 ml) was added. The mixture was stirred at -78 °C for three hours. After carbon dioxide gas was bubbled into the reaction mixture for 15 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (1.20 ml) was added. The mixture was stirred and allowed to warm to room temperature overnight. Brine (5 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.45 g of pure [(3R,4R)-3-hydroxy-4-methyl-6-(3-(tert-butoxycarbonyl)amino)benzyl-N-MeNle]-1-[α-methylene-Sar]-3-cyclosporin [Molecular Formula: C₇₃H₁₂₄N₁₂O₁₄; Exact Mass: 1392.94; MS (m/z): 1393.88 (M+1)⁺].

### [(3R,4R)-3-Hydroxy-4-methyl-6-(3-amino)benzyl-N-MeNle]-1-[α-methylene-Sar]-3-cyclosporin

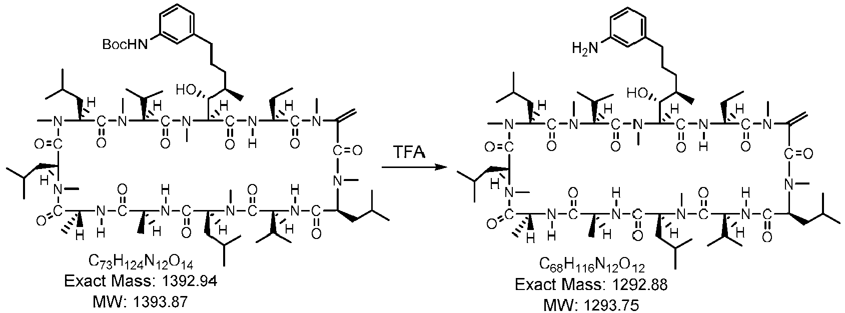

[(3R,4R)-3-Hydroxy-4-methyl-6-(3-(tert-butoxycarbonyl)amino)benzyl-N-MeNle]-1-[α-methylene-Sar]-3-cyclosporin (0.80 g, 0.57 mmol) was dissolved in dichloromethane (15 ml) and put into ice-water bath. Trifluoroacetic acid (7 ml) was added. The mixture was stirred at room temperature for one hour. Another dichloromethane (50 ml) was added. The mixture was washed with brine (30 ml) and saturated sodium bicarbonate solution (30 ml), dried over magnesium sulfate and evaporated under reduced pressure. The residue was purified by chromatography (dichloromethane/methanol) to give 0.20 g of pure [(3R,4R)-3-hydroxy-4-methyl-6-(3-amino) benzyl-N-MeNle]-1-[α-methylene-Sar]-3-cyclosporin [Molecular Formula: C₆₈H₁₁₆N₁₂O₁₂; Exact Mass: 1292.88; MS (m/z): 1293.68 (M+1)⁺].

### [(3R,4R)-3-Hydroxy-4-methyl-6-(3-amino)benzyl-N-MeNle]-1-[(S)-(4-hydroxybutylthio)methyl-Sar]-cyclos porin

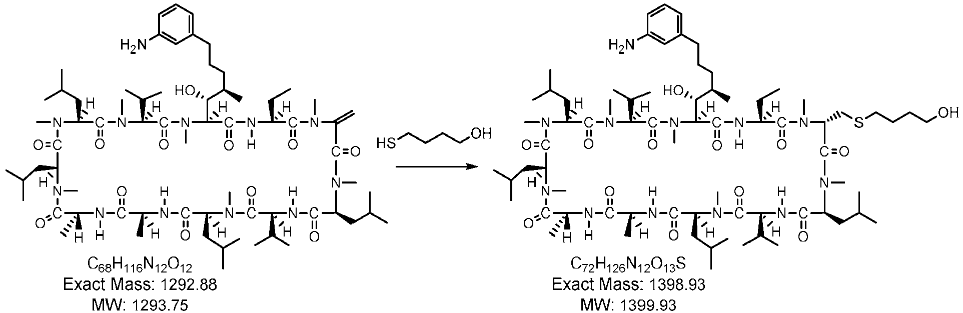

To a solution of [(3R,4R)-3-hydroxy-4-methyl-6-(3-amino) benzyl-N-MeNle]-1-[α-methylene-Sar]-3-cyclosporin (0.20 g, 0.15 mmol) in methanol (10 ml) were added 4-mercapto-1-butanol (0.07 g, 0.66 mmol) and lithium hydroxide (19 mg, 0.79 mmol). The reaction mixture was stirred at room temperature overnight. Most of the methanol was evaporated under reduced pressure. Ethyl acetate (30 ml) and brine (30 ml) were added and the mixture was separated. The organic layer was dried over magnesium sulfate and evaporated under reduced pressure. The residue was purified by chromatography (dichloromethane/methanol) to give pure [(3R,4R)-3-hydroxy-4-methyl-6-(3-amino)benzyl-N-MeNle]-1-[(S)-(4-hydroxy butylthio)methyl-Sar]-3-cyclosporin [Molecular Formula: C₇₂H₁₂₆N₁₂O₁₃S; Exact Mass: 1398.93; MS (m/z): 1399.62 (M+1)⁺; HPLC RT: 11.33 min. (C8 reverse phase column: 250 mm; acetonitrile/water (0.05% trifluoroacetic acid); operation temperature: 64 °C; detector: 210 nm)].

### Example 5

### [(3R,4R)-3-Hydroxy-4-methyl-6-(3-(N,N-diisopropylamino)carbonyl)benzyl-N-MeNle]-1-[(S)-(4-hydroxybutylthio)methyl-Sarl-3-cyclosporin

### (3-Methoxycarbonylbenzyl)triphenylphosphonium bromide

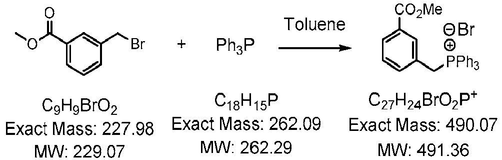

Methyl (3-bromomethyl)benzoate (6.00 g, 26.20 mmol) and triphenylphosphine (6.86 g, 26.18 mmol) were added to toluene (60 ml). The mixture was stirred and heated to reflux overnight. After cooled to room temperature, the precipitate was filtered off, washed with toluene and hexane and dried in vacuum to give 11.05 g product. [Molecular Formula: C₂₇H₂₄BrO₂P; Exact Mass: 490.07; MS (m/z): 411.25 (M+1)⁺-Br].

### [(3R,4R)-3-Acetyloxy-4-methyl-6-(3-methoxycarbonylphenyl)methylene-N-MeNle]-1-cyclosporin

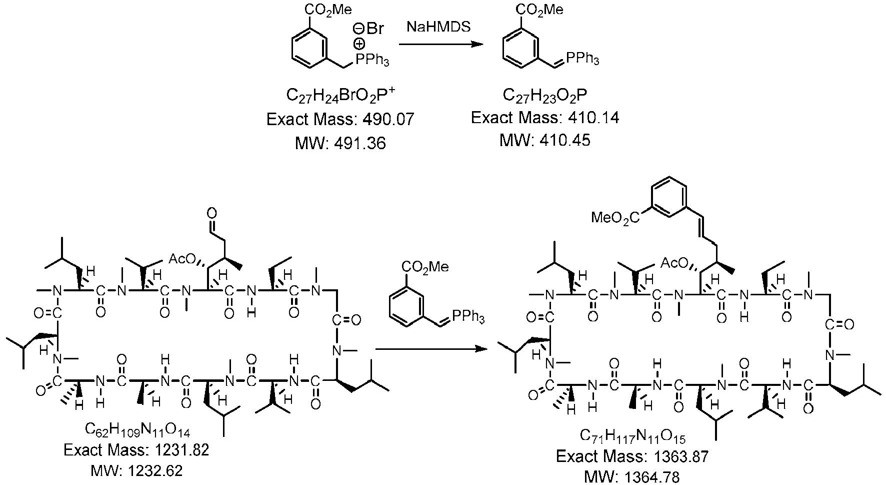

To a mixture of (3-methoxycarbonylbenzyl)triphenylphosphonium bromide (4.00 g, 8.14 mmol) in anhydrous tetrahydrofuran (120 ml) under nitrogen were added sodium bis(trimethylsilyl)amide (1.0 M in THF, 10 ml, 10.00 mmol). The reaction mixture was stirred at room temperature for one hour and cooled to -40 °C. A solution of [(3R,4R)-3-acetyloxy-4-methyl-6-oxo-N-MeNle]-1-cyclosporin (5.00 g, 4.05 mmol) in anhydrous tetrahydrofuran (25 ml) was added. The mixture was stirred another two hours at -30 °C. Then saturated ammonium chloride solution (20 ml) was added to quench the reaction. Most of tetrahydrofuran was evaporated under reduced pressure. Ethyl acetate (150 ml) and brine (50 ml) were added and the mixture was separated. The organic layer was dried over magnesium sulfate and evaporated under reduced pressure. The residue was purified by chromatography (dichloromethane/methanol) to give 2.00 g of pure [(3R,4R)-3-acetyloxy-4-methyl-6-(3-methoxycarbonylphenyl)methylene-N-MeNle]-1-cyclosporin [Molecular Formula: C₇₁H₁₁₇N₁₁O₁₅; Exact Mass: 1363.87; MS (m/z): 1364.61 (M+1)⁺].

### [(3R, 4R)-3-Acetyloxy-4-methyl-6-(3-methoxycarbonyl)benzyl-N-MeNle]-1-cyclosporin

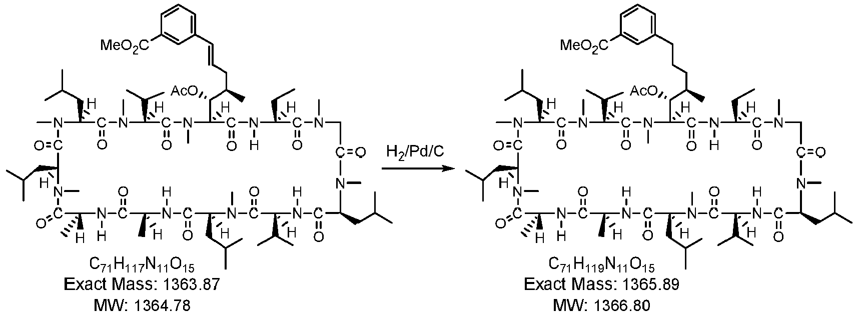

To a solution of [(3R,4R)-3-acetyloxy-4-methyl-6-(3-methoxycarbonylphenyl) methylene-N-MeNle]-1-cyclosporin (2.00 g, 1.46 mmol) in methanol (50 ml) were added palladium (10 wt% on carbon, 20 mg) and acetic acid (5 drops). The mixture was stirred at room temperature under hydrogen for two hours. The mixture was filtered and the filtrate was evaporated under reduced pressure to give crude [(3R,4R)-3-acetyloxy-4-methyl-6-(3-methoxycarbonyl)benzyl-N-MeNle]-1-cyclosporin [Molecular Formula: C₇₁H₁₁₉N₁₁O₁₅; Exact Mass: 1365.89; MS (m/z): 1366.73 (M+1)⁺].

### [(3R, 4R)-3-Hydroxy-4-methyl-6-(3-carboxy)benzyl-N-MeNle]-1-cyclosporin

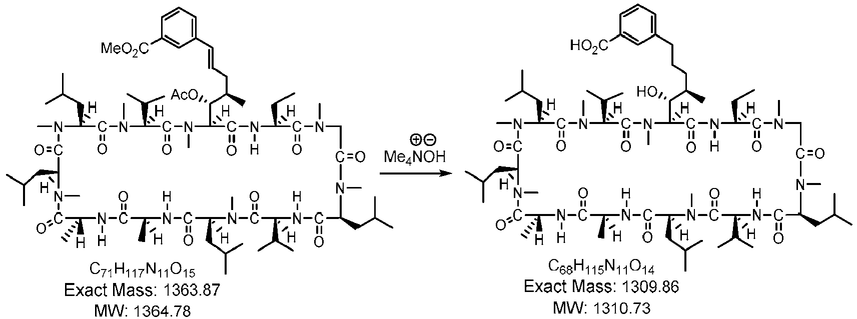

[(3R,4R)-3-Acetyloxy-4-methyl-6-(3-methoxycarbonyl)benzyl-N-MeNle]-1-cyclosporin (0.25 g, 0.18 mmol) was dissolved in methanol (4 ml). Water (2 ml) and tetramethylammonium hydroxide pentahydrate (70 mg) were added. The mixture was stirred at room temperature overnight. Then most of the methanol was evaporated. The PH of the mixture was adjusted to 6 with acetic acid. Ethyl acetate (50 ml) and brine (10 ml) were added and the mixture was separated. The organic layer was dried over magnesium sulfate and evaporated under reduced pressure. The residue was used for next step without further purification. [(3R, 4R)-3-hydroxy-4-methyl-6-(3-carboxy)benzyl-N-MeNle]-1-cyclosporin [Molecular Formula: C₆₈H₁₁₅N₁₁O₁₄; Exact Mass: 1309.86; MS (m/z): 1310.61 (M+1)⁺].

### [(3R, 4R)-3-Hydroxy-4-methyl-6-(3-methoxycarbonyl)benzyl-N-MeNle]-1-cyclosporin

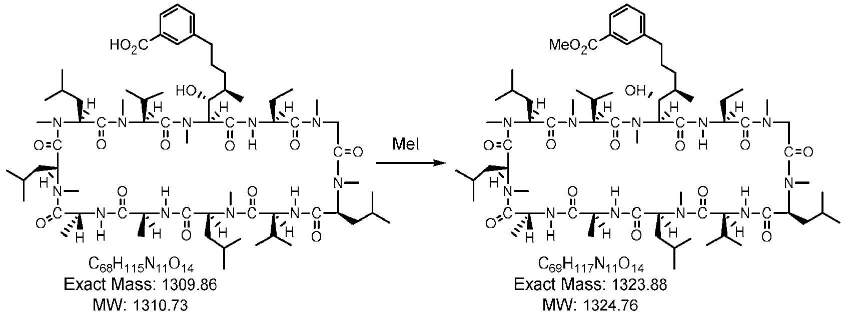

To the solution of [(3R,4R)-3-hydroxy-4-methyl-6-(3-carboxy)benzyl-N-MeNle]-1-cyclosporin(1.80 g, 1.37 mmol) in N,N-dimethylformamide (25 ml) were added potassium carbonate (0.38 g, 2.75 mmol) and iodomethane (0.50 g, 3.52 mmol). The mixture was stirred at room temperature for three hours. Ethyl acetate (100 ml) and water (50 ml) were added and the mixture was separated. The organic layer was dried over magnesium sulfate and evaporated under reduced pressure. The residue was purified by chromatography (dichloromethane/methanol) to give 1.60 g of pure [(3R, 4R)-3-hydroxy-4-methyl-6-(3-methoxycarbonyl)benzyl-N-MeNle]-1-cyclosporin [Molecular Formula: C₆₉H₁₁₇N₁₁O₁₄; Exact Mass: 1323.88; MS (m/z): 1324.64 (M+1)⁺].

### [(3R, 4R)-3-Hydroxy-4-methyl-6-((3-N,N-diisopropylaminocarbonyl))benzyl-N-MeNle]-1-[α-methylene-Sar]-3-cyclosporin

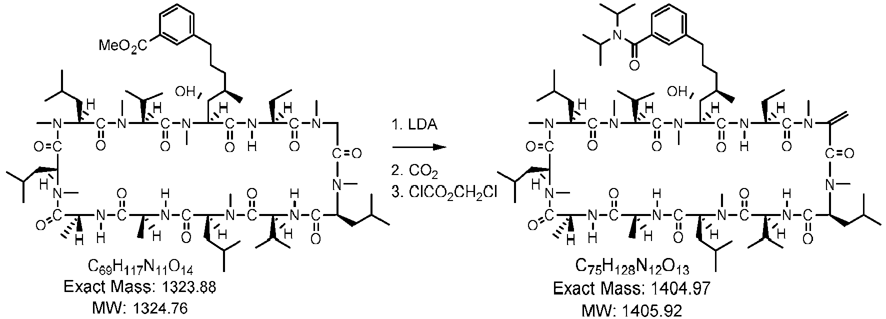

n-Butyllithium (2.65 M, 5 ml, 13.33 mmol) was added to a solution of diisopropylamine (1.37 g, 13.30 mmol) in tetrahydrofuran (50 ml) at -78 °C under nitrogen. After the reaction mixture was stirred for one hour, a solution of [(3R,4R)-3-hydroxy-4-methyl-6-(3-methoxycarbonyl)benzyl-N-MeNle]-1-cyclosporin (1.60 g, 1.20 mmol) in tetrahydrofuran (5 ml) was added. The mixture was stirred at -78 °C for three hours. After carbon dioxide gas was bubbled into the reaction mixture for 15 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (2.00 ml) was added. The mixture was stirred and allowed to warm to room temperature overnight. Brine (5 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.60 g of pure [(3R, 4R)-3-hydroxy-4-methyl-6-(3-(N,N-diisopropylamino)carbonyl)benzyl-N-MeNle]-1-[a-methylene-Sar]-3-cyclosporin [Molecular Formula: C₇₅H₁₂₈N₁₂O₁₃; Exact Mass: 1404.97; MS (m/z): 1405.67 (M+1)⁺].

### [(3R, 4R)-3-Hydroxy-4-methyl-6-(3-(N,N-diisopropylamino)carbonyl)benzyl-N-MeNle]-1-[(S)-(4-hydroxybutylthio)methyl-Sar]-3-cyclosporin

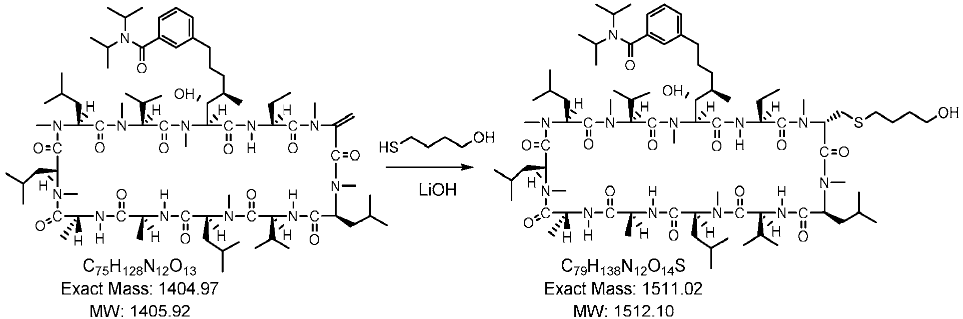

To a solution of [(3R,4R)-3-hydroxy-4-methyl-6-(3-(N,N-diisopropyamino) carbonyl)benzyl-N-MeNle]-1-[α-methylene-Sar]-3-cyclosporin (0.60 g, 0.43 mmol) in methanol (25 ml) were added 4-mercapto-1-butanol (0.45 g, 4.25 mmol) and lithium hydroxide (0.10 g, 4.25 mmol). The reaction mixture was stirred at room temperature overnight. Most of the methanol was evaporated under reduced pressure. Ethyl acetate (60 ml) and brine (30 ml) were added and the mixture was separated. The organic layer was dried over magnesium sulfate and evaporated under reduced pressure. The residue was purified by chromatography (dichloromethane/methanol) to give pure [(3R, 4R)-3-hydroxy-4-methyl-6-(3-(N,N-diisopropyamino)carbonyl)benzyl-N-MeNle]-1-[(S)-(4-hydroxybutylthio)methyl-Sar]-3-cyclosporin [Molecular Formula: C₇₉H₁₃₈N₁₂O₁₄S; Exact Mass: 1511.02; MS (m/z): 1511.70 (M+1)⁺; HPLC RT: 17.00 min. (C8 reverse phase column: 250 mm; acetonitrile/water (0.05% trifluoroacetic acid); operation temperature: 64 °C; detector: 210 nm)].

### Example 6

### [(3R,4R)-3-Hydroxy-4-methyl-6-(3-carboxy)benzyl-N-MeNle]-1-[(S)-(4-hydroxybutylthio)methyl-Sar]-3-cyclosporin

### [(3R, 4R)-3-Hydroxy-4-methyl-6-(3-carboxy)benzyl-N-MeNle]-1-[α-methylene-Sar]-3-cyclosporin

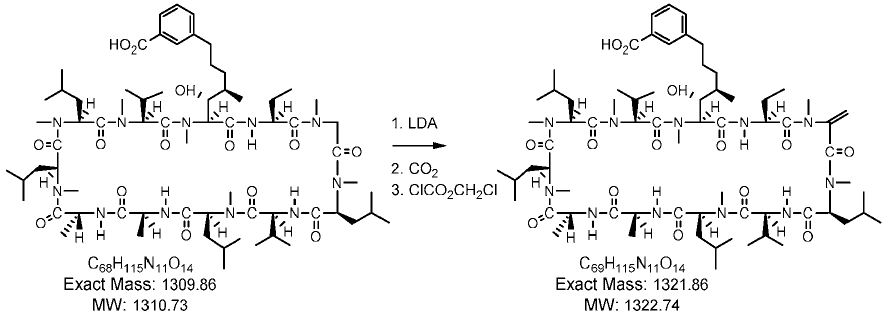

n-Butyllithium (2.65 M, 7.7 ml, 20.41 mmol) was added to a solution of diisopropylamine (2.05 g, 20.30 mmol) in tetrahydrofuran (50 ml) at -78 °C under nitrogen. After the reaction mixture was stirred for one hour, a solution of [(3R,4R)-3-hydroxy-4-methyl-6-(3-carboxy)benzyl-N-MeNle]-1-cyclosporin (2.40 g, 1.20 mmol) in tetrahydrofuran (10 ml) was added. The mixture was stirred at -78 °C for three hours. After carbon dioxide gas was bubbled into the reaction mixture for 15 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (3.00 ml) was added. The mixture was stirred and allowed to warm to room temperature overnight. Brine (5 ml) was added to quench the reaction. Most of tetrahydrofuran was removed under reduced pressure. Ethyl acetate (80 ml) and brine (50 ml) were added and the mixture was separated. The organic layer was dried over magnesium sulfate and evaporated under reduced pressure to give 1.50 g of crude product [(3R, 4R)-3-hydroxy-4-methyl-6-(3-carboxy)benzyl-N-MeNle]-1-[a-methylene-Sar]-3-cyclosporin [Molecular Formula: C₆₉H₁₁₅N₁₁O₁₄; Exact Mass: 1321.86; MS (m/z): 1322.45 (M+1)⁺].

### [(3R, 4R)-3-Hydroxy-4-methyl-6-(3-methoxycarbonyl)benzyl-N-MeNle]-1-[α-methylene-Sar]-3-cyclosporin

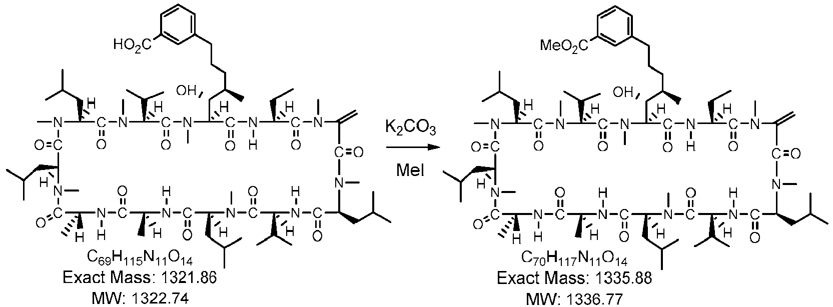

To the solution of [(3R, 4R)-3-hydroxy-4-methyl-6-(3-carboxy)benzyl-N-MeNle]-1-[α-methylene-Sar]-3-cyclosporin(1.32 g, 1.00 mmol) in N,N-dimethylformamide (15 ml) were added potassium carbonate (0.38 g, 2.75 mmol) and iodomethane (0.50 g, 3.52 mmol). The mixture was stirred at room temperature for three hours. Ethyl acetate (100 ml) and water (50 ml) were added and the mixture was separated. The organic layer was dried over magnesium sulfate and evaporated under reduced pressure. The residue was purified by chromatography (dichloromethane/methanol) to give 1.60 g of pure [(3R,4R)-3-hydroxy-4-methyl-6-(3-methoxycarbonyl)benzyl-N-MeNle]-1-[α-methylene-Sar]-3-cyclosporin [Molecular Formula: C₇₀H₁₁₇N₁₁O₁₄; Exact Mass: 1335.88; MS (m/z): 1336.64 (M+1)⁺].

### [(3R, 4R)-3-Hydroxy-4-methyl-6-(3-methoxycarbonyl)benzyl-N-MeNle]-1-[(S)-(4-hydroxybutylthio)methyl-Sar]-3-cyclosporin

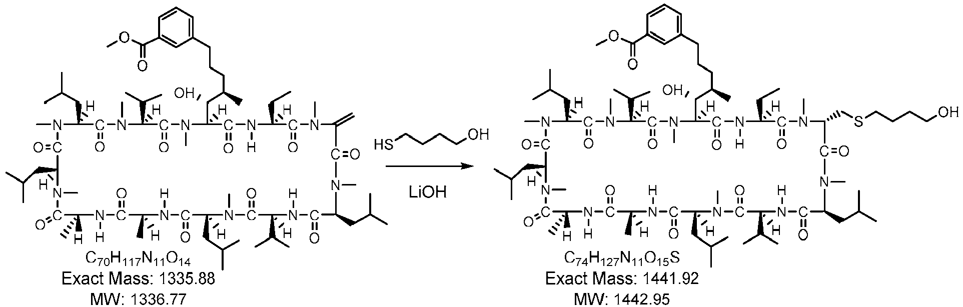

To a solution of [(3R,4R)-3-hydroxy-4-methyl-6-(3-methoxycarbonyl)benzyl-N-MeNle]-1-[α-methylene-Sar]-3-cyclosporin (0.60 g, 0.45 mmol) in methanol (25 ml) were added 4-mercapto-1-butanol (0.45 g, 4.25 mmol) and lithium hydroxide (0.10 g, 4.25 mmol). The reaction mixture was stirred at room temperature overnight. Most of the methanol was evaporated under reduced pressure. Ethyl acetate (60 ml) and brine (30 ml) were added and the mixture was separated. The organic layer was dried over magnesium sulfate and evaporated under reduced pressure. The residue was purified by chromatography (dichloromethane/methanol) to give pure [(3R,4R)-3-hydroxy-4-methyl-6-(3-methoxycarbonyl)benzyl-N-MeNle]-1-[(S)-(4-hydroxybutylthio)methyl-Sar]-3-cyclosporin [Molecular Formula: C₇₄H₁₂₇N₁₁O₁₅S; Exact Mass: 1441.92; MS (m/z): 1422.65 (M+1)⁺].

### Examples 7

### [(3R,4R)-3-Hydroxy-4-methyl-6-(3-carboxy)benzyl-N-MeNle]-1-[(S)-(4-hydroxybutylthio )methyl-Sar]-3-cyclosporin

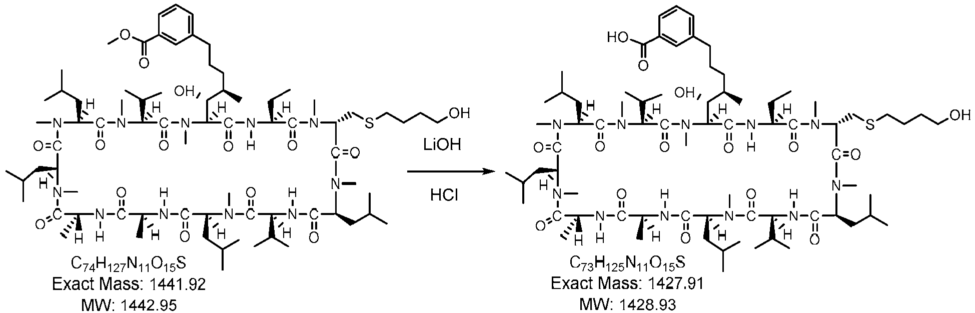

To a solution of [(3R,4R)-3-hydroxy-4-methyl-6-(3-methoxycarbonyl)benzyl-N-MeNle]-1-[(S)-(4-hydroxybutylthio)methyl-Sar]-3-cyclosporin (85 mg, 0.06 mmol) in methanol (5 ml) was added a solution of lithium hydroxide (15 mg) in water (5 ml). The mixture was stirred at room temperature overnight. Most of the methanol was evaporated under reduced pressure. The PH of aqueous mixture was adjusted to 6 with 1.0 N hydrochloric acid. Ethyl acetate (60 ml) and brine (10 ml) were added and the mixture was separated. The organic layer was dried over magnesium sulfate and evaporated under reduced pressure. The residue was purified to give pure [(3R,4R)-3-hydroxy-4-methyl-6-(3-carboxy)benzyl-N-MeNle]-1-[(S)-(4-hydroxybutylthio)methyl-Sar]-3-cyclosporin [Molecular Formula: C₇₃H₁₂₅N₁₁O₁₅S; Exact Mass: 1427.91; MS (m/z): 1428.63 (M+1)⁺; HPLC RT: 14.37 min. (C8 reverse phase column: 250 mm; acetonitrile/water (0.05% trifluoroacetic acid); operation temperature: 64 °C; detector: 210 nm)].

### Examples 8

### [(3R, 4R)-3-Hydroxy-4-methyl-6-(2-hydroxyethyl)-N-MeNle]-1-[(S)-(4-hydroxybutylthio)methyl-Sar]-3-cyclosporin

### [(3R, 4R)-3-Acetyloxy-4-methyl-(6-methoxycarbonylmethylene)-N-MeNle]-1-cyclosporin

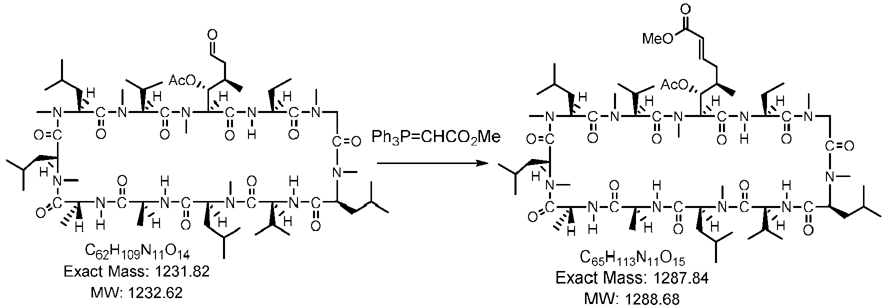

To a solution of methoxycarbonylmethylenetriphenylphosphorane (6.20 g, 18.54 mmol) in anhydrous tetrahydrofuran (100 ml) under nitrogen was added [(3R,4R)-3-acetyloxy-4-methyl-6-oxo-N-MeNle]-1-cyclosporin (4.10 g, 3.33 mmol). The mixture was stirred and heated to reflux for six hours. Then saturated ammonium chloride solution (20 ml) was added to quench the reaction. Most of tetrahydrofuran was evaporated under reduced pressure. Ethyl acetate (150 ml) and brine (50 ml) were added and the mixture was separated. The organic layer was dried over magnesium sulfate and evaporated under reduced pressure. The residue was purified by chromatography (dichloromethane/methanol) to give 3.60 g of pure [(3R,4R)-3-acetyloxy-4-methyl-(6-methoxycarbonylmethylene)-N-MeNle]-1-cyclosporin [Molecular Formula: C₆₅H₁₁₃N₁₁O₁₅; Exact Mass: 1287.84; MS (m/z): 1288.61 (M+1)⁺; HPLC RT: 16.43 min. (C8 reverse phase column: 250 mm; acetonitrile/water (0.05% trifluoroacetic acid); operation temperature: 64 °C; detector: 210 nm)].

### [(3R, 4R)-3-Acetyloxy-4-methyl-6-(methoxycarbonylmethyl)-N-MeNle]-1-cyclosporin

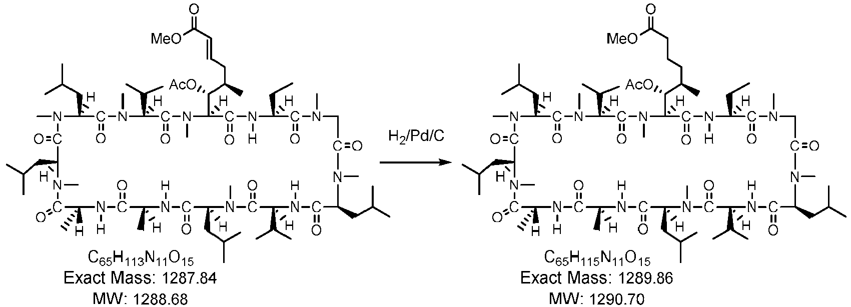

To a solution of [(3R,4R)-3-acetyloxy-4-methyl-6-(methoxycarbonylmethylene)-N-MeNle]-1-cyclosporin (3.80 g, 2.95 mmol) in methanol (100 ml) were added palladium (10 wt% on carbon, 250 mg) and acetic acid (5 drops). The mixture was stirred at room temperature under hydrogen for eight hours. The mixture was filtered and the filtrate was evaporated under reduced pressure to give crude [(3R,4R)-3-acetyloxy-4-methyl-6-(methoxycarbonymethyl)-N-MeNle]-1-cyclosporin [Molecular Formula: C₆₅H₁₁₅N₁₁O₁₅; Exact Mass: 1289.86; MS (m/z): 1290.59 (M+1)⁺; HPLC RT: 16.50 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(2-hydroxyethyl)-N-MeNle]-1-cyclosporin

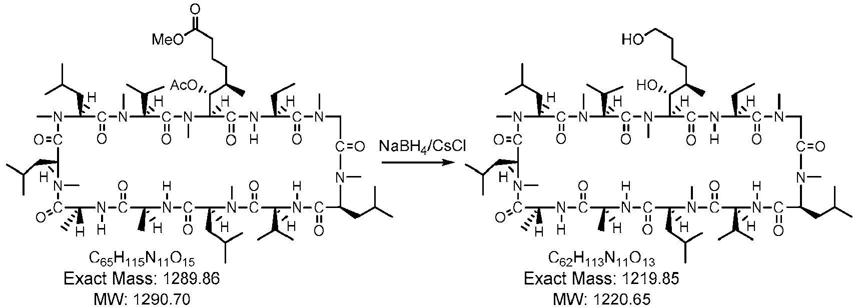

To a solution of [(3R,4R)-3-acetyloxy-4-methyl-6-(methoxycarbonymethyl)-N-MeNle]-1-cyclosporin (3.50 g, 2.71 mmol) in methanol (100 ml) was added cessium chloride (5.00 g, 29.78 mmol). Then sodium borohydride (10.00 g, 262.95 mmol) was added portions in 30 minutes. After the mixture was stirred for another eight hours at room temperature, most of the methanol was evaporated under reduced pressure. Ethyl acetate (150 ml) and saturated sodium bicarbonate solution (150 ml) were added and the mixture was separated. The organic layer was washed with brine, dried over magnesium sulfate and evaporated under reduced pressure. The residue was purified by chromatography (dichloromethane/methanol) to give 2.85 g of [(3R,4R)-3-hydroxy-4-methyl-6-(2-hydroxyethyl)-N-MeNle]-1-cyclosporin [Molecular Formula: C₆₂H₁₁₃N₁₁O₁₃; Exact Mass: 1219.85; MS (m/z): 1220.68 (M+1)⁺; HPLC RT: 15.80 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(2-hydroxyethyl)-N-MeNle]-1-[α-methylene-Sar]-3-cyclosporin

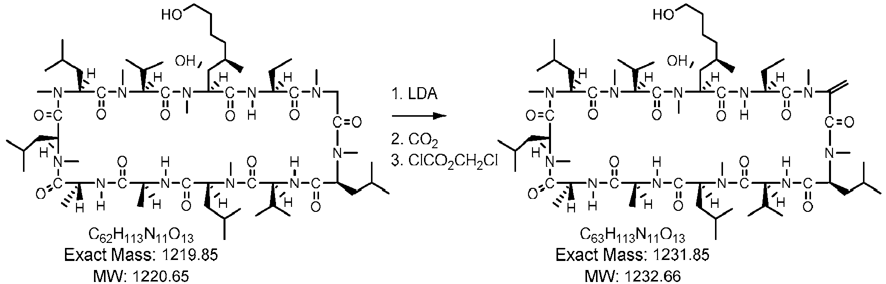

n-Butyllithium (2.65 M, 9.60 ml, 25.30 mmol) was added to a solution of diisopropylamine (2.56 g, 25.30 mmol) in tetrahydrofuran (80 ml) at -78 °C under nitrogen. After the reaction mixture was stirred for one hour, a solution of [(3R,4R)-3-hydroxy-4-methyl-6-(2-hydroxyethyl)-N-MeNle]-1-cyclosporin (2.85 g, 2.30 mmol) in tetrahydrofuran (15 ml) was added. The mixture was stirred at -78 °C for three hours. After carbon dioxide gas was bubbled into the reaction mixture for 15 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (3.00 ml) was added. The mixture was stirred and allowed to warm to room temperature overnight. Brine (5 ml) was added to quench the reaction. Most of tetrahydrofuran was removed under reduced pressure. Ethyl acetate (80 ml) and brine (50 ml) were added and the mixture was separated. The organic layer was dried over magnesium sulfate and evaporated under reduced pressure. The residue was purified by chromatography (dichloromethane/methanol) to give 1.0 g of product [(3R,4R)-3-hydroxy-4-methyl-6-(2-hydroxyethyl)-N-MeNle]-1-[α-methylene-Sar]-3-cyclosporin [Molecular Formula: C₆₃H₁₁₃N₁₁O₁₃; Exact Mass: 1231.85; MS (m/z): 1232.55 (M+1)⁺ ; HPLC RT: 15.14 min. (C8 reverse phase column: 250 mm; acetonitrile/water (0.05% trifluoroacetic acid); operation temperature: 64 °C; detector: 210 nm)].

### [(3R, 4R)-3-Hydroxy-4-methyl-6-(2-hydroxyethyl)-N-MeNle]-1-[(S)-(4-hydroxybutylthio)methyl-Sar]-3-cyclosporin

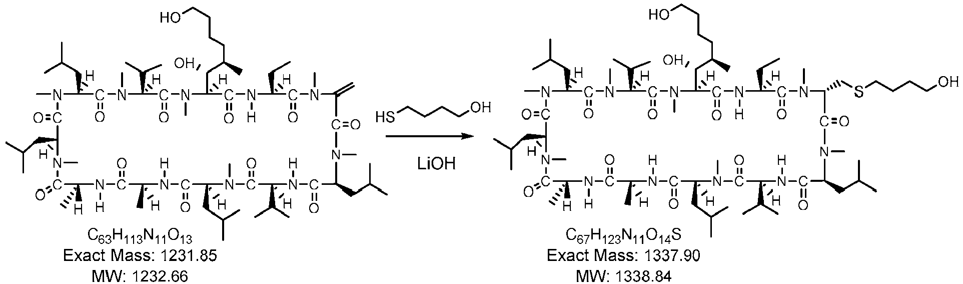

To a solution of [(3R,4R)-3-hydroxy-4-methyl-6-(2-hydroxyethyl)-N-MeNle]-1-[α-methylene-Sar]-3-cyclosporin (0.60 g, 0.45 mmol) in methanol (50 ml) were added 4-mercapto-1-butanol (0.45 g, 4.25 mmol) and lithium hydroxide (0.10 g, 4.25 mmol). The reaction mixture was stirred at room temperature for overnight. Most of the methanol was evaporated under reduced pressure. Ethyl acetate (60 ml) and brine (30 ml) were added and the mixture was separated. The organic layer was dried over magnesium sulfate and evaporated under reduced pressure. The residue was purified by chromatography (dichloromethane/methanol) to give pure [(3R,4R)-3-hydroxy-4-methyl-6-(2-hydroxyethyl)-N-MeNle]-1-[(S)-(4hydroxybutylthio) methyl-Sar]-3-cyclosporin [Molecular Formula: C₆₇H₁₂₃N₁₁O₁₄S; Exact Mass: 1337.90; MS (m/z): 1338.74 (M+1)⁺; HPLC RT: 12.98 min. (C8 reverse phase column: 250 mm; acetonitrile/water (0.05% trifluoroacetic acid); operation temperature: 64 °C; detector: 210 nm)].

### Example 9

### [8-(2-Acetamidoethyl)-6,7-dihydro-MeBmt]-1-[(R)-2-methoxymethyl-Sar]-3-cyclosporin

### (3-Cyanopropyl)triphenylphosphonium bromide

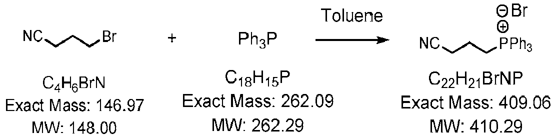

4-Bromobutyronitrile (10 ml, 0.10 mol) and triphenylphosphine (36.23 g, 0.10 mol) were added to toluene (200 ml). The mixture was stirred and heated to reflux overnight. After cooled to room temperature, the precipitate was filtered off, washed with toluene and hexane and dried in vacuum to give 26.60 g product. [Molecular Formula: C₂₂H₂₁NP⁺; Exact Mass: 330.14; MS (m/z): 330.23 (M)⁺].

### [8-Cyanomethyl-3-acetyl-MeBmt]-1-cyclosporin

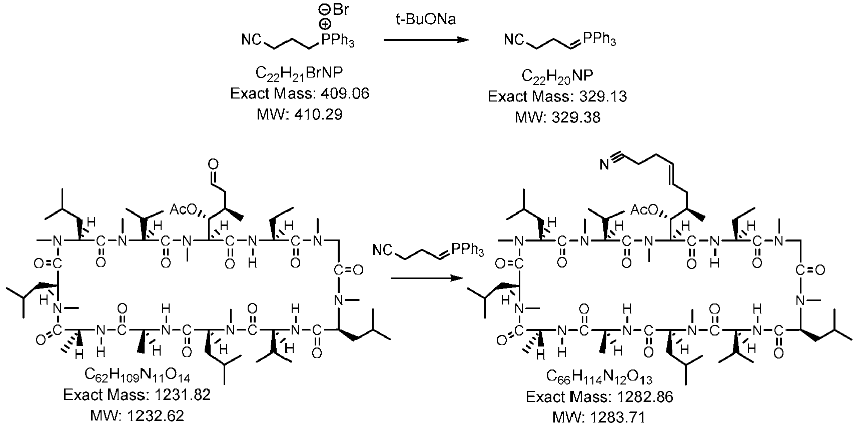

To a dried flask were added (3-cyanopropyl)triphenylphosphonium bromide (7.98 g, 19.50 mmol) and anhydrous tetrahydrofuran (60 ml) under nitrogen. The reaction mixture was put into an ice-water bath and sodium tert-butoxide (2.19 g, 22.75 mmol) was added. After the mixture was stirred for two hours, a solution of [(3R, 4R)-3-acetyloxy-4-methyl-6-oxo-N-MeNle]-1-cyclosporin (4.00 g, 3.25 mmol) in anhydrous tetrahydrofuran (20 ml) was added. The mixture was stirred another five hours at 0 °C. Then saturated ammonium chloride solution (20 ml) was added to quench the reaction. Most of tetrahydrofuran was evaporated under reduced pressure. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 2.00 g of pure [8-cyanomethyl-3-acetyl-MeBmt]-1-cyclosporin [Molecular Formula: C₆₆H₁₁₄N₁₂O₁₃; Exact Mass: 1282.86; MS (m/z): 1283.51 (M+1)⁺, 1305.73 (M+Na)⁺; HPLC RT: 16.50 min. (C8 reverse phase column: 250 mm; acetonitrile/water (0.05% trifluoroacetic acid); operation temperature: 64 °C; detector: 210 nm)].

### [8-Cyanomethyl-MeBmt]-1-cyclosporin

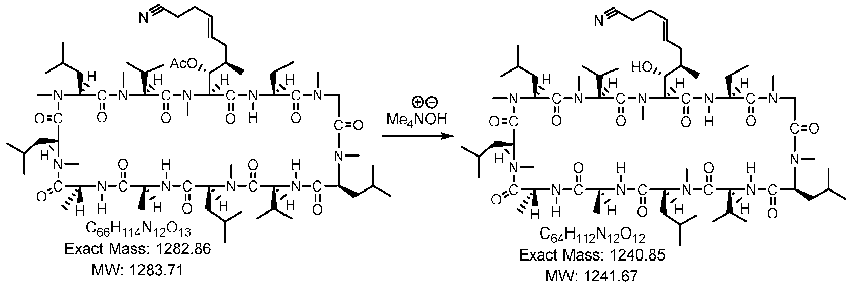

[8-Cyanomethyl-3-acetyl-MeBmt]-1-cyclosporin (2.00 g, 1.56 mmol) was dissolved in methanol (20 ml). Water (10 ml) and tetramethylammonium hydroxide pentahydrate (0.85 g, 4.68 mmol) were added. The mixture was stirred at room temperature for two hours. Then most of the methanol was evaporated. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 1.70 g of pure [8-cyanomethyl-MeBmt]-1-cyclosporin [Molecular Formula: C₆₄H₁₁₂N₁₂O₁₂; Exact Mass: 1240.85; MS (m/z): 1241.54 (M+1)⁺, 1263.73 (M+Na)⁺; HPLC RT: 14.80 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-cyclosporin

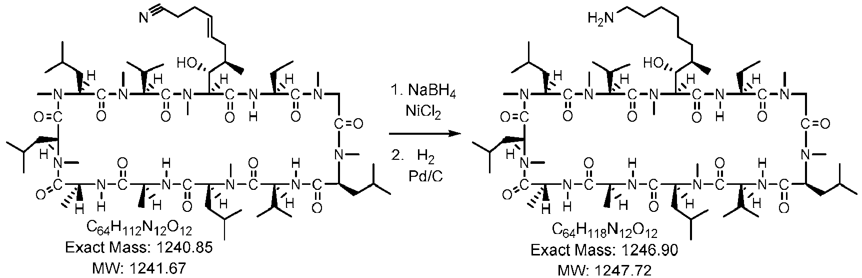

To a solution of [8-cyanomethyl-MeBmt]-1-cyclosporin (2.00 g, 1.61 mmol) in methanol (50 ml) under nitrogen was added nickel (II) chloride hexahydrate (0.19 g, 0.81 mmol). The reaction mixture was put into ice-water bath. Sodium borohydride (3.05 g, 80.50 mmol) was added in four batches in two hours. After the mixture was stirred for another two hours at 0 °C, water (10 ml) was added. Most of the methanol was evaporated under reduced pressure. Ethyl acetate (50 ml) and saturated sodium bicarbonate solution (50 ml) were added and the mixture was separated. The organic layer was washed with brine, dried over magnesium sulfate and evaporated under reduced pressure. The residue was dissolved in methanol (30 ml). Palladium (10 wt% on carbon, 20 mg) and acetic acid (5 drops) were added. The mixture was stirred at room temperature under hydrogen for two hours. The mixture was filtered and the filtrate was evaporated under reduced pressure to give crude [8-(2-aminoethyl)-6,7-dihydro-MeBmt]-1-cyclosporin [Molecular Formula: C₆₄H₁₁₈N₁₂O₁₂; Exact Mass: 1246.90; MS (m/z): 1247.69 (M+1)⁺; HPLC RT: 11.13 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-cyclosporin

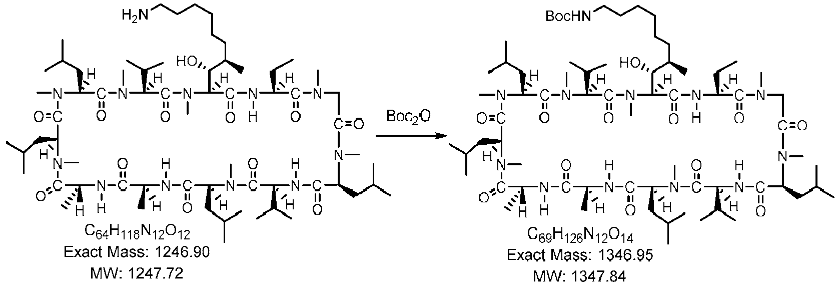

[8-(2-Aminoethyl)-6,7-dihydro-MeBmt]-1-cyclosporin (crude, 1.61 mmol) was dissolved in tetrahydrofuran (20 ml). Saturated sodium bicarbonate solution (20 ml) and di-tert-butyldicarbonate (0.39 g, 1.77 mmol) were added. The mixture was stirred at room temperature for two hours. Most of tetrahydrofuran was evaporated under reduced pressure. Ethyl acetate (30 ml) and brine (30 ml) were added and the mixture was separated. The organic layer was dried over magnesium sulfate and evaporated under reduced pressure. The residue was purified by chromatography (hexane/acetone) to give 2.00 g of pure [8-(2-(tert-butoxycarbonyl)aminoethyl)-6,7-dihydro-MeBmt]-1-cyclosporin [Molecular Formula: C₆₉H₁₂₆N₁₂O₁₄; Exact Mass: 1346.95; MS (m/z): 1347.54 (M+1)⁺, 1369.78 (M+Na)⁺; HPLC RT: 17.08 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(S)-2-methoxycarbonyl-Sar]-3-cyclosporin

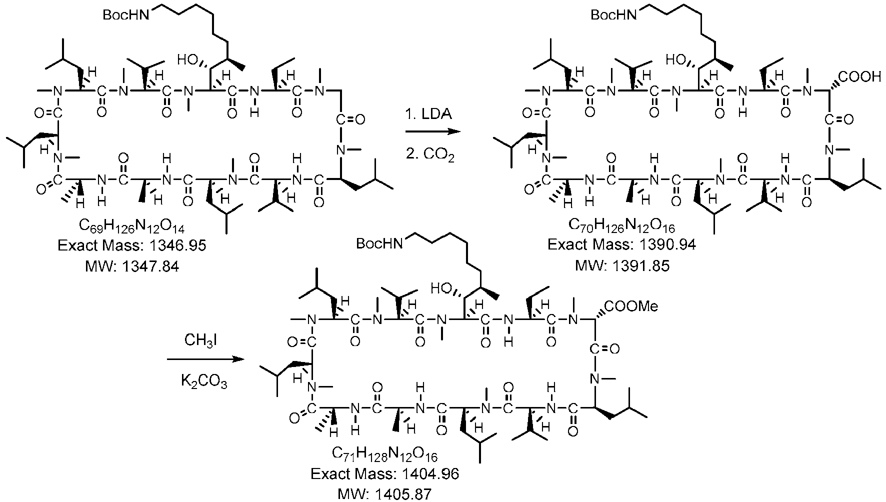

n-Butyllithium (2.20 M, 3.38 ml, 7.42 mmol) was added to a solution of diisopropylamine (1.06 ml, 7.42 mmol) in tetrahydrofuran (20 ml) at -78 °C under nitrogen. After the reaction mixture was stirred for an hour, a solution of [8-(2-(tert-butoxycarbonyl)aminoethyl)-6,7-dihydro-MeBmt]-1-cyclosporin (1.00 g, 0.74 mmol) in tetrahydrofuran (10 ml) was added over ten minutes. The mixture was stirred at -78 °C for two hours. After carbon dioxide gas was bubbled into the reaction mixture for 20 minutes, the mixture was stirred at -78 °C for another hour. Then the cooling bath was removed and the reaction mixture was allowed to warm up to 0 °C slowly. Most of tetrahydrofuran was removed under vacuum at room temperature. The residue was quenched by the addition of saturated citric acid solution and the pH of the aqueous layer was adjusted to 3∼4 with 1 N hydrochloric acid and the precipitated oil was extracted with ethyl acetate (100 ml). The aqueous layer was extracted with ethyl acetate (100 ml × 3). The combined ethyl acetate layers were washed with brine, dried over magnesium sulfate and evaporated under reduced pressure to give 0.50 g of [8-(2-(tert-butoxycarbonyl)aminoethyl)-6,7-dihydro-MeBmt]-1-[(S)-2-carboxy-Sar]-3-cyclosporin [Molecular Formula: C₇₀H₁₂₆N₁₂O₁₆; Exact Mass: 1390.94; MS (m/z): 1391.60 (M+1)⁺; HPLC RT: 15.57 min. (C8 reverse phase column: 250 mm; acetonitrile/water (0.05% trifluoroacetic acid); operation temperature: 64 °C; detector: 210 nm)].

[8-(2-(tert-Butoxycarbonyl)aminoethyl)-6,7-dihydro-MeBmt]-1-[(S)-2-carboxy-Sar]-3-cyclosporin (0.50 g, 0.36 mmol) was dissolved in acetone (10 ml). Iodomethane (0.03 ml, 0.54 mmol) and potassium carbonate (0.08 g, 0.54 mmol) were added. The mixture was stirred at room temperature for two hours. Most of acetone was evaporated under reduced pressure. Then ethyl acetate (40 ml) and water (40 ml) were added and the mixture was separated. The ethyl acetate layer was dried over magnesium sulfate and evaporated under reduced pressure to give 0.51 g of crude [8-(2-(tert-butoxycarbonyl)aminoethyl)-6,7-dihydro-MeBmt]-1-[(S)-2-methoxycarbonyl-Sar]-3-cyclosporin [Molecular Formula: C₇₁H₁₂₈N₁₂O₁₆; Exact Mass: 1404.96; MS (m/z): 1405.64 (M+1)⁺, 1427.86 (M+Na)⁺; HPLC RT: 16.65 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-2-hydroxymethyl-Sar]-3-cyclosporin

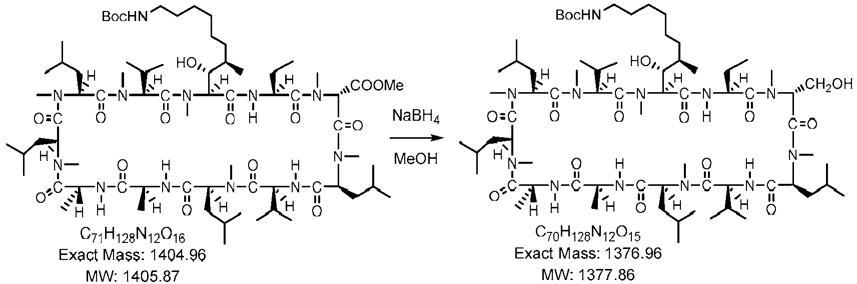

[8-(2-(tert-Butoxycarbonyl)aminoethyl)-6,7-dihydro-MeBmt]-1-[(S)-2-methoxy carbonyl-Sar]-3-cyclosporin (0.51 g, 0.36 mmol) was dissolved in tetrahydrofuran (10 ml). Sodium borohydride (0.68 g, 18.00 mmol) and cesium chloride (0.49 g, 1.51 mmol) were added. The mixture was stirred at room temperature and methanol (10 ml) was added dropwise in one hour. The mixture was stirred for another two hours. Most of solvents were evaporated under reduced pressure. Then ethyl acetate (40 ml) and water (40 ml) were added and the mixture was separated. The ethyl acetate layer was dried over magnesium sulfate and evaporated under reduced pressure to give 0.50 g of crude [8-(2-(tert-butoxycarbonyl)aminoethyl)-6,7-dihydro-MeBmt]-1-[(R)-2-hydroxymethyl-Sar]-3-cyclosporin [Molecular Formula: C₇₀H₁₂₈N₁₂O₁₅; Exact Mass: 1376.96; MS (m/z): 1377.71 (M+1)⁺, 1388.85 (M+Na)⁺; HPLC RT: 16.49 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-2-hydroxymethyl-Sar]-3-cyclosporin

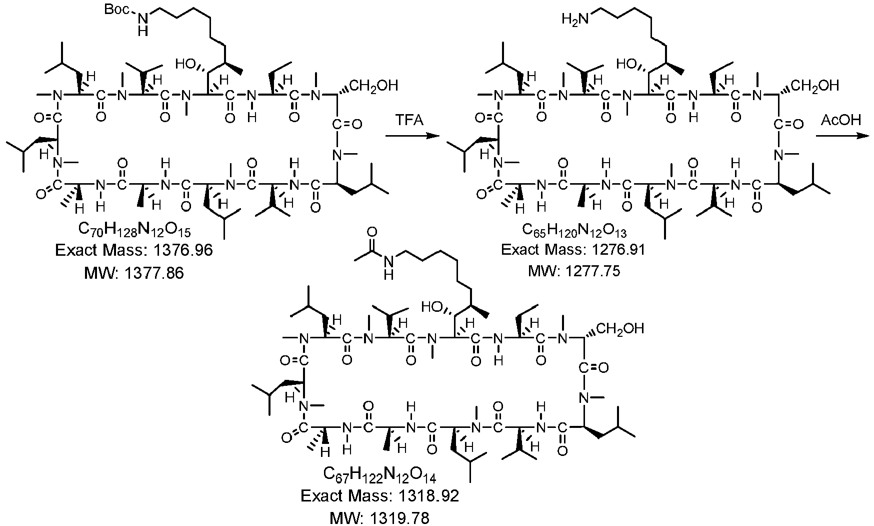

[8-(2-(tert-Butoxycarbonyl)aminoethyl)-6,7-dihydro-MeBmt]-1-[(R)-2-hydroxy methyl-Sar]-3-cyclosporin(0.50 g, 0.36 mmol) was dissolved in dichloromethane (10 ml) and put into ice-water bath. Trifluoroacetic acid (5 ml) was added. The mixture was stirred at room temperature for one hour. Another dichloromethane (20 ml) was added. The mixture was washed with brine (30 ml), saturated sodium bicarbonate solution (30 ml) and dried over magnesium sulfate and evaporated under reduced pressure to give crude [8-(2-aminoethyl)-6,7-dihydro-MeBmt]-1-[(R)-2-hydroxymethyl-Sar]-3-cyclosporin [Molecular Formula: C₆₅H₁₂₀N₁₂O₁₃; Exact Mass: 1276.91; MS (m/z): 1277.75 (M+1)⁺; HPLC RT: 9.13 min. (C8 reverse phase column: 250 mm; acetonitrile/water (0.05% trifluoroacetic acid); operation temperature: 64 °C; detector: 210 nm)]. The crude [8-(2-aminoethyl)-6,7-dihydro-MeBmt]-1-[(R)-2-hydroxymethyl-Sar]-3-cyclosporin was dissolved in dichloromethane (10 ml). Acetic acid (0.12 ml, 2.20 mmol), HBTU (0.41 g, 1.08 mmol), 1-hydroxybenzotriazole (0.15 g, 1.08 mmol) and pyridine (0.50 ml) were added. The mixture was stirred at room temperature for two hours. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 27 mg of pure [8-(2-acetamidoethyl)-6,7-dihydro-MeBmt]-1-[(R)-2-hydroxymethyl-Sar]-3-cyclosporin [Molecular Formula: C₆₇H₁₂₂N₁₂O₁₄; Exact Mass: 1318.92; MS (m/z): 1319.72 (M+1)⁺, 1341.91 (M+Na)⁺; HPLC RT: 14.12 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-2-methoxymethyl-Sar]-3-cyclosporin

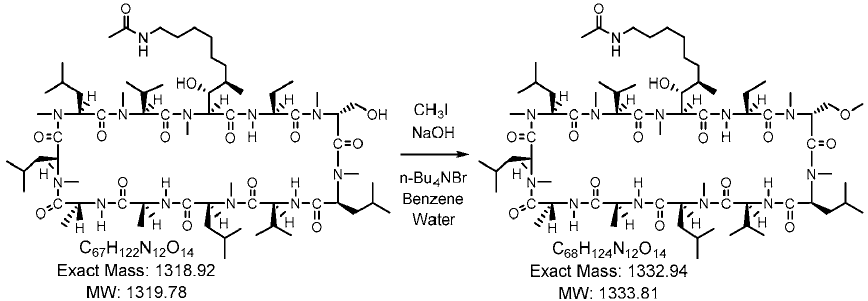

[8-(2-Acetamidoethyl)-6,7-dihydro-MeBmt]-1-[(R)-2-hydroxymethyl-Sar]-3-cyclosporin (0.20 g, 0.15 mmol) was dissolved in benzene (10 ml). Iodomethane (0.21 g, 1.52 mmol), tetra-n-butylammonium bromide (0.49 g, 1.52 mmol), sodium hydroxide (1.00 g, 2.50 mmol) and water (2 ml) were added. The mixture was stirred at room temperature overnight. Then ethyl acetate (50 ml) and brine (50 ml) were added and separated. The ethyl acetate layer was dried over magnesium sulfate and evaporated under reduced pressure. The residue was purified by chromatography (dichloromethane/methanol) to give 60 mg of pure [8-(2-acetamidoethyl)-6,7-dihydro-MeBmt]-1-[(R)-2-methoxymethyl-Sar]-3-cyclosporin [Molecular Formula: C₆₈H₁₂₄N₁₂O₁₄; Exact Mass: 1332.94; MS (m/z): 1333.69 (M+1)⁺, 1355.86 (M+Na)⁺; HPLC RT: 16.18 min. (C8 reverse phase column: 250 mm; acetonitrile/water (0.05% trifluoroacetic acid); operation temperature: 64 °C; detector: 210 nm)].

### Examples 10

### [8-(2-Acetamidoethyl)-6,7-dihydro-MeBmt]-1-[(S)-(4-hydroxybutylthio)methyl-Sar]-3-cyclosporin

### [8-(2-(tert-Butoxycarbonyl)aminoethyl)-6,7-dihydro-MeBmt]-1-[α-methylene-Sar]-3-cyclosporin

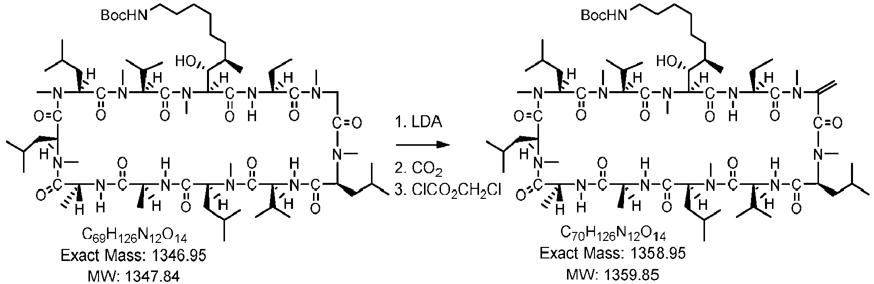

n-Butyllithium (2.20 M, 6.75 ml, 14.85 mmol) was added to a solution of diisopropylamine (2.11 ml, 14.85 mmol) in tetrahydrofuran (40 ml) at -78 °C under nitrogen. After the reaction mixture was stirred for an hour, a solution of [8-(2-(tert-butoxycarbonyl)aminoethyl)-6,7-dihydro-MeBmt]-1-cyclosporin (2.00 g, 1.48 mmol) in tetrahydrofuran (15 ml) was added over ten minutes. The mixture was stirred at -78 °C for two hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (1.32 ml, 14.85 mmol) was added. The mixture was stirred and allowed to warm to room temperature overnight. Brine (5 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.60 g of pure [8-(2-(tert-butoxycarbonyl)aminoethyl)-6,7-dihydro-MeBmt]-1-[α-methylene-Sar]-3-cyclosporin [Molecular Formula: C₇₀H₁₂₆N₁₂O₁₄; Exact Mass: 1358.95; MS (m/z): 1359.59 (M+1)⁺, 1381.81 (M+Na)⁺; HPLC RT: 18.44 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[α-methylene-Sar]-3-cyclosporin

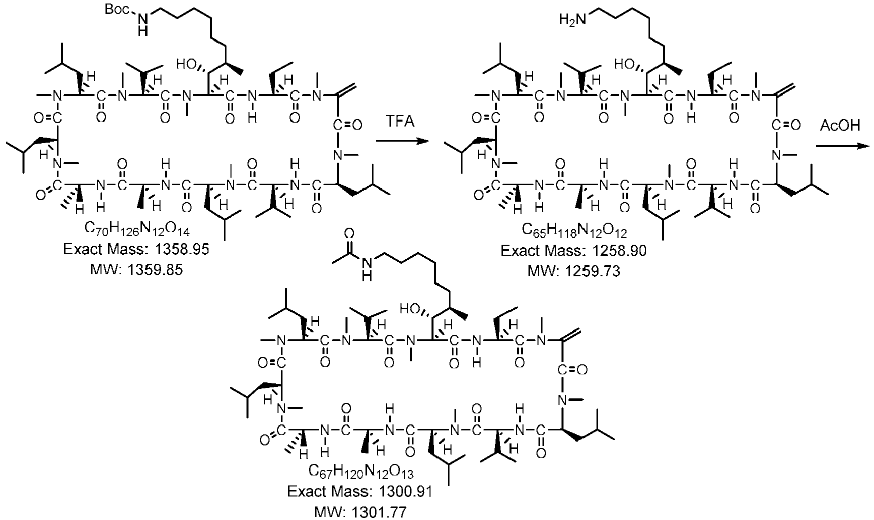

[8-(2-(tert-Butoxycarbonyl)aminoethyl)-6,7-dihydro-MeBmt]-1-[α-methylene-Sar]-3-cyclosporin (0.60 g, 0.44 mmol) was dissolved in dichloromethane (10 ml) and put into ice-water bath. Trifluoroacetic acid (5 ml) was added. The mixture was stirred at room temperature for one hour. Another dichloromethane (20 ml) was added. The mixture was washed with brine (30 ml), saturated sodium bicarbonate solution (30 ml) and dried over magnesium sulfate and evaporated under reduced pressure to give crude [8-(2-aminoethyl)-6,7-dihydro-MeBmt]-1-[α-methylene-Sar]-3-cyclosporin [Molecular Formula: C₆₅H₁₁₈N₁₂O₁₂; Exact Mass: 1258.90; MS (m/z): 1259.77 (M+1)⁺, 1281.84 (M+Na)⁺; HPLC RT: 13.00 min. (C8 reverse phase column: 250 mm; acetonitrile/water (0.05% trifluoroacetic acid); operation temperature: 64 °C; detector: 210 nm)]. The crude [8-(2-aminoethyl)-6,7-dihydro-MeBmt]-1-[α-methylene-Sar]-3-cyclosporin was dissolved in dichloromethane (10 ml). Acetic acid (0.12 ml, 2.20 mmol), HBTU (0.50 g, 1.32 mmol), 1-hydroxybenzotriazole (0.18 g, 1.32 mmol) and pyridine (1 ml) were added. The mixture was stirred at room temperature for one hour. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 0.30 g of pure [8-(2-acetamidoethyl)-6,7-dihydro-MeBmt]-1-[α-methylene-Sar]-3-cyclosporin [Molecular Formula: C₆₇H₁₂₀N₁₂O₁₃; Exact Mass: 1300.91; MS (m/z): 1301.66 (M+1)⁺, 1323.92 (M+Na)⁺; HPLC RT: 17.41 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-(4-hydroxybutylthio)methyl-Sar]-3-cyclosporin

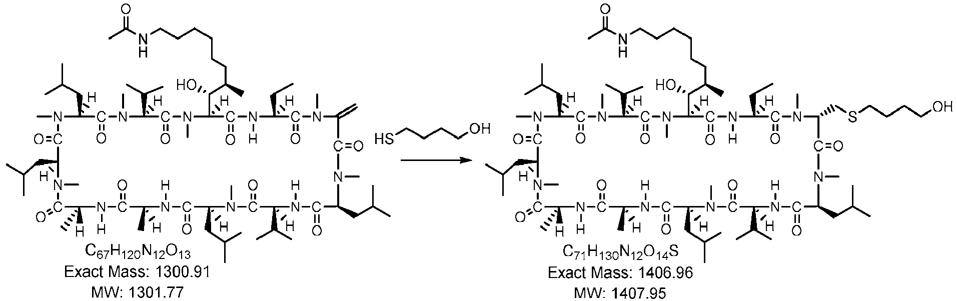

To a solution of [8-(2-acetamidoethyl)-6,7-dihydro-MeBmt]-1-[α-methylene-Sar]-3-cyclosporin (0.30 g, 0.23 mmol) in methanol (10 ml) were added 4-mercapto-1-butanol (0.14 ml, 1.38 mmol) and lithium hydroxide (0.06 g, 2.31 mmol). The reaction mixture was stirred at room temperature for five hours. Most of the methanol was evaporated under reduced pressure. Ethyl acetate (30 ml) and brine (30 ml) were added and the mixture was separated. The organic layer was dried over magnesium sulfate and evaporated under reduced pressure. The residue was purified by chromatography (hexane/acetone from 90/10 to 75/25) to give 14 mg of pure [8-(2-acetamidoethyl)-6,7-dihydro-MeBmt]-1-[(S)-(4-hydroxybutylthio)methyl-Sar]-3-cyclosporin [Molecular Formula: C₇₁H₁₃₀N₁₂O₁₄S; Exact Mass: 1406.95; MS (m/z): 1407.70 (M+1)⁺, 1429.89 (M+Na)⁺; HPLC RT: 15.02 min. (C8 reverse phase column: 250 mm; acetonitrile/water (0.05% trifluoroacetic acid); operation temperature: 64 °C; detector: 210 nm)].

### Example 11

### [8-(2-Hydroxyethyl)-6,7-dihydro-MeBmt]-1-[(S)-(4-hydroxybutylthio)methyl-Sar]-3-cyclosporin

### (4-Ethoxy-4-oxobutyl)triphenylphosphonium bromide

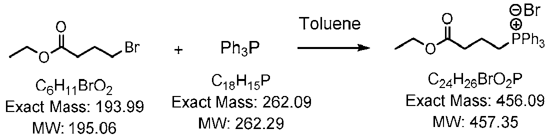

Ethyl 4-bromobutyrate (14.40 ml, 0.10 mol) and triphenylphosphine (26.29 g, 0.10 mol) were added to toluene (200 ml). The mixture was stirred and heated to reflux for twenty four hours. After cooled to room temperature, the mixture was stirred at room temperature for a weekend. The precipitate was filtered off, washed with toluene and hexane and dried in vacuum to give 20.0 g product [Molecular Formula: C₂₄H₂₆O₂P⁺; Exact Mass: 377.17; MS (m/z): 377.20 (M)⁺].

### [8-(2-Ethoxy-2-oxoethyl)-3-acetyl-MeBmt]-1-cyclosporin

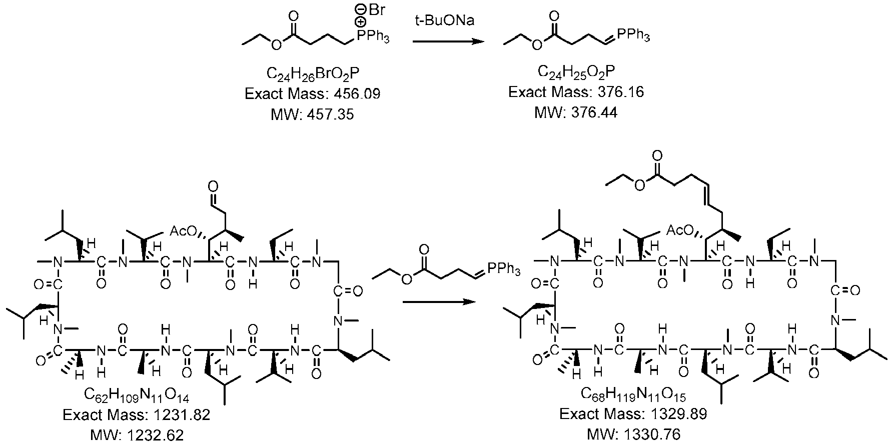

To a dried flask were added (4-ethoxy-4-oxobutyl)triphenylphosphonium bromide (8.88 g, 19.48 mmol) and anhydrous tetrahydrofuran (60 ml) under nitrogen. The reaction mixture was put into an ice-water bath and sodium tert-butoxide (2.18 g, 22.74 mmol) was added. After the mixture was stirred for two hours, a solution of [(3R,4R)-3-aceyloxy-4-methyl-6-oxo-N-MeNle]-1-cyclosporin (4.00 g, 3.25 mmol) in anhydrous tetrahydrofuran (20 ml) was added. The mixture was stirred another 5 hours at 0°C. Most of tetrahydrofuran was evaporated under reduced pressure. Dichloromethane (100 ml) and brine (100 ml) were added and the mixture was separated. The organic layer was dried over magnesium sulfate and evaporated under reduced pressure. The residue was purified by chromatography (dichloromethane/methanol) to give 2.82 g of [8-(2-ethoxy-2-oxoethyl)-3-acetyl-MeBmt]-1-cyclosporin [Molecular Formula: C₆₈H₁₁₉N₁₁O₁₅; Exact Mass: 1329.89; MS (m/z): 1330.51 (M+1)⁺, 1352.69 (M+Na)⁺; HPLC RT: 17.72 min. (C8 reverse phase column: 250 mm; acetonitrile/water (0.05% trifluoroacetic acid); operation temperature: 64 °C; detector: 210 nm)].

### [8-(2-Hydroxyethyl)-MeBmt]-1-cyclosporin

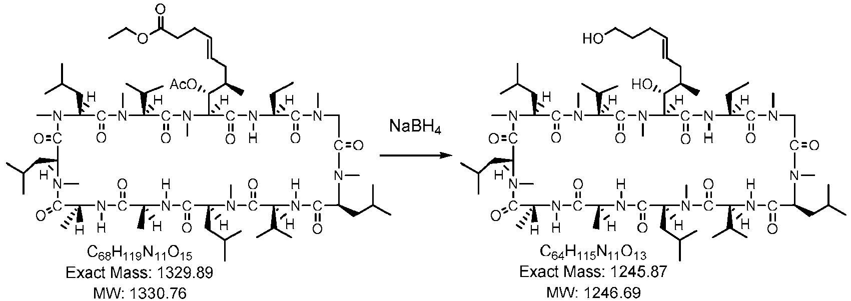

[8-(2-Ethoxy-2-oxoethyl)-3-acetyl-MeBmt]-1-cyclosporin (2.82 g, 2.12 mmol) was dissolved in tetrahydrofuran (40 ml). Sodium borohydride (8.02 g, 212.04 mmol) and cesium chloride (3.56 g, 21.20 mmol) were added. The mixture was stirred at room temperature and methanol (40 ml) was added dropwise in two hours. The mixture was stirred for another three hours. Most of solvents were evaporated under reduced pressure. Then ethyl acetate (100 ml) and water (100 ml) were added and the mixture was separated. The ethyl acetate layer was dried over magnesium sulfate and evaporated under reduced pressure. The residue was purified by chromatography (dichloromethane/methanol) to give 2.70 g of [8-(2-hydroxyethyl)-MeBmt]-1-cyclosporin [Molecular Formula: C₆₄H₁₁₅N₁₁O₁₃; Exact Mass: 1245.87; MS (m/z): 1246.56 (M+1)⁺, 1413.96 (M+Na)⁺; HPLC RT: 14.15 min. (C8 reverse phase column: 250 mm; acetonitrile/water (0.05% trifluoroacetic acid); operation temperature: 64 °C; detector: 210 nm)].

### [8-(2-Hydroxyethyl)-6,7-dihydro-MeBmt]-1-cyclosporin

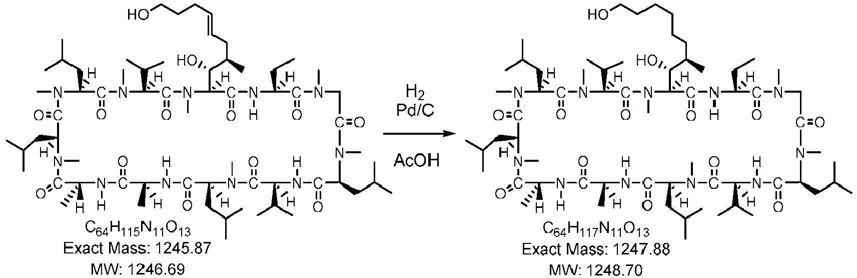

[8-(2-hydroxyethyl)-MeBmt]-1-cyclosporin (2.70 g, 2.17 mmol) was dissolved in methanol (50 ml). Palladium (10 wt% on carbon, 50 mg) and acetic acid (6 drops) were added. The mixture was stirred at room temperature overnight under hydrogen. The mixture was filtered and the filtrate was evaporated under reduced pressure to give 1.50 g of crude [8-(2-hydroxyethyl)-6,7-dihydro-MeBmt]-1-cyclosporin [Molecular Formula: C₆₄H₁₁₇N₁₁O₁₃; Exact Mass: 1247.88; MS (m/z): 1248.66 (M+1)⁺, 1270.77 (M+Na)⁺; HPLC RT: 14.32 min. (C8 reverse phase column: 250 mm; acetonitrile/water (0.05% trifluoroacetic acid); operation temperature: 64 °C; detector: 210 nm)].

### [8-(2-Hydroxyethyl)-6,7-dihydro-MeBmt]-1-[α-methylene-Sar]-3-cyclosporin

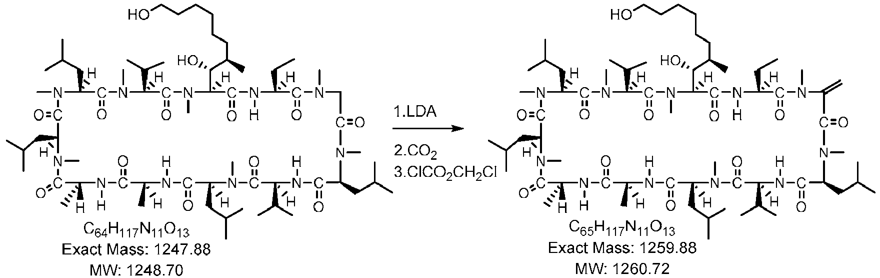

n-Butyllithium (2.65 M, 5.00 ml, 13.23 mmol) was added to a solution of diisopropylamine (1.87 ml, 13.23 mmol) in tetrahydrofuran (40 ml) at -78 °C under nitrogen. After the reaction mixture was stirred for an hour, a solution of [8-(2-hydroxyethyl)-6,7-dihydro-MeBmt]-1-cyclosporin (1.50 g, 1.20 mmol) in tetrahydrofuran (15 ml) was added over ten minutes. The mixture was stirred at -78 °C for two hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (1.07 ml, 12.03 mmol) was added. The mixture was stirred and allowed to warm to room temperature overnight. Brine (5 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.50 g of pure [8-(2-hydroxyethyl)-6,7-dihydro-MeBmt]-1-[α-methylene-Sar]-3-cyclosporin [Molecular Formula: C₆₅H₁₁₇N₁₁O₁₃; Exact Mass: 1259.88; MS (m/z): 1260.61 (M+1)⁺, 1282.78 (M+Na)⁺; HPLC RT: 16.09 min. (C8 reverse phase column: 250 mm; acetonitrile/water (0.05% trifluoroacetic acid); operation temperature: 64 °C; detector: 210 nm)].

### [8-(2-Hydroxyethyl)-6,7-dihydro-MeBmt]-1-[(S)-(4-hydroxybutylthio)methyl-Sar]-3-cyclosporin

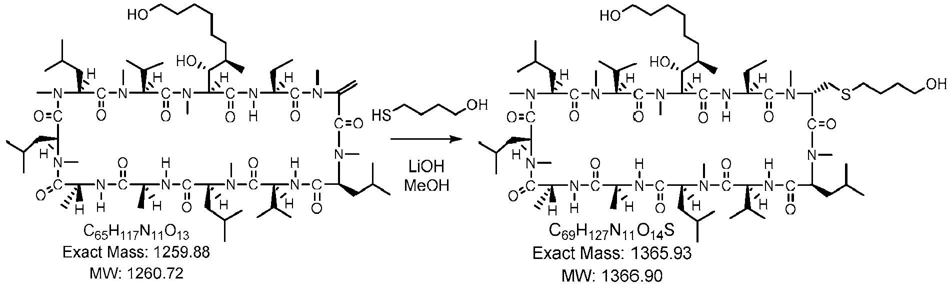

To a solution of [8-(2-hydroxyethyl)-6,7-dihydro-MeBmt]-1-[α-methylene-Sar]-3-cyclosporin (0.50 g, 0.40 mmol) in methanol (10 ml) were added 4-mercapto-1-butanol (0.25 ml, 2.38 mmol) and lithium hydroxide (0.10 g, 4.00 mmol). The reaction mixture was stirred at room temperature for four hours. Most of the methanol was evaporated under reduced pressure. Ethyl acetate (30 ml) and brine (30 ml) were added and the mixture was separated. The organic layer was dried over magnesium sulfate and evaporated under reduced pressure. The residue was purified by chromatography (hexane/acetone) to give 80 mg of pure [8-(2-hydroxyethyl)-6,7-dihydro-MeBmt]-1-[(S)-(4-hydroxybutylthio)methyl-Sar]-3-cyclosporin [Molecular Formula: C₆₉H₁₂₇N₁₁O₁₄S; Exact Mass: 1365.93; MS (m/z): 1366.64 (M+1)⁺, 1388.84 (M+Na)⁺; HPLC RT: 13.94 min. (C8 reverse phase column: 250 mm; acetonitrile/water (0.05% trifluoroacetic acid); operation temperature: 64 °C; detector: 210 nm)].

### Example 12

### [8-(2-(2-Ethoxy-2-oxoethyl)amino)ethyl-6,7-dihydro-MeBmt]-1-[(S)-(4-hydroxybutylthio)methyl-Sar]-3-cyclosporin (12a) and [8-(2-bis(2-ethoxy-2-oxoethyl)amino)ethyl-6,7-dihydro-MeBmt]-1-[(S)-(4-hydroxybutylthio)methyl-Sar]-3-cyclosporin (12b)

### [8-(2-(tert-Butoxycarbonyl)aminoethyl)-6,7-dihydro-MeBmt]-1-[(S)-(4-hydroxybutylthio)methyl-Sar]-3-cyclosporin

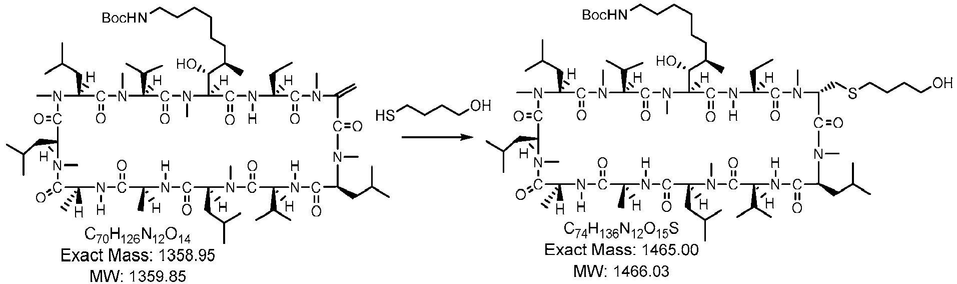

To a solution of [8-(2-(tert-butoxycarbonyl)aminoethyl)-6,7-dihydro-MeBmt]-1-[α-methylene-Sar]-3-cyclosporin (0.25 g, 0.18 mmol) in methanol (15 ml) were added 4-mercapto-1-butanol (0.12 ml, 1.10 mmol) and lithium hydroxide (0.04 g, 1.84 mmol). The reaction mixture was stirred at room temperature overnight. Most of the methanol was evaporated under reduced pressure. Ethyl acetate (30 ml) and brine (30 ml) were added and the mixture was separated. The organic layer was dried over magnesium sulfate and evaporated under reduced pressure. The residue was purified by chromatography (hexane/acetone from 90/10 to 75/25) to give 100 mg of pure [8-(2-(tert-butoxycarbonyl)aminoethyl)-6,7-dihydro-MeBmt]-1-[(S)-(4-hydroxybutylthio)methyl-Sar]-3-cyclosporin [Molecular Formula: C₇₄H₁₃₆N₁₂O₁₅S; Exact Mass: 1465.00; MS (m/z): 1465.53 (M+1)⁺, 1487.84 (M+Na)⁺; HPLC RT: 16.77 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(S)-(4-hydroxybutylthio)methyl-Sar]-3-cyclosporin

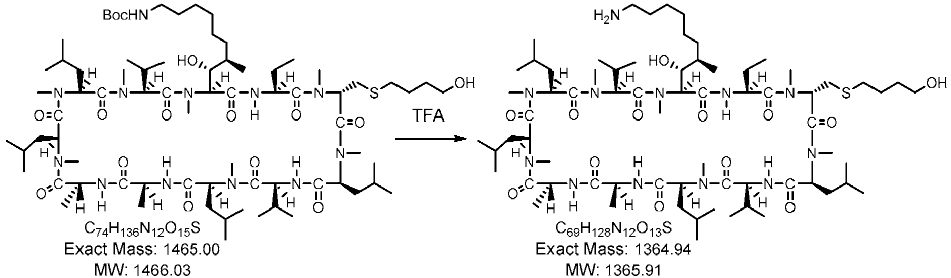

[8-(2-(tert-Butoxycarbonyl)aminoethyl)-6,7-dihydro-MeBmt]-1-[(S)-(4-hydroxybutyl thio)methyl -Sar]-3-cyclosporin (0.10 g, 0.07 mmol) was dissolved in dichloromethane (8 ml) and put into ice-water bath. Trifluoroacetic acid (4 ml) was added. The mixture was stirred at 0 °C for two hours. Another dichloromethane (10 ml) was added. The mixture was washed with brine (30 ml), saturated sodium bicarbonate solution (30 ml), dried over magnesium sulfate and evaporated under reduced pressure to give crude [8-(2-aminoethyl)-6,7-dihydro-MeBmt]-1-[(S)-(4-hydroxybutylthio)methyl-Sar]-3-cyclosporin [Molecular Formula: C₆₉H₁₂₈N₁₂O₁₃S; Exact Mass: 1364.94; MS (m/z): 1365.75 (M+1)⁺; HPLC RT: 10.57 min. (C8 reverse phase column: 250 mm; acetonitrile/water (0.05% trifluoroacetic acid); operation temperature: 64 °C; detector: 210 nm)].

### [8-(2-(2-Ethoxy-2-oxoethyl)amino)ethyl-6,7-dihydro-MeBmt]-1-[(S)-(4-hydroxybutylthio)methyl-Sar]-3-cyclosporin (12a) and [8-(2-bis(2-ethoxy-2-oxoethyl)amino)ethyl-6,7-dihydro-MeBmt]-1-[(S)-(4-hydroxybutylthio)methyl-Sar]-3-cyclosporin (12b)

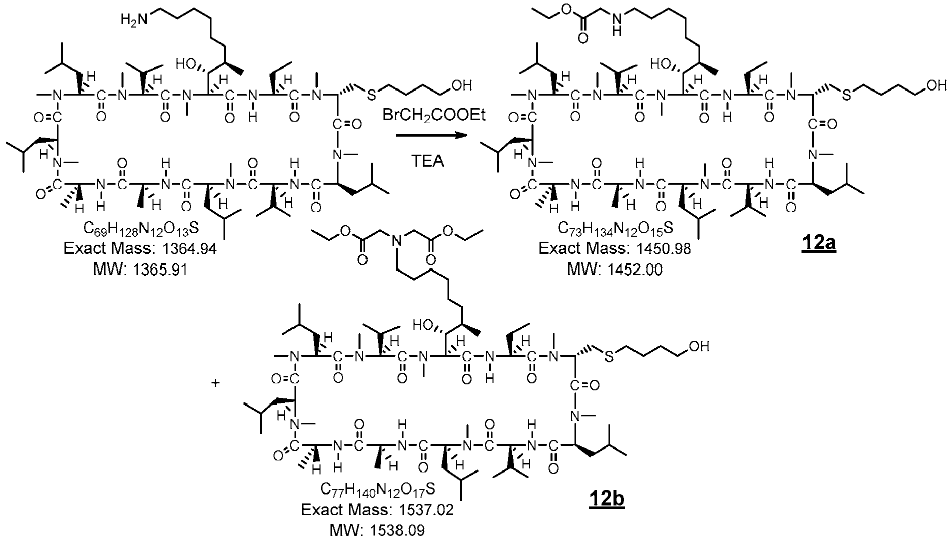

Crude [8-(2-aminoethyl)-6,7-dihydro-MeBmt]-1-[(S)-(4-hydroxybutylthio)methyl-Sar]-3-cyclosporin from last step was dissolved in dichloromethane (10 ml). Ethyl bromoacetate (48 mg, 0.34 mmol), and triethylamine (0.50 ml) were added. The mixture was stirred and heated to reflux for three hours. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 10 mg of [8-(2-((2-ethoxy-2-oxoethyl)amino)ethyl)-6,7-dihydro-MeBmt]-1-[(S)-(4-hydroxybutylthio)methyl-Sar]-3-cyclosporin [Molecular Formula: C₇₃H₁₃₄N₁₂O₁₅S; Exact Mass: 1450.98; MS (m/z): 1451.70 (M+1)⁺, 1473.87 (M+Na)⁺; HPLC RT: 12.23 min. (C8 reverse phase column: 250 mm; acetonitrile/water (0.05% trifluoroacetic acid); operation temperature: 64 °C; detector: 210 nm)] and 30 mg of [8-(2-(bis(2-ethoxy-2-oxoethyl)amino)ethyl)-6,7-dihydro-MeBmt]-1-[(S)-(4-hydroxybutylthio)methyl-Sar]-3-cyclosporin [Molecular Formula: C₇₇H₁₄₀N₁₂O₁₇S; Exact Mass: 1537.02; MS (m/z): 1337.70 (M+1)⁺, 1559.90 (M+Na)⁺; HPLC RT: 14.42 min. (C8 reverse phase column: 250 mm; acetonitrile/water (0.05% trifluoroacetic acid); operation temperature: 64 °C; detector: 210 nm)].

### Example 13

### [8-(2-(Carboxymethyl)amino)ethyl-6,7-dihydro-MeBmt]-1-[(S)-(4-hydroxybutylthio)methyl-Sar]-3-cydosporin

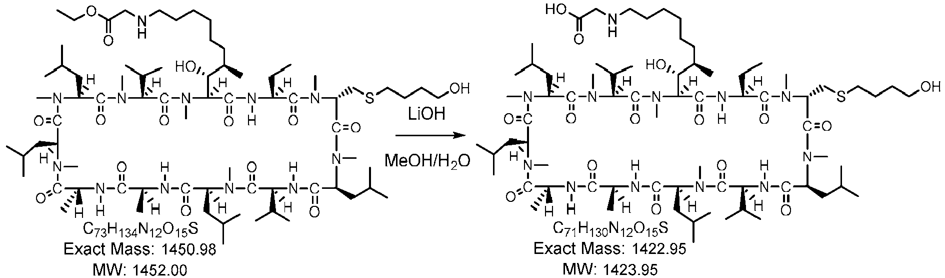

[8-(2-(2-Ethoxy-2-oxoethyl)amino)ethyl-6,7-dihydro-MeBmt]-1-[(S)-(4-hydroxybutyl thio)methyl-Sar]-3-cyclosporin (10 mg, 6.9 × 10⁻³ mmol) was dissolved in methanol (3 ml). Lithium hydroxide (2 mg, 0.08 mmol) and water (3 ml) were added. The mixture was stirred at room temperature for one hour. Then most of the methanol was evaporated under reduced pressure. Ethyl acetate (10 ml) and brine (10 ml) were added and the PH of the aqueous layer was adjusted to 3 by adding hydrochloric acid solution (1.0 N). After separated, the ethyl acetate layer was dried over magnesium sulfate and evaporated under reduced pressure. The residue was purified by chromatography to give [8-(2-((carboxymethyl)amino)ethyl)-6,7-dihydro-MeBmt]-1-[(S)-(4-hydroxybutylthio)methyl-Sar]-3-cyclosporin [Molecular Formula: C₇₁H₁₃₀N₁₂O₁₅S; Exact Mass: 1422.95; MS (m/z): 1423.59 (M+1)⁺, 1445.84 (M+Na)⁺; HPLC RT: 10.41 min. (C8 reverse phase column: 250 mm; acetonitrile/water (0.05% trifluoroacetic acid); operation temperature: 64 °C; detector: 210 nm)]

### Example 14

### [8-(2-Bis(carboxymethyl)amino)ethyl-6,7-dihydro-MeBmt]-1-[(S)-(4-hydroxybutylthio)methyl-Sar]-3-cyclosporin

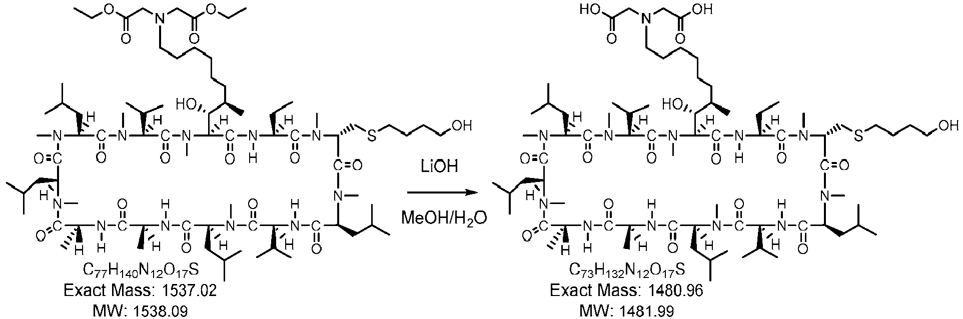

[8-(2-Bis(2-ethoxy-2-oxoethyl)amino)ethyl-6,7-dihydro-MeBmt]-1-[(S)-(4-hydroxybutylthio) methyl-Sar]-3-cyclosporin (30 mg, 0.02 mmol) was dissolved in methanol (3 ml). Lithium hydroxide (6 mg, 0.23 mmol) and water (3 ml) were added. The mixture was stirred at room temperature for one hour. Then most of the methanol was evaporated under reduced pressure. Ethyl acetate (10 ml) and brine (10 ml) were added and the PH of the aqueous layer was adjusted to 3 by adding hydrochloric acid solution (1.0 N). After separated, the ethyl acetate layer was dried over magnesium sulfate and evaporated under reduced pressure. The residue was purified by chromatography to give [8-(2-(bis(carboxymethyl)amino)ethyl)-6,7-dihydro-MeBmt]-1-[(S)-(4-hydroxybutylthio)methyl-Sar]-3-cyclosporin [Molecular Formula: C₇₃H₁₃₂N₁₂O₁₇S; Exact Mass: 1480.96; MS (m/z): 1481.70 (M+1)⁺, 1503.87 (M+Na)⁺; HPLC RT: 9.88 min. (C8 reverse phase column: 250 mm; acetonitrile/water (0.05% trifluoroacetic acid); operation temperature: 64 °C; detector: 210 nm)].

### Example 15

### [8-(2-(4-(Methoxycarbonyl)benzyl)amino)ethyl-6,7-dihydro-MeBmt]-1-[(S)-(4-hydroxybutylthio)methyl-Sar]-3-cyclosporin

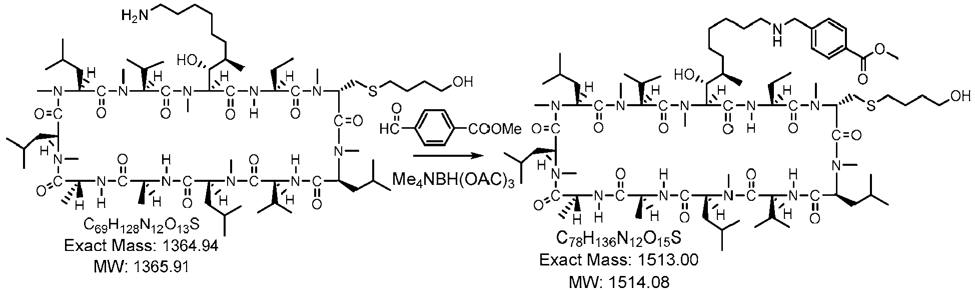

[8-(2-Aminoethyl)-6,7-dihydro-MeBmt]-1-[(S)-(4-hydroxybutylthio)methyl-Sar]-3-cyclosporin (0.50 g, 0.37 mmol) was dissolved in dichloromethane (50 ml). 4-Methyl 4-formylbenzoate (0.08 g, 0.46 mmol), tetramethylammonium triacetoxyborohydride (0.12 g, 0.46 mmol) and acetic acid (5 drops) were added. The mixture was stirred at room temperature for one hour. Then dichloromethane (30 ml) and saturated sodium bicarbonate solution (50 ml) were added and separated. The dichloromethane layer was dried over magnesium sulfate and evaporated under reduced pressure. The residue was purified by chromatography (dichloromethane/methanol) to give 140 mg of [8-(2-(4-(methoxycarbonyl)benzyl)amino)ethyl-6,7-dihydro-MeBmt]-1-[(S)-(4-hydroxybutylthio)methyl-Sar]-3-cyclosporin [Molecular Formula: C₇₈H₁₃₆N₁₂O₁₅S; Exact Mass: 1513.00; MS (m/z): 1513.77 (M+1)⁺, 1535.80 (M+Na)⁺; HPLC RT: 13.40 min. (C8 reverse phase column: 250 mm; acetonitrile/water (0.05% trifluoroacetic acid); operation temperature: 64 °C; detector: 210 nm)].

### Example 16

### [8-(2-(4-Carboxybenzyl)amino)ethyl)-6,7-dihydro-MeBmt]-1-[(S)-(4-hydroxybutylthio)methyl-Sar]-3-cyclosporin

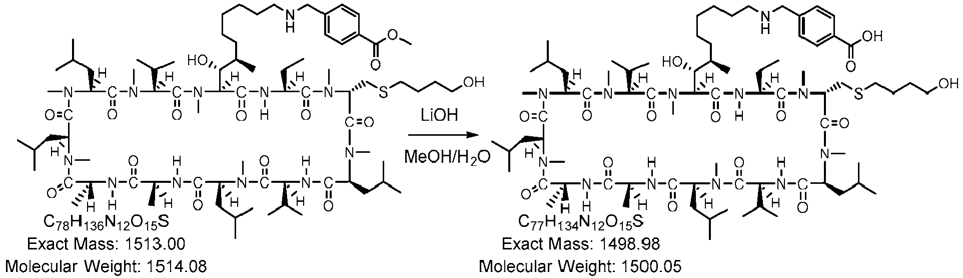

[8-(2-(4-(Methoxycarbonyl)benzyl)amino)ethyl-6,7-dihydro-MeBmt]-1-[(S)-(4-hydroxy butylthio)methyl-Sar]-3-cyclosporin (0.14 g, 0.09 mmol) was dissolved in methanol (5 ml). Lithium hydroxide (0.01 g, 0.40 mmol) and water (5 ml) were added. The mixture was stirred at room temperature for two hours. Then most of the methanol was evaporated under reduced pressure. Ethyl acetate (10 ml) and brine (10 ml) were added and the PH of the aqueous layer was adjusted to 3 by adding hydrochloric acid solution (1.00 N). After separated, the ethyl acetate layer was dried over magnesium sulfate and evaporated under reduced pressure. The residue was purified by chromatography to give [8-(2-(4-carboxybenzyl)amino)ethyl-6,7-dihydro-MeBmt]-1-[(S)-(4-hydroxybutylthio)methyl-Sar]-3-cyclosporin [Molecular Formula: C₇₇H₁₃₄N₁₂O₁₅S; Exact Mass: 1498.98; MS (m/z): 1499.62 (M+1)⁺, 1521.77 (M+Na)⁺; HPLC RT: 11.76 min. (C8 reverse phase column: 250 mm; acetonitrile/water (0.05% trifluoroacetic acid); operation temperature: 64 °C; detector: 210 nm)].

### Example 17

### [8-(2-(4-(Dimethylcarbamoyl))benzyl)amino)ethyl-6,7-dihydro-MeBmt]-1-[(S)-(4-hydroxybutylthio)methyl-Sar]-3-cyclosporin

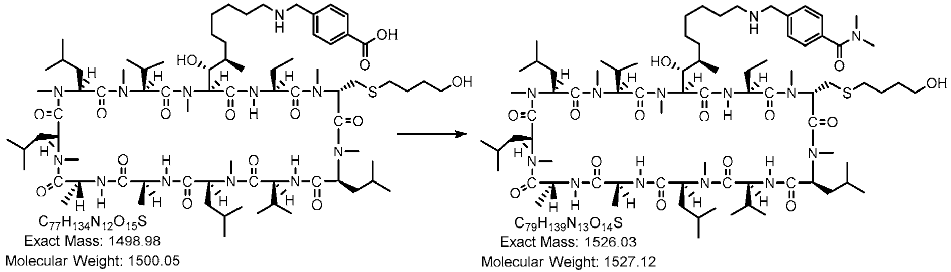

[8-(2-(4-Carboxybenzyl)amino)ethyl-6,7-dihydro-MeBmt]-1-[(S)-(4-hydroxybutylthio)methyl-Sar]-3-cyclosporin (0.13 g, 0.09 mmol) was dissolved in dichloromethane (10 ml). Dimethylamine hydrochloride (0.04, 0.45 mmol), HBTU (0.10 g, 0.27 mmol), 1-hydroxybenzotriazole (0.04 g, 0.27 mmol) and triethylamine (0.5 ml) were added. The mixture was stirred at room temperature for two hours. Then dichloromethane (30 ml) and brine (50 ml) were added and separated. The dichloromethane layer was dried over magnesium sulfate and evaporated under reduced pressure. The residue was purified by chromatography to give [8-(2-(4-(dimethylcarbamoyl))benzyl)amino)ethyl-6,7-dihydro-MeBmt]-1-[(S)-(4-hydroxybutylthio) methyl-Sar]-3-cyclosporin [Molecular Formula: C₇₉H₁₃₉N₁₃O₁₄S; Exact Mass: 1526.03; MS (m/z): 1526.72 (M+1)⁺, 1548.84 (M+Na)⁺; HPLC RT: 11.90 min. (C8 reverse phase column: 250 mm; acetonitrile/water (0.05% trifluoroacetic acid); operation temperature: 64 °C; detector: 210 nm)].

### Example 18

### [8-(2-Bis(4-(methoxycarbonyl)benzyl)amino))ethyl-6,7-dihydro-MeBmt]-1-[(S)-(4-hydroxybutylthio)methyl-Sar]-3-cyclosporin

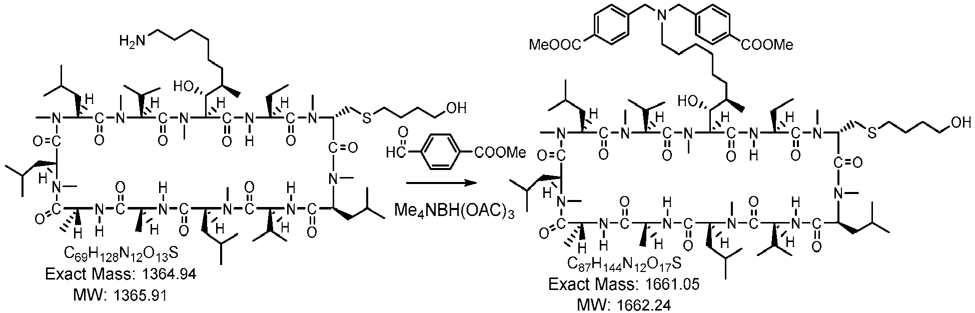

[8-(2-Aminoethyl)-6,7-dihydro-MeBmt]-1-[(S)-(4-hydroxybutylthio)methyl-Sar]-3-cyclosporin (0.50 g, 0.37 mmol) was dissolved in dichloromethane (50 ml). 4-Methyl 4-formylbenzoate (0.30 g, 1.83 mmol), tetramethylammonium triacetoxyborohydride (0.48 g, 1.83 mmol) and acetic acid (10 drops) were added. The mixture was stirred at room temperature for one hour. Then dichloromethane (30 ml) and saturated sodium bicarbonate solution (50 ml) were added and separated. The dichloromethane layer was dried over magnesium sulfate and evaporated under reduced pressure. The residue was purified by chromatography (dichloromethane/methanol) to give 174 mg of [8-(2-bis(4-(methoxycarbonyl)benzyl)amino))ethyl-6,7-dihydro-MeBmt]-1-[(S)-(4-hydroxybutylthio)methyl-Sar]-3-cyclosporin [Molecular Formula: C₈₇H₁₄₄N₁₂O₁₇S; Exact Mass: 1661.05; MS (m/z): 1661.66 (M+1)⁺, 1684.06 (M+Na)⁺; HPLC RT: 15.32 min. (C8 reverse phase column: 250 mm; acetonitrile/water (0.05% trifluoroacetic acid); operation temperature: 64 °C; detector: 210 nm)].

### Example 19

### [8-(2-(Bis(4-carboxybenzyl)amino)ethyl)-6,7-dihydro-MeBmt]-1-[(S)-(4-hydroxybutylthio)methyl-Sar]-3-cyclosporin

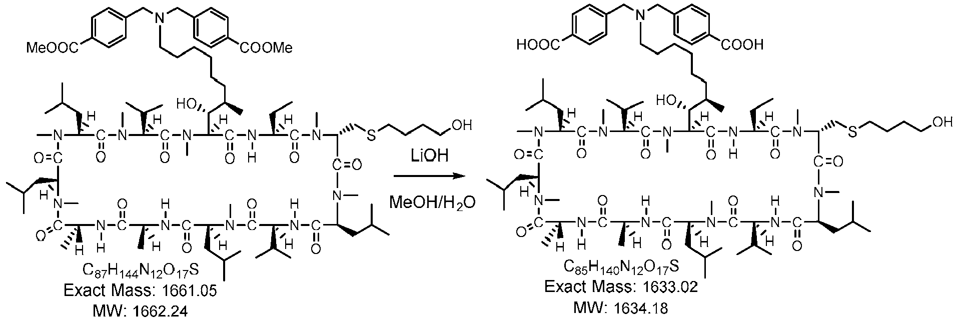

[8-(2-Bis(4-(methoxycarbonyl)benzyl)amino))ethyl-6,7-dihydro-MeBmt]-1-[(S)-(4-hydroxybutylthio)methyl-Sar]-3-cyclosporin (174 mg, 0.10 mmol) was dissolved in methanol (5 ml). Lithium hydroxide (12 mg, 0.46 mmol) and water (5 ml) were added. The mixture was stirred at room temperature for two hours. Then most of the methanol was evaporated under reduced pressure. Ethyl acetate (10 ml) and brine (10 ml) were added and the PH of the aqueous layer was adjusted to 3 by adding hydrochloric acid solution (1.00 N). After separated, the ethyl acetate layer was dried over magnesium sulfate and evaporated to give 100 mg of [8-(2-(bis(4-carboxybenzyl)amino)ethyl)-6,7-dihydro-MeBmt]-1-[(S)-(4-hydroxybutylthio)methyl-Sar]-3-cyclosporin [Molecular Formula: C₈₅H₁₄₀N₁₂O₁₇S; Exact Mass: 1633.02; MS (m/z): 1633.56 (M+1)⁺, 1655.68 (M+Na)⁺; HPLC RT: 11.64 min. (C8 reverse phase column: 250 mm; acetonitrile/water (0.05% trifluoroacetic acid); operation temperature: 64 °C; detector: 210 nm)].

### Example 20

### [8-(2-Bis(4-(dimethylcarbamoyl))benzyl)amino)ethyl-6,7-dihydro-MeBmt]-1-[(S)-(4-hydroxybutylthio)methyl-Sar]-3-cyclosporin

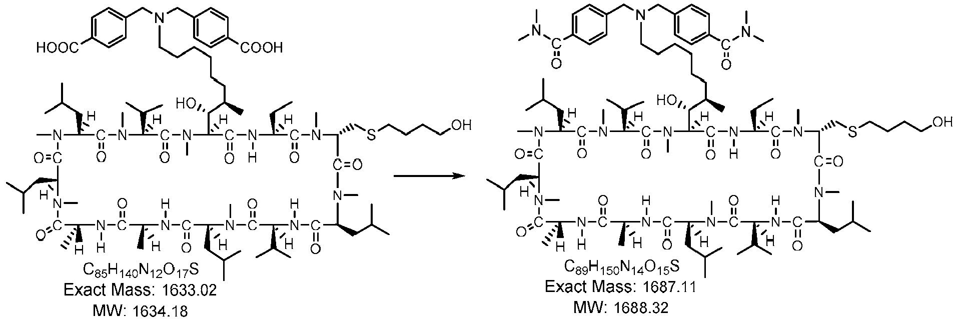

[8-(2-(Bis(4-carboxybenzyl)amino)ethyl)-6,7-dihydro-MeBmt]-1-[(S)-(4-hydroxybutylthio) methyl-Sar]-3-cyclosporin (0.10 g, 0.06 mmol) was dissolved in dichloromethane (10 ml). Dimethylamine hydrochloride (0.03, 0.37 mmol), HBTU (0.12 g, 0.31 mmol), 1-hydroxybenzotriazole (0.04 g, 0.31 mmol) and triethylamine (0.5 ml) were added. The mixture was stirred at room temperature for two hours. Then dichloromethane (30 ml) and brine (50 ml) were added and separated. The dichloromethane layer was dried over magnesium sulfate and evaporated under reduced pressure. The residue was purified by chromatography to give [8-(2-(Bis(4-(dimethylcarbamoyl))benzyl)amino)ethyl)-6,7-dihydro-MeBmt]-1-[(S)-(4-hydroxybutyl thio)methyl-Sar]-3-cyclosporin [Molecular Formula: C₈₉H₁₅₀N₁₄O₁₅S; Exact Mass: 1687.11; MS (m/z): 1687.73 (M+1)⁺, 1709.88 (M+Na)⁺; HPLC RT: 12.44 min. (C8 reverse phase column: 250 mm; acetonitrile/water (0.05% trifluoroacetic acid); operation temperature: 64 °C; detector: 210 nm)].

### Example 21

### [8-(2-(3-Carboxybenzamido)ethyl)-6,7-dihydro-MeBmt]-1-[(S)-(4-hydroxybutylthio)methyl-Sar]-3-cyclosporin

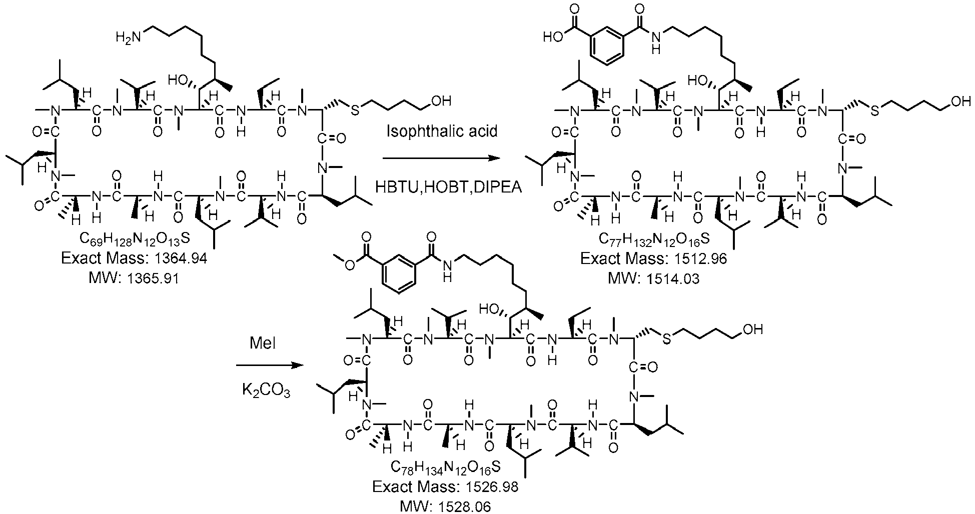

[8-(2-aminoethyl)-6,7-dihydro-MeBmt]-1-[(S)-(4-hydroxybutylthio)methyl-Sar]-3-cyclosporin (0.19 g, 0.14 mmol) was dissolved in dichloromethane (8 ml). Isophthalic acid (0.07 g, 0.41 mmol), HBTU (0.16 g, 0.41 mmol), 1-hydroxybenzotriazole (0.06 g, 0.41 mmol) and N-ethyldiisopropylamine (0.50 ml) were added. The mixture was stirred at room temperature for two hours. Then dichloromethane (50 ml) and brine (50 ml) were added. The PH of the aqueous layer was adjusted to 4 by adding hydrochloric acid solution (1.0 N). After the mixture was separated, the dichloromethane layer was dried over magnesium sulfate and evaporated under reduced pressure to give crude [8-(2-(3-carboxybenzamido)ethyl)-6,7-dihydro-MeBmt]-1-[(S)-(4-hydroxybutylthio)methyl-Sar]-3-cyclosporin [Molecular Formula: C₇₇H₁₃₂N₁₂O₁₆S; Exact Mass: 1512.96; MS (m/z): 1513.66 (M+1)⁺, 1535.80 (M+Na)⁺; HPLC RT: 14.15 min. (C8 reverse phase column: 250 mm; acetonitrile/water (0.05% trifluoroacetic acid); operation temperature: 64 °C; detector: 210 nm)].

Crude [8-(2-(3-carboxybenzamido)ethyl)-6,7-dihydro-MeBmt]-1-[(S)-(4-hydroxybutyl thio)methyl-Sar]-3-cyclosporin was dissolved in acetone (10 ml). Iodomethane (0.06 g, 0.41mmol) and potassium carbonate (0.06 g, 0.41 mmol) were added. The mixture was stirred at room temperature for two hours. Then most of solvent was evaporated. Ethyl acetate (50 ml) and brine (50 ml) were added and the mixture was separated. The ethyl acetate layer was dried over magnesium sulfate and evaporated under reduced pressure. The residue was purified by column (hexane/acetone) to give 44 mg of [8-(2-(3-(methoxycarbonyl)benzamido)ethyl)-6,7-dihydro-MeBmt]-1-[(S)-(4-hydroxybutylthio)methyl-Sar]-3-cyclosporin. [Molecular Formula: C₇₈H₁₃₄N₁₂O₁₆S; Exact Mass: 1526.98; MS (m/z): 1527.62 (M+1)⁺, 1549.81 (M+Na)⁺; HPLC RT: 16.08 min. (C8 reverse phase column: 250 mm; acetonitrile/water (0.05% trifluoroacetic acid); operation temperature: 64 °C; detector: 210 nm)].

### Example 22

### [8-(2-(3-Carboxybenzamido)ethyl)-6,7-dihydro-MeBmt]-1-[(S)-(4-hydroxybutylthio)methyl-Sar]-3-cyclosporin

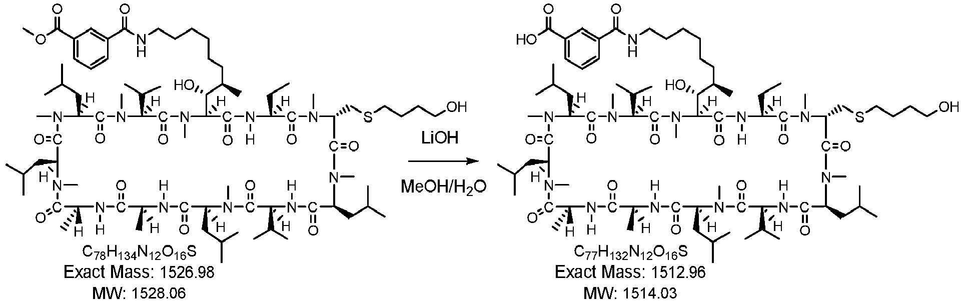

[8-(2-(3-(Methoxycarbonyl)benzamido)ethyl)-6,7-dihydro-MeBmt]-1-[(S)-(4-hydroxy butylthio) methyl-Sar]-3-cyclosporin (44 mg, 0.03 mmol) was dissolved in methanol (3 ml). Lithium hydroxide (20 mg, 0.83 mmol) and water (3 ml) were added. The mixture was stirred at room temperature for two hours. Then most of the methanol was evaporated under reduced pressure. Ethyl acetate (10 ml) and brine (10 ml) were added and the PH of the aqueous layer was adjusted to 4 by adding hydrochloric acid solution (1.0 N). After the mixture separated, the ethyl acetate layer was dried over magnesium sulfate and evaporated under reduced pressure. The residue was purified by chromatography (C8 reverse phase column) to give 16 mg of [8-(2-(3-carboxybenzamido)ethyl)-6,7-dihydro-MeBmt]-1-[(S)-(4-hydroxybutylthio)methyl-Sar]-3-cyclosporin [Molecular Formula: C₇₇H₁₃₂N₁₂O₁₆S; Exact Mass: 1512.96; MS (m/z): 1513.66 (M+1)⁺, 1535.80 (M+Na)⁺; HPLC RT: 14.15 min. (C8 reverse phase column: 250 mm; acetonitrile/water (0.05% trifluoroacetic acid); operation temperature: 64 °C; detector: 210 nm)].

### Example 23

### [8-(2-(4-(Methoxycarbonyl)benzamido)ethyl)-6,7-dihydro-MeBmt]-1-[(S)-(4-hydroxybutylthio)methyl-Sar]-3-cyclosporin

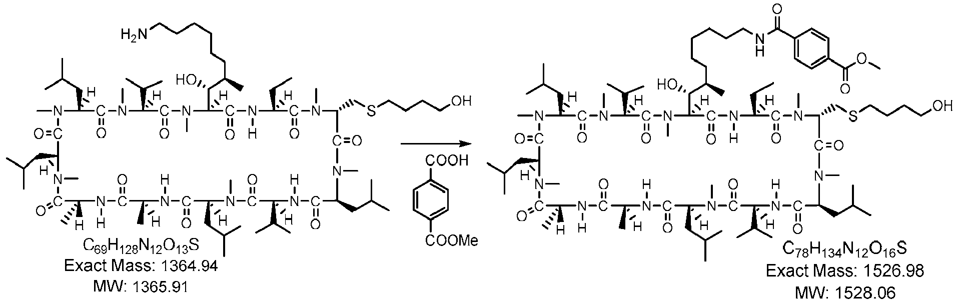

[8-(2-Aminoethyl)-6,7-dihydro-MeBmt]-1-[(S)-(4-hydroxybutylthio)methyl-Sar]-3-cyclosporin (0.60 g, 0.44 mmol) was dissolved in dichloromethane (30 ml). Methyl hydrogen terephthalate (0.24 g, 1.32 mmol), HBTU (0.52 g, 1.32 mmol), 1-hydroxybenzotriazole (0.16 g, 1.32 mmol) and N,N-diisopropylethylamine (3.0 ml) were added. The mixture was stirred at room temperature for two hours. Then dichloromethane (100 ml) and brine (100 ml) were added and separated. The dichloromethane layer was dried over magnesium sulfate and evaporated under reduced pressure. The residue was purified by chromatography to give 0.34 g of [8-(2-(4-(methoxycarbonyl)benzamido)ethyl)-6,7-dihydro-MeBmt]-1-[(S)-(4-hydroxybutylthio)methyl-Sar]-3-cyclosporin [Molecular Formula: C₇₈H₁₃₄N₁₂O₁₆S; Exact Mass: 1526.98; MS (m/z): 1527.69 (M+1)⁺, 1549.95 (M+Na)⁺; HPLC RT: 16.08 min. (C8 reverse phase column: 250 mm; acetonitrile/water (0.05% trifluoroacetic acid); operation temperature: 64 °C; detector: 210 nm)].

### Example 24

### [8-(2-(4-Carboxybenzamido)ethyl)-6,7-dihydro-MeBmt]-1-[(S)-(4-hydroxybutylthio)methyl-Sar]-3-cyclosporin

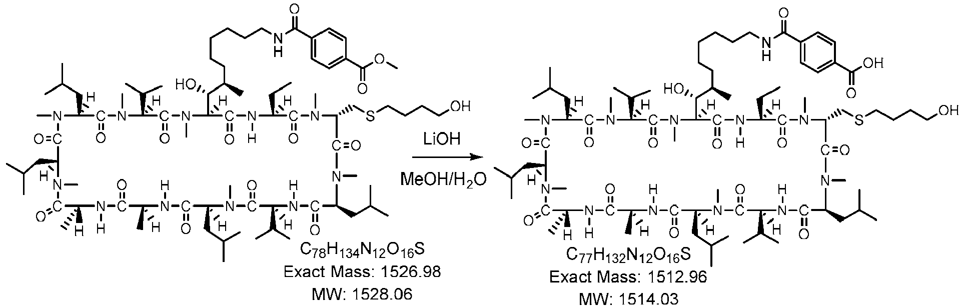

[8-(2-(4-(Methoxycarbonyl)benzamido)ethyl)-6,7-dihydro-MeBmt]-1-[(S)-(4-hydroxybutylthio) methyl-Sar]-3-cyclosporin (0.34 mg, 0.24 mmol) was dissolved in methanol (5 ml). Lithium hydroxide (20 mg, 0.83 mmol) and water (5 ml) were added. The mixture was stirred at room temperature for two hours. Then most of the methanol was evaporated under reduced pressure. Ethyl acetate (20 ml) and brine (20 ml) were added and the PH of the aqueous layer was adjusted to 3 by adding hydrochloric acid solution (1.00 N). After separated, the ethyl acetate layer was dried over magnesium sulfate and evaporated under reduced pressure. The residue was purified by chromatography (C18 reverse phase column) to give 0.22 g of [8-(2-(4-carboxybenzamido)ethyl)-6,7-dihydro-MeBmt]-1-[(S)-(4-hydroxybutylthio)methyl-Sar]-3-cyclosporin [Molecular Formula: C₇₇H₁₃₂N₁₂O₁₆S; Exact Mass: 1512.96; MS (m/z): 1513.66 (M+1)⁺, 1535.80 (M+Na)⁺; HPLC RT: 13.98 min. (C8 reverse phase column: 250 mm; acetonitrile/water (0.05% trifluoroacetic acid); operation temperature: 64 °C; detector: 210 nm)].

### Example 25

### [8-(2-(4-(Dimethylcarbamoyl)benzamido)ethyl)-6,7-dihydro-MeBmt]-1-[(S)-(4-hydroxybutylthio)methyl-Sar]-3-cyclosporin

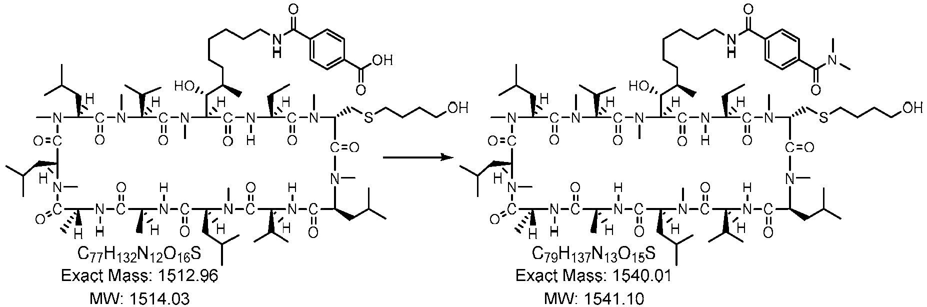

[8-(2-(4-Carboxybenzamido)ethyl)-6,7-dihydro-MeBmt]-1-[(S)-(4-hydroxybutylthio)methyl-Sar]-3-cyclosporin (0.22 g, 0.15 mmol) was dissolved in dichloromethane (5 ml). Dimethylamine hydrochloride (0.04, 0.49 mmol), HBTU (0.17 g, 0.45 mmol), 1-hydroxybenzotriazole (0.06 g, 0.45 mmol) and pyridine (0.5 ml) were added. The mixture was stirred at room temperature overnight. Then dichloromethane was evaporated under reduced pressure. Ethyl acetate (50 ml) and brine (50 ml) were added and separated. The dichloromethane layer was dried over magnesium sulfate and evaporated under reduced pressure. The residue was purified by chromatography to give [8-(2-(4-(dimethylcarbamoyl) benzamido)ethyl)-6,7-dihydro-MeBmt]-1-[(S)-(4-hydroxybutylthio)methyl-Sar]-3-cyclosporin [Molecular Formula: C₇₉H₁₃₇N₁₃O₁₅S; Exact Mass: 1540.01; MS (m/z): 1540.89 (M+1)⁺, 1563.06 (M+Na)⁺; HPLC RT: 14.54 min. (C8 reverse phase column: 250 mm; acetonitrile/water (0.05% trifluoroacetic acid); operation temperature: 64 °C; detector: 210 nm)].

### Example 26

### [8-(2-(2-Hydroxyacetamido)ethyl)-6,7-dihydro-MeBmt]-1-[(S)-(4-hydroxybutylthio)methyl-Sar]-3-cyclosporin

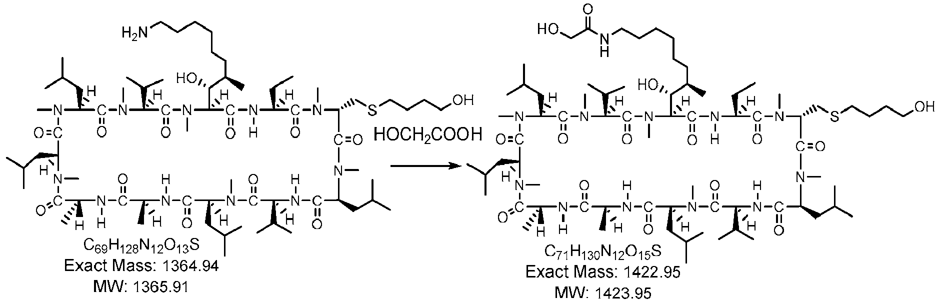

[8-(2-Aminoethyl)-6,7-dihydro-MeBmt]-1-[(S)-(4-hydroxybutylthio)methyl-Sar]-3-cyclosporin (0.26 g, 0.19 mmol) was dissolved in dichloromethane (20 ml). Glycolic acid (0.04 g, 0.53 mmol), HBTU (0.22 g, 0.58 mmol), 1-hydroxybenzotriazole (0.08 g, 0.58 mmol) and pyridine (1.00 ml) were added. The mixture was stirred at room temperature for two hours. Then dichloromethane (30 ml) and brine (50 ml) were added and separated. The dichloromethane layer was dried over magnesium sulfate and evaporated under reduced pressure. The residue was purified by chromatography (dichloromethane/methanol) to give pure [8-(2-(2-hydroxyacetamido)ethyl)-6,7-dihydro-MeBmt]-1-[(S)-(4-hydroxybutylthio)methyl-Sar]-3-cyclosporin [Molecular Formula: C₇₁H₁₃₀N₁₂O₁₅S; Exact Mass: 1422.95; MS (m/z): 1423.80 (M+1)⁺, 1445.95 (M+Na)⁺; HPLC RT: 12.73 min. (C8 reverse phase column: 250 mm; acetonitrile/water (0.05% trifluoroacetic acid); operation temperature: 64 °C; detector: 210 nm)].

### Example 27

### [8-(2-Acetamidoethyl)-6,7-dihydro-MeBmt]-1-[(R)-(4-acetoxybutoxy)methyl-Sar]-3-cyclosporin (26a)

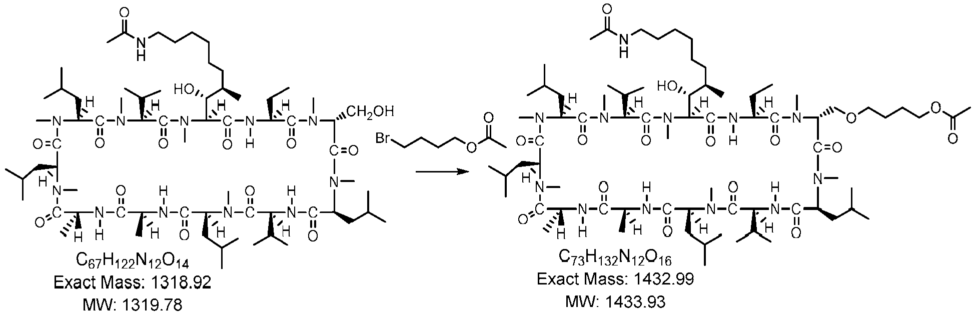

[8-(2-Acetamidoethyl)-6,7-dihydro-MeBmt]-1-[(R)-hydroxymethyl-Sar]-3-cyclosporin (0.14 g, 0.11 mmol) was dissolved in benzene (10 ml). 4-Bromobutyl acetate (0.21 g, 1.06 mmol), tetra-n-butylammonium bromide (0.34 g, 1.06 mmol), tetramethylammonium hydroxide pentahydrate (0.19 g, 1.06 mmol) and sodium hydroxide solution (2.0 ml, 45%) were added. The mixture was stirred at 50 °C overnight. Then ethyl acetate (50 ml) and brine (50 ml) were added and separated. The ethyl acetate layer was dried over magnesium sulfate and evaporated under reduced pressure. The residue was purified by chromatography (dichloromethane/methanol) to give 50 mg of pure [8-(2-acetamidoethyl)-6,7-dihydro-MeBmt]-1-[(R)-(4-acetoxybutoxy)methyl-Sar]-3-cyclosporin [Molecular Formula: C₇₃H₁₃₂N₁₂O₁₆; Exact Mass: 1432.99; MS (m/z): 1433.73 (M+1)⁺, 1455.99 (M+Na)⁺; HPLC RT: 17.43 min. (C8 reverse phase column: 250 mm; acetonitrile/water (0.05% trifluoroacetic acid); operation temperature: 64 °C; detector: 210 nm)].

### Example 28

### [8-(2-Acetamidoethyl)-6,7-dihydro-MeBmt]-1-[(R)-(4-hydroxybutoxy)methyl-Sar]-3-cyclosporin

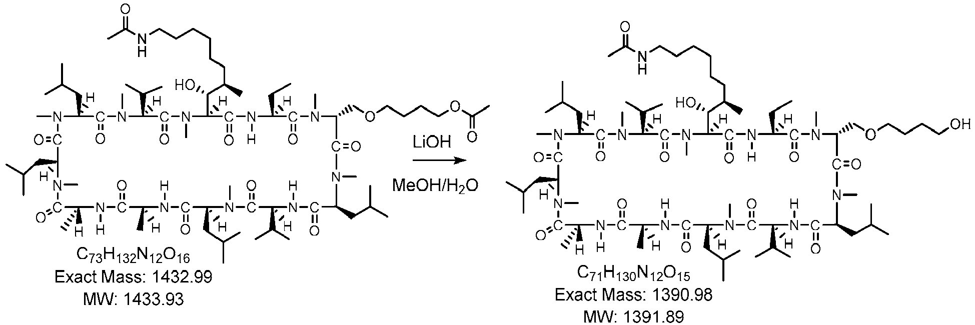

[8-(2-Acetamidoethyl)-6,7-dihydro-MeBmt]-1-[(R)-(4-acetoxybutoxy)methyl-Sar]-3-cyclosporin (50 mg, 0.03 mmol) was dissolved in methanol (5 ml). Lithium hydroxide (20 mg, 0.83 mmol) and water (5 ml) were added. The mixture was stirred at room temperature for two hours. Then most of the methanol was evaporated under reduced pressure. Ethyl acetate (20 ml) and brine (30 ml) were added and the PH of the aqueous layer was adjusted to 3 by adding hydrochloric acid solution (1.00 N). After separated, the ethyl acetate layer was dried over magnesium sulfate and evaporated under reduced pressure. The residue was purified by C18 reverse phase chromatography to give 15 mg of [8-(2-acetamidoethyl)-6,7-dihydro-MeBmt]-1-[(R)-(4-hydroxybutoxy)methyl-Sar]-3-cyclosporin [Molecular Formula: C₇₁H₁₃₀N₁₂O₁₅; Exact Mass: 1390.98; MS (m/z): 1391.80 (M+1)⁺, 1413.98 (M+Na)⁺; HPLC RT: 15.50 min. (C8 reverse phase column: 250 mm; acetonitrile/water (0.05% trifluoroacetic acid); operation temperature: 64 °C; detector: 210 nm)].

### Example 29

### [8-(3-Acetamidopropyl)-6,7-dihydro-MeBmt]-1-[(S)-(3-(N-morpholino)propylthio)methyl-Sar]-3-cyclosporin

### (4-Cyanobutyl)triphenylphosphonium bromide

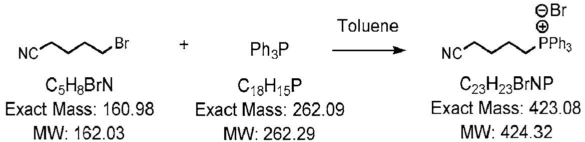

5-Bromovaleronitrile (1.62 g, 10.00 mmol) and triphenylphosphine (2.62 g, 10.00 mmol) were added to toluene (20 ml). The mixture was stirred and heated to reflux overnight. After cooled to room temperature, the precipitate was filtered off, washed with toluene and hexane and dried in vacuum to give 3.00 g product. [Molecular Formula: C₂₃H₂₃BrNP; Exact Mass: 423.08; MS (m/z): 344.28 (M-Br)⁺],

### [8-(2-Cyanoethyl)-3-acetyl-MeBmt]-1-cyclosporin

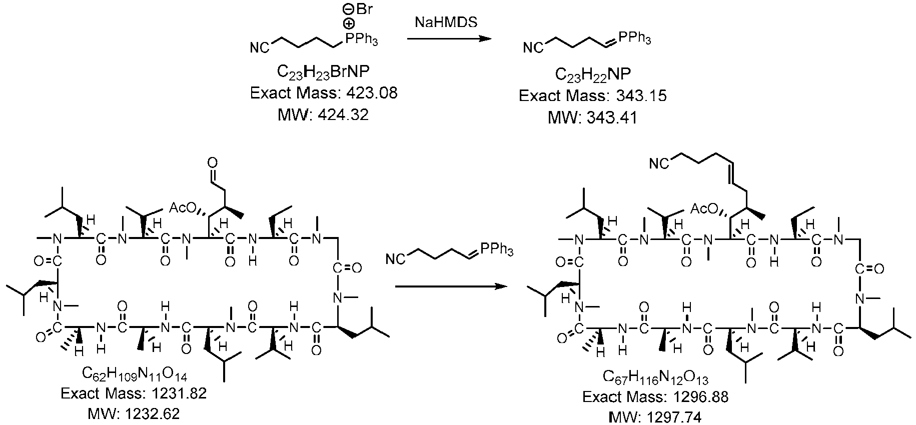

To a solution of (4-cyanobutyl)triphenylphosphonium bromide (6.40 g, 15.08 mmol) in anhydrous tetrahydrofuran (180 ml) under nitrogen was added sodium bis(trimethylsilyl)amide (2.0 M in THF, 11.2 ml, 22.40 mmol). The reaction mixture was stirred at room temperature for one hour and cooled to -30 °C. A solution of [(3R, 4R)-3-acetyloxy-4-methyl-6-oxo-N-MeNle]-1-cyclosporin(8.20 g, 6.65 mmol) in anhydrous tetrahydrofuran (25 ml) was added. The mixture was stirred another two hours at -30 °C. Then saturated ammonium chloride solution (20 ml) was added to quench the reaction. Most of tetrahydrofuran was evaporated under reduced pressure. Ethyl acetate (250 ml) and brine (100 ml) were added and the mixture was separated. The organic layer was dried over magnesium sulfate and evaporated under reduced pressure. The residue was purified by chromatography (dichloromethane/methanol) to give 5.60 g of pure [8-(2-cyanoethyl)-3-acetyl-MeBmt]-1-cyclosporin [Molecular Formula: C₆₇H₁₁₆N₁₂O₁₃; Exact Mass: 1296.88; MS (m/z): 1297.57 (M+1)⁺].

### [8-(2-Cyanoethyl)-MeBmt]-1-cyclosporin

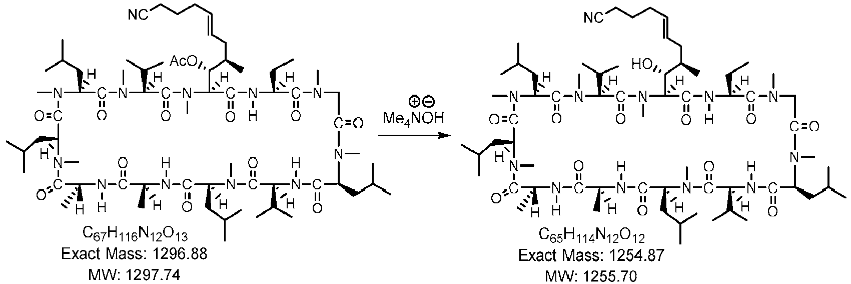

[8-(2-Cyanoethyl)-3-acetyl-MeBmt]-1-cyclosporin (5.00 g, 3.86 mmol) was dissolved methanol (100 ml). Water (50 ml) and tetramethylammonium hydroxide pentahydrate (4.40 g, 24.22 mmol) were added. The mixture was stirred at room temperature overnight. Then most of the methanol was evaporated. Ethyl acetate (250 ml) and brine (100 ml) were added and the mixture was separated. The organic layer was dried over magnesium sulfate and evaporated under reduced pressure. The residue was purified by chromatography (dichloromethane/methanol) to give 3.60 g of pure [8-(2-cyanoethyl)-MeBmt]-1-cyclosporin [Molecular Formula: C₆₅H₁₁₄N₁₂O₁₂; Exact Mass: 1254.87; MS (m/z): 1255.51 (M+1)⁺; HPLC RT: 14.90 min. (C8 reverse phase column: 250 mm; acetonitrile/water (0.05% trifluoroacetic acid); operation temperature: 64 °C; detector: 210 nm)].

### [8-(3-Aminopropyl)-6,7-dihydro-MeBmt]-1-cyclosporin

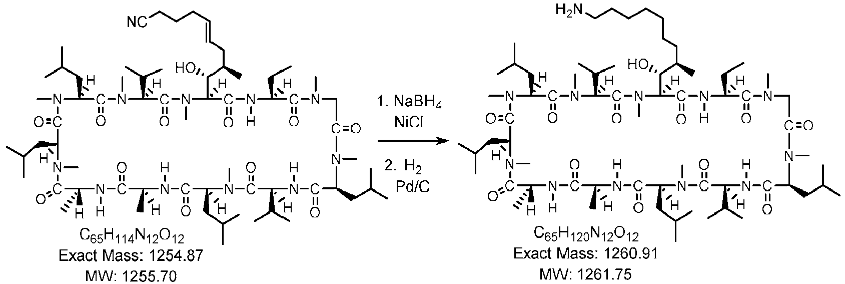

To a solution of [8-(2-cyanoethyl)-MeBmt]-1-cyclosporin (2.40 g, 1.91 mmol) in methanol (120 ml) under nitrogen was added nickel(II) chloride hexahydrate (0.38 g, 1.62 mmol). Sodium borohydride (2.00 g, 52.63 mmol) was added in four batches in 30 minutes. After the mixture was stirred for another hour, most of the methanol was evaporated under reduced pressure. Ethyl acetate (100 ml) and saturated sodium bicarbonate solution (50 ml) were added and the mixture was separated. The organic layer was washed with brine, dried over magnesium sulfate and evaporated under reduced pressure. The residue was dissolved in methanol (30 ml). Palladium (10 wt% on carbon, 150 mg) and acetic acid (5 drops) were added. The mixture was stirred at room temperature under hydrogen for four hours. The mixture was filtered and the filtrate was evaporated under reduced pressure to give crude [8-(3-aminopropyl)-6,7-dihydro-MeBmt]-1-cyclosporin [Molecular Formula: C₆₅H₁₂₀N₁₂O₁₂; Exact Mass: 1260.90; MS (m/z): 1261.69 (M+1)⁺; HPLC RT: 11.23 min. (C8 reverse phase column: 250 mm; acetonitrile/water (0.05% trifluoroacetic acid); operation temperature: 64 °C; detector: 210 nm)].

### [8-(3-Acetamidopropyl)-6,7-dihydro-MeBmt]-1-cyclosporin

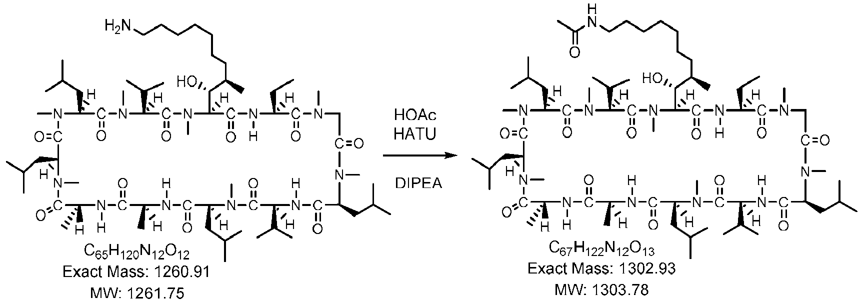

To a solution of [8-(3-aminopropyl)-6,7-dihydro-MeBmt]-1-cyclosporin (2.40 g, 1.90 mmol) and acetic acid (240 mg, 60 mmol) in dichloromethane (100 ml) were added diisopropylethyl amine (386 mg, 1.99 mmol) and HATU (1.20 g, 3.15 mmol). After stirred at room temperature for two hours, the reaction mixture was washed with saturated sodium bicarbonate solution, brine, dried over magnesium sulfate and evaporated under reduced pressure. The residue was purified by chromatography (dichloromethane/methanol) to give 2.00 g of pure [8-(3-acetamidopropyl)-6,7-dihydro-MeBmt]-1-cyclosporin [Molecular Formula: C₆₇H₁₂₂N₁₂O₁₃; Exact Mass: 1302.93; MS (m/z): 1303.63(M+1)⁺. HPLC RT: 13.74 min. (C8 reverse phase column: 250 mm; acetonitrile/water (0.05% trifluoroacetic acid); operation temperature: 64 °C; detector: 210 nm)].

### [8-(3-Acetamidopropyl)-6,7-dihydro-MeBmt]-1-[α-methylene-Sar]-3-cyclosporin

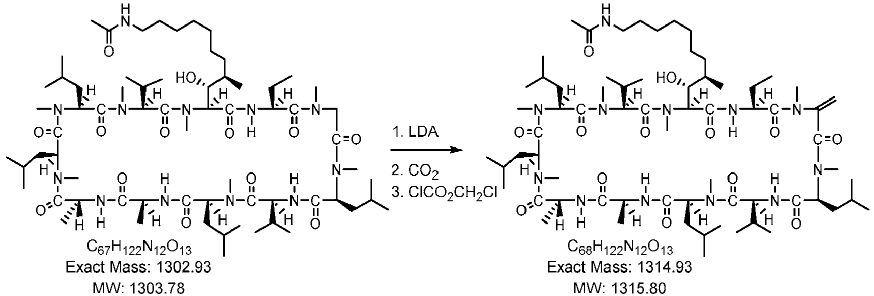

n-Butyllithium (2.65 M, 12 ml, 31.80 mmol) was added to a solution of diisopropylamine (3.23 g, 32 mmol) in tetrahydrofuran (100 ml) at-78 °C under nitrogen. After the reaction mixture was stirred for one hour, a solution of [8-(3-acetamidopropyl)-6,7-dihydro-MeBmt]-1-cyclosporin (3.80 g, 2.92 mmol) in tetrahydrofuran (15 ml) was added over ten minutes. The mixture was stirred at -78 °C for three hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (3.70 ml) was added. The mixture was stirred and allowed to warm to room temperature overnight. Brine (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 1.20 g of pure [8-(3-acetamidopropyl)-6,7-dihydro-MeBmt]-1-[α-methylene-Sar]-3-cyclosporin [Molecular Formula: C₆₈H₁₂₂N₁₂O₁₃; Exact Mass: 1314.93; MS (m/z): 1315.61 (M+1)⁺; HPLC RT: 16.44 min. (C8 reverse phase column: 250 mm; acetonitrile/water (0.05% trifluoroacetic acid); operation temperature: 64 °C; detector: 210 nm)].

### [8-(3-Acetamidopropyl)-6,7-dihydro-MeBmt]-1-[(S)-(3-(N-morpholino)propylthio)methyl-Sar]-3-cyclosporin

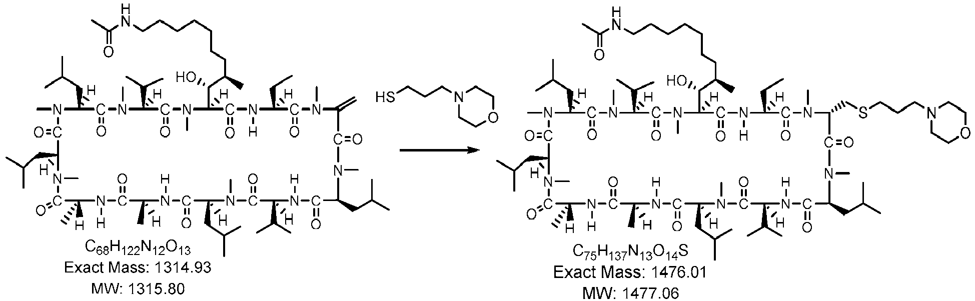

To a solution of [8-(3-acetamidopropyl)-6,7-dihydro-MeBmt]-1-[(S)-(α-methylene-Sar]-3-cyclosporin (0.50 g, 0.38 mmol) in methanol (60 ml) were added 3-morpholinopropanethiol (0.48 g, 3.00 mmol) and lithium hydroxide (92 mg, 3.83 mmol). The reaction mixture was stirred at room temperature overnight. Most of the methanol was evaporated under reduced pressure. Ethyl acetate (80 ml) and brine (30 ml) were added and the mixture was separated. The organic layer was dried over magnesium sulfate and evaporated under reduced pressure. The residue was purified by chromatography (dichloromethane/methanol) to give 130 mg of pure [8-(3-acetamidopropyl)-6,7-dihydro-MeBmt]-1-[(S)-(3-(N-morpholino)propylthio)methyl-Sar]-3-cyclosporin [Molecular Formula: C₇₅H₁₃₇N₁₃O₁₄S; Exact Mass: 1476.01; MS (m/z): 1476.82 (M+1)⁺; HPLC RT: 12.61 min. (C8 reverse phase column: 250 mm; acetonitrile/water (0.05% trifluoroacetic acid); operation temperature: 64 °C; detector: 210 nm)].

### Example 30

### [8-(3-Acetamidopropyl)-6,7-dihydro-MeBmt]-1-[(S)-(4-hydroxybutylthio)methyl-Sar]-3-cyclosporin

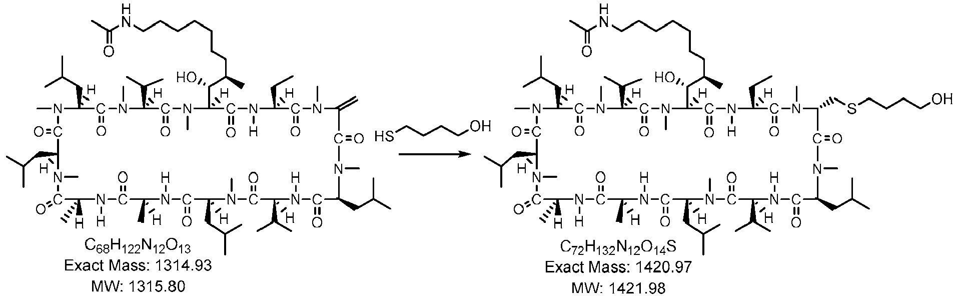

To a solution of [8-(3-acetamidopropyl)-6,7-dihydro-MeBmt]-1-[(S)-(α-methylene-Sar]-3-cyclosporin (0.50 g, 0.38 mmol) in methanol (60 ml) were added 4-mercapto-1-butanol (0.28 g, 2.64 mmol) and lithium hydroxide (92 mg, 3.83 mmol). The reaction mixture was stirred at room temperature overnight. Most of the methanol was evaporated under reduced pressure. Ethyl acetate (80 ml) and brine (30 ml) were added and the mixture was separated. The organic layer was dried over magnesium sulfate and evaporated under reduced pressure. The residue was purified by chromatography (dichloromethane/methanol) to give 115 mg of pure [8-(3-acetamidopropyl)-6,7-dihydro-MeBmt]-1-[(S)-(4-hydroxybutylthio)methyl-Sar]-3-cyclosporin [Molecular Formula: C₇₂H₁₃₂N₁₂O₁₄S; Exact Mass: 1420.97; MS (m/z): 1421.80 (M+1)⁺; HPLC RT: 15.49 min. (C8 reverse phase column: 250 mm; acetonitrile/water (0.05% trifluoroacetic acid); operation temperature: 64 °C; detector: 210 nm)].

### Example 31

### [8-(3-(4-Carboxybenzamido)propyl)-6,7-dihydro-MeBmt]-1-[(S)-(4-hydroxybutylthio)methyl-Sar]-3-cyclosporin

### [8-(3-(4-Methoxycarbonylbenzamido)propyl)-6,7-dihydro-MeBmt]-1-cyclosporin

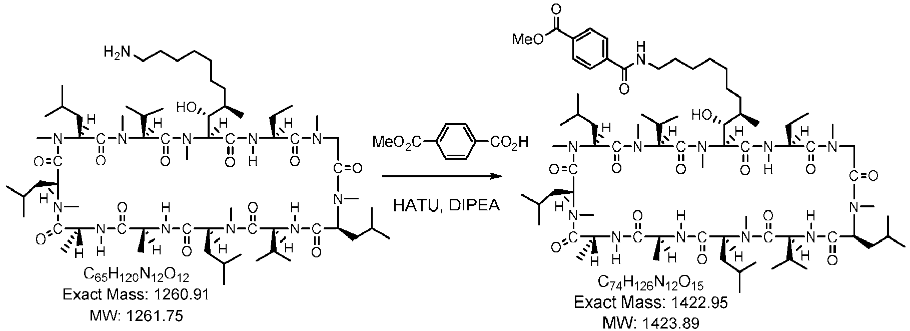

To a solution of [8-(3-aminopropyl)-6,7-dihydro-MeBmt]-1-cyclosporin (4.00 g, 3.17 mmol) and methyl hydrogen terephthalate (0.85 g, 4.72 mmol) in dichloromethane (100 ml) were added diisopropylethyl amine (770 mg, 5.96 mmol) and HATU (2.40 g, 6.30 mmol). The reaction mixture was stirred at room temperature for two hours and then washed with saturated sodium bicarbonate solution, brine, dried over magnesium sulfate and evaporated under reduced pressure. The residue was purified by chromatography (dichloromethane/methanol) to give 2.00 g of pure [8-(3-(4-methoxycarbonylbenzamido)propyl)-6,7-dihydro-MeBmt]-1-cyclosporin [Molecular Formula: C₇₄H₁₂₆N₁₂O₁₅; Exact Mass: 1422.95; MS (m/z): 1423.70(M+1)⁺. HPLC RT: 16.74 min. (C8 reverse phase column: 250 mm; acetonitrile/water (0.05% trifluoroacetic acid); operation temperature: 64 °C; detector: 210 nm)].

### [8-(3-(4-Carboxybenzamido)propyl)-6,7-dihydro-MeBmt]-1-cyclosporin

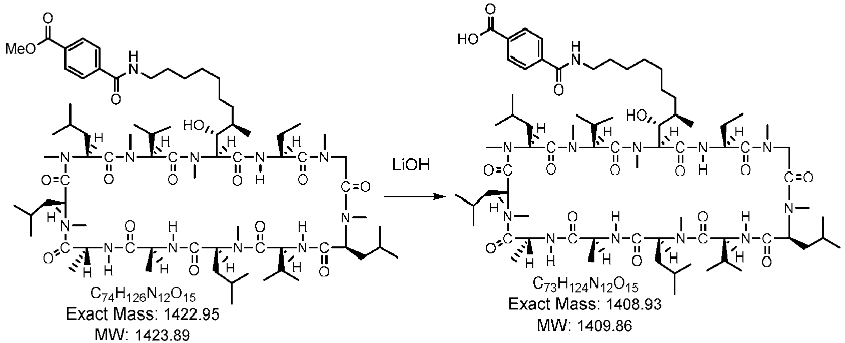

To a solution of [8-(3-(4-methoxycarbonylbenzamido)propyl)-6,7-dihydro-MeBmt]-1-cyclosporin (2.40 g, 1.68 mmol) in methanol (80 ml) and water (20 ml) was added lithium hydroxide (0.28 g, 11.66 mmol). The reaction mixture was stirred at room temperature for two hours and evaporated under reduced pressure. Ethyl acetate (10 ml) and brine (10 ml) were added and the PH of the aqueous layer was adjusted to 5 by adding hydrochloric acid solution (1.00 N). After separated, the ethyl acetate layer was dried over magnesium sulfate and evaporated to give 2.3 g of [8-(3-(4-carboxybenzamido)propyl)-6,7-dihydro-MeBmt]-1-cyclosporin. [Molecular Formula: C₇₃H₁₂₄N₁₂O₁₅; Exact Mass: 1408.93; MS (m/z): 1409.70(M+1)⁺. HPLC RT: 14.93 min. (C8 reverse phase column: 250 mm; acetonitrile/water (0.05% trifluoroacetic acid); operation temperature: 64 °C; detector: 210 nm)].

### [8-(3-(4-Carboxybenzamido)propyl)-6,7-dihydro-MeBmt]-1-[α-methylene-Sar]-3-cyclosporin

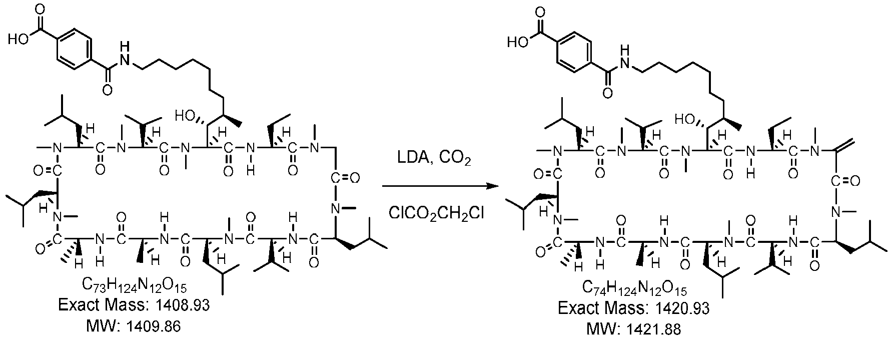

n-Butyllithium (2.65 M, 7.6 ml, 20.14 mmol) was added to a solution of diisopropylamine (2.02 g, 20.00 mmol) in tetrahydrofuran (120 ml) at -78 °C under nitrogen. After the reaction mixture was stirred for one hour, a solution of [8-(3-(4-carboxybenzamido)propyl)-6,7-dihydro-MeBmt]-1-cyclosporin (2.10 g, 1.49 mmol) in tetrahydrofuran (15 ml) was added over ten minutes. The mixture was stirred at -78 °C for three hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (2.20 ml) was added. The mixture was stirred and allowed to warm to room temperature overnight. Brine (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography to give 1.20 g of pure [8-(3-(4-carboxybenzamido)propyl)-6,7-dihydro-MeBmt]-1-[α-methylene-Sar]-3-cyclosporin [Molecular Formula: C₇₄H₁₂₄N₁₂O₁₅; Exact Mass: 1420.93; MS (m/z): 1421.61 (M+1)⁺; HPLC RT: 16.56 min. (C8 reverse phase column: 250 mm; acetonitrile/water (0.05% trifluoroacetic acid); operation temperature: 64 °C; detector: 210 nm)].

### [8-(3-(4-Carboxybenzamido)propyl)-6,7-dihydro-MeBmt]-1-[(S)-(4-hydroxybutylthio)methyl-Sar]-3-cyclosporin

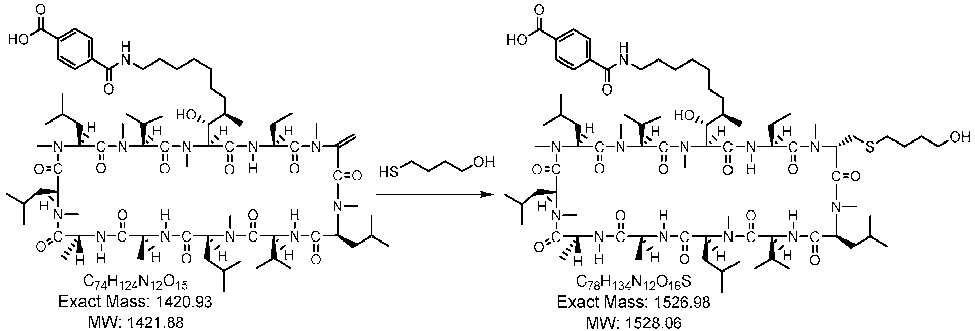

To a solution of [8-(3-(4-carboxybenzamido)propyl)-6,7-dihydro-MeBmt]-1-[α-methylene-Sar]-3-cyclosporin (0.50 g, 0.35 mmol) in methanol (30 ml) were added 4-mercapto-1-butanol (0.28 g, 2.64 mmol) and lithium hydroxide (92 mg, 3.83 mmol). The reaction mixture was stirred at room temperature overnight. Most of the methanol was evaporated under reduced pressure. Ethyl acetate (80 ml) and brine (30 ml) were added and the mixture was separated. The organic layer was dried over magnesium sulfate and evaporated under reduced pressure. The residue was purified by chromatography (dichloromethane/methanol) to give 115 mg of pure [8-(3-(4-carboxybenzamido)propyl)-6,7-dihydro-MeBmt]-1-[(S)-(4-hydroxybutylthio) methyl-Sar]-3-cyclosporin [Molecular Formula: C₇₈H₁₃₄N₁₂O₁₆S; Exact Mass: 1526.98; MS (m/z): 1527.84 (M+1)⁺; HPLC RT: 14.80 min. (C8 reverse phase column: 250 mm; acetonitrile/water (0.05% trifluoroacetic acid); operation temperature: 64 °C; detector: 210 nm)].

### Example 32

### [8-(3-(4-(Diethylcarbamoylbenzamido)propyl)-6,7-dihydro-MeBmt]-1-[(S)-(4-hydroxybutylthio)methyl-Sar]-3-cyclosporin

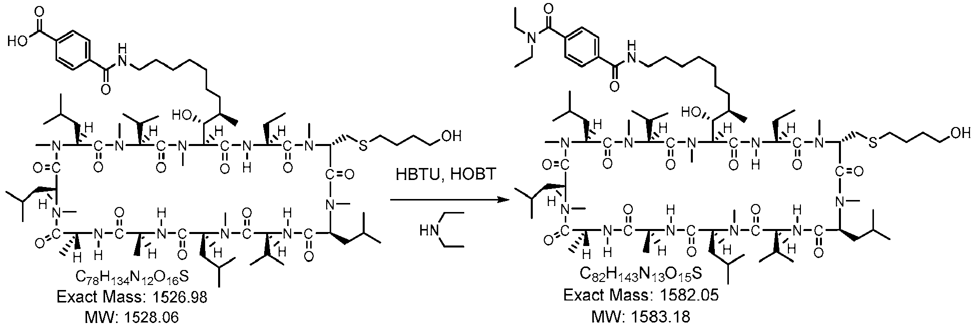

To a solution of [8-(3-(4-carboxybenzamido)propyl)-6,7-dihydro-MeBmt]-1-[(S)-(4-hydroxybutylthio)methyl-Sar]-3-cyclosporin (0.10 g, 0.07 mmol) and diethylamine (15 mg, 0.21 mmol) in dichloromethane (10 ml) were added diisopropylethyl amine (25 mg, 0.19 mmol), HOBT (29 mg, 0.19 mmol) and HBTU (73 mg, 0.19 mmol). After stirred at room temperature for two hours, the reaction mixture was washed with saturated sodium bicarbonate solution and brine, dried over magnesium sulfate and evaporated under reduced pressure. The residue was purified by chromatography (dichloromethane/methanol) to give 42 mg of pure [8-(3-(4-diethylcarbamoybenzamido)propyl)-6,7-dihydro-MeBmt]-1--[(S)-(4-hydroxybutylthio) methyl-Sar]-3-cyclosporin [Molecular Formula: C₈₂H₁₄₃N₁₃O₁₅S; Exact Mass: 1582.05; MS (m/z): 1582.70(M+1)⁺. HPLC RT: 16.08 min. (C8 reverse phase column: 250 mm; acetonitrile/water (0.05% trifluoroacetic acid); operation temperature: 64 °C; detector: 210 nm)].

### Example 33

### [8-(3-(3-Methoxycarbonylbenzamido)propyl)-6,7-dihydro-MeBmt]-1-[(S)-(4-hydroxybutylthio)methyl-Sar]-3-cyclosporin

### [8-(3-(3-Methoxycarbonylbenzamido)propyl)-6,7-dihydro-MeBmt]-1-cyclosporin

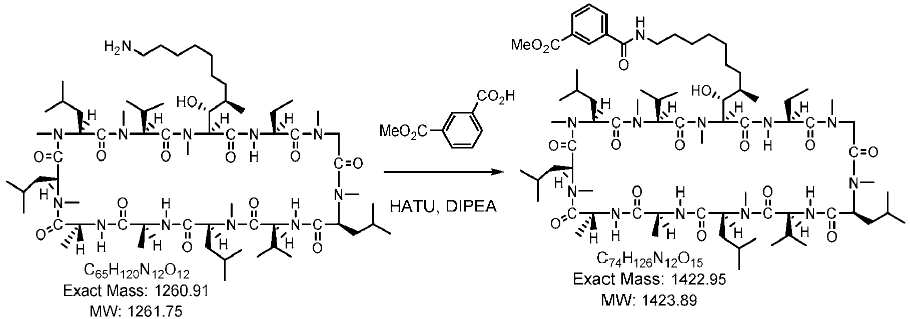

To a solution of [8-(3-aminopropyl)-6,7-dihydro-MeBmt]-1-cyclosporin (4.33 g, 3.43 mmol) and methyl hydrogen isophthalate (0.92 g, 5.11 mmol) in dichloromethane (100 ml) were added diisopropylethyl amine (770 mg, 5.96 mmol) and HATU (2.40 g, 6.30 mmol). The reaction mixture was stirred at room temperature for two hours and then washed with saturated sodium bicarbonate solution, brine, dried over magnesium sulfate and evaporated under reduced pressure. The residue was purified by chromatography (dichloromethane/methanol) to give 2.00 g of pure [8-(3-(3-methoxycarbonylbenzamido)propyl)-6,7-dihydro-MeBmt]-1-cyclosporin [Molecular Formula: C₇₄H₁₂₆N₁₂O₁₅; Exact Mass: 1422.95; MS (m/z): 1423.70(M+1)⁺. HPLC RT: 16.82 min. (C8 reverse phase column: 250 mm; acetonitrile/water (0.05% trifluoroacetic acid); operation temperature: 64 °C; detector: 210 nm)].

### [8-(3-(3-Carboxybenzamido)propyl)-6,7-dihydro-MeBmt]-1-cyclosporin

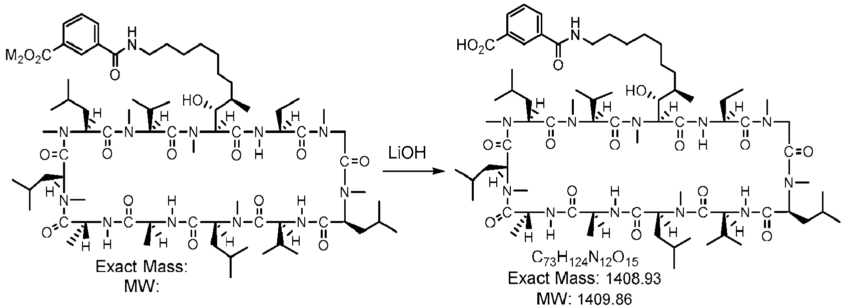

To a solution of [8-(3-(3-methoxycarbonylbenzamido)propyl)-6,7-dihydro-MeBmt]-1-cyclosporin (2.50 g, 1.76 mmol) in methanol (80 ml) were added water (20 ml) and lithium hydroxide (0.28 g, 11.66 mmol). The reaction mixture was stirred at room temperature for two hours and evaporated under reduced pressure. Ethyl acetate (10 ml) and brine (10 ml) were added and the PH of the aqueous layer was adjusted to 5 by adding hydrochloric acid solution (1.00 N). After separated, the ethyl acetate layer was dried over magnesium sulfate and evaporated to give 2.1 g of [8-(3-(3-carboxybenzamido)propyl)-6,7-dihydro-MeBmt]-1-cyclosporin. [Molecular Formula: C₇₃H₁₂₄N₁₂O₁₅; Exact Mass: 1408.93; MS (m/z): 1409.70(M+1)⁺. HPLC RT: 15.11 min. (C8 reverse phase column: 250 mm; acetonitrile/water (0.05% trifluoroacetic acid); operation temperature: 64 °C; detector: 210 nm)].

### [8-(3-(3-Methoxycarbonylbenzamido)propyl)-6,7-dihydro-MeBmt]-1-[α-methylene-Sar]-3-cyclosporin

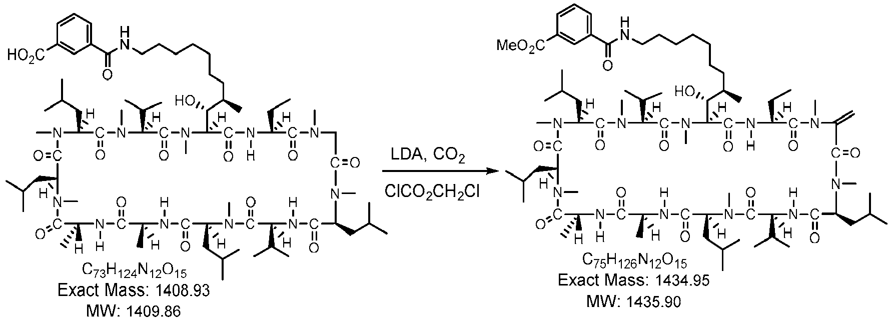

n-Butyllithium (2.65 M, 7.9 ml, 20.94 mmol) was added to a solution of diisopropylamine (2.12 g, 21.00 mmol) in tetrahydrofuran (120 ml) at -78 °C under nitrogen. After the reaction mixture was stirred for one hour, a solution of [8-(3-(3-carboxybenzamido)propyl)-6,7-dihydro-MeBmt]-1-cyclosporin (2.10 g, 1.49 mmol) in tetrahydrofuran (15 ml) was added over ten minutes. The mixture was stirred at -78 °C for three hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (2.20 ml) was added. The mixture was stirred and allowed to warm to room temperature overnight. Methanol (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 1.20 g of pure [8-(3-(3-methoxycarbonylbenzamido)propyl)-6,7-dihydro-MeBmt]-1-[α-methylene-Sar]-3-cyclosporin [Molecular Formula: C₇₅H₁₂₆N₁₂O₁₅; Exact Mass: 1434.95; MS (m/z): 1435.61 (M+1)⁺; HPLC RT: 18.26 min. (C8 reverse phase column: 250 mm; acetonitrile/water (0.05% trifluoroacetic acid); operation temperature: 64 °C; detector: 210 nm)].

### [8-(3-(3-Methoxycarbonylbenzamido)propyl)-6,7-dihydro-MeBmt]-1-[(S)-(4-hydroxybutylthio)methyl-Sar]-3-cyclosporin

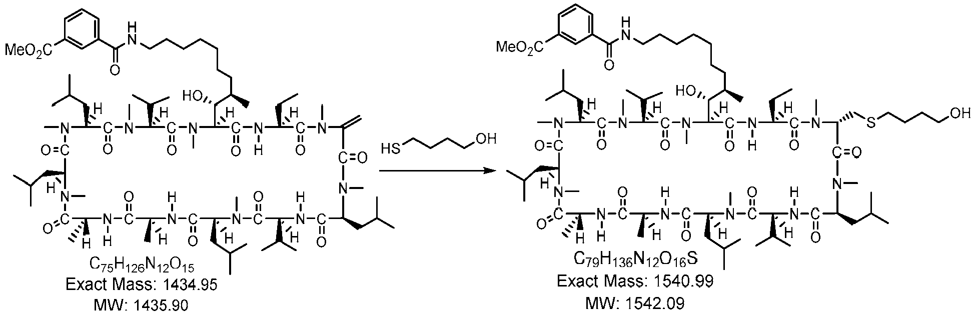

To a solution of [8-(3-(3-methoxycarbonylbenzamido)propyl)-6,7-dihydro-MeBmt]-1-[α-methylene-Sar]-3-cyclosporin (0.62 g, 0.43 mmol) in methanol (30 ml) were added 4-mercapto-1-butanol (0.28 g, 2.64 mmol) and lithium hydroxide (103 mg, 4.29 mmol). The reaction mixture was stirred at room temperature overnight. Most of the methanol was evaporated under reduced pressure. Ethyl acetate (80 ml) and brine (30 ml) were added and the mixture was separated. The organic layer was dried over magnesium sulfate and evaporated under reduced pressure. The residue was purified by chromatography (dichloromethane/methanol) to give 115 mg of pure [8-(3-(3-methoxycarbonylbenzamido)propyl)-6,7-dihydro-MeBmt]-1-[(S)-(4-hydroxybutylthio)methyl-Sar]-3-cyclosporin [Molecular Formula: C₇₉H₁₃₆N₁₂O₁₆S; Exact Mass: 1540.99; MS (m/z): 1541.84 (M+1)⁺; HPLC RT: 16.83 min. (C8 reverse phase column: 250 mm; acetonitrile/water (0.05% trifluoroacetic acid); operation temperature: 64 °C; detector: 210 nm)].

### Example 34

### [8-(3-(3-Carboxylbenzamido)propyl)-6,7-dihydro-MeBmt]-1-[(S)-(4-hydroxybutylthio)methyl-Sar]-3-cyclosporin

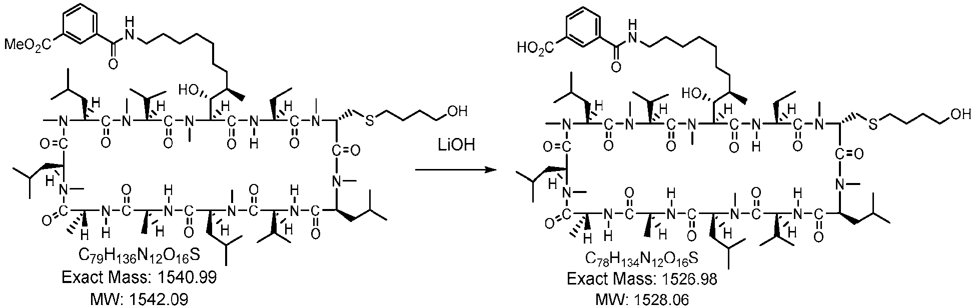

To a solution of [8-(3-(3-methoxycarbonylbenzamido)propyl)-6,7-dihydro-MeBmt]-1-[(S)-(4-hydroxybutylthio)methyl-Sar]-3-cyclosporin (0.20 g, 0.13 mmol) in methanol (8 ml) were added water (3 ml) and lithium hydroxide (15 mg, 0.63 mmol). The reaction mixture was stirred at room temperature for eight hours and evaporated under reduced pressure. Ethyl acetate (10 ml) and brine (10 ml) were added and the PH of the aqueous layer was adjusted to 5 by adding hydrochloric acid solution (1.00 N). After separated, the ethyl acetate layer was dried over magnesium sulfate and evaporated to give [8-(3-(3-carboxylbenzamido)propyl)-6,7-dihydro-MeBmt]-1-[(S)-(4-hydroxybutylthio)methyl-Sar]-3-cyclosporin [Molecular Formula: C₇₈H₁₃₄N₁₂O₁₆S; Exact Mass: 1526.98; MS (m/z): 1527.84 (M+1)⁺; HPLC RT: 14.99 min. (C8 reverse phase column: 250 mm; acetonitrile/water (0.05% trifluoroacetic acid); operation temperature: 64 °C; detector: 210 nm)].

### Example 35

### [8-(4-Acetamidobutyl)-6,7-dihydro-MeBmt]-1-[(S)-(4-hydroxybutylthio)methyl-Sar]-3-cyclosporin

### (5-Cyanopentyl)triphenylphosphonium bromide

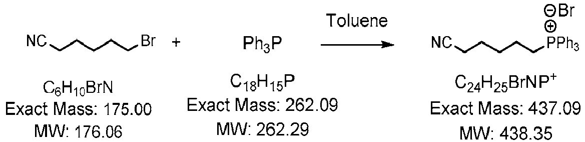

6-Bromohexanenitrile (10.00 g, 56.80 mmol) and triphenylphosphine (14.90 g, 56.80 mmol) were dissolved in toluene (100 ml). The mixture was stirred and heated to reflux for three days. After cooled to room temperature, most of toluene was decanted. The residue was dried in vacuum for six hours. Then hexane (160 ml) was added and the mixture was stirred for a weekend at room temperature. The precipitate was filtered off and dried in vacuum to give 21.0 g product. [Molecular Formula: C₂₄H₂₅NP⁺; Exact Mass: 358.17; MS (m/z): 358.20 (M)⁺].

### [8-(3-Cyanopropyl)-3-acetyl-MeBmt]-1-cyclosporin

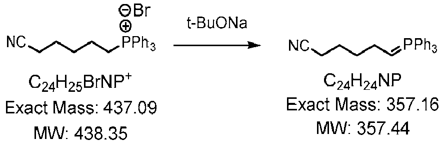

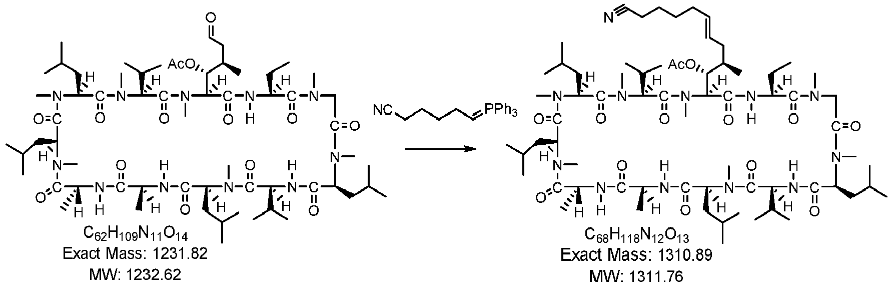

To a dried flask were added (5-cyanopentyl)triphenylphosphonium bromide (14.00 g, 32.04 mmol) and anhydrous tetrahydrofuran (50 ml) under nitrogen. The reaction mixture was put into an ice-water bath and sodium tert-butoxide (4.30 g, 44.84 mmol) was added. After the mixture was stirred for three hours, a solution of [(3R,4R)-3-acetyloxy-4-methyl-6-oxo-N-MeNle]-1-cyclosporin (7.90 g, 6.40 mmol) in anhydrous tetrahydrofuran (20 ml) was added. The mixture was stirred another three hours at 0°C. Then saturated ammonium chloride solution (20 ml) was added to quench the reaction. Most of tetrahydrofuran was evaporated under reduced pressure. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 7.19 g of [8-(3-cyanopropyl)-3-acetyl-MeBmt]-1-cyclosporin [Molecular Formula: C₆₈H₁₁₈N₁₂O₁₃; Exact Mass: 1310.89; MS (m/z): 1311.58 (M+1)⁺, 1333.74 (M+Na)⁺; HPLC RT: 17.19 min.(C8 reverse phase column: 250 mm; acetonitrile/water (0.05% trifluoroacetic acid); operation temperature: 64 °C; detector: 210 nm)].

### [8-(3-Cyanopropyl)-MeBmt]-1-cyclosporin

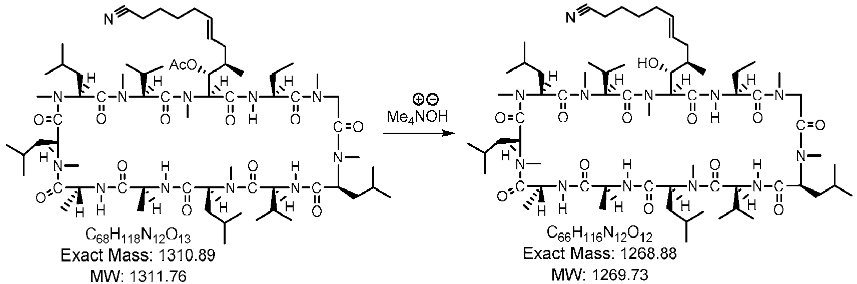

[8-(3-Cyanopropyl)-3-acetyl-MeBmt]-1-cyclosporin (7.19 g, 5.48 mmol) was dissolved methanol (40 ml). Water (20 ml) and tetramethylammonium hydroxide pentahydrate (2.98 g, 16.45 mmol) were added. The mixture was stirred at room temperature for two hours. Then most of the methanol was evaporated. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 4.50 g of pure [8-(3-cyanopropyl)-MeBmt]-1-cyclosporin [Molecular Formula: C₆₆H₁₁₆N₁₂O₁₂; Exact Mass: 1268.88; MS (m/z): 1269.62 (M+1)⁺, 1291.76 (M+Na)⁺; HPLC RT: 15.78 min.(C8 reverse phase column: 250 mm; acetonitrile/water (0.05% trifluoroacetic acid); operation temperature: 64 °C; detector: 210 nm)].

### [8-(4-Aminobutyl)-6,7-dihydro-MeBmt]-1-cyclosporin

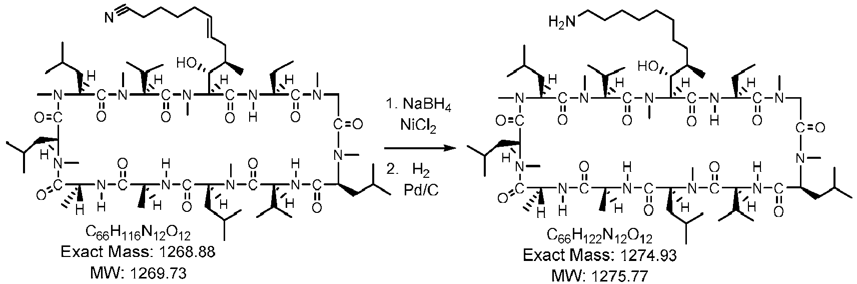

To a solution of [8-(3-cyanopropyl)-MeBmt]-1-cyclosporin (4.50 g, 3.63 mmol) in methanol (60 ml) under nitrogen was added nickel (II) chloride hexahydrate (0.43 g, 1.81 mmol). The reaction mixture was put into ice-water bath. Sodium borohydride (7.04 g, 181.33 mmol) was added in four batches in two hours. After the mixture was stirred for another two hours at 0°C, water (10 ml) was added. Most of the methanol was evaporated under reduced pressure. Ethyl acetate (120 ml) and saturated sodium bicarbonate solution (120 ml) were added and the mixture was separated. The organic layer was washed with brine, dried over magnesium sulfate and evaporated under reduced pressure. The residue was dissolved in methanol (60 ml). Palladium (10 wt% on carbon, 50 mg) and acetic acid (6 drops) were added. The mixture was stirred at room temperature under hydrogen overnight. The mixture was filtered and the filtrate was evaporated under reduced pressure to give crude 4.63 g of 8-(4-aminobutyl)-6,7-dihydro-MeBmt]-1-cyclosporin [Molecular Formula: C₆₆H₁₂₂N₁₂O₁₂; Exact Mass: 1274.93; MS (m/z): 1275.71 (M+1)⁺; HPLC RT: 11.95 min. (C8 reverse phase column: 250 mm; acetonitrile/water (0.05% trifluoroacetic acid); operation temperature: 64 °C; detector: 210 nm)].

### [8-(4-(tert-Butoxycarbonyl)aminobutyl)-6,7-dihydro-MeBmt]-1-cyclosporin

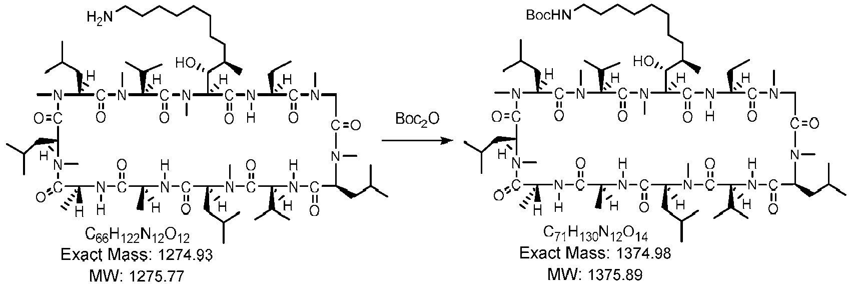

[8-(4-Aminobutyl)-6,7-dihydro-MeBmt]-1-cyclosporin (4.63 g, 3.63 mmol) was dissolved in tetrahydrofuran (50 ml). Saturated sodium bicarbonate solution (25 ml) and di-tert-butyldicarbonate were (0.87 g, 3.99 mmol) were added. The mixture was stirred at room temperature for two hours. Most of tetrahydrofuran was evaporated under reduced pressure. Ethyl acetate (30 ml) and brine (30 ml) were added and the mixture was separated. The organic layer was dried over magnesium sulfate and evaporated under reduced pressure. The residue was purified by chromatography (hexane/acetone) to give 3.00 g of pure [8-(4-(tert-butoxycarbonyl)aminobutyl)-6,7-dihydro-MeBmt]-1-cyclosporin [Molecular Formula: C₇₁H₁₃₀N₁₂O₁₄; Exact Mass: 1374.98; MS (m/z): 1375.64 (M+1)⁺, 1397.85 (M+Na)⁺; HPLC RT: 17.81 min. (C8 reverse phase column: 250 mm; acetonitrile/water (0.05% trifluoroacetic acid); operation temperature: 64 °C; detector: 210 nm)].

### [8-(4-(tert-Butoxycarbonyl)aminobutyl)-6,7-dihydro-MeBmt]-1-[α-methylene-Sar]-3-cyclosporin

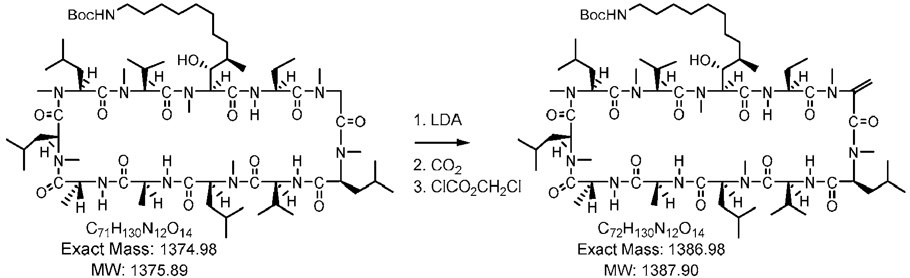

n-Butyllithium (2.65 M, 8.23 ml, 21.82 mmol) was added to a solution of diisopropylamine (3.09 ml, 21.82 mmol) in tetrahydrofuran (60 ml) at -78 °C under nitrogen. After the reaction mixture was stirred for an hour, a solution of [8-(4-(tert-butoxycarbonyl)aminobutyl)-6,7-dihydro-MeBmt]-1-cyclosporin (3.00 g, 2.18 mmol) in tetrahydrofuran (20 ml) was added over ten minutes. The mixture was stirred at -78 °C for two hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (1.93 ml, 21.82 mmol) was added. The mixture was stirred and allowed to warm to room temperature overnight. Brine (10 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.40 g of pure [8-(4-(tert-butoxycarbonyl)aminobutyl)-6,7-dihydro-MeBmt]-1-[α-methylene-Sar]-3-cyclosporin [Molecular Formula: C₇₂H₁₃₀N₁₂O₁₄; Exact Mass: 1386.98; MS (m/z): 1387.61 (M+1)⁺, 1409.80 (M+Na)⁺; HPLC RT: 19.01 min. (C8 reverse phase column: 250 mm; acetonitrile/water (0.05% trifluoroacetic acid); operation temperature: 64 °C; detector: 210 nm)].

### [8-(4-(tert-Butoxycarbonyl)aminobutyl)-6,7-dihydro-MeBmt]-1-[(S)-(4-hydroxybutylthio)methyl-Sar]-3-cyclosporin

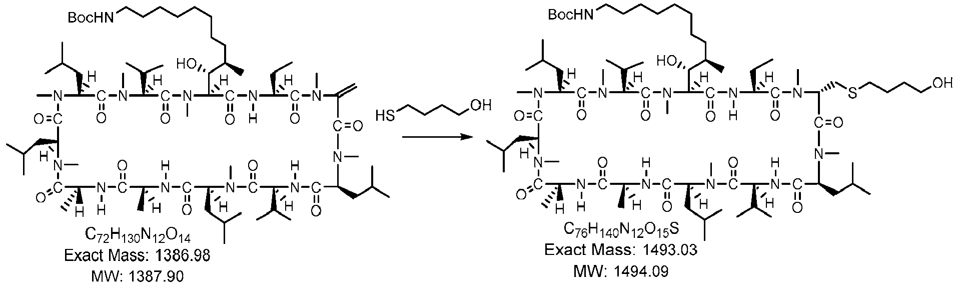

To a solution of [8-(4-(tert-butoxycarbonyl)aminobutyl)-6,7-dihydro-MeBmt]-1-[α-methylene-Sar]-3-cyclosporin (0.40 g, 0.29 mmol) in methanol (15 ml) were added 4-mercapto-1-butanol (0.18 ml, 1.73 mmol) and lithium hydroxide (0.07 g, 2.88 mmol). The reaction mixture was stirred at room temperature for seven hours. Most of the methanol was evaporated under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone from 90/10 to 75/25) to give 200 mg of pure [8-(4-(tert-butoxycarbonyl)aminobutyl)-6,7-dihydro-MeBmt]-1-[(S)-(4-hydroxybutylthio)methyl-Sar]-3-cyclosporin [Molecular Formula: C₇₆H₁₄₀N₁₂O₁₅S; Exact Mass: 1493.03; MS (m/z): 1493.63 (M+1)⁺, 1515.88 (M+Na)⁺; HPLC RT: 17.65 min.(C8 reverse phase column: 250 mm; acetonitrile/water (0.05% trifluoroacetic acid); operation temperature: 64 °C; detector: 210 nm)].

### [8-(4-Acetamidobutyl)-6,7-dihydro-MeBmt]-1-[(S)-(4-hydroxybutylthio)methyl-Sar]-3-cyclosporin

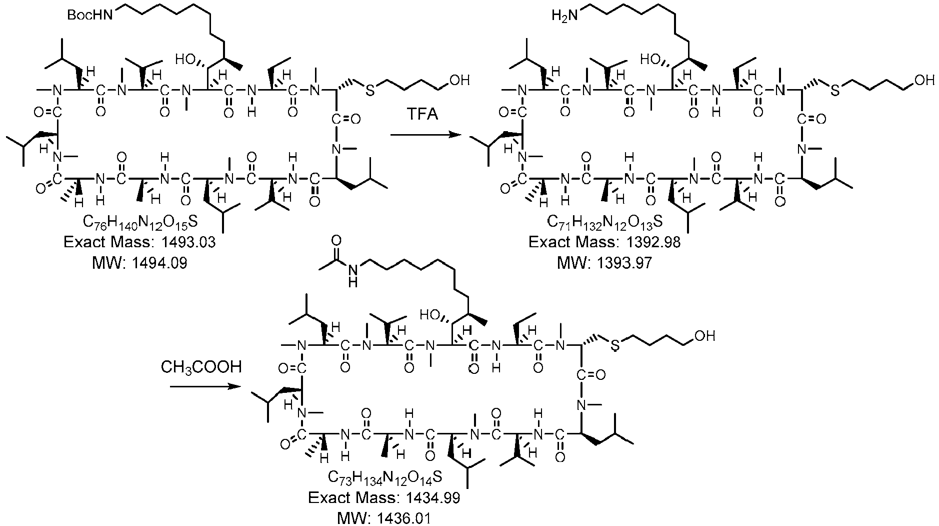

[8-(4-(tert-Butoxycarbonyl)aminobutyl)-6,7-dihydro-MeBmt]-1-[(S)-(4-hydroxybutyl thio)methyl-Sar]-3-cyclosporin (0.20 g, 0.16 mmol) was dissolved in dichloromethane (15 ml) and put into ice-water bath. Trifluoroacetic acid (5 ml) was added. The mixture was stirred at 0 °C for one hour. Another dichloromethane (20 ml) was added. The mixture was washed with brine (30 ml), saturated sodium bicarbonate solution (30 ml) and dried over magnesium sulfate and evaporated under reduced pressure to give crude [8-(4-aminobutyl)-6,7-dihydro-MeBmt]-1-[(S)-(4-hydroxybutylthio)methyl-Sar]-3-cyclosporin [Molecular Formula: C₇₁H₃₂N₁₂O₁₃S; Exact Mass: 1392.98; MS (m/z): 1393.80 (M+1)⁺; HPLC RT: 11.38 min. (C8 reverse phase column: 250 mm; acetonitrile/water (0.05% trifluoroacetic acid); operation temperature: 64 °C; detector: 210 nm)]. The crude [8-(4-aminobutyl)-6,7-dihydro-MeBmt]-1-[(S)-(4-hydroxybutyl thio)methyl-Sar]-3-cyclosporin was dissolved in dichloromethane (15 ml). Acetic acid (48 mg, 0.80 mmol), HBTU (0.18 g, 0.48 mmol), 1-hydroxybenzotriazole (0.06 g, 0.48 mmol) and pyridine 0.50 ml) were added. The mixture was stirred at room temperature for two hours. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 27 mg of [8-(4-acetamidobutyl)-6,7-dihydro-MeBmt]-1-[(S)-(4-hydroxybutylthio)methyl-Sar]-3-cyclosporin [Molecular Formula: C₇₃H₁₃₄N₁₂O₁₄S; Exact Mass: 1434.99; MS (m/z): 1435.69 (M+1)⁺, 1457.87 (M+Na)⁺; HPLC RT: 16.01 min. (C8 reverse phase column: 250 mm; acetonitrile/water (0.05% trifluoroacetic acid); operation temperature: 64 °C; detector: 210 nm)].

### Example 36

### [8-(2-Acetamidoethyl)-6,7-dihydro-MeBmt]-1-[(S)-(4-hydroxybutylthio)methyl-Sar]-3-[(γ-hydroxy)-N-MeLeu]-4-cyclosporin

### [3-Chloroacetyl-MeBmt]-1-[(γ-hydroxy)-N-MeLeu]-4-cyclosporin

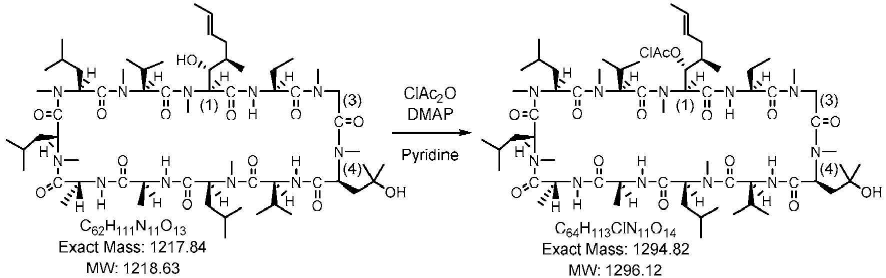

To a solution of [(γ-hydroxy)-N-MeLeu]-4-cyclosporin (12.17 g, 1.00 mmol) in N,N-dimethylformamide (300 ml) were added N,N-dimethylaminopyridine (0.12 g, 0.10 mmol), anhydrous pyridine (16.00 g, 0.20 mol) and chloroacetic anhydride (72.05 g, 0.54 mol) at -35 °C. The reaction mixture was stirred at room temperature overnight. The mixture was poured into 800 ml ice-water and stirred until the ice was melted. Ethyl acetate (500 ml) was added and the mixture was separated. The ethyl acetate layer was washed with water (100 ml), saturated sodium bicarbonate solution (100 ml), brine (100 ml), dried over magnesium sulfate and evaporated under reduced pressure. The residue was purified by chromatography (hexane/acetone) to give 9.40 g of pure [3-chloroacetyl-MeBmt]-1-[(γ-hydroxy)-N-MeLeu]-4-cyclosporin [Molecular Formula: C₆₄H₁₁₃ClN₁₁O₁₄; Exact Mass: 1294.82; MS (m/z): 1295.50 (M+1)⁺].

[(y-Hydroxy)-N-MeLeu]-4-cyclosporin was prepared by *Sebekia benihana* biotransformation according to a method described by Kuhnt M. et al., 1996, Microbial Biotransformation Products of Cyclosporin A, J. Antibiotics, 49 (8), 781.

### [(3R,4R)-3-Chloroacetyloxy-4-methyl-6-oxo-N-MeNle]-1-[(γ-hydroxy)-N-MeLeu]-4-cyclosporin

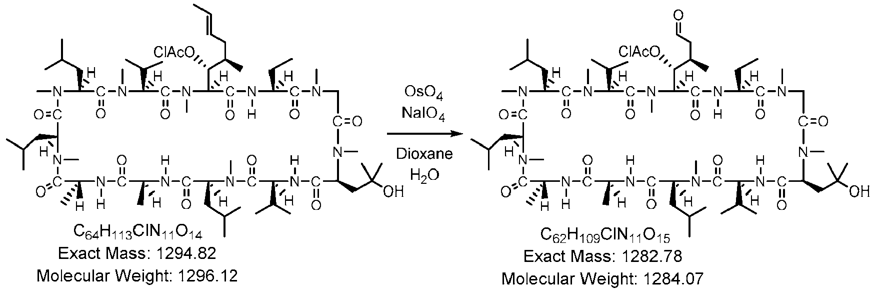

To a solution of [3-chloroacetyl-MeBmt]-1-[(γ-hydroxy)-N-MeLeu]-4-cyclosporin (9.50 g, 7.34 mmol) in dioxane (125 ml) were added water (100 ml), osmium(VIII) oxide solution (0.4% in water, 35 ml) and sodium metaperiodate (6.60 g, 30.90 mmol). The reaction mixture was stirred at room temperature for five hours. Ethyl acetate (100 ml) and brine (100 ml) were added and the mixture was separated. The organic layer was washed with saturated sodium bicarbonate solution, brine, dried over magnesium sulfate and evaporated under reduced pressure. The residue was purified by chromatography (hexane/acetone) to give 5.00 g of pure [(3R,4R)-3-chloroacetyloxy-4-methyl-6-oxo-N-MeNle]-1-[(γ-hydroxy)-N-MeLeu]-4-cyclosporin [Molecular Formula: C₆₂H₁₀₉ClN₁₁O₁₅; Exact Mass: 1282.78; MS (m/z): 1283.47 (M+1)⁺; HPLC RT: 14.74 min. (C8 reverse phase column: 250 mm; acetonitrile/water (0.05% trifluoroacetic acid); operation temperature: 64 °C; detector: 210 nm)].

### [3-Chloroacetyl-8-cyanomethyl-MeBmt]-1-[(γ-hydroxy)-N-MeLeu]-4-cyclosporin

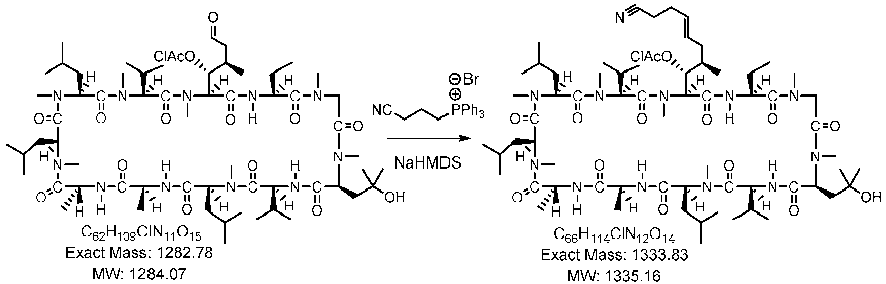

To a solution of (3-cyanopropyl)triphenylphosphonium bromide (3.07 g, 7.48 mmol) in anhydrous tetrahydrofuran (120 ml) under nitrogen was added sodium bis(trimethylsilyl)amide (1.00 M in tetrahydrofuran, 14 ml, 14.00 mmol). The reaction mixture was stirred at room temperature for one hour and cooled to -30 °C. A solution of [(3R, 4R)-3-chloroaceyloxy-4-methyl-6-oxo-N-MeNle]-1-[(γ-hydroxy)-N-MeLeu]-4-cyclosporin (4.80 g, 3.74 mmol) in anhydrous tetrahydrofuran (15 ml) was added. The mixture was stirred another two hours at -30 °C. Then saturated ammonium chloride solution (20 ml) was added to quench the reaction. Most of tetrahydrofuran was evaporated under reduced pressure. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 2.00 g of pure [3-chloroacetyl-8-cyanomethyl-MeBmt]-1-[(y-hydroxy)-N-MeLeu]-4-cyclosporin [Molecular Formula: C₆₆H₁₁₄ClN₁₂O₁₄; Exact Mass: 1333.83; MS (m/z): 1334.56 (M+1)⁺].

### [3-Acetyl-8-(2-aminoethyl)-6,7-dihydro-MeBmt]-1-[(γ-hydroxy)-N-MeLeu]-4-cyclosporin

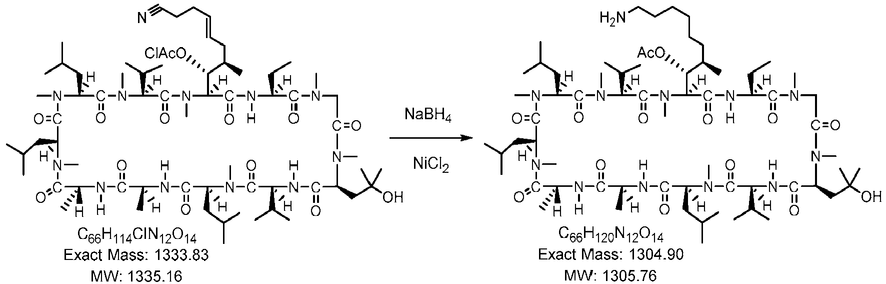

To a solution of [3-chloroacetyl-8-cyanomethyl-MeBmt]-1-[(γ-hydroxy)-N-MeLeu]-4-cyclosporin (0.85 g, 0.63 mmol) in methanol (100 ml) were added nickel (II) chloride hexahydrate (0.19 g, 0.81 mmol). Then sodium borohydride (0.38 g, 10.00 mmol) was added portions in 30 minutes. After the mixture was stirred for another hour at room temperature, most of the methanol was evaporated under reduced pressure. Ethyl acetate (50 ml) and saturated sodium bicarbonate solution (50 ml) were added and the mixture was separated. The organic layer was washed with brine, dried over magnesium sulfate and evaporated under reduced pressure. The residue was purified by chromatography (dichloromethane/methanol) to give 0.61 g of [3-acetyl-8-(2-aminoethyl)-6,7-dihydro-MeBmt]-1-[(γ-hydroxy)-N-MeLeu]-4-cyclosporin [Molecular Formula: C₆₆H₁₂₀N₁₂O₁₄; Exact Mass: 1304.90; MS (m/z): 1305.68 (M+1)⁺; HPLC RT: 12.80 min. (C8 reverse phase column: 250 mm; acetonitrile/water (0.05% trifluoroacetic acid); operation temperature: 64 °C; detector: 210 nm)].

### [8-(2-Acetamidoethyl)-3-acetyl-6,7-dihydro-MeBmt]-1-[(y-hydroxy)-N-MeLeu]-4-cyclosporin

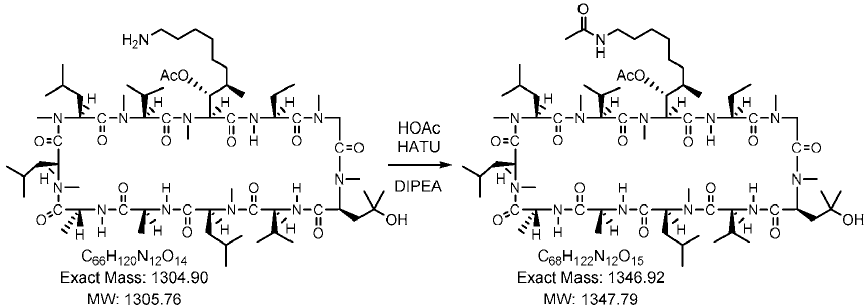

To a solution of [3-acetyl-8-(2-aminoethyl)-6,7-dihydro-MeBmt]-1-[(γ-hydroxy)-N-MeLeu]-4-cyclosporin (0.80 g, 0.61 mmol) and acetic acid (0.11 g, 1.83 mmol) in dichloromethane (60 ml) were added diisopropylethylamine (0.25 g, 1.93 mmol) and HATU (0.70 g, 1.83 mmol). The reaction mixture was stirred at room temperature for three hours and then washed with saturated sodium bicarbonate solution, brine, dried over magnesium sulfate and evaporated under reduced pressure. The residue was purified by chromatography (dichloromethane/methanol) to give 0.55 g of pure [8-(2-acetamidoethyl)-3-acetyl-6,7-dihydro-MeBmt]-1-[(γ-hydroxy)-N-MeLeu]-4-cyclosporin [Molecular Formula: C₆₈H₁₂₂N₁₂O₁₅; Exact Mass: 1346.92; MS (m/z): 1347.63 (M+1)⁺; HPLC RT: 13.74 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(γ-hydroxy)-N-MeLeu]-4-cyclosporin

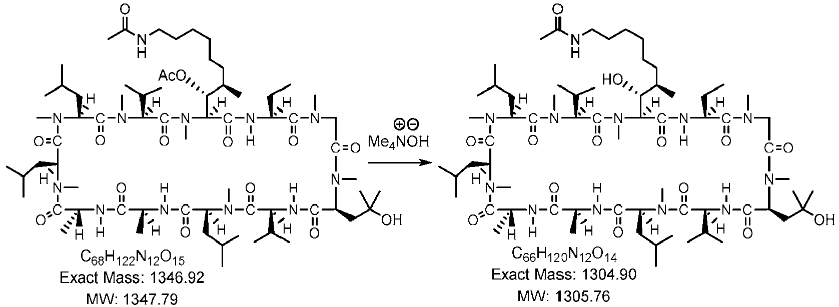

[8-(2-Acetamidoethyl)-3-acetyl-6,7-dihydro-MeBmt]-1-[(γ-hydroxy)-N-MeLeu]-4-cyclosporin (0.50 g, 0.37 mmol) was dissolved in methanol (50 ml). Tetramethylammonium hydroxide pentahydrate (0.22 g, 1.21 mmol) was added. The mixture was stirred at room temperature for two days. Then most of the methanol was evaporated. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 0.22 g of [8-(2-acetamidoethyl)-6,7-dihydro-MeBmt]-1-[(γ-hydroxy)-N-MeLeu]-4-cyclosporin [Molecular Formula: C₆₆H₁₂₀N₁₂O₁₄; Exact Mass: 1304.90; MS (m/z): 1305.72 (M+1)⁺; HPLC RT: 12.80 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[α-methylene-Sar]-3-[(γ-hydroxy)-N-MeLeu]-4-cyclosporin

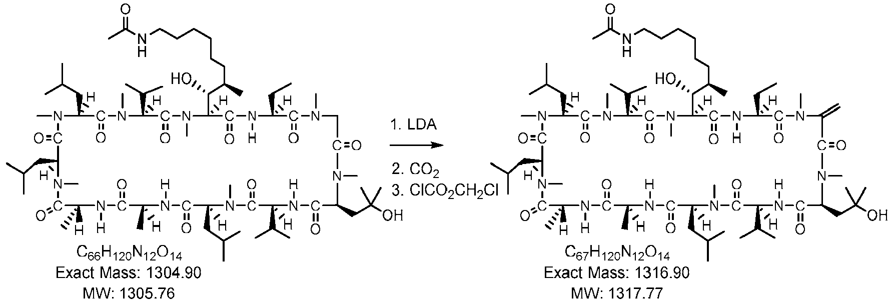

n-Butyllithium (2.65 M, 5.3 ml, 14.04 mmol) was added to a solution of diisopropylamine (2.11 ml, 14.85 mmol) in tetrahydrofuran (50 ml) at -78 °C under nitrogen. After the reaction mixture was stirred for an hour, a solution of [8-(2-acetamidoethyl)-6,7-dihydro-MeBmt]-1-[(γ-hydroxy)-N-MeLeu]-4-cyclosporin (1.60 g, 1.23 mmol) in tetrahydrofuran (6 ml) was added over ten minutes. The mixture was stirred at -78 °C for three hours. After carbon dioxide gas was bubbled into the reaction mixture for 30 minutes, the mixture was stirred at -78 °C for another hour. Then the cooling bath was removed and the reaction mixture was allowed to warm up to room temperature to let unreacted carbon dioxide come out. The mixture was cooled to -78 °C and chloromethyl chloroformate (1.32 ml, 14.85 mmol) was added. The mixture was stirred and allowed to warm to room temperature overnight. Brine (5 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 0.50 g of pure [8-(2-acetamidoethyl)-6,7-dihydro-MeBmt]-1-[α-methylene-Sar]-3-[(γ-hydroxy)-N-MeLeu]-4-cyclosporin [Molecular Formula: C₆₇H₁₂₀N₁₂O₁₄; Exact Mass: 1316.90; MS (m/z): 1317.66 (M+1)⁺; HPLC RT: 14.32 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-(4-hydroxybutylthio)methyl-Sar]-3-[(γ-hydroxy)-N-MeLeu]-4-cyclosporin

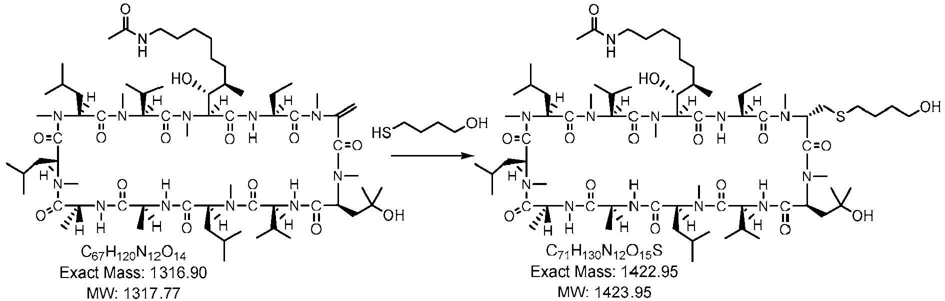

To a solution of [8-(2-acetamidoethyl)-6,7-dihydro-MeBmt]-1-[α-methylene-Sar]-3-[(y-hydroxy)-N-MeLeu]-4-cyclosporin (0.30 g, 0.23 mmol) in methanol (20 ml) were added 4-mercapto-1-butanol (0.14 ml, 1.38 mmol) and lithium hydroxide (54 mg, 2.31 mmol). The reaction mixture was stirred at room temperature for five hours. Most of the methanol was evaporated under reduced pressure. Ethyl acetate (30 ml) and brine (30 ml) were added and the mixture was separated. The organic layer was dried over magnesium sulfate and evaporated under reduced pressure. The residue was purified by chromatography (dichloromethane/methanol) to give 120 mg of pure [8-(2-acetamidoethyl)-6,7-dihydro-MeBmt]-1-[(S)-(4-hydroxybutylthio)methyl-Sar]-3-[(γ-hydroxy)-N-MeLeu]-4-cyclosporin [Molecular Formula: C₇₁H₁₃₀N₁₂O₁₅S; Exact Mass: 1422.95; MS (m/z): 1423.74 (M+1)⁺; HPLC RT: 11.98 min. (C8 reverse phase column: 250 mm; acetonitrile/water (0.05% trifluoroacetic acid); operation temperature: 64 °C; detector: 210 nm)].

### Examples 37

### [8-(3-Acetamidopropyl)-6,7-dihydro-MeBmt]-1-[(R)-(3-hydroxypropyl)thio-Sar]-3-cyclosporin

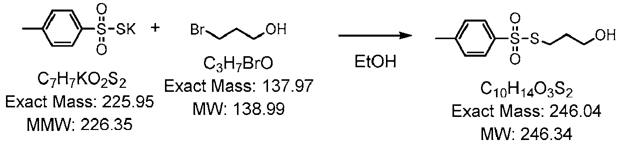

p-Toluenethiosulfonic acid potassium salt (4.5 g, 19.91 mmol) and 3-bromo-1-propanol (2.8 g, 20.29 mmol) were added to ethanol (50 ml). The reaction mixture was stirred and heated to reflux for four hours. Most of ethanol was evaporated under reduced pressure. The residue was mixed with ethyl acetate (100 ml). The ethyl acetate layer was washed with brine, dried over magnesium sulfate and evaporated to give 14.79 g of crude S-(3-hydroxypropyl) 4-methylbenzenesulfonothioate.

### S-(3-((Tetrahydro-2H-pyran-2-yl)oxy)propyl) 4-methylbenzenesulfonothioate

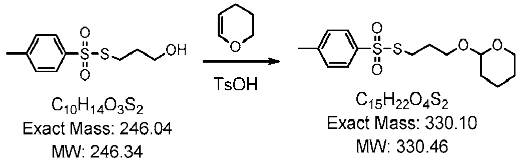

S-(3-hydroxypropyl) 4-methylbenzenesulfonothioate (7.20 g, 29.26 mmol) was dissolved in dichloromethane (100 ml). 3,4-Dihydro-2H-pyran (3.00 g, 35.66 mmol) and p-toluenesulfonic acid monohydrate (1.00 g, 5.26 mmol) were added. The mixture was stirred at room temperature overnight. The dichloromethane was washed with sodium bicarbonate solution and brine, dried over magnesium sulfate and evaporated under reduced pressure. The residue was purified by chromatography to give 6.50 g of pure S-(3-((tetrahydro-2H-pyran-2-yl)oxy)propyl) 4-methylbenzenesulfonothioate. [Molecular Formula: C₁₅H₂₂O₄S₂; Exact Mass: 330.10; MS (m/z): 330.95 (M+1)⁺.

### [8-(3-Aminopropyl)-6,7-dihydro-MeBmt]-1-[(R)-(3-((tetrahydro-2H-pyran-2-yl)oxy)propyl)thio-Sar]-3-cyclosporin

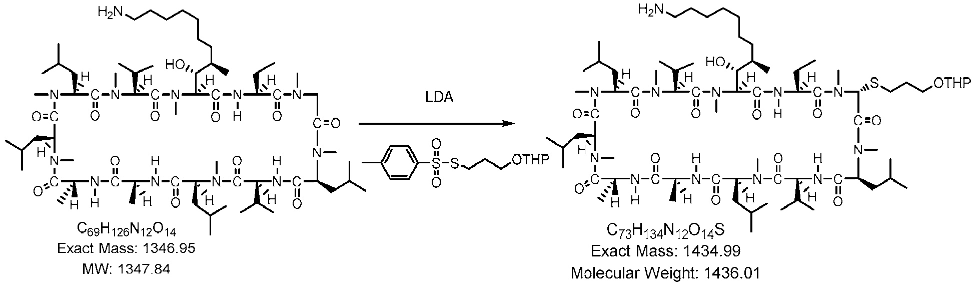

n-Butyllithium (2.60 M, 6.40 ml, 16.64 mmol) was added to a solution of diisopropylamine (2.32 ml, 16.33 mmol) in tetrahydrofuran (50 ml) at -78 °C under nitrogen. After the reaction mixture was stirred for an hour, a solution of [8-(3-(aminopropyl)-6,7-dihydro-MeBmt]-1-cyclosporin (1.50 g, 1.11 mmol) in tetrahydrofuran (15 ml) was added over ten minutes. The mixture was stirred at -78 °C for two hours. S-(3-((Tetrahydro-2H-pyran-2-yl)oxy)propyl) 4-methylbenzenesulfonothioate (2.70 g, 8.18 mmol) in tetrahydrofuran (10 ml) was added and the mixture was stirred at -78 °C for another two hours. Then the cooling bath was removed and the reaction mixture was allowed to warm up to room temperature and stirred for another two hours. Saturated ammonium chloride solution (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (dichloromethane/methanol) to give 0.38 g of [8-(3-aminopropyl)-6,7-dihydro-MeBmt]-1-[(R)-(3-((tetrahydro-2H-pyran-2-yl)oxy)propyl)thio-Sar]-3-cyclosporin [Molecular Formula: C₇₃H₁₃₄N₁₂O₁₄S; Exact Mass: 1434.99; MS (m/z): 1435.70 (M+1)⁺; HPLC RT: 14.93 min. (C8 reverse phase column: 250 mm; acetonitrile/water (0.05% trifluoroacetic acid); operation temperature: 64 °C; detector: 210 nm)].

### [8-(3-Acetamidopropyl)-6,7-dihydro-MeBmt]-1-[(R)-(3-((tetrahydro-2H-pyran-2-yl)oxy)propyl)thio-Sar]-3-cyclosporin

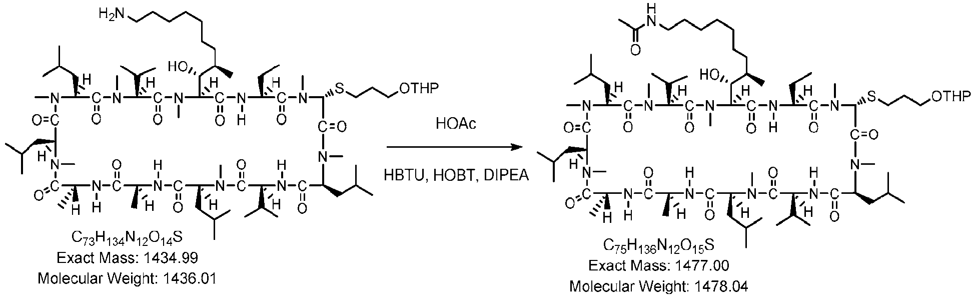

To a solution of [8-(3-aminopropyl)-6,7-dihydro-MeBmt]-1-[(R)-(3-((tetrahydro-2H-pyran-2-yl)oxy)propyl)thio-Sar]-3-cyclosporin (0.50 g, 0.35 mmol) and acetic acid (50 mg, 0.83 mmol) in dichloromethane (50 ml) were added diisopropylethylamine (90 mg, 0.70 mmol), HOBT (85 mg, 0.56 mmol) and HBTU (237 mg, 0.63 mmol). The reaction mixture was stirred at room temperature for two hours and then washed with saturated sodium bicarbonate solution, brine, dried over magnesium sulfate and evaporated under reduced pressure. The residue was purified by chromatography (dichloromethane/methanol) to give 142 mg of pure [8-(3-acetamidopropyl)-6,7-dihydro-MeBmt]-1-[(R)-(3-((tetrahydro-2H-pyran-2-yl)oxy)propyl)thio-Sar]-3-cyclosporin [Molecular Formula: C₇₅H₁₃₅N₁₂O₁₅S; Exact Mass: 1477.00; MS (m/z): 1477.65(M+1)⁺. HPLC RT: 18.17min (C8 reverse phase column: 250 mm; acetonitrile/water (0.05% trifluoroacetic acid); operation temperature: 64 °C; detector: 210 nm)].

### [8-(3-Acetamidopropyl)-6,7-dihydro-MeBmt]-1-[(R)-(3-hydroxypropyl)thio-Sar]-3-cyclosporin

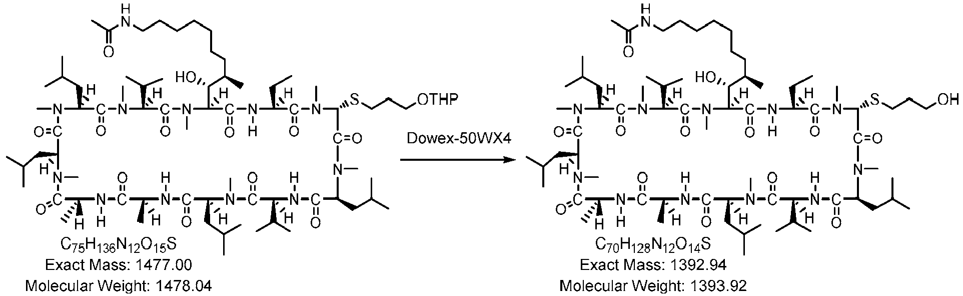

To a solution of [8-(3-acetamidopropyl)-6,7-dihydro-MeBmt]-1-[(R)-(3-((tetrahydro-2H-pyran-2-yl)oxy)propyl)thio-Sar]-3-cyclosporin (0.14 g, 0.09 mmol) in methanol (15 ml) was added Dowex-50WX4 (200 mg). The reaction mixture was stirred at room temperature for four hours and then filtered. Most the methanol was evaporated under reduced pressure and the residue was purified by chromatography (dichloromethane/methanol) to give 48 mg of pure [8-(3-acetamidopropyl)-6,7-dihydro-MeBmt]-1-[(R)-(3-hydroxypropyl)thio-Sar]-3-cyclosporin [Molecular Formula: C₇₀H₁₂₈N₁₂O₁₄S; Exact Mass: 1392.94; MS (m/z): 1393.66(M+1)⁺. HPLC RT: 15.38min (C8 reverse phase column: 250 mm; acetonitrile/water (0.05% trifluoroacetic acid); operation temperature: 64 °C; detector: 210 nm)].

### Examples 36

### [8-(2-Acetamidoethyl)-6,7-dihydro-MeBmt]-1-[(R)-(4-hydroxybutyl)thio-Sar]-3-cyclosporin

### 4-(Tosylthio)butyl benzoate

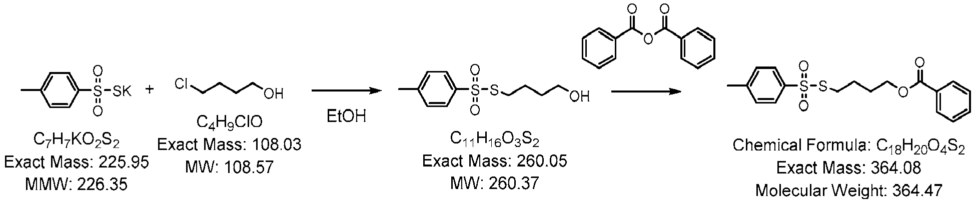

P-Toluenethiosulfonic acid potassium salt (20.00 g, 88.36 mmol) and 4-chloro-1-butanol (9.60 g, 88.36 mmol) were added to ethanol (160 ml). The reaction mixture was stirred and heated to reflux for four hours. Most of ethanol was evaporated under reduced pressure. The residue was mixed with ethyl acetate (100 ml). The ethyl acetate layer was washed with brine, dried over magnesium sulfate and evaporated to give 14.79 g of crude S-(4-hydroxybutyl) 4-methylbenzenesulfonothioate.

S-(4-Hydroxybutyl) 4-methylbenzenesulfonothioate (14.79 g, 56.90 mmol) was dissolved in acetone (60ml). Benzoic anhydride (25.74, 113.80 mmol), 4-(dimethylamino)pyridine (1.39 g, 11.38 mmol) and pyridine (30 ml) were added. The mixture was stirred at room temperature overnight. Most of acetone and pyridine were evaporated under reduced pressure. Ethyl acetate (100 ml) was added. The ethyl acetate layer was washed with hydrochloric acid solution (1.00 N), brine, saturated sodium bicarbonate solution and brine, dried over magnesium sulfate and evaporated under reduced pressure. The residue was purified by chromatography (hexane/acetone) to give 3.30 g of pure 4-(tosylthio)butyl benzoate [Molecular Formula: C₁₈H₂₀O₄S₂; Exact Mass: 364.08; MS (m/z): 364.57 (M+1)⁺; HPLC RT: 19.44 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(4-(benzoyloxy)butyl)thio-Sar1-3-cyclosporin

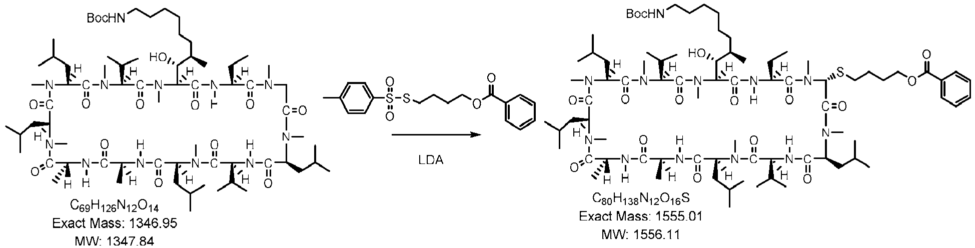

n-Butyllithium (2.60 M, 6.28 ml, 16.33 mmol) was added to a solution of diisopropylamine (2.32 ml, 16.33 mmol) in tetrahydrofuran (50 ml) at -78 °C under nitrogen. After the reaction mixture was stirred for an hour, a solution of [8-(2-(tert-butoxycarbonyl)aminoethyl)-6,7-dihydro-MeBmt]-1-cyclosporin (2.20 g, 1.63 mmol) in tetrahydrofuran (15 ml) was added over ten minutes. The mixture was stirred at -78 °C for two hours. 4-(Tosylthio)butyl benzoate (3.56 g, 9.78 mmol) in tetrahydrofuran (10 ml) was added and the mixture was stirred at -78 °C for another two hours. Then the cooling bath was removed and the reaction mixture was allowed to warm up to room temperature and stirred for another two hours. Saturated ammonium chloride solution (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure to give 0.38 g of crude [8-(2-(tert-butoxycarbonyl)aminoethyl)-6,7-dihydro-MeBmt]-1-[(R)-(4-(benzoyloxy)butyl)thio-Sar]-3-cyclosporin [Molecular Formula: C₈₀H₁₃₈N₁₂O₁₆S; Exact Mass: 1555.01; MS (m/z): 1555.72 (M+1)⁺; HPLC RT: 19.21 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(4-hydroxybutyl)thio-Sar]-3-cyclosporin

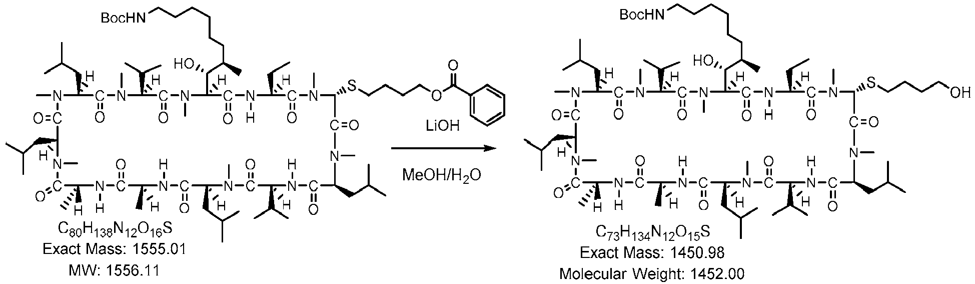

[8-(2-(tert-Butoxycarbonyl)aminoethyl)-6,7-dihydro-MeBmt]-1-[(R)-(4-(benzoyloxy)butyl)thio-Sar]-3-cyclosporin from prior step was dissolved in methanol (10 ml). Lithium hydroxide (2.00 g, 83.33 mmol) and water (10 ml) were added. The mixture was stirred at room temperature for two hours. Then most of the methanol was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 0.38 g of pure [8-(2-(tert-butoxycarbonyl)aminoethyl)-6,7-dihydro-MeBmt]-1-[(R)-(4-hydroxybutyl)thio-Sar]-3-cyclosporin [Molecular Formula: C₇₃H₁₃₄N₁₂O₁₅S; Exact Mass: 1450.98; MS (m/z): 1451.68 (M+1)⁺, 1473.79 (M+Na)⁺; HPLC RT: 17.06 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(R)-(4-hydroxybutyl)thio-Sar]-3-cyclosporin

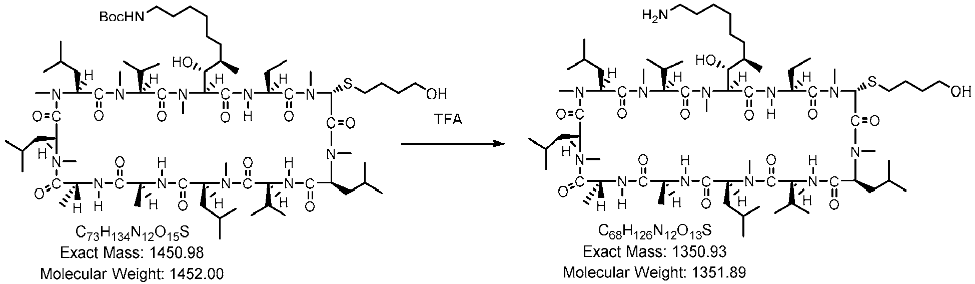

[8-(2-(tert-Butoxycarbonyl)aminoethyl)-6,7-dihydro-MeBmt]-1-[(R)-(4-hydroxybutyl)thio-Sar]-3-cyclosporin (0.38 g, 0.26 mmol) was dissolved in dichloromethane (12 ml) and put into ice-water bath. Trifluoroacetic acid (4 ml) was added. The mixture was stirred at 0 °C for three hours. Another dichloromethane (50 ml) was added. The mixture was washed with brine (50 ml), saturated sodium bicarbonate solution (50 ml) and dried over magnesium sulfate and evaporated under reduced pressure to give crude [8-(2-aminoethyl)-6,7-dihydro-MeBmt]-1-[(R)-(4-hydroxybutyl)thio-Sar]-3-cyclosporin [Molecular Formula: C₆₈H₁₂₆N₁₂O₁₃S; Exact Mass: 1350.93; MS (m/z): 1351.67 (M+1)⁺; HPLC RT: 10.72 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-(4-hydroxybutyl)thio-Sar]-3-cyclosporin

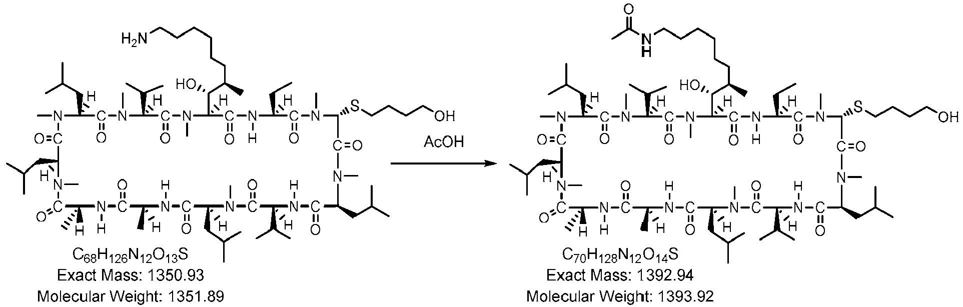

Crude [8-(2-aminoethyl)-6,7-dihydro-MeBmt]-1-[(R)-(4-hydroxybutyl)thio-Sar]-3-cyclosporin was dissolved dichloromethane (15 ml). Acetic acid (0.08 g, 1.33 mmol), HBTU (0.30 g, 0.79 mmol), 1-hydroxybenzotriazole (0.11 g, 0.79 mmol) and diisopropylethylamine (0.50 ml) were added. The mixture was stirred at room temperature for two hours. Then most of solvent was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 25 mg of pure [8-(2-acetamidoethyl)-6,7-dihydro-MeBmt]-1-[(R)-(4-hydroxybutyl)thio-Sar]-3-cyclosporin [Molecular Formula: C₇₀H₁₂₉N₁₂O₁₄S; Exact Mass: 1392.94; MS (m/z): 1393.98 (M+1)⁺, 1415.92 (M+Na)⁺; HPLC RT: 15.09 min. (C8 reverse phase column: 250 mm; acetonitrile/water (0.05% trifluoroacetic acid); operation temperature: 64 °C; detector: 210 nm)].

### Examples 39

### [8-(2-Acetamidoethyl)-6,7-dihydro-MeBmt]-1-[(R)-(5-hydroxypentyl)thio-Sar]-3-cyclosporin

### 5-(Tosylthio)pentyl benzoate

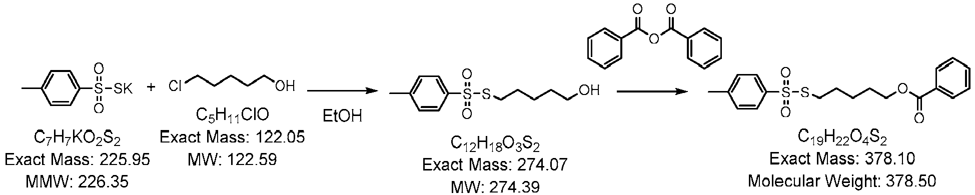

P-Toluenethiosulfonic acid potassium salt (11.80 g, 47.71 mmol) and 5-chloro-1-pentanol (5.84 g, 47.71 mmol) were added to ethanol (100 ml). The reaction mixture was stirred and heated to reflux overnight. Most of ethanol was evaporated under reduced pressure. The residue was mixed with ethyl acetate (100 ml). The ethyl acetate layer was washed with brine, dried over magnesium sulfate and evaporated to give 9.16 g of crude S-(5-hydroxypentyl) 4-methylbenzenesulfonothioate.

S-(5-Hydroxypentyl) 4-methylbenzenesulfonothioate (9.16 g, 33.42 mmol) was dissolved in acetone (60ml). Benzoic anhydride (15.12, 66.83 mmol), 4-(dimethylamino)pyridine (0.82 g, 6.71 mmol) and pyridine (30 ml) were added. The mixture was stirred at room temperature overnight. Most of acetone and pyridine were evaporated under reduced pressure. Ethyl acetate (100 ml) was added. The ethyl acetate layer was washed with hydrochloric acid solution (1.00 N), brine, saturated sodium bicarbonate solution and brine, dried over magnesium sulfate and evaporated under reduced pressure. The residue was purified by chromatography (hexane/acetone) to give 7.78 g of pure 5-(tosylthio)pentyl benzoate [Molecular Formula: C₁₉H₂₂O₄S₂; Exact Mass: 378.10; MS (m/z): 378.57 (M+1)⁺; HPLC RT: 20.24 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(5-(benzoyloxy)pentyl)thio-Sar]-3-cyclosporin

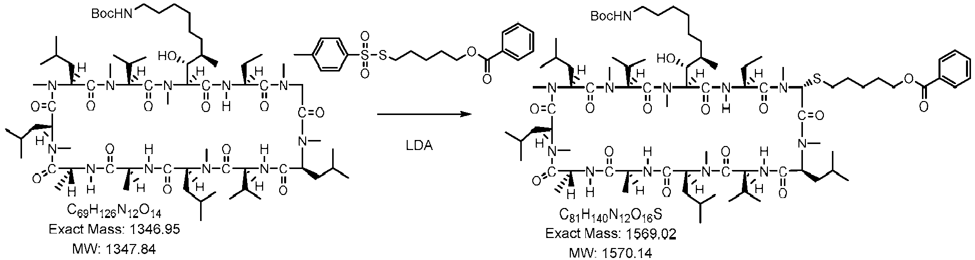

n-Butyllithium (2.65 M, 6.16 ml, 16.33 mmol) was added to a solution of diisopropylamine (2.32 ml, 16.33 mmol) in tetrahydrofuran (50 ml) at -78 °C under nitrogen. After the reaction mixture was stirred for an hour, a solution of [8-(2-(tert-butoxycarbonyl)aminoethyl)-6,7-dihydro-MeBmt]-1-cyclosporin (2.20 g, 1.63 mmol) in tetrahydrofuran (15 ml) was added over ten minutes. The mixture was stirred at -78 °C for two hours. 5-(Tosylthio)pentyl benzoate (3.71 g, 9.80 mmol) in tetrahydrofuran (10 ml) was added and the mixture was stirred at -78 °C for another two hours. Then the cooling bath was removed and the reaction mixture was allowed to warm up to room temperature and stirred for another two hours. Saturated ammonium chloride solution (20 ml) was added to quench the reaction. Most of tetrahydrofuran was removed under reduced pressure. Ethyl acetate (80 ml) and brine (80 ml) were added and the mixture was separated. The organic layer was dried over magnesium sulfate and evaporated under reduced pressure to give 0.38 g of crude [8-(2-(tert-butoxycarbonyl)aminoethyl)-6,7-dihydro-MeBmt]-1-[(R)-(5-(benzoyloxy)pentyl)thio-Sar]-3-cyclosporin [Molecular Formula: C₈₁H₁₄₀N₁₂O₁₆S; Exact Mass: 1569.02; MS (m/z): 1569.73 (M+1)⁺; HPLC RT: 19.56 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(5-hydroxypentyl)thio-Sar]-3-cyclosporin

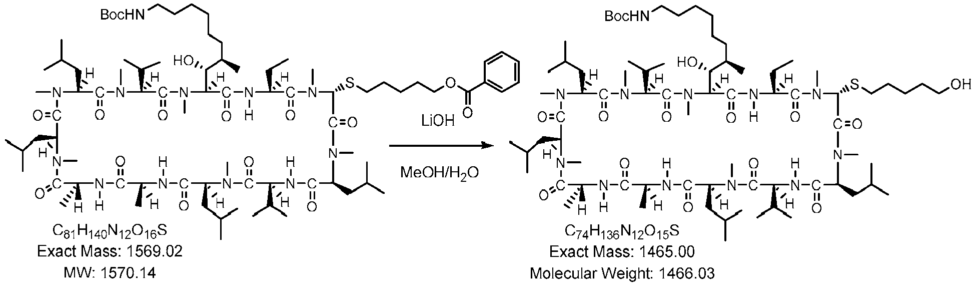

[8-(2-(tert-Butoxycarbonyl)aminoethyl)-6,7-dihydro-MeBmt]-1-[(R)-(5-(benzoyloxy)pentyl)thio -Sar]-3-cyclosporin from prior step was dissolved in methanol (25 ml). Lithium hydroxide (1.25 g, 52.08 mmol) and water (25 ml) were added. The mixture was stirred at room temperature for two hours. Then most of the methanol was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 0.22 g of pure [8-(2-(tert-butoxycarbonyl)aminoethyl)-6,7-dihydro-MeBmt]-1-[(R)-(5-hydroxypentyl)thio-Sar]-3-cyclosporin [Molecular Formula: C₇₄H₁₃₆N₁₂O₁₅S; Exact Mass: 1465.00; MS (m/z): 1465.68 (M+1)⁺, 1487.79 (M+Na)⁺; HPLC RT: 17.44 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(R)-(5-hydroxypentyl)thio-Sar]-3-cyclosporin

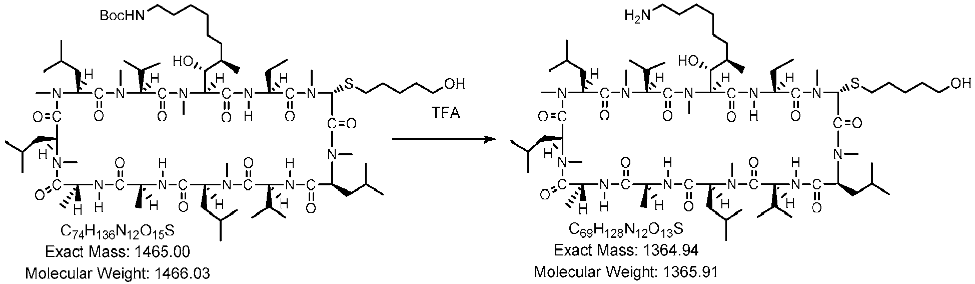

[8-(2-(tert-Butoxycarbonyl)aminoethyl)-6,7-dihydro-MeBmt]-1-[(R)-(5-hydroxypentyl)thio-Sar]-3-cyclosporin (0.22 g, 0.15 mmol) was dissolved in dichloromethane (6 ml) and put into ice-water bath. Trifluoroacetic acid (2 ml) was added. The mixture was stirred at 0 °C for three hours. Another dichloromethane (50 ml) was added. The dichloromethane layer was washed with brine (50 ml), saturated sodium bicarbonate solution (50 ml) and dried over magnesium sulfate and evaporated under reduced pressure to give crude [8-(2-aminoethyl)-6,7-dihydro-MeBmt]-1-[(R)-(5-hydroxypentyl)thio-Sar]-3-cyclosporin [Molecular Formula: C₆₉H₁₂₈N₁₂O₁₃S; Exact Mass: 1364.94; MS (m/z): 1365.67 (M+1)⁺; HPLC RT: 11.53 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-(5-hydroxypentyl)thio-Sar]-3-cyclosporin

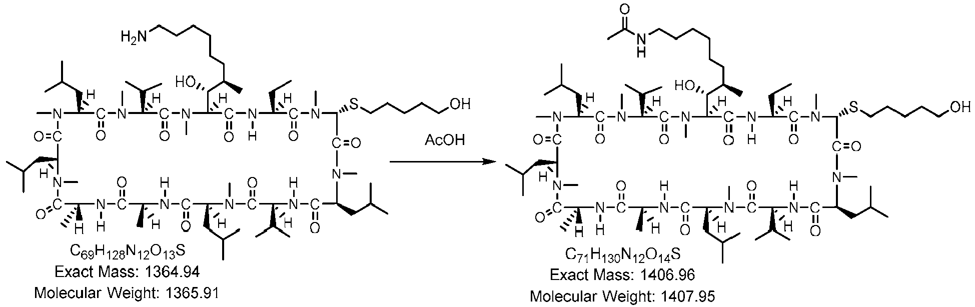

Crude [8-(2-aminoethyl)-6,7-dihydro-MeBmt]-1-[(R)-(5-hydroxypentyl)thio-Sar]-3-cyclosporin was dissolved dichloromethane (15 ml). Acetic acid (0.05 g, 0.83 mmol), HBTU (0.17 g, 0.45mmol), 1-hydroxybenzotriazole (0.06 g, 0.45 mmol) and pyridine (0.50 ml) were added. The mixture was stirred at room temperature for three hours. Then most of solvent was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 12 mg of pure [8-(2-acetamidoethyl)-6,7-dihydro-MeBmt]-1-[(R)-(5-hydroxypentyl)thio-Sar]-3-cyclosporin [Molecular Formula: C₇₁H₁₃₀N₁₂O₁₄S; Exact Mass: 1406.96; MS (m/z): 1407.77 (M+1)⁺, 1429.96 (M+Na)⁺; HPLC RT: 15.72 min. (C8 reverse phase column: 250 mm; acetonitrile/water (0.05% trifluoroacetic acid); operation temperature: 64 °C; detector: 210 nm)].

### Examples 40

### [8-(2-Acetamidoethyl)-6,7-dihydro-MeBmt]-1-[(R)-(6-hydroxyhexyl)thio-Sar]-3-cyclosporin

### 6-(Tosylthio)hexyl benzoate

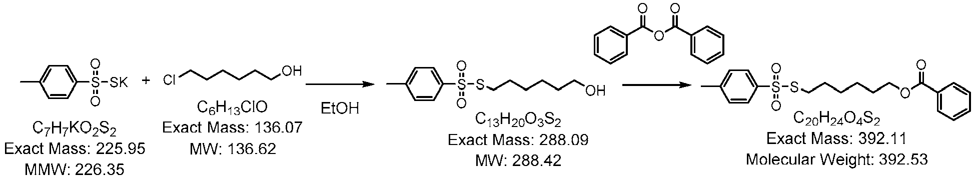

P-Toluenethiosulfonic acid potassium salt (18.00 g, 79.52 mmol) and 6-chloro-1-hexanol (10.86 g, 79.52 mmol) were added to ethanol (160 ml). The reaction mixture was stirred and heated to reflux for five hours. Most of ethanol was evaporated under reduced pressure. The residue was mixed with ethyl acetate (200 ml). The ethyl acetate layer was washed with brine, dried over magnesium sulfate and evaporated to give 15.33 g of crude S-(6-hydroxyhexyl) 4-methylbenzenesulfonothioate.

S-(6-Hydroxyhexyl) 4-methylbenzenesulfonothioate (15.33 g, 53.23 mmol) was dissolved in acetone (60ml). Benzoic anhydride (24.08, 106.46 mmol), 4-(dimethylamino)pyridine (1.30 g, 10.65 mmol) and pyridine (30 ml) were added. The mixture was stirred at room temperature overnight. Most of acetone and pyridine were evaporated under reduced pressure. Ethyl acetate (100 ml) was added. The ethyl acetate layer was washed with hydrochloric acid solution (1.00 N), brine, saturated sodium bicarbonate solution and brine, dried over magnesium sulfate and evaporated under reduced pressure. The residue was purified by chromatography (hexane/acetone) to give 5.41 g of pure 6-(tosylthio)hexyl benzoate [Molecular Formula: C₂₀H₂₄O₄S₂; Exact Mass: 392.11; MS (m/z): 392.57 (M+1)⁺; HPLC RT: 20.86 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(6-(benzoyloxy)hexyl)thio-Sar]-3-cyclosporin

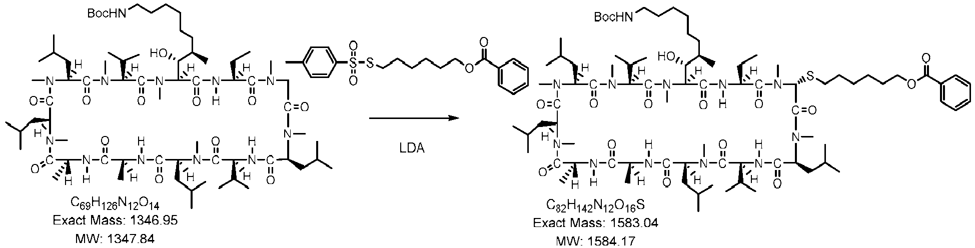

n-Butyllithium (2.60 M, 6.28 ml, 16.33 mmol) was added to a solution of diisopropylamine (2.32 ml, 16.33 mmol) in tetrahydrofuran (50 ml) at -78 °C under nitrogen. After the reaction mixture was stirred for an hour, a solution of [8-(2-(tert-butoxycarbonyl)aminoethyl)-6,7-dihydro-MeBmt]-1-cyclosporin (2.20 g, 1.63 mmol) in tetrahydrofuran (15 ml) was added over ten minutes. The mixture was stirred at -78 °C for two hours. 6-(Tosylthio)hexyl benzoate (3.83 g, 9.78 mmol) in tetrahydrofuran (10 ml) was added and the mixture was stirred at -78 °C for another two hours. Then the cooling bath was removed and the reaction mixture was allowed to warm up to room temperature and stirred for another two hours. Saturated ammonium chloride solution (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure to give crude [8-(2-(tert-butoxycarbonyl)aminoethyl)-6,7-dihydro-MeBmt]-1-[(R)-(6-(benzoyloxy)hexyl)thio-Sar]-3-cyclosporin [Molecular Formula: C₈₂H₁₄₂N₁₂O₁₆S; Exact Mass: 1583.04; MS (m/z): 1583.75(M+1)⁺; HPLC RT: 19.27 min. (C8 reverse phase column: 250 mm; acetonitrile/water (0.05% trifluoroacetic acid); operation temperature: 64 °C; detector: 210 nm)].

### [8-(2-(tert-Butoxycarbonyl)aminoethyl)-6,7-dihydro-MeBmt]-1-[(R)-(6-hydroxyhexyl)thio-Sar]-3-cyclosporin

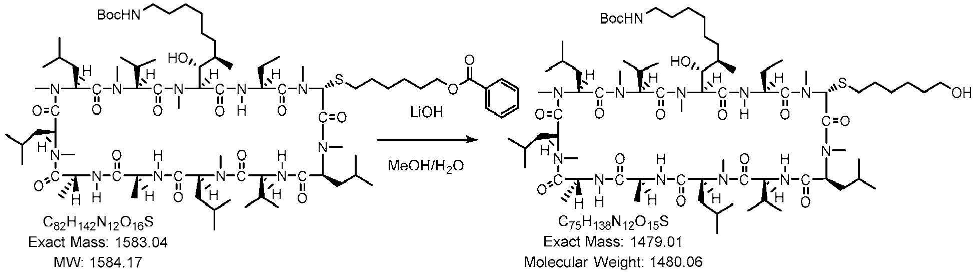

[8-(2-(tert-Butoxycarbonyl)aminoethyl)-6,7-dihydro-MeBmt]-1-[(R)-(6-(benzoyloxy)hexyl)thio-Sar]-3-cyclosporin was dissolved in methanol (10 ml). Lithium hydroxide (1.25 g, 52.08 mmol) and water (10 ml) were added. The mixture was stirred at room temperature for two hours. Then most of the methanol was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 0.34 g of pure [8-(2-(tert-butoxycarbonyl)aminoethyl)-6,7-dihydro-MeBmt]-1-[(R)-(6-hydroxyhexyl)thio-Sar]-3-cyclosporin [Molecular Formula: C₇₅H₁₃₈N₁₂O₁₅S; Exact Mass: 1479.01; MS (m/z): 1479.86 (M+1)⁺, 1501.80 (M+Na)⁺; HPLC RT: 17.86 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(R)-(6-hydroxyhexyl)thio-Sar]-3-cyclosporin

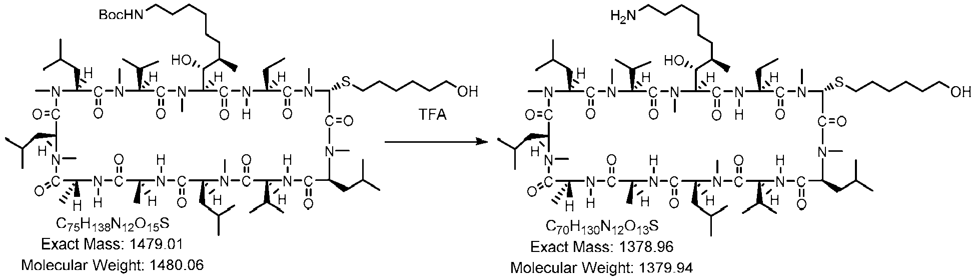

[8-(2-(tert-Butoxycarbonyl)aminoethyl)-6,7-dihydro-MeBmt]-1-[(R)-(6-hydroxyhexyl)thio-Sar]-3-cyclosporin (0.34 g, 0.15 mmol) was dissolved in dichloromethane (15 ml) and put into ice-water bath. Trifluoroacetic acid (5 ml) was added. The mixture was stirred at 0 °C for three hours. Another dichloromethane (50 ml) was added. The dichloromethane layer was washed with brine (50 ml), saturated sodium bicarbonate solution (50 ml) and dried over magnesium sulfate and evaporated under reduced pressure to give 0.14 g of crude [8-(2-aminoethyl)-6,7-dihydro-MeBmt]-1-[(R)-(6-hydroxyhexyl)thio-Sar]-3-cyclosporin [Molecular Formula: C₇₀H₁₃₀N₁₂O₁₃S; Exact Mass: 1378.96; MS (m/z): 1379.67 (M+1)⁺; HPLC RT: 12.45 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-(6-hydroxyhexyl)thio-Sar]-3-cyclosporin

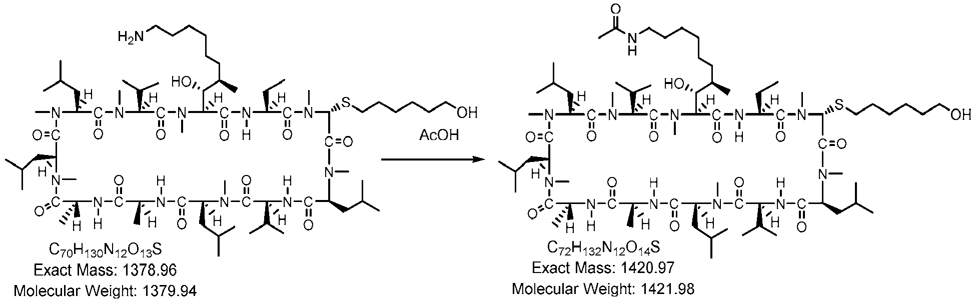

Crude [8-(2-aminoethyl)-6,7-dihydro-MeBmt]-1-[(R)-(6-hydroxyhexyl)thio-Sar]-3-cyclosporin dissolved dichloromethane (15 ml). Acetic acid (0.14 g, 2.43 mmol), HBTU (0.55 g, 1.46 mmol), 1-hydroxybenzotriazole (0.20 g, 1.46 mmol) and pyridine (1.0 ml) were added. The mixture was stirred at room temperature overnight. Then most of solvent was evaporated under reduced pressure. Ethyl acetate (50 ml) and brine (50 ml) were added and separated. The ethyl acetate layer was dried over magnesium sulfate and evaporated under reduced pressure. The residue was purified by chromatography (hexane/acetone) to give 14 mg of pure [8-(2-acetamidoethyl)-6,7-dihydro-MeBmt]-1-[(R)-(6-hydroxyhexyl)thio-Sar]-3-cyclosporin [Molecular Formula: C₇₂H₁₃₂N₁₂O₁₄S; Exact Mass: 1420.97; MS (m/z): 1422.08 (M+1)⁺, 1443.93 (M+Na)⁺; HPLC RT: 16.23 min. (C8 reverse phase column: 250 mm; acetonitrile/water (0.05% trifluoroacetic acid); operation temperature: 64 °C; detector: 210 nm)].

### Examples 41

### [8-(2-Acetamidoethyl)-6,7-dihydro-MeBmt]-1-[(S)-((4-hydroxy-2-methylbutan-2-yl)thio)methyl-Sar]-3-cyclosporin

### 3-Methyl-3-mercaptobutyricic acid

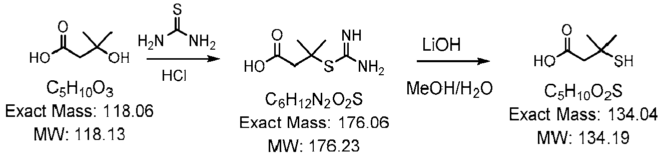

3-Hydroxy-3-methylbutyric acid (10.00 g, 84.65 mmol), hydrochloric acid (36%, 30 ml) and thiourea (7.08 g, 93.12 mmol) were mixed and heated to reflux for twenty four hours. Then the mixture was evaporated under reduced pressure. Methanol (10 ml) and a solution of lithium hydroxide (6.09 g, 253.95 mmol) in water (10 ml) were added. The mixture was stirred and heated to reflux overnight. After cooled to room temperature, the mixture was filtered. The filtrate was evaporated under reduced pressure to give crude product.

### [8-(2-(tert-Butoxycarbonyl)aminoethyl)-6,7-dihydro-MeBmt]-1-[(S)-((1-carboxy-2-methylpropan-2-yl)thio)methyl-Sar]-3-cyclosporin

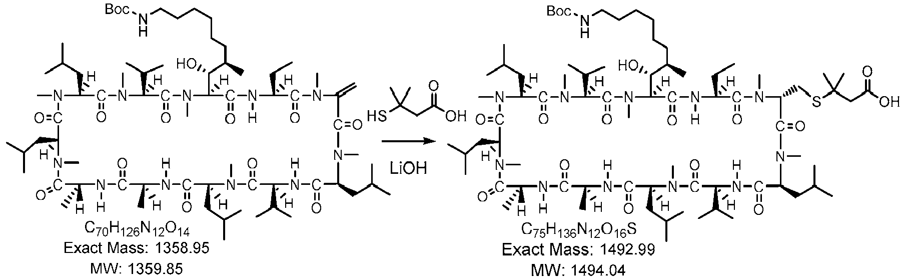

To a solution of [8-(2-(tert-butoxycarbonyl)aminoethyl)-6,7-dihydro-MeBmt]-1-[α-methylene-Sar]-3-cyclosporin (1.00 g, 0.74 mmol) in methanol (10 ml) were added 3-methyl-3-mercaptobutyricic acid (0.30 g, 2.20 mmol) and lithium hydroxide (0.18 g, 7.36 mmol). The reaction mixture was stirred at room temperature overnight. Most of the methanol was evaporated under reduced pressure. Ethyl acetate (50 ml) and brine (50 ml) were added and the PH of the aqueous layer was adjusted to 3 by adding hydrochloric acid solution (1.0 N). The ethyl acetate layer was dried over magnesium sulfate and evaporated under reduced pressure to give crude [8-(2-(tert-butoxycarbonyl)aminoethyl)-6,7-dihydro-MeBmt]-1-[(S)-((1-carboxy-2-methylpropan-2-yl)thio)methyl-Sar]-3-cyclosporin [Molecular Formula: C₇₅H₁₃₆N₁₂O₁₆S; Exact Mass: 1492.99; MS (m/z): 1493.63 (M+1)⁺, 1515.88 (M+Na)⁺].

### [8-(2-(tert-Butoxycarbonyl)aminoethyl)-6,7-dihydro-MeBmt]-1-[(S)-((4-methoxy-2-methyl-4-oxobutan-2-yl)thio)methyl-Sar]-3-cyclosporin

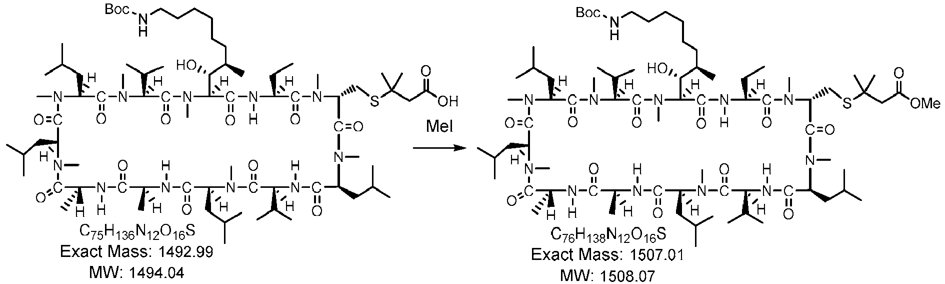

Crude [8-(2-(tert-butoxycarbonyl)aminoethyl)-6,7-dihydro-MeBmt]-1-[(S)-((1-carboxy-2-methylpropan-2-yl)thio)methyl-Sar]-3-cyclosporin was dissolved in acetone (10 ml). Iodomethane (0.14 ml, 2.22 mmol) and potassium carbonate (0.31 g, 2.22 mmol) were added. The mixture was stirred at room temperature for two hours. Most of acetone was evaporated under reduced pressure. Then ethyl acetate (50 ml) and water (50 ml) were added and the mixture was separated. The ethyl acetate layer was dried over magnesium sulfate and evaporated under reduced pressure The residue was purified by chromatography (hexane/acetone) to give 0.16 g of pure [8-(2-(tert-butoxycarbonyl)aminoethyl)-6,7-dihydro-MeBmt]-1-[(S)-((4-methoxy-2-methyl-4-oxobutan-2-yl)thio)methyl-Sar]-3-cyclosporin [Molecular Formula: C₇₆H₁₃₈N₁₂O₁₆S; Exact Mass: 1507.01; MS (m/z): 1507.65 (M+1)⁺, 1529.95 (M+Na)⁺; HPLC RT: 18.34 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(S)-((4-methoxy-2-methyl-4-oxobutan-2-yl)thio)methyl-Sar]-3-cyclosporin

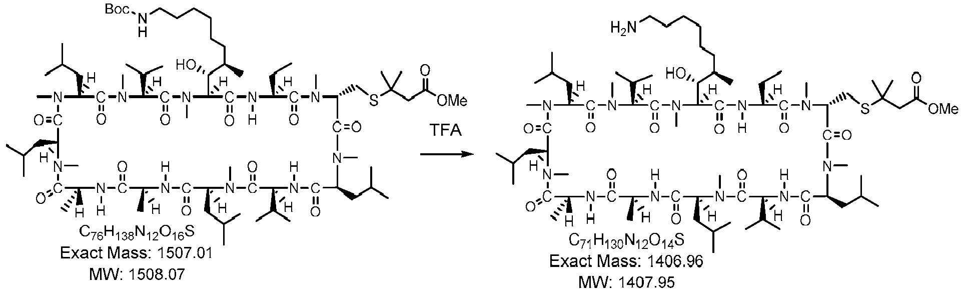

[8-(2-(tert-Butoxycarbonyl)aminoethyl)-6,7-dihydro-MeBmt]-1-[(S)-(4-methoxy-2-methyl-4-oxobutan-2-ylthio)methyl-Sar]-3-cyclosporin (0.16 g, 0.11 mmol) was dissolved in dichloromethane (6 ml) and put into ice-water bath. Trifluoroacetic acid (2 ml) was added. The mixture was stirred at 0 °C for three hours. Another dichloromethane (20 ml) was added. The mixture was washed with brine (30 ml), saturated sodium bicarbonate solution (30 ml) and dried over magnesium sulfate and evaporated under reduced pressure to give crude [8-(2-aminoethyl)-6,7-dihydro-MeBmt]-1-[(S)-((4-methoxy-2-methyl-4-oxobutan-2-yl)thio)methyl-Sar]-3-cyclosporin. Molecular Formula: C₇₁H₁₃₀N₁₂O₁₄S; Exact Mass: 1406.96; MS (m/z): 1407.65 (M+1)⁺; HPLC RT: 13.66 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-((4-methoxy-2-methyl-4-oxobutan-2-yl)thio)methyl-Sar]-3-cyclosporin

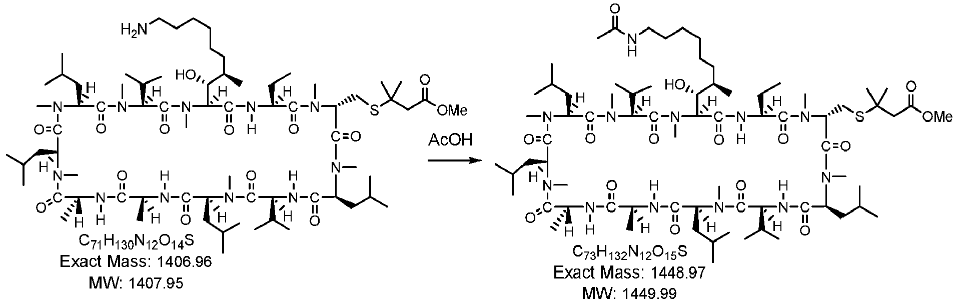

Crude [8-(2-aminoethyl)-6,7-dihydro-MeBmt]-1-[(S)-((4-methoxy-2-methyl-4-oxobutan-2-yl)thio)methyl-Sar]-3-cyclosporin was dissolved dichloromethane (10 ml). Acetic acid (0.03 ml, 20.53 mmol), HBTU (0.12 g, 0.32 mmol), 1-hydroxybenzotriazole (0.04 g, 0.32 mmol) and pyridine (0.50 ml) were added. The mixture was stirred at room temperature for two hours. Then dichloromethane (30 ml) and brine (50 ml) were added and separated. The dichloromethane layer was dried over magnesium sulfate and evaporated under reduced pressure to give crude [8-(2-acetamidoethyl)-6,7-dihydro-MeBmt]-1-[(S)-((4-methoxy-2-methyl-4-oxobutan-2-yl)thio)methyl-Sar]-3-cyclosporin [Molecular Formula: C₇₃H₁₃₂N₁₂O₁₅S; Exact Mass: 1448.97; MS (m/z): 1449.72 (M+1)⁺, 1471.91 (M+Na)⁺; HPLC RT: 17.19 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-((4-hydroxy-2-methylbutan-2-yl)thio)methyl-Sar]-3-cyclosporin

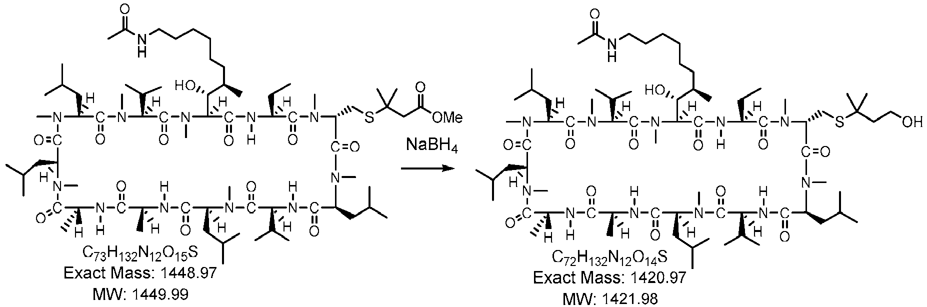

Crude [8-(2-acetamidoethyl)-6,7-dihydro-MeBmt]-1-[(S)-((4-methoxy-2-methyl-4-oxobutan-2-yl)thio)methyl-Sar]-3-cyclosporin was dissolved in tetrahydrofuran (10 ml). Sodium borohydride (0.50 g, 12.88 mmol) and cesium chloride (0.10 g, 0.59 mmol) were added. The mixture was stirred at room temperature and methanol (10 ml) was added dropwise over two hours. Then the mixture was stirred overnight. Most of solvents were evaporated under reduced pressure. Ethyl acetate (50 ml) and brine (50 ml) were added and the mixture was separated. The ethyl acetate layer was dried over magnesium sulfate and evaporated under reduced pressure. The residue was purified by chromatography (dichloromethane/methanol) to give 34 mg of pure [8-(2-acetamidoethyl)-6,7-dihydro-MeBmt]-1-[(S)-((4-hydroxy-2-methylbutan-2-yl)thio)methyl-Sar]-3-cyclosporin [Molecular Formula: C₇₂H₁₃₂N₁₂O₁₄S; Exact Mass: 1420.97; MS (m/z): 1421.87(M+1)⁺, 1444.00 (M+Na)⁺; HPLC RT: 15.67 min. (C8 reverse phase column: 250 mm; acetonitrile/water (0.05% trifluoroacetic acid); operation temperature: 64 °C; detector: 210 nm)].

### Examples 42

### [8-(2-Acetamidoethyl)-6,7-dihydro-MeBmt]-1-[(S)-((5-hydroxy-2-methylpentan-2-yl)thio)methyl-Sar]-3-cyclosporin

### 4-Mercapto-4-methylpentanoic acid

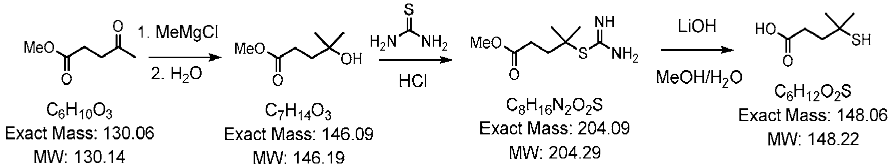

Methyl levulinate (10.00 g, 76.84 mmol) was dissolved in anhydrous tetrahydrofuran (50 ml) under nitrogen. The mixture was put into a dry ice-acetone bath and methylmagnesium chloride (3.00 M, 25.6 ml, 76.84 mmol) was added slowly. The mixture was stirred at -78 °C for two hours and allowed to warm to room temperature overnight. Saturated ammonium chloride solution (20 ml) was added to quench the reaction. Most of tetrahydrofuran was removed under reduced pressure. Ethyl acetate (50 ml) and brine (50 ml) were added and the mixture was separated. The organic layer was dried over magnesium sulfate and evaporated under reduced pressure. The residue was purified by chromatography (hexane/acetone) to give 8.13 g of pure methyl 4-hydroxy-4-methylpentanoate [Molecular Formula: C₇H₁₄O₃; Exact Mass: 146.09; MS (m/z): 146.64 (M+1)⁺],

Methyl 4-hydroxy-4-methylpentanoate (8.13 g, 55.70 mmol), hydrochloric acid (36%, 25 ml) and thiourea (4.66 g, 61.27 mmol) were mixed and heated to reflux for twenty four hours. Then the mixture was evaporated under reduced pressure. Methanol (10 ml) and a solution of lithium hydroxide (4.01 g, 167.09 mmol) in water (10 ml) were added. The mixture was stirred and heated to reflux overnight. After cooled to room temperature, the mixture was filtered. The filtrate was evaporated under reduced pressure to give crude product.

### [8-(2-(tert-Butoxycarbonyl)aminoethyl)-6,7-dihydro-MeBmt]-1-[(S)-((4-carboxy-2-methylbutan-2-yl)thio)methyl-Sar]-3-cyclosporin

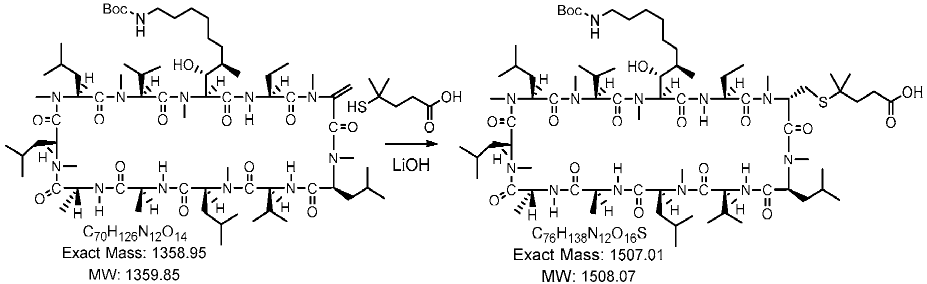

To a solution of [8-(2-(tert-butoxycarbonyl)aminoethyl)-6,7-dihydro-MeBmt]-1-[α-methylene-Sar]-3-cyclosporin (1.91 g, 1.41 mmol) in methanol (20 ml) were added 4-mercapto-4-methylpentanoic acid (0.62 g, 4.22 mmol) and lithium hydroxide (0.20 g, 8.43 mmol). The reaction mixture was stirred at room temperature for two days. Most of the methanol was evaporated under reduced pressure. Ethyl acetate (100 ml) and brine (100 ml) were added and the PH of the aqueous layer was adjusted to 3 by adding hydrochloric acid solution (1.00 N). After separated, the ethyl acetate layer was dried over magnesium sulfate and evaporated under reduced pressure to give crude [8-(2-(tert-butoxycarbonyl)aminoethyl)-6,7 -dihydro-MeBmt]-1-[(S)-((4-carboxy-2-methylbutan-2-yl)thio)methyl-Sar]-3-cyclosporin [Molecular Formula: C₇₆H₁₃₈N₁₂O₁₆S; Exact Mass: 1507.01; MS (m/z): 1507.65 (M+1)⁺, 1529.95 (M+Na)⁺],

### [8-(2-(tert-Butoxycarbonyl)aminoethyl)-6,7-dihydro-MeBmt]-1-[(S)-((5-methoxy-2-methyl-5-oxopentan-2-yl)thio)methyl-Sar]-3-cyclosporin

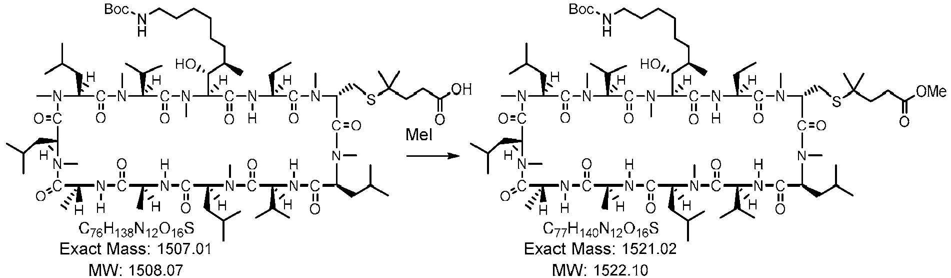

Crude [8-(2-(tert-butoxycarbonyl)aminoethyl)-6,7-dihydro-MeBmt]-1-[(S)-((4-carboxy-2-methyl butan-2-yl)thio)methyl-Sar]-3-cyclosporin was dissolved in acetone (20 ml). Iodomethane (0.56 ml, 8.88 mmol) and potassium carbonate (1.24 g, 8.88 mmol) were added. The mixture was stirred at room temperature for a weekend. Most of acetone was evaporated under reduced pressure. Then ethyl acetate (50 ml) and water (50 ml) were added and the mixture was separated. The ethyl acetate layer was dried over magnesium sulfate and evaporated under reduced pressure The residue was purified by chromatography (hexane/acetone) to give 0.45 g of pure [8-(2-(tert-butoxycarbonyl)aminoethyl)-6,7-dihydro-MeBmt]-1-[(S)-((5-methoxy-2-methyl-5-oxopentan-2-yl)thio)methyl-Sar]-3-cyclosporin [Molecular Formula: C₇₇H₁₄₀N₁₂O₁₆S; Exact Mass: 1521.02; MS (m/z): 1521.61 (M+1)⁺, 1543.72 (M+Na)⁺; HPLC RT: 18.31 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(S)-((5-methoxy-2-methyl-5-oxopentan-2-yl)thio)methyl-Sar]-3-cyclosporin

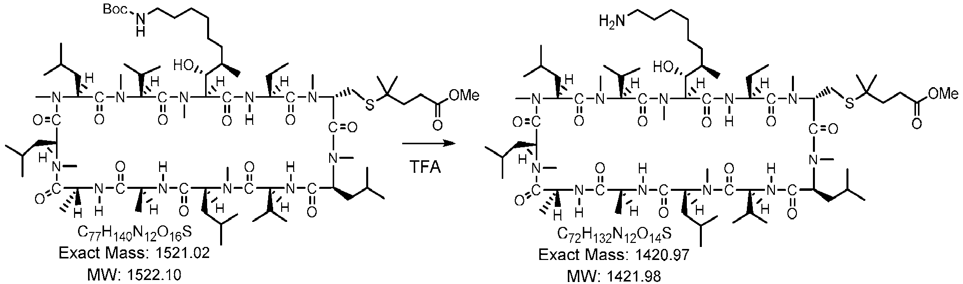

[8-(2-(tert-Butoxycarbonyl)aminoethyl)-6,7-dihydro-MeBmt]-1-[(S)-((5-methoxy-2-methyl-5-oxopentan-2-yl)thio)methyl-Sar]-3-cyclosporin (0.45 g, 0.30 mmol) was dissolved in dichloromethane (15 ml) and put into ice-water bath. Trifluoroacetic acid (5 ml) was added. The mixture was stirred at 0 °C for two hours. Another dichloromethane (50 ml) was added. The mixture was washed with brine (50 ml), saturated sodium bicarbonate solution (50 ml) and dried over magnesium sulfate and evaporated under reduced pressure to give crude [8-(2-aminoethyl)-6,7-dihydro-MeBmt]-1-[(S)-((5-methoxy-2-methyl-5-oxopentan-2-yl)thio) methyl-Sar]-3-cyclosporin [Molecular Formula: C₇₂H₁₃₂N₁₂O₁₄S; Exact Mass: 1420.97; MS (m/z): 1421.67 (M+1)⁺; HPLC RT: 12.97 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-((5-methoxy-2-methyl-5-oxopentan-2-yl)thio)methyl-Sar]-3-cyclosporin

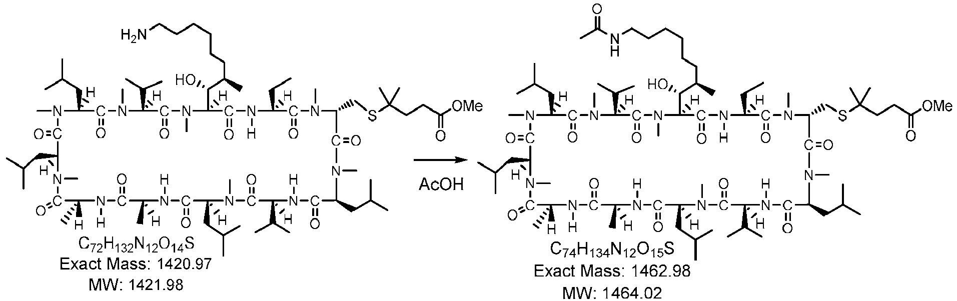

Crude [8-(2-aminoethyl)-6,7-dihydro-MeBmt]-1-[(S)-((5-methoxy-2-methyl-5-oxopentan-2-yl)thio)methyl-Sar]-3-cyclosporin was dissolved dichloromethane (15 ml). Acetic acid (0.09 g, 1.48 mmol), HBTU (0.34 g, 0.89 mmol), 1-hydroxybenzotriazole (0.12 g, 0.89 mmol) and triethylamine (0.50 ml) were added. The mixture was stirred at room temperature for two hours. Then dichloromethane (50 ml) and brine (50 ml) were added and separated. The dichloromethane layer was dried over magnesium sulfate and evaporated under reduced pressure. The residue was purified by chromatography (hexane/acetone) to give 0.20 mg of pure [8-(2-acetamidoethyl)-6,7-dihydro-MeBmt]-1-[(S)-((5-methoxy-2-methyl-5-oxopentan-2-yl)thio)methyl-Sar]-3-cyclosporin [Molecular Formula: C₇₄H₁₃₄N₁₂O₁₅S; Exact Mass: 1462.98; MS (m/z): 1463.74 (M+1)⁺, 1485.81 (M+Na)⁺; HPLC RT: 17.11 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(S)-((5-hydroxy-2-methylpentan-2-yl)thio)methyl-Sar]-3-cyclosporin

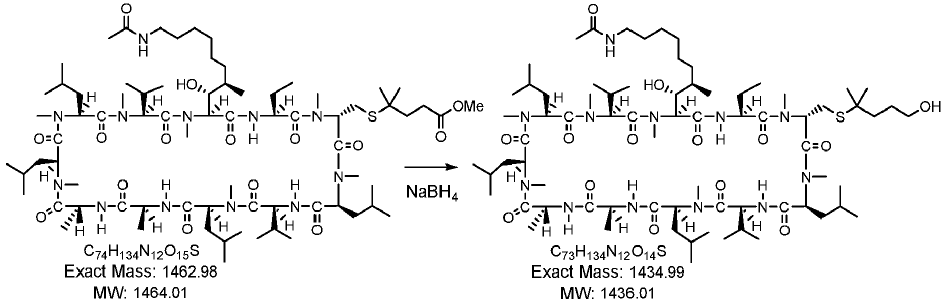

[8-(2-Acetamidoethyl)-6,7-dihydro-MeBmt]-1-[(S)-((5-methoxy-2-methyl-5-oxopentan-2-yl) thio)methyl-Sar]-3-cyclosporin (0.20 g, 0.14 mmol) was dissolved in tetrahydrofuran (10 ml). Sodium borohydride (1.00 g, 64.40 mmol) and cesium chloride (0.20 g, 0.59 mmol) were added. The mixture was stirred at room temperature and methanol (10 ml) was added dropwise over two hours. Then the mixture was stirred overnight. Most of solvents were evaporated under reduced pressure. Ethyl acetate (50 ml) and brine (50 ml) were added and the mixture was separated. The ethyl acetate layer was dried over magnesium sulfate and evaporated under reduced pressure. The residue was purified by chromatography (dichloromethane/methanol) to give 34 mg of pure [8-(2-acetamidoethyl)-6,7-dihydro-MeBmt]-1-[(S)-((5-hydroxy-2-methylpentan-2-yl)thio)methyl-Sar]-3-cyclosporin [Molecular Formula: C₇₃H₁₃₄N₁₂O₁₄S; Exact Mass: 1434.99; MS (m/z): 1435.86 (M+1)⁺, 1457.87 (M+Na)⁺; HPLC RT: 17.03 min. (C8 reverse phase column: 250 mm; acetonitrile/water (0.05% trifluoroacetic acid); operation temperature: 64 °C; detector: 210 nm)].

### Reference Example 1

### [8-(2-Acetamidoethyl)-6,7-dihydro-MeBmt]-1-[(R)-2-methyl-Sar]-3-cyclosporin(CPI-431-32(CRV431))

### [(R)-2-Methyl-Sar]-3-cyclosporin

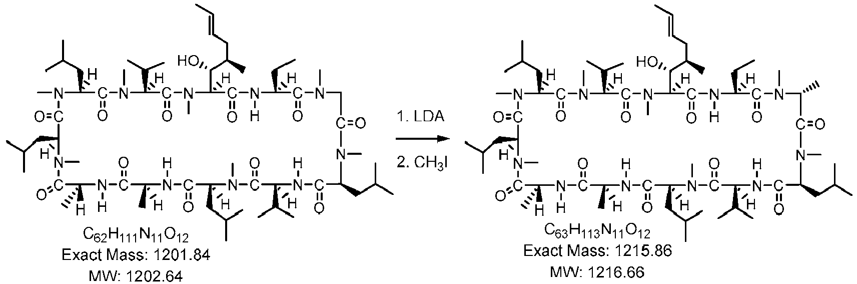

n-Butyllithium (2.20 M, 75.60 ml, 166.39 mmol) was added to a solution of diisopropylamine (23.60 ml, 166.39 mmol) in tetrahydrofuran (150 ml) at -78 °C under nitrogen. After the reaction mixture was stirred for an hour, a solution of cyclosporine (20.00 g, 16.64 mmol) in tetrahydrofuran (50 ml) was added over ten minutes. The mixture was stirred at -78 °C for two hours. After iodomethane (10.36 ml, 166.39 mmol) was added, the mixture was stirred at -78 °C for another two hours and allowed to warm to room temperature overnight. Brine (20 ml) was added to quench the reaction. Most of tetrahydrofuran was removed under reduced pressure. Ethyl acetate (100 ml) and brine (100 ml) were added and the mixture was separated. The organic layer was dried over magnesium sulfate and evaporated under reduced pressure. The residue was purified by chromatography (hexane/acetone) to give 5.49 g of [(R)-2-methyl-Sar]-3-cyclosporin [Molecular Formula: C₆₃H₁₁₃N₁₁O₁₂; Exact Mass: 1215.86; MS (m/z): 1216.63 (M+1)⁺, 1238.79 (M+Na)⁺; HPLC RT: 17.53 min. (C8 reverse phase column: 250 mm; acetonitrile/water (0.05% trifluoroacetic acid); operation temperature: 64 °C; detector: 210 nm)].

### [3-Acetyt-MeBmt]-1-[(R)-2-methyt-Sar]-3-cydosporin

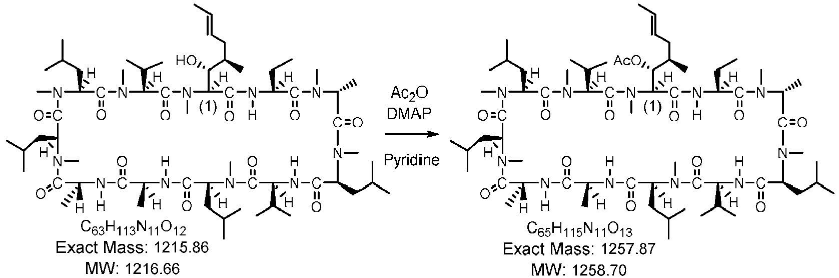

To a dried flask under nitrogen were added [(R)-2-methyl-Sar]-3-cyclosporin (5.49 g, 4.52 mmol), N,N-dimethylaminopyridine (0.06 g, 0.45 mmol), anhydrous pyridine (60 ml) and acetic anhydride (28.21 ml, 299.00 mol). The reaction mixture was stirred overnight at room temperature. The mixture was poured into ice-water (300 ml) and stirred until the ice was melted. Ethyl acetate (100 ml) was added and the mixture was separated. The ethyl acetate layer was washed with 1 N hydrochloric acid solution (50 ml x 2), saturated sodium bicarbonate solution (50 ml), brine (50 ml), dried over magnesium sulfate and evaporated under reduced pressure. The residue was purified by chromatography (hexane/acetone/methanol) to give 4.67 g of [3-acetyl-MeBmt]-1-[(R)-2-methyl-Sar]-3-cyclosporin [Molecular Formula: C₆₅H₁₁₅N₁₁O₁₃; Exact Mass: 1257.87; MS (m/z): 1258.54 (M+1)⁺, 1280.71 (M+Na)⁺; HPLC RT: 19.31 min. (C8 reverse phase column: 250 mm; acetonitrile/water (0.05% trifluoroacetic acid); operation temperature: 64 °C; detector: 210 nm)].

### [(3R,4R)-3-Acetyloxy-4-methyl-6-oxo-N-MeNle]-1-[(R)-2-methyl-Sar]-3-cyclosporin

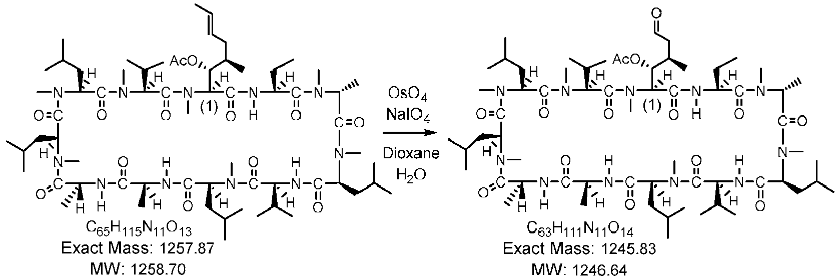

To a solution of [3-acetyl-MeBmt]-1-[(R)-2-methyl-Sar]-3-cyclosporin (4.67 g, 3.71 mmol) in dioxane (100 ml) were added water (20 ml), osmium(VIII) oxide solution (15.74 mM, 23.50 ml, 0.37 mmol) and sodium metaperiodate (3.18 g, 14.85 mmol). The reaction mixture was stirred at room temperature for five hours. Ethyl acetate (100 ml) and brine (100 ml) were added and the mixture was separated. The organic layer was washed with saturated sodium bicarbonate solution, brine, dried over magnesium sulfate and evaporated under reduced pressure. The residue was purified by chromatography (hexane/acetone) to give 4.10 g of [(3R, 4R)-3-acetyloxy-4-methyl-6-oxo-N-MeNle]-1-[(R)-2-methyl-Sar]-3-cyclosporin [Molecular Formula: C₆₃H₁₁₁N₁₁O₁₄; Exact Mass: 1245.83 ; MS (m/z): 1246.54 (M+1)⁺, 1268.71 (M+Na)⁺; HPLC RT: 17.27 min. (C8 reverse phase column: 250 mm; acetonitrile/water (0.05% trifluoroacetic acid); operation temperature: 64 °C; detector: 210 nm)].

### [8-Cyanomethyl-3-acetyl-MeBmt]-1-[(R)-2-methyl-Sar]-3-cyclosporin

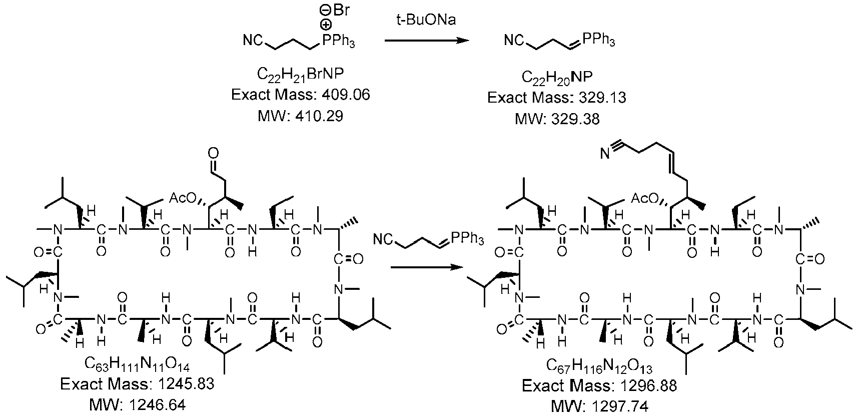

To a dried flask were added (3-cyanopropyl)triphenylphosphonium bromide (7.98 g, 19.50 mmol) and anhydrous tetrahydrofuran (60 ml) under nitrogen. The reaction mixture was put into an ice-water bath and sodium tert-butoxide (2.19 g, 22.75 mmol) was added. After the mixture was stirred for two hours, a solution of [(3R, 4R)-3-acetyloxy-4-methyl-6-oxo-N-MeNle]-1-[(R)-2-methyl-Sar]-3-cyclosporin (4.10 g, 3.29 mmol) in anhydrous tetrahydrofuran (20 ml) was added. The mixture was stirred another five hours at 0°C. Then saturated ammonium chloride solution (20 ml) was added to quench the reaction. Most of tetrahydrofuran was evaporated under reduced pressure. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 2.00 g of pure [8-cyanomethyl-3-acetyl-MeBmt]-1-[(R)-2-methyl-Sar]-3-cyclosporin [Molecular Formula: C₆₇H₁₁₆N₁₂O₁₃; Exact Mass: 1296.88; MS (m/z): 1297.55 (M+1)⁺, 1319.787 (M+Na)⁺; HPLC RT: 17.56 min. (C8 reverse phase column: 250 mm; acetonitrile/water (0.05% trifluoroacetic acid); operation temperature: 64 °C; detector: 210 nm)].

### [8-(2-Aminoethyl)-3-acetyl-6,7-dihydro-MeBmt]-1-[(R)-2-methyl-Sar]-3-cyclosporin

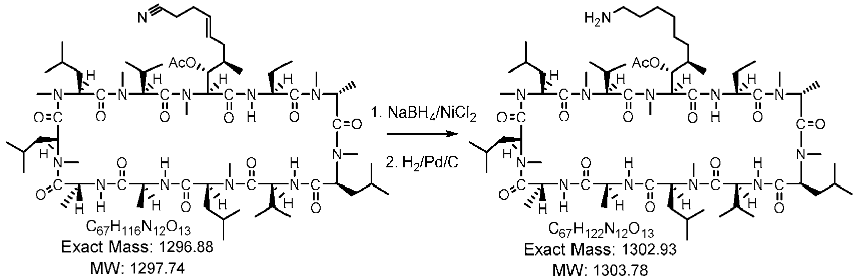

To a solution of [8-cyanomethyl-3-acetyl-MeBmt]-1-[(R)-2-methyl-Sar]-3-cyclosporin (2.00 g, 1.54 mmol) in methanol (50 ml) under nitrogen was added nickel (II) chloride hexahydrate (0.04 g, 0.15 mmol).The reaction mixture was put into ice-water bath. Sodium borohydride (3.05 g, 80.50 mmol) was added in four batches in two hours. After the mixture was stirred for another two hours at 0°C, water (10 ml) was added. Most of the methanol was evaporated under reduced pressure. Ethyl acetate (50 ml) and saturated sodium bicarbonate solution (50 ml) were added and the mixture was separated. The organic layer was washed with brine, dried over magnesium sulfate and evaporated under reduced pressure. The residue was dissolved in methanol (30 ml). Palladium (10 wt% on carbon, 20 mg) and acetic acid (5 drops) were added. The mixture was stirred at room temperature under hydrogen for two hours. Then the mixture was filtered and the filtrate was evaporated under reduced pressure to give 2.20 g of crude [8-(2-aminoethyl)-3-acetyl-6,7-dihydro-MeBmt]-1-[(R)-2-methyl-Sar]-3-cyclosporin [Molecular Formula: C₆₇H₁₂₂N₁₂O₁₃; Exact Mass: 1302.93; MS (m/z): 1303.75 (M+1)⁺; HPLC RT: 14.72 min. (C8 reverse phase column: 250 mm; acetonitrile/water (0.05% trifluoroacetic acid); operation temperature: 64 °C; detector: 210 nm)].

### [8-(2-Aminoethyl)-6,7-dihydro-MeBmt]-1-[(R)-2-methyl-Sar]-3-cyclosporin

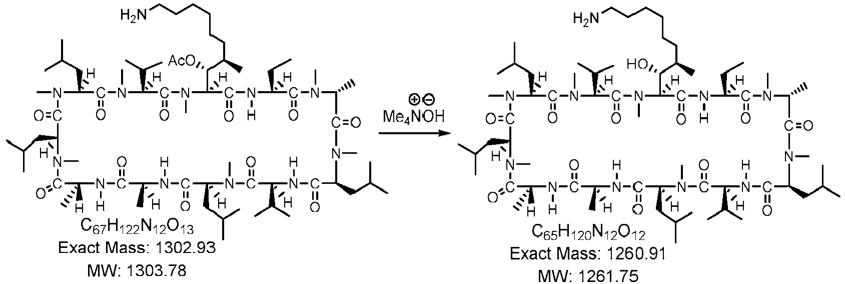

[8-(2-Aminoethyl)-3-acetyl-6,7-dihydro-MeBmt]-1-[(R)-2-methyl-Sar]-3-cyclosporin (1.60 g, 1.24 mmol) was dissolved in methanol (20 ml). Water (10 ml) and tetramethylammonium hydroxide pentahydrate (0.67 g, 3.72 mmol) were added. The mixture was stirred at room temperature for two hours. Then most of the methanol was evaporated. Ethyl acetate (100 ml) and brine (100 ml) were added and the mixture was separated. The organic layer was dried over magnesium sulfate and evaporated under reduced pressure. The residue was purified by chromatography (dichloromethane/methanol) to give 1.20 g of pure [8-(2-aminoethyl)-6,7-dihydro-MeBmt]-1-[(R)-2-methyl-Sar]-3-cyclosporin [Molecular Formula: C₆₅H₁₂₀N₁₂O₁₂; Exact Mass: 1260.91; MS (m/z): 1261.72 (M+1)⁺; HPLC RT: 12.11 min. (C8 reverse phase column: 250 mm; acetonitrile/water (0.05% trifluoroacetic acid); operation temperature: 64 °C; detector: 210 nm)].

### [8-(2-Acetamidoethyl)-6,7-dihydro-MeBmt]-1-[(R)-2-methyl-Sar]-3-cyclosporin

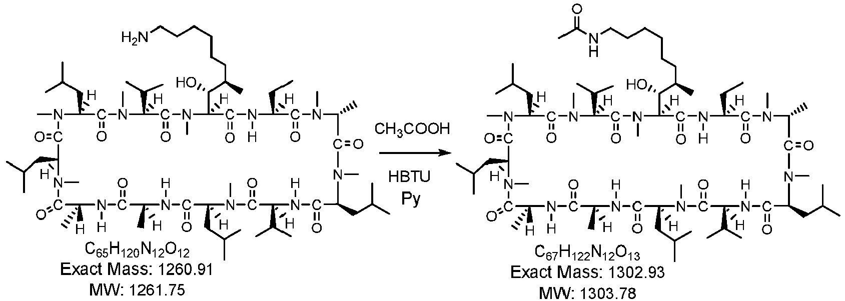

[8-(2-Aminoethyl)-6,7-dihydro-MeBmt]-1-[(R)-2-methyl-Sar]-3-cyclosporin (0.60 g, 0.48 mmol) was dissolved in dichloromethane (25 ml). Acetic acid (0.14 ml, 2.38 mmol), HBTU (0.54 g, 1.43 mmol), 1-hydroxybenzotriazole (0.19 g, 1.43 mmol) and pyridine (1.00 ml) were added. The mixture was stirred at room temperature for three hours. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 0.11 g of pure [8-(2-acetamidoethyl)-6,7-dihydro-MeBmt]-1-[(R)-2-methyl-Sar]-3-cyclosporin [Molecular Formula: C₆₇H₁₂₂N₁₂O₁₃; Exact Mass: 1302.93; MS (m/z): 1303.66 (M+1)⁺, 1325.86 (M+Na)⁺; HPLC RT: 16.56 min. (C8 reverse phase column: 250 mm; acetonitrile/water (0.05% trifluoroacetic acid); operation temperature: 64 °C; detector: 210 nm)]. This product was prepared using a method analogous to the procedure described in US Patent No. 9,200,038 B2 and US 2013/0190223 A1 (which are incorporated herein by references).

### Reference Example 2

### [8-(2-Acetamidoethyl)-6,7-dihydro-MeBmt]-1-cyclosporin

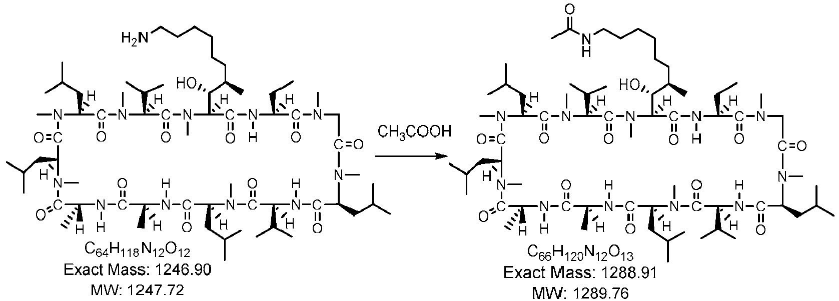

[8-(2-Aminoethyl)-6,7-dihydro-MeBmt]-1-cyclosporin (0.50 g, 0.40 mmol) was dissolved dichloromethane (20 ml). Acetic acid (0.11 ml, 2.00 mmol), HBTU (0.46 g, 1.20 mmol), 1-hydroxybenzotriazole (0.16 g, 1.20 mmol) and pyridine (0.50 ml) were added. The mixture was stirred at room temperature for one hour. Then dichloromethane (30 ml) and brine (50 ml) were added and separated. The dichloromethane layer was dried over magnesium sulfate and evaporated under reduced pressure. The residue was purified by chromatography (dichloromethane/methanol) to give 0.20 g of pure [8-(2-acetamidoethyl)-6,7-dihydro-MeBmt]-1-cyclosporin [Molecular Formula: C₆₆H₁₂₀N₁₂O₁₃; Exact Mass: 1288.91; MS (m/z): 1289.96 (M+1)⁺, 1311.82 (M+Na)⁺; HPLC RT: 14.92 min. (C8 reverse phase column: 250 mm; acetonitrile/water (0.05% trifluoroacetic acid); operation temperature: 64 °C; detector: 210 nm)].

### Reference Example 3

### [α-Methylene-Sar]-3-cyclosporin

### [α-Methoxycarbonyl-Sar]-3-cyclosporin

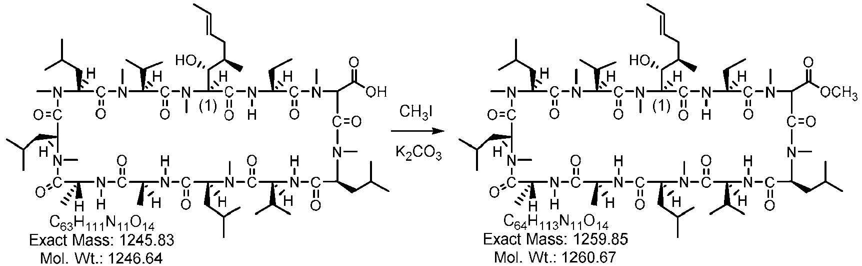

[α-Carboxy-sar]-3-cyclosporin (5.00 g, 4.01 mmol) was dissolved in N,N-dimethylformamide (30 ml). Iodomethane (2.85 g, 20.10 mmol) and potassium carbonate (1.38 g, 10.00 mmol) were added. The mixture was stirred at room temperature for two hours. Then ethyl acetate (60 ml) and water (60 ml) were added and the mixture was separated. The ethyl acetate layer was washed with brine, dried over magnesium sulfate and evaporated under reduced pressure to give 5.32 g of crude product, which was directly used for the next step without purification (yield: ∼ 100%) [Molecular Formula: C₆₄H₁₁₃N₁₁O₁₄; Exact Mass: 1259.85; MS (m/z): 1260.7 (M+1)⁺, 1282.7 (M+Na)⁺],

### [(R)-α-Hydroxymethyl-Sar]-3-cyclosporin

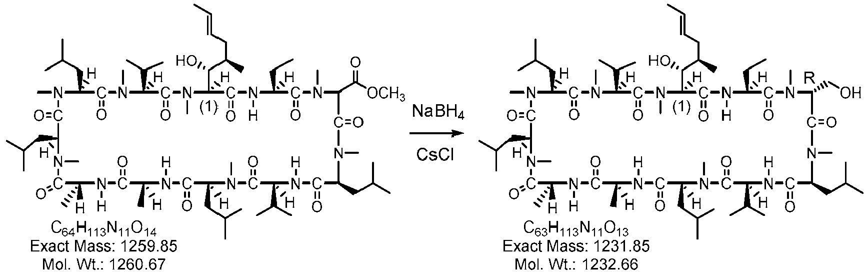

[α-Methoxycarbonyl-Sar]-3-cyclosporin (2.00 g, 1.59 mmol) was dissolved in tetrahydrofuran (30 ml). Cesium chloride (1.33 g, 7.90 mmol) and sodium borohydride (0.60 g, 15.89 mmol) were added. Then methanol (30 ml) was added dropwise to the mixture over two hours. After addition, the mixture was stirred at room temperature overnight. Most of solvent was then evaporated under reduced pressure. Ethyl acetate (50 ml) and water (50 ml) were added. The ethyl acetate layer was separated and washed with brine, dried over magnesium sulfate and evaporated under reduced pressure to give 1.99 g of crude product, which was purified by on silica gel column with dichloromethane/methanol (from 100:0 to 95:5) to give 1.50 g of pure product (yield: 76%) [Molecular Formula: C₆₃H₁₁₃N₁₁O₁₃; Exact Mass: 1231.85; MS (m/z): 1232.7 (M+1)⁺, 1254.7 (M+Na)⁺].

### [α-Methylmethanesulfonate-Sar]-3-cyclosporin

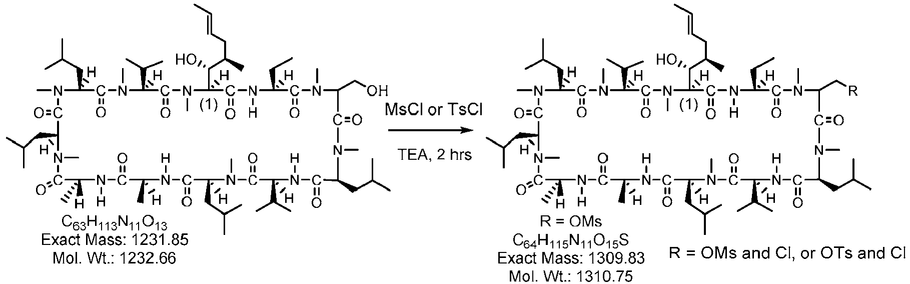

To a solution of [α-hydroxymethyl-Sar]-3-cyclosporin (30 mg, 0.024 mmol) in dichloromethane (2 ml) at 0 °C were added triethylamine (52.8 µl, 0.38 mmol) and methanesulfonyl chloride (23 mg, 0.20 mmol). After stirred at room temperature for two hours, the mixture was washed with brine, dried over magnesium sulfate and evaporated under reduced pressure to give 33 mg of crude product, which was directly used in next step reaction without further purification [Molecular Formula: C₆₄H₁₁₅N₁₁O₁₅S; Exact Mass: 1309.83; MS (m/z): 1310.7 (M+1)⁺].

### [α-Chloromethyl-Sar]-3-cyclosporin

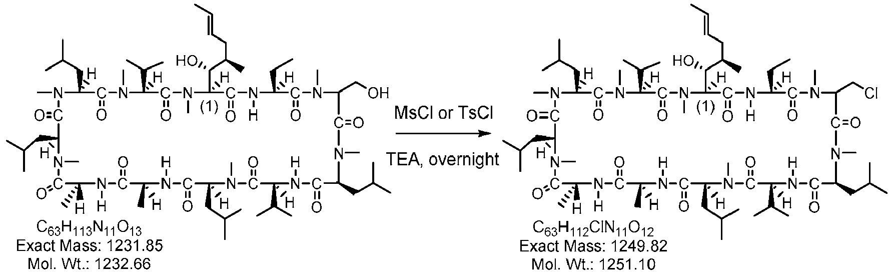

To a solution of [α-hydroxymethyl-Sar]-3-cyclosporin (30 mg, 0.024 mmol) in dichloromethane (2 ml) at 0 °C were added triethylamine (52.8 µL, 0.384 mmol, 16 equivalents) and methanesulfonyl chloride (23 mg, 0.20 mmol). After stirred at room temperature overnight, the mixture was washed with brine, dried over magnesium sulfate and evaporated under reduced pressure to give 30 mg of crude product, which was directly used in next step reaction without further purification [Molecular Formula: C₆₃H₁₁₂ClN₁₁O₁₂; Exact Mass: 1249.82; MS (m/z): 1250.7 (M+1)⁺, 1272.9 (M+Na)⁺],

### [α-Methylene-Sar]-3-cyclosporin

### Method 1

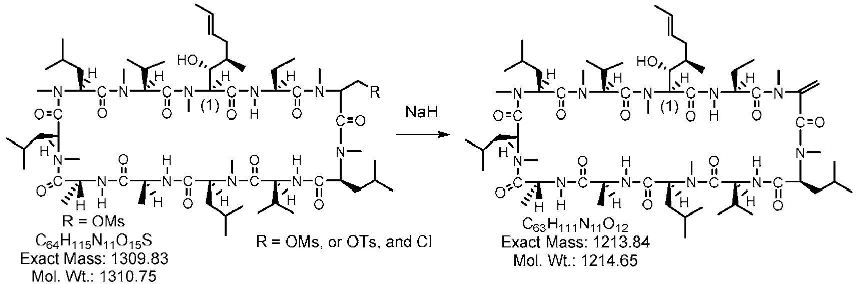

To a solution of either [α-methanesulfonatemethyl-Sar]-3-cyclosporin (33 mg, 0.025 mmol) or [α-chloromethyl-Sar]-3-cyclosporin (30 mg, 0.025 mmol) in tetrahydrofuran (3 ml) was added sodium hydride (15.3 mg, 60% in oil, 0.38 mmol, 10 equivalents) at 0 °C. The mixture was stirred at 0 °C for one hour and then warmed up to room temperature for 30 minutes. After removal of solvent, the residue was dissolved in dichloromethane (20 ml). The dichloromethane layer was washed with 1 N hydrochloric acid, saturated sodium bicarbonate solution and brine, dried over magnesium sulfate and evaporated under reduced pressure. The residue was purified by chromatography on silica gel using dichloromethylene/methanol (20/1) to give 16 mg of product (yield: 54%) [Molecular Formula: C₆₃H₁₁₁N₁₁O₁₂; Exact Mass: 1213.84; MS (m/z): 1214.7 (M+1)⁺, 1236.7 (M+Na)⁺; TLC R_{f}: 0.55 (ethyl acetate/methanol = 20/1); HPLC RT: 7.0 min (C8 reverse phase column: 150 mm; acetonitrile/water (0.05% trifluoroacetic acid); operation temperature: 64 °C; detector: 210 nm)].

### [α-Methylene-Sar]-3-cyclosporin

### Method 2

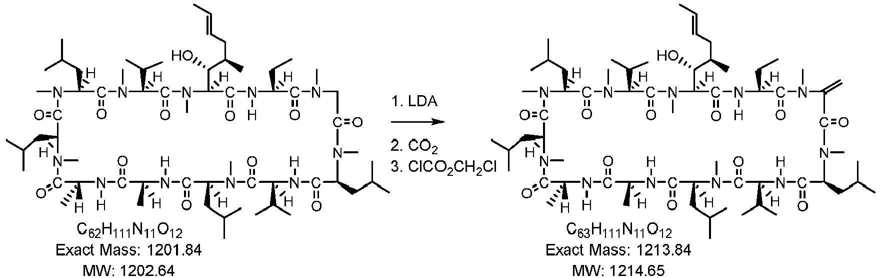

[α-Methylene-Sar]-3-cyclosporin can also be prepared using a method analogous to the procedure described in WO2012/051194A1 (which is incorporated herein by reference).

### Reference Example 4

### [α-Methylene-Sar]-3-[(γ-hydroxy)-NMeLeu]-4-cyclosporin

### [α-Methoxycarbonyl-Sar]-3-[(γ-hydroxy)-NMeLeu]-4-cyclosporin

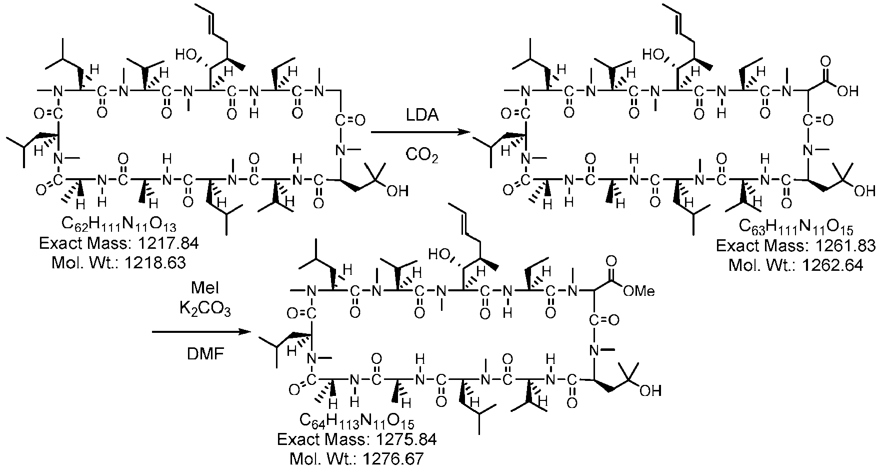

To a solution of LDA (2.0 M in tetrahydrofuran, 23 ml, 46 mmol) in tetrahydrofuran (80 ml) at -78 °C under nitrogen, [(y-hydroxy)-N-MeLeu]-4-cyclosporin (4.40 g, 3.61 mmol) in tetrahydrofuran (15 ml) was added over 3 min. After the mixture was stirred at -78 °C for 3 hours, carbon dioxide gas was bubbled into the reaction mixture for 1 hour. Then the mixture was allowed to warm to room temperature slowly and kept stirring for 3 hours. Most of tetrahydrofuran was evaporated. Dichloromethane (100 ml) and water (50 ml) were added. The PH of the mixture was adjusted to around 5 by adding aqueous citric acid solution. The mixture was separated and the organic layer was washed with brine, dried over magnesium sulfate and evaporated under reduced pressure to give 3.20 g of crude product as acid, which was used for next step without purification [Molecular Formula: C₆₃H₁₁₁N₁₁O₁₅; Exact Mass: 1261.83; MS (m/z): 1262.49 (M+1)⁺], To a mixture of [α-carboxy-Sar]-3-[(γ-hydroxy)-N-MeLeu]-4-cyclosporin (3.20 g 2.53 mmol) and potassium carbonate (1.30 g, 9.40 mmol) in N,N-dimethylformamide (20 ml) was added iodomethane (1.80 g, 12.70 mmol). The mixture was stirred overnight at room temperature. Dichloromethane (80 ml) and water (50 ml) were added and the mixture was separated. The dichloromethane layer was washed with water (25 ml) and brine (25 ml), dried over magnesium sulfate and evaporated under reduced pressure to give crude 3.00 g of product [Molecular Formula: C₆₄H₁₁₃N₁₁O₁₅; Exact Mass: 1275.84; MS (m/z): 1276.75 (M+1)⁺],

[(y-Hydroxy)-N-MeLeu]-4-cyclosporin was prepared by *Sebekia benihana* biotransformation according to a method described by Kuhnt M. et al., 1996, Microbial Biotransformation Products of Cyclosporin A, J. Antibiotics, 49 (8), 781.

### [(R)-α-Hydroxymethyl-Sar]-3-[(γ-hydroxy)-NMeLeu]-4-cyclosporin

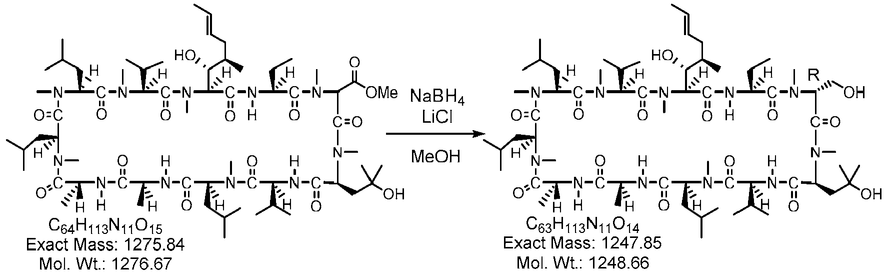

To a suspension of [α-methoxycarbonyl-Sar]-3-[(γ-hydroxy)-N-MeLeu]-4-cyclosporin (3.00 g, 2.35 mmol) and lithium chloride (1.50 g, 35.30 mmol) in methanol (100 ml) was added sodium borohydride (2.50 g, 66.10 mmol) in portions. The mixture was stirred overnight at room temperature. Most of solvent was evaporated under reduced pressure. Dichloromethane (80 ml) and water (50 ml) were added and the mixture was separated. The dichloromethane layer was washed with brine, dried over magnesium sulfate and evaporated under reduced pressure. The residue was purified by chromatography (dichloromethane/methanol = 96/4) to give 1.30 g of product [Molecular Formula: C₆₃H₁₁₃N₁₁O₁₄; Exact Mass: 1247.85; MS (m/z): 1248.48 (M+1)⁺; ¹H NMR spectrum (600 MHz, CDCl₃, δ in ppm): 0.68 (d, J = 5.4Hz, 3H), 0.80-1.00 (m, 30H), 1.07 (d, J = 6.0Hz, 3H), 1.16 - 1.29 (m, 10H), 1.32 (d, J = 7.2Hz, 3H), 1.39-1.46 (m, 2H), 1.59-1.63 (m, 6H), 1.68-1.83 (m, 7H), 2.02-2.11 (m, 4H), 2.31-2.33 (m, 1H), 2.37-2.42 (m, 2H), 2.67 (s, 6H), 3.09 (s, 3H), 3.19 (s, 3H), 3.20 (s, 3H), 3.22 (s, 3H), 3.47 (s, 3H), 3.72-3.75 (m, 1H), 3.82 (br, 1H), 3.97-3.99 (m, 1H), 4.07-4.10 (m, 1H), 4.50-4.52 (m, 1H), 4.65-4.67 (t, J = 8.4 Hz, 1H), 4.79-4.81 (m, 1H), 4.90-4.95 (m, 2H), 5.00 -5.05 (m, 2H), 5.09 (d, J = 10.8Hz, 1H), 5.30-5.35 (m, 2H), 5.46 (d, J = 6.0Hz, 1H), 5.52-5.53 (m, 1H), 5.66-5.68 (m, 1H), 7.12 (d, J = 7.8Hz, 1H), 7.47 (d, J = 8.4Hz, 1H), 7.60 (d, J = 7.2Hz, 1H), 7.87-7.89 (d, J = 9.6Hz, 1H)].

### [α-Methylene-Sar]-3-[(γ-hydroxy)-NMeLeu]-4-cyclosporin

### Method 1

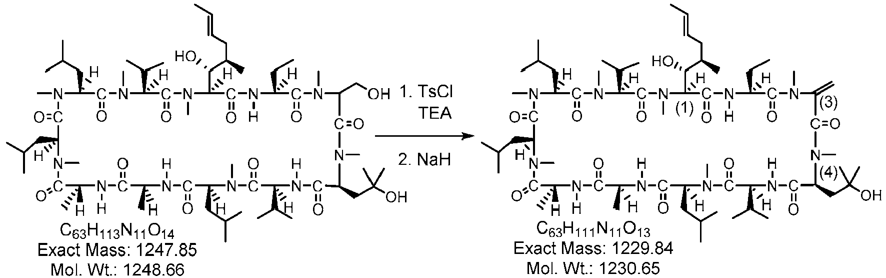

To a solution of [α-hydroxymethyl-Sar]-3-[(γ-hydroxy)-NMeLeu]-4-cyclosporin (0.25 g, 0.20 mmol) in dichloromethane (10 mL) at room temperature were added triethylamine (0.33 mL, d 0.726, 2.40 mmol) and triethylamine hydrochloride (95.6 mg, 1.00 mmol), followed by adding p-toluenesulfonyl chloride (0.23 g, 1.20 mmol) under stirring. The mixture was stirred at room temperature overnight. Then the reaction mixture was washed with brine, dried over magnesium sulfate and the solvent was evaporated under reduced pressure. The reaction mixture of [α-chloromethyl-Sar]-3-[(γ-hydroxy)-NMeLeu]-4-cyclosporin [Molecular formula: C₆₃H₁₁₂CIN₁₁O₁₃; Exact Mass: 1265.81; MS (m/z): 1266.32 (M+1)⁺, 1288.43 (M+Na)⁺] and [α-p-toluenesulfonylmethyl-Sar]-3-[(y-hydroxy)-NMeLeu]-4-cyclosporin [Molecular formula: C₇₀H₁₁₉N₁₁O₁₆S; Exact Mass: 1401.856; MS (m/z): 1402.34 (M+1)⁺, 1424.62 (M+Na)⁺] was directly used in next step reaction without further purification. To a solution of the above mixture in tetrahydrofuran (20 ml) was added sodium hydride (320 mg, 60% in oil, 8 mmol) at 0 °C. The mixture was stirred at 0 °C for one hour and then warmed up to room temperature for 30 minutes. The reaction was quenched with a saturated ammonia chloride solution. After removing tetrahydrofuran, the crude product was extracted with ethyl acetate. The ethyl acetate layer was washed with brine, dried over magnesium sulfate and evaporated under reduced pressure. The residue was purified by chromatography on silica gel using ethyl acetate/methanol (20/1) to give 45 mg of product (yield: 18 %) [Molecular formula: C₆₃H₁₁₁N₁₁O₁₃; Exact Mass: 1229.84; MS (m/z): 1230.6 (M+1)⁺, 1252.82 (M+Na)⁺; TLC R_{f}: 0.50 (ethyl acetate/methanol = 10/1); HPLC RT: 15.38 min (C8 reverse phase column: 250 mm; acetonitrile/water (0.05% trifluoroacetic acid); operation temperature: 64 °C; detector: 210 nm); ¹H NMR spectrum (600 MHz, CDCl₃, δ in ppm): 0.72 (d, J = 5.4Hz, 3H), 0.84-1.00 (m, 30H), 1.17-1.26 (m, 15H), 1.34 (d, J = 6.0 Hz, 3H), 1.44 -1.47 (m, 2H), 1.59-1.62 (m, 6H), 1.69-1.76 (m, 4H), 1.94-1.99 (m, 1H), 2.09-2.13 (m, 3H), 2.34-2.37 (m, 3H), 2.65(s, 3H), 2.67 (s, 3H), 3.09 (s, 3H)), 3.10 (s, 3H), 3.19 (s, 3H), 3.44 (s, 3H), 3.46 (s, 3H), 3.80 (m, 1H), 3.91 (m, 1H), 4.47-4.50 (m, 1H), 4.68-4.71(t, J = 9.0Hz, 1H), 4.78-4.81 (m, 1H), 4.98-5.02 (m, 2H), 5.06-5.11 (m, 3H), 5.24 (s, 1H), 5.32 (m, 2H), 5.41-5.43 (m, 2H), 5.64-5.66 (m, 1H), 7.11 (d, J = 7.2Hz, 1H), 7.49 (d, J = 7.2Hz, 1H), 7.74 (d, J = 8.4Hz, 1H), 7.84 (d, J = 9.6Hz, 1H)].

### [α-Methylene-Sar]-3-[(γ-hydroxy)-NMeLeu]-4-cyclosporin

### Method 2

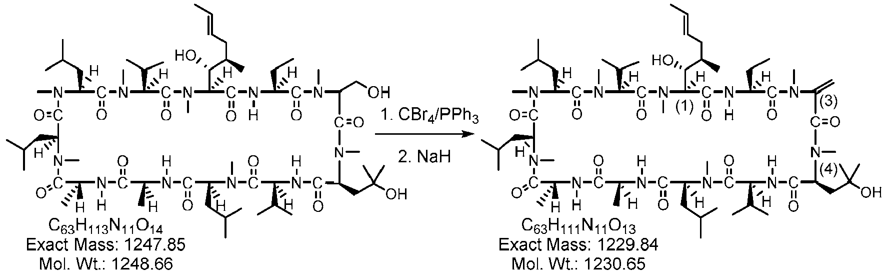

[(R)-α-Hydroxymethyl-Sar]-3-[(γ-hydroxy)-NMeLeu]-4-cyclosporin (crude, 2.00 g), carbon tetrabromide (2.66 g, 8.02 mmol) and triphenylphosphine (2.11 g, 8.02 mmol) were dissolved in dichloromethane (30 ml). The mixture was stirred under nitrogen at room temperature for two hours. Then the mixture was added into a suspension of sodium hydride (60% dispersion in mineral oil) (0.77 g, 19.25 mmol) in tetrahydrofuran (30 ml) under nitrogen at 0 °C. The mixture was stirred at 0 °C for one hour. Most of solvents then were evaporated under reduced pressure. The residue was treated with water (10 ml) slowly at 0 °C. Ethyl acetate (30 ml) and water (30 ml) were added and the mixture was separated. The ethyl acetate layer was washed with brine, dried over magnesium sulfate and evaporated under reduced pressure. The residue was purified by chromatography (hexane/acetone from 90/10 to 70/30) to give 0.68 g product of [α-methylene-Sar]-3-[(γ-hydroxy)-NMeLeu]-4-cyclosporin [Molecular Formula: C₆₃H₁₁₁N₁₁O₁₃; Exact Mass: 1229.84; MS (m/z): 1230.50 (M+1)⁺, 1252.68 (M+Na)⁺; TLC R_{f}: 0.50 (ethyl acetate/methanol = 10/1); HPLC RT: 15.36 min (C8 reverse phase column: 250 mm; acetonitrile/water (0.05% trifluoroacetic acid); operation temperature: 64 °C; detector: 210 nm)].

### [α-Methylene-Sar]-3-[(γ-hydroxy)-NMeLeu]-4-cyclosporin

### Method 3

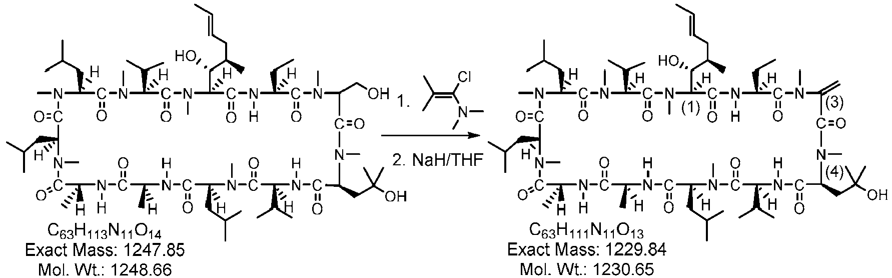

To a solution of [(R)-α-hydroxymethyl-Sar]-3-[(γ-hydroxy)-NMeLeu]-4-cyclosporin (0.25 g, 0.20 mmol) in methylene chloride (10 mL) was added dropwise 1-chloro-N,N,2-trimethyl-1-propenylamine (131 µl, d 1.01, 1.0 mmol) at 0 °C under nitrogen atmosphere. After stirred for 30 minutes at 0 °C, the mixture was allowed to warm to room temperature and stirred for another hour. The reaction mixture was washed with sodium bicarbonate solution, brine, dried over magnesium sulfate and evaporated under reduced pressure. The crude product containing [α-chloromethyl-Sar]-3-[(γ-hydroxy)-NMeLeu]-4-cyclosporin [Molecular formula: C₆₃H₁₁₂ClN₁₁O₁₃; Exact Mass: 1265.81; MS (m/z): 1266.32 (M+1)⁺, 1288.43 (M+Na)⁺] was used in next step reaction without further purification. To a solution of the above crude product in tetrahydrofuran (20 ml) was added sodium hydride (320 mg, 60% in oil, 8 mmol) at 0 °C under stirring. The mixture was stirred at 0 °C for one hour and then warmed up to room temperature for another 30 minutes. The reaction was then quenched with a saturated ammonia chloride solution. After removing tetrahydrofuran, the residue was extracted with ethyl acetate. The ethyl acetate layer was washed with brine, dried over magnesium sulfate and evaporated under reduced pressure. The residue was purified by chromatography on silica gel using ethyl acetate/methanol (20/1) to give 33 mg of product (yield: 13 %) [Molecular formula: C₆₃H₁₁₁N₁₁O₁₃; Exact Mass: 1229.84; MS (m/z): 1230.45(M+1)⁺, 1252.65 (M+Na)⁺; TLC R_{f}: 0.50 (ethyl acetate/methanol = 10/1); HPLC RT: 15.36 min (C8 reverse phase column: 250 mm; acetonitrile/water (0.05% trifluoroacetic acid); operation temperature: 64 °C; detector: 210 nm)].

### [α-Methylene-Sar]-3-[(γ-hydroxy)-NMeLeu]-4-cyclosporin

### Method 4

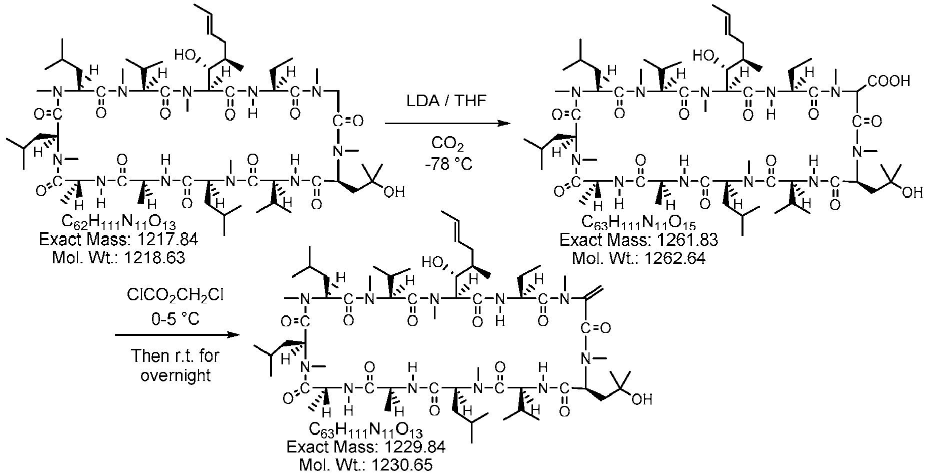

n-Butyllithium (2.2 M, 49.30 ml, 108.46 mmol) was added into a solution of diisopropylamine (15.39 ml, 108.46 mmol) in tetrahydrofuran (150 ml) at-78 °C under nitrogen. After the reaction mixture was stirred for an hour, a solution of [(y-hydroxy)-NMeLeu]-4-cyclosporin (12.00 g, 9.86 mmol) in tetrahydrofuran (30 ml) was added over 10 min. The stirring was continued at -78 °C for two hours. Carbon dioxide gas was bubbled through the reaction mixture for two hour and the mixture was stirred at -78 °C for another hour. Then the cooling bath was removed and the reaction mixture was allowed to warm up to room temperature slowly with bubbling out of unreacted carbon dioxide. The mixture was cooled to about 0-5 °C by ice bath and chloromethyl chloroformate (13.98 g, 108.46 mmol) was added. The mixture was allowed to warm to room temperature and stirred overnight. Water (30 ml) was added to quench the reaction. Most of solvent was then evaporated under reduced pressure. Ethyl acetate (100 ml) and water (80 ml) were added. The ethyl acetate layer was separated and washed with brine, dried over magnesium sulfate and evaporated under reduced pressure. The residue was purified by chromatography with hexane/acetone (from 90:10 to 70:30) as eluent to give 4.74 g of pure product of [α-Methylene-Sar]-3-[(γ-hydroxy)-NMeLeu]-4-cyclosporin* [Molecular Formula: C₆₃H₁₁₁N₁₁O₁₃; Exact Mass: 1229.84; MS (m/z): 1230.39 (M+1)⁺, 152.59 (M+Na)⁺; TLC R_{f}: 0.50 (ethyl acetate/methanol = 10/1); HPLC RT: 15.38 min (C8 reverse phase column: 250 mm; acetonitrile/water (0.05% trifluoroacetic acid); operation temperature: 64 °C; detector: 210 nm)]. This [α-Methylene-Sar]-3-[(γ-hydroxy)-NMeLeu]-4-cyclosporin prepared using a method analogous to the procedure described in WO2012/051194A1.

### Example 43

### Anti HCV Activity of Cyclosporin Derivatives

Anti-HCV activity of cyclosporine derivatives were evaluated in the HCV subgenomic replicon assay. The assay use the cell line ET (luc-ubi-neo/ET), which is a Huh7 human hepatoma cell line harboring an HCV replicon with a stable luciferase (Luc) reporter. HCV RNA replication was assessed by quantifying HCV replicon-derived luciferase activity. The antiviral activity of cyclosporine analogs were evaluated after drug treatment to derive EC₅₀ and EC₉₀ values by using the luciferase end point (Krieger, N., et al., 2001, J. Virol. 75, 4614-4624; Pietschmann, T., et al., 2002, J. Virol. 76, 4008-4021; each of which is incorporated herein by reference). Cytotoxicity was assessed in parallel.

**Table 2. Testing results of certain representative compounds**

| Compound | Antiviral activity |
|---|---|
| Cyclosporine A | * |
| [N-MeIle]-4-cyclosporin (SDZ-NIM-811) | ** |
| [N-MeVal]-4-cyclosporin (SDZ 220-384) | ** |
| (R)-2-(N,N-Dimethylamino)ethylthio-Sar]-3-[(γ-hydroxy)-N-MeLeu]-4-cyclosporin (SCY-635) | *** |
| [D-N-MeAla]-3-[N-EtVal]-4-cyclosporin (Alisporivir, Debio-025) | **** |
| (R)-2-(N,N-Diethylamino)ethylthio-Sar]-3-[N-MeIle]-4-cyclosporin | **** |
| (S)-2-(N,N-Diethylamino)ethylthio-Sar]-3-[N-MeIle]-4-cyclosporin | No activity |
| [8-(2-Acetamidoethyl)aminoethyl)-6,7-dihydro-MeBmt]-1-[(R)-2-methyl-Sar]-3-cyclosporin (CPI-431-32(CRV431)) | **** |
| [8-(2-Acetamidoethyl)-6,7-dihydro-MeBmt]-1-cyclosporin | **** |
| [8-(2-Acetamidoethyl)-6,7-dihydro-MeBmt]-1-[(R)-Methyl]-3-cyclosporin | **** |
| [8-(2-Acetamidoethyl)-6,7-dihydro-MeBmt]-1-[(γ-hydroxy)-N-MeLeu]-4-cyclosporin | **** |
| [8-(2-Acetamidoethyl)-6,7-dihydro-MeBmt]-1-[(S)-(4-hydroxybutylthio)methyl-Sar]-3-[(γ-hydroxy)-N-MeLeu]-4-cyclosporin | **** |
| [8-(2-Acetamidoethyl)-6,7-dihydro-MeBmt]-1-[(R)-hydroxymethyl-Sar]-3-cyclosporin | **** |
| [8-(2-Acetamidoethyl)-6,7-dihydro-MeBmt]-1-[(S)-(4-hydroxybutylthio)methyl-Sar]-3-cyclosporin | **** |

| | |
|---|---|
| Antiviral activity: ****IC₅₀ < 35 nM; ***35 nM < IC₅₀ < 90 nM; **90 nM < IC₅₀ < 250 nM; * 250 nM < IC₅₀ < 450 nM; No activity > 1500 nM. | |

### Example 44

### Cyclophilin A and D (CyPA & CyPD) PPIase Inhibition Assay

Inhibition of CyPA isomerase activity was assessed using the α-chymotrypsin-coupled assay adapted to a 96-well plate format. Human recombinant CypA (Atgen) was dissolved to 10 nM in isomerase buffer (50 mM Hepes, 100 mM NaCl, 1 mg/ml bovine serum albumin, 1 mg/ml α-chymotrypsin; pH 8). Succinyl-AAPF-pNA peptide substrate (Sigma) was dissolved to 3.2 mM in dried LiCl/trifluoroethanol. Each test compound was prepared at 10 concentrations in DMSO, and then diluted into CypA-isomerase buffer to 0.05-1000 nM (reaction mix). All solutions were equilibrated, and reactions conducted at 5°C. Reactions were initiated by mixing 95 µL reaction mix with 5 µL peptide preloaded in multiple wells of 96-well plates and measuring OD405 nm in each well at 6-sec intervals for 6 min using a BMG Polarstar Galaxy plate reader. Data were fitted with Graphpad Prism 6.0 to obtain first-order rate constants. Enzyme catalyzed rate constants were calculated by subtracting the rate constant from uncatalyzed reactions (no CypA), and the catalytic rate constants plotted as a function of inhibitor concentration to obtain IC50s (see Gallay et al., 2015, PLOS ONE).

**Table 3. EC₅₀ based on PPIase Inhibition of Cyclophilin A (CyPA) for certain representative compounds**

| Compound | PPIase Inhibition of CyPA |
|---|---|
| Cyclosporine A | * |
| (R)-2-(N,N-Dimethylamino)ethylthio-Sar]-3-[(y-hydroxy)-N-MeLeu]-4-cyclosporin (SCY-635) | ** |
| [D-N-MeAla]-3-[N-EtVal]-4-cyclosporin (Alisporivir, Debio-025) | *** |
| [8-(2-Acetamidoethyl)aminoethyl)-6,7-dihydro-MeBmt]-1-[(R)-2-methyl-Sar]-3-cyclosporin (CPI-431-32(CRV431)) | *** |
| [(S)-(4-Hydroxylbutylthio)methyl-Sar]-3-[(γ-hydroxy)-N-MeLeu]-4-cyclosporin | *** |
| [8-(2-Acetamidoethyl)-6,7-dihydro-MeBmt]-1-[(S)-(4-hydroxybutylthio)methyl-Sar]-3-cyclosporin | *** |

| | |
|---|---|
| CyPA PPIase inhibition activity: *** IC₅₀ < 4.0 nM; **4.0 nM < IC₅₀ < 10.0 nM; * 10.0 nM < IC₅₀ < 20.0 nM. | |

**Table 4. EC₅₀ based on PPIase Inhibition of Cyclophilin D (CyPD) for certain representative compounds**

| Compound | PPIase Inhibition of CyPD EC₅₀ (nM) |
|---|---|
| Cyclosporine A | * |
| (R)-2-(N,N-Dimethylamino)ethylthio-Sar]-3-[(γ-hydroxy)-N-MeLeu]-4-cyclosporin (SCY-635) | ** |
| [D-N-MeAla]-3-[N-EtVal]-4-cyclosporin (Alisporivir, Debio-025) | *** |
| [8-(2-Acetamidoethyl)aminoethyl)-6,7-dihydro-MeBmt]-1-[(R)-2-methyl-Sar]-3-cyclosporin (CPI-431-32(CRV431)) | *** |
| [(S)-(4-Hydroxylbutylthio)methyl-Sar]-3-[(γ-hydroxy)-N-MeLeu]-4-cyclosporin | *** |
| [8-(2-Acetamidoethyl)-6,7-dihydro-MeBmt]-1-[(S)-(4-hydroxybutylthio)methyl-Sar]-3-cyclosporin | *** |

| | |
|---|---|
| CyPD PPIase inhibition activity: *** IC₅₀ < 4.0 nM; **4.0 nM < IC₅₀ < 10.0 nM; * 10.0 nM < IC₅₀ < 20.0 nM. | |

The present application and invention further includes the subject matter of the following numbered clauses:
1. A compound of Formula (I): 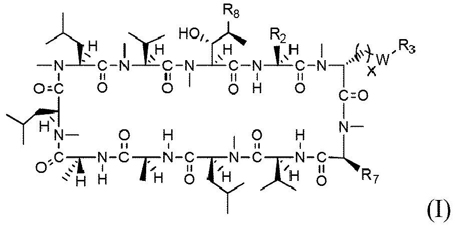 or pharmaceutically acceptable salt thereof, wherein:
x is 0 or 1;
R₈ is alkyl, alkenyl, alkynyl, cycloalkyl, cycloalkenyl, aryl, or heteroaryl; wherein R₈ is substituted by one or more R₁; provided that R₈-R₁ is not n-butyl or (*E*)-but-2-enyl;
R₂ is ethyl, 1-hydroxyethyl, isopropyl or n-propyl;
W is O, S, or CH₂;
R₃ is H, alkyl or substituted alkyl, alkenyl or substituted alkenyl, alkynyl or substituted alkynyl, cycloalkyl or substituted cycloalkyl, cycloalkenyl or substituted cycloalkenyl, aryl or substituted aryl, or heteroaryl or substituted heteroaryl; wherein R₃ is optionally substituted by one or more R₁;
each occurrence of R₁ is independently H, halogen, aryl or substituted aryl, heteroaryl or substituted heteroaryl, CN, OR_{A}, SR_{A}, NR_{A}R_{B}, -NR_{A}(CH₂)_{O}OR_{B}, -N((CH₂)_{O}OR_{A})((CH₂)_{O}OR_{B}) -C(=O)R_{A}, -C(=O)OR_{A}, -OC(=O)R_{A}, -OC(=O)(C₁-C₆ alkyl-RH), -OC(=O)(CH₂)_{O}OR_{A},-C(=O)NR_{A}R_{B}, -NR_{A}C(=O)R_{B}, -N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B},-N(C(=O)(CH₂)_{O}OR_{B})₂, -NR_{A}C(=O)(CH₂)_{O}NR_{A}R_{B}, -NR_{A}(CH₂)_{O}C(=O)OR_{B},-N((CH₂)_{O}C(=O)OR_{A})((CH₂)_{O}C(=O)OR_{B}), -NR_{A}(CH₂)_{O}C(=O)NR_{A}R_{B},-N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B},-C(=O)N((CH₂)_{O}OR_{B})₂, -N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B},-NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, NR_{C}(CH₂)ₘNR_{C}(CH₂)ₘNR_{A}R_{B}, 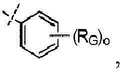 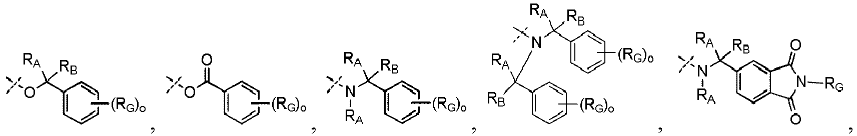 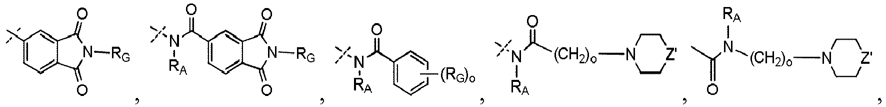 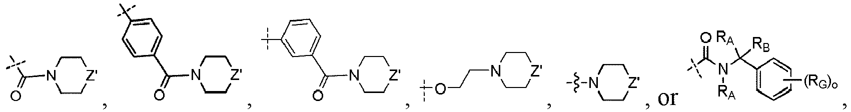 wherein said aryl or heteroaryl is optionally substituted by one or more groups which may be the same or different selected from the group consisting of halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A}; 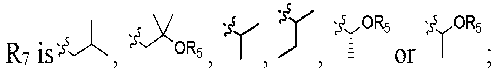
each R₅ is independently H, alkyl or substituted alkyl, alkenyl or substituted alkenyl, alkynyl or substituted alkynyl, cycloalkyl or substituted cycloalkyl, cycloalkenyl or substituted cycloalkenyl, or aryl or substituted aryl;
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl or 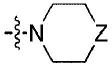 wherein Z is CH₂, O, S, NH, NCH₃, NEt, N-isopropyl, N-isopropyl, N-neoPentyl, N-CH₂CH₂OH, or N-CH₂CH₂OMe;
each occurrence of R_{G} is independently R_{A}, OR_{A}, SR_{A}, NR_{A}R_{B}, -(CH₂)_{O}R_{A},-(CH₂)_{O}C(=O)OR_{A}, -(CH₂)_{O}C(=O)NR_{A}R_{B}, C(=O)OR_{A}, OC(=O)R_{A}, NR_{A}C(=O)R_{B}, NR_{A}C(=O)(CH₂)_{O}OR_{A}, C(=O)O(CH₂)_{O}OR_{A}, C(=O)OR_{B}, C(=O)NR_{A}R_{B}, C(=O)NR_{A}(CH₂)_{O}OR_{B}, C(=O)N((CH₂)_{O}OR_{A})((CH₂)_{O}OR_{B}), C(=O)N((CH₂)_{O}C(=O)OR_{A})((CH₂)_{O}C(=O)OR_{B}), C(=O)N((CH₂)_{O}NR_{A}R_{B})((CH₂)_{O}NR_{A}R_{B}), C(=O)N((CH₂)_{O}OC(=O)(CH₂)_{O}OR_{A})((CH₂)_{O}OC(=O)(CH₂)_{O}OR_{B}), C(=O)N((CH₂)_{O}NR_{A}C(=O)(CH₂)_{O}OR_{B})((CH₂)_{O}NR_{A}C(=O)(CH₂)_{O}OR_{B}), C(=O)NR_{A}(CH₂)ₒNR_{A}R_{B}, , 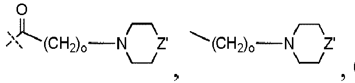 C(=O)NR_{A}(CH₂)ₒOC(=O)R_{B}, C(=O)NR_{A}(CH₂)ₒC(=O)OR_{B}, C(=O)NR_{A}(CH₂)ₒC(=O)NR_{A}R_{B}, C(=O)NR_{A}(CH₂)ₒOC(=O)(CH₂)ₒOR_{B}, or 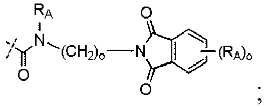
each occurrence of R_{H} is independently halogen;
each occurrence Z' is independently CH₂, O, S, NR_{A}, N(CH₂)_{O}OR_{A}, N(CH₂)_{O}NR_{A}R_{B}, N(CH₂)_{O}COOR_{A}, N(CH₂)_{O}OC(=O)R_{A}, N(CH₂)_{O}CONR_{A}R_{B}, N(CH₂)_{O}NR_{A}C(=O)R_{B}, or N(CH₂)_{O}OC(=O)(CH₂)_{O}OR_{A};
each occurrence of o is independently 0, 1, 2, 3, 4, 5, or 6;
each occurrence of p is independently an integer of 0, 1, 2, 3, 4, or 5; and
each occurrence of m is independently an integer of 1, 2, 3, 4 or 5.
2. The compound of clause 1, wherein R₈ is (C₁-C₁₂)alkyl, (C₂-C₁₂)alkenyl, (C₂-C₁₂)alkynyl, (C₃-C₁₂)cycloalkyl, or phenyl or CH₂-phenyl optionally substituted by one or more groups which may be the same or different selected from halogen, hydroxy, (C₁-C₆)alkyl.
3. The compound of any one of the preceding clauses, wherein R₈ is (C₁-C₁₄)alkyl or (C₁-C₁₄)alkenyl.
4. The compound of any one of the preceding clauses, wherein R₈ is (C₁-C₆) linear alkyl or (C₇-C₁₂) linear alky.
5. The compound of any one of the preceding clauses, wherein R₈ is a -(CH₂)₃₋₁₁-alkyl chain.
6. The compound of any one of the preceding clauses, wherein R₂ is ethyl.
7. The compound of any one of the preceding clauses, wherein R₇ is 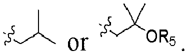 8. The compound of any one of the preceding clauses, wherein W is O.
9. The compound of any one of the preceding clauses, wherein W is S.
10. The compound of any one of the preceding clauses, wherein R₃ is H, (C₁-C₁₂)alkyl, (C₂-C₁₂)alkenyl, (C₂-C₁₂)alkynyl, (C₃-C₁₂)cycloalkyl, or phenyl or CH2-phenyl, optionally substituted by one or more groups which may be the same or different selected from the group consisting of halogen, hydroxy, and (C₁-C₆)alkyl.
11. The compound of clause 1, having the structure of Formulae (II) or (III): 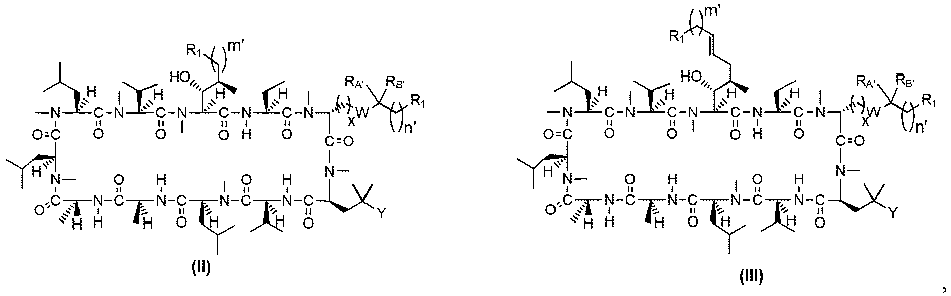 or pharmaceutically acceptable salt thereof, wherein:
Y is H or OR₅; wherein R₅ is H or methyl;
m' and n' are each independently 0, 1, 2, 3, 4, 5, 6, 7, 8, 9, 10, 11, or 12;
each occurrence of R_{A}, and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
   or R_{A'} and R_{B'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl; and
each occurrence of R_{D} is independently halogen, hydroxy, O(C₁-C₄)alkyl, C(=O)(C₁-C₄)alkyl, C(=O)O(C₁-C₄)alkyl or 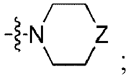 wherein Z is CH₂, O, S, NH, NCH₃, NEt, N-isopropyl, N-isopropyl, N-neoPentyl, N-CH₂CH₂OH, or N-CH₂CH₂OMe.
12. The compound of clause 11, having structure of Formula (IV) or (V): 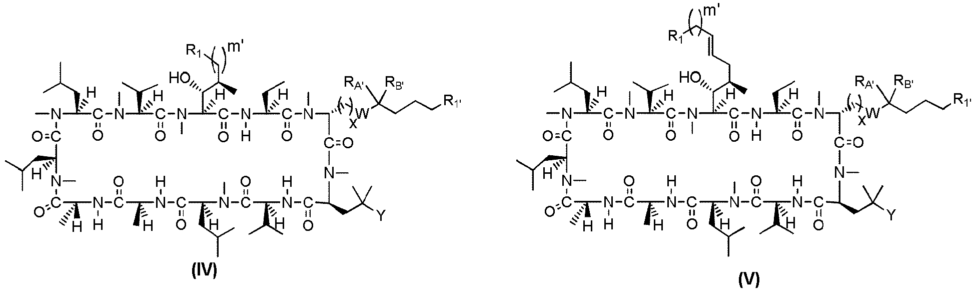 ; wherein each occurrence of R_{1'} is independently H, halogen, aryl or substituted aryl, heteroaryl or substituted heteroaryl, OR_{A}, SR_{A}, NR_{A}R_{B}, -NR_{A}(CH₂)_{O}OR_{B}, -N((CH₂)_{O}OR_{A})((CH₂)_{O}OR_{B})-C(=O)R_{A}, -C(=O)OR_{A}, -OC(=O)R_{A}, -OC(=O)(C₁-C₆ alkyl-R_{H}), -OC(=O)(CH₂)ₒOR_{A},-C(=O)NR_{A}R_{B}, -NR_{A}C(=O)R_{B}, -N(C(=O)R_{A})(C(=O)R_{B}), -N(C(=O)(C₁-C₆ alkyl-R_{H}))(C(=O)(C₁-C₆ alkyl-R_{H})), -N(C(=O)(C₁-C₆ alkyl-R_{H}))₂, -NR_{A}C(=O)(CH₂)_{O}OR_{B}, -NR_{A}C(=O)(CH₂)_{O}OR_{B},-N(C(=O)(CH₂)_{O}OR_{B})₂, -NR_{A}C(=O)(CH₂)_{O}NR_{A}R_{B}, -NR_{A}(CH₂)_{O}C(=O)OR_{B},-N((CH₂)_{O}C(=O)OR_{A})((CH₂)_{O}C(=O)OR_{B}), -NR_{A}(CH₂)_{O}C(=O)NR_{A}R_{B},-N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B},-C(=O)N((CH₂)_{O}OR_{B})₂, -N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B},-NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, NR_{C}(CH₂)ₘNR_{C}(CH₂)ₘNR_{A}R_{B}, 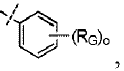 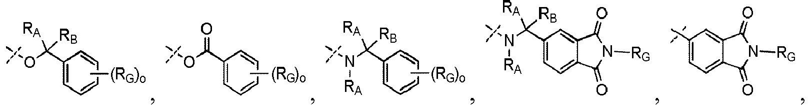 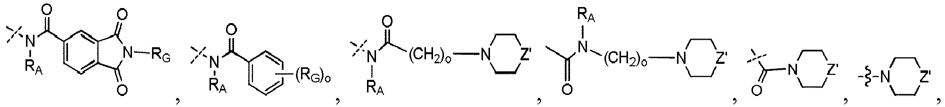 or 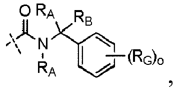 wherein said aryl or heteroaryl is optionally substituted by one or more groups which may be the same or different selected from the group consisting of halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A.}
13. The compound of clause 12, wherein R_{1'} is OR_{A}, OCOCH₂OR_{A}, SR_{A}, NHR_{A}, N(R_{A})₂, or NHCOCH₂OR_{A}.
14. The compound of clause 12, wherein R_{1'} is OAc, OCOCH₂Cl, OCOCH₂CH₃, OCOCHMe₂, OCOCMe₃, OCOCH=CH₂, NHCH₂CH₂OH, NHCH₂CH₂OMe, N(CH₂CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂.
15. The compound of clause 11 or 12, wherein R_{A'} and R_{B'} are each independently H, Me, Et, n-Propyl, isoProyl, isoButyl, neoPentyl, cyclopentyl, cyclohexyl, CH₂CH₂OH, CH₂CH₂OMe, 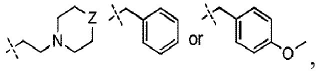 wherein Z is CH₂, O, S, NH, NCH₃, NEt, N-isopropyl, N-isopropyl, N-neoPentyl, N-CH₂CH₂OH, or N-CH₂CH₂OMe.
16. The compound of clause 12, wherein R₁ is -NR_{A}C(=O)R_{B} and R_{1'} is OR_{A}.
17. The compound of clause 16, wherein R₁ is -NHC(=O)R_{B} and R_{1'} is OH.
18. The compound of clause 1, having structure of Formula (VI): 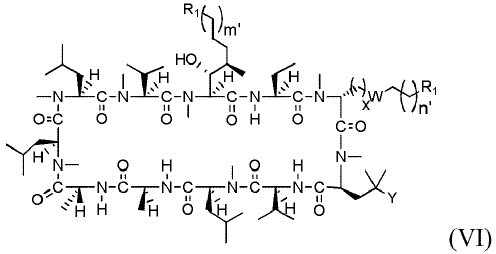 wherein
W is CH₂, O or S;
Y is H or OR₅; wherein R₅ is H or methyl;
m' and n' are each independently 0, 1, 2, 3, 4, 5, 6, 7, 8, 9, or 10; and
each occurrence of R₁ is independently H, halogen, aryl or substituted aryl, heteroaryl or substituted heteroaryl, OR_{A}, SR_{A}, NR_{A}R_{B}, -C(=O)R_{A}, -C(=O)OR_{A}, -C(=O)NR_{A}R_{B}, - NR_{A}C(=O)R_{B}, -NR_{A}C(=O)(CH₂)ₒR_{B}, -NR_{A}C(=O)(CH₂)ₒOR_{B}, -NR_{A}C(=O)(CH₂)ₒNR_{A}R_{B}, or -C(=O)NR_{A}(CH₂)ₒR_{B}.
19. The compound of any one of the preceding clauses, wherein R₁ is selected from the group consisting of H, OR_{A}, SR_{A}, NR_{A}R_{B}, -C(=O)R_{A}, -C(=O)OR_{A}, -C(=O)NR_{A}R_{B}, - NR_{A}C(=O)R_{B}, -NR_{A}C(=O)(CH₂)_{O}R_{B}, -NR_{A}C(=O)(CH₂)_{O}OR_{B}, -NR_{A}C(=O)(CH₂)_{O}NR_{A}R_{B},-C(=O)NR_{A}(CH₂)_{O}R_{B},
-C(=O)NR_{A}(CH₂)_{O}OR_{B}, and -C(=O)NR_{A}(CH₂)_{O}NR_{A}R_{B}.
20. The compound of any one of the preceding clauses, wherein R₁ is selected from the group consisting of O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, and NR_{C}(CH₂)ₘNR_{C}(CH₂)ₘNR_{A}R_{B}.
21. The compound of any one of the preceding clauses, wherein R₁ is selected from the group consisting of OR_{A}, SR_{A}, NR_{A}R_{B}, -C(=O)R_{A}, -C(=O)OR_{A}, and -C(=O)NR_{A}R_{B}.
22. The compound of any one of the preceding clauses, wherein R₁ is OH, OMe, OEt, O-isopropyl, O-isoButyl, O-neoPentyl, O-cyclopentyl, O-cyclohexyl, SH, SMe, S-isopropyl, S-isoButyl, S-neoPentyl, S-cyclopentyl, S-cyclohexyl, 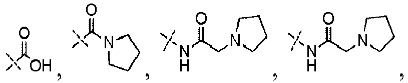 or 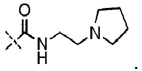 23. The compound of any one of the preceding clauses, wherein R₁ is 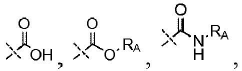 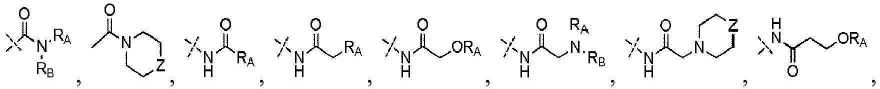 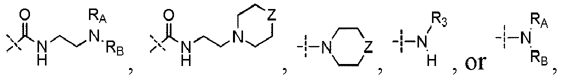 wherein Z is CH₂, O, S, NH, NCH₃, NEt, N-isopropyl, N-isopropyl, N-neoPentyl, N-CH₂CH₂OH, or N-CH₂CH₂OMe.
24. The compound of any one of the preceding clauses, wherein R₁ is selected from the group consisting of H, OH, OMe, OEt, O-isoProyl, O-isoButyl, O-neoPentyl, O-cyclopentyl, O-cyclohexyl, OCH₂CH₂OH, OCH₂CH₂OCH₃, OCH₂COOH, OCH₂COOCH₃, OCH₂CONH₂, OCH₂CONHMe, OCH₂CONMe₂, OCH₂CONHCH₂CH₂OH, OCH₂CONHCH₂CH₂OMe, OAc, OOCCH₂Cl, OOCCH₂OR_{A}, OOCCH₂CH₃, OOCCHMe₂, OOCCMe₃, and OC(=O)CCH=CH₂.
25. The compound of any one of the preceding clauses, wherein R₁ is selected from the group consisting of: 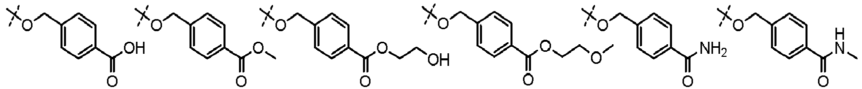 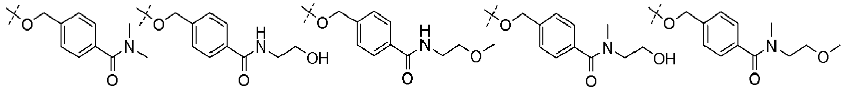 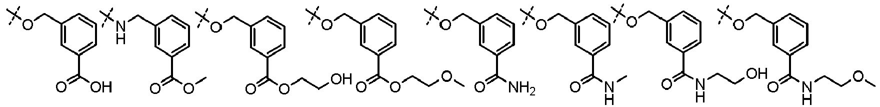 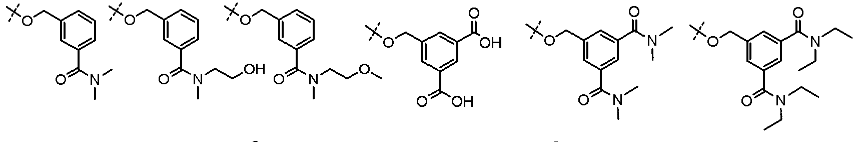 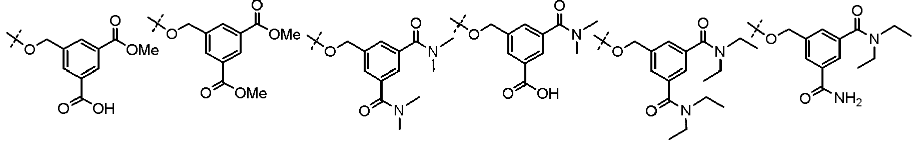 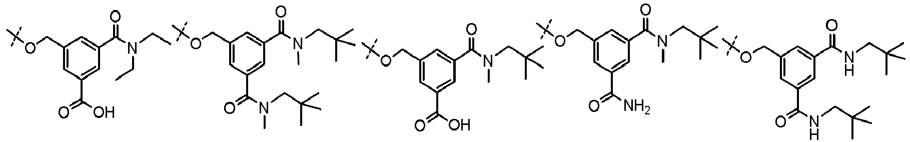 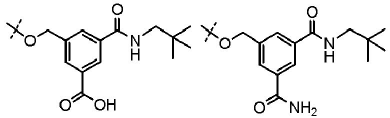 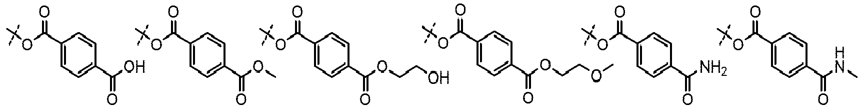 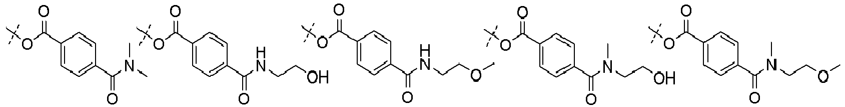 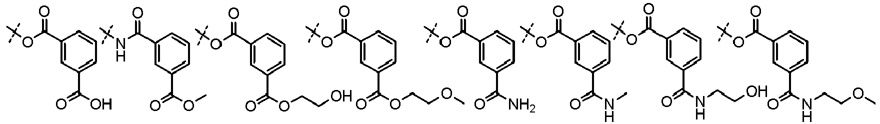 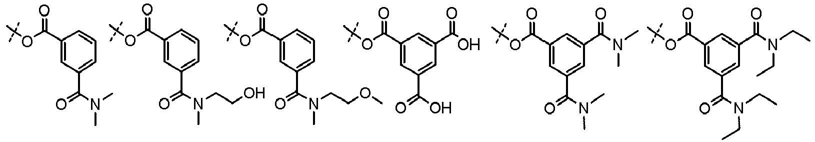 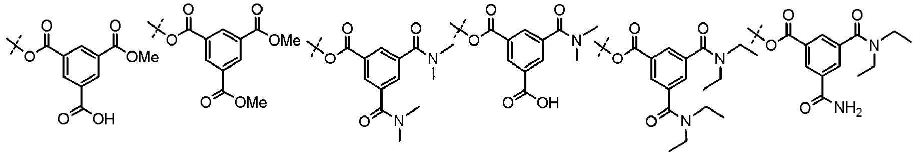 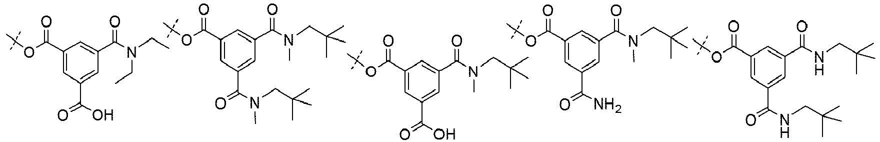 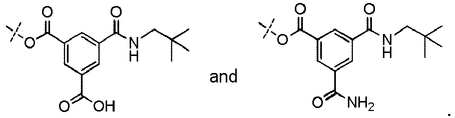 26. The compound of any one of the preceding clauses, wherein R₁ is SH, SMe, SEt, S-isoProyl, S-isoButyl, S-neoPentyl, O-cyclopentyl, or S-cyclohexyl.
27. The compound of any one of the preceding clauses, wherein R₁ is selected from the group consisting of NH₂, NHCH₃, NHCH₂CH₃, NHCH₂CHOH, NHCH₂CH₂OMe, NMe₂, NEt₂, NHCH₂CHMe₂, NHCH₂CMe₃, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂OR_{A}, NHCOCH₂CH₃, NHCOCHMe₂, NHCOCMe₃, NHCOCH=CH₂, N(COCH₂Cl)₂, N(COCH₂OR_{A})₂, N(COCH₂CH₃)₂, N(COCHMe₂)₂, N(COCMe₃)₂, and N(COCH=CH₂)₂.
28. The compound of any one of the preceding clauses, wherein R₁ is 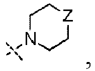 wherein Z is CH₂, O, S, NH, NMe, NEt, N-isopropyl, N-neoPentyl, N-CH₂CH₂OH, NCH₂CH₂OCH₃, NCH₂CH₂OCH₃, NCH₂COOH, NCH₂COOMe, N-CH₂CONH₂, NCH₂CONHMe, or NCH₂CONMe₂.
29. The compound of any one of the preceding clauses, wherein R₁ is selected from the group consisting of: 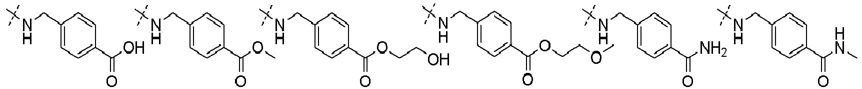 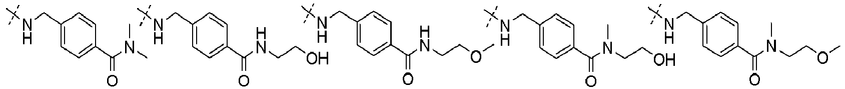 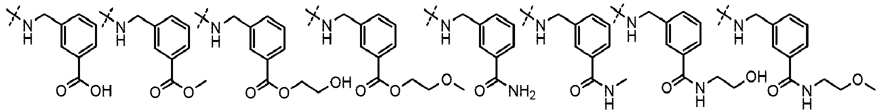 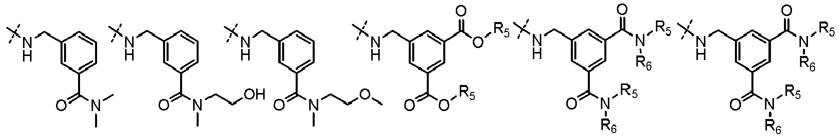 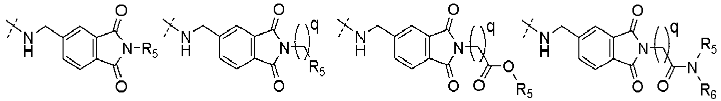 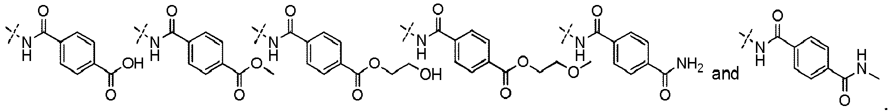 30. The compound of any one of the preceding clauses, wherein R₁ is selected from the group consisting of: 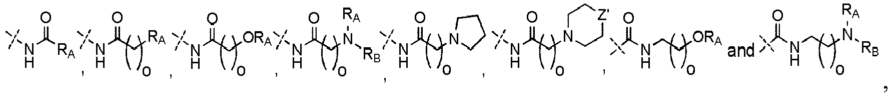 wherein each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNR_{A}C(=O)R_{B}, or N(CH₂)ₒOC(=O)(CH₂)ₒOR_{A}.
31. The compound of any one of the preceding clauses, wherein R₁ is -COOH, -COOMe,-COOEt, -CONH₂, -CONHMe, -CONMe₂, -CONHEt, -CONEt₂, -CONHCH₂CH₂OH,-CONHCH₂CH₂OMe, -CON(CH₂CH₂OH)₂, -CON(CH₂CH₂OMe)₂, or -CONMe₂.
32. The compound of any one of the preceding clauses, wherein R₁ is selected from the group consisting of: 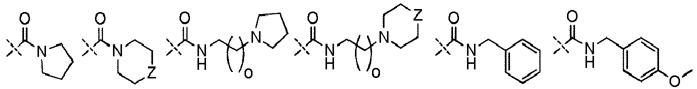 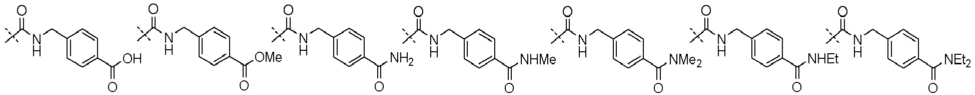 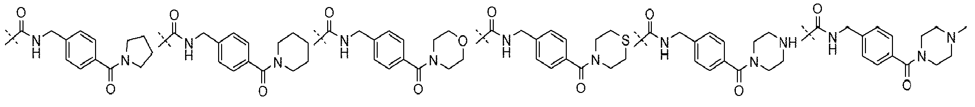 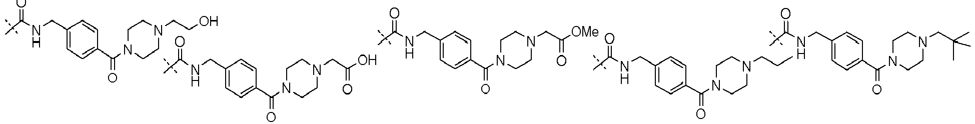 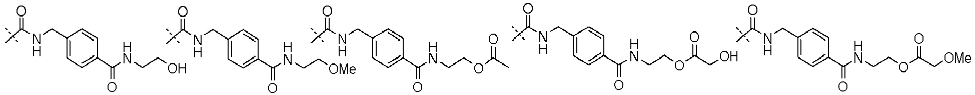 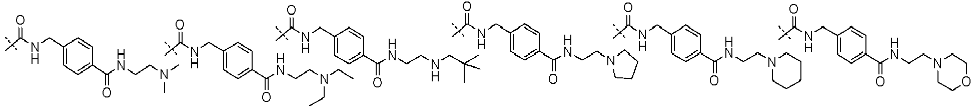 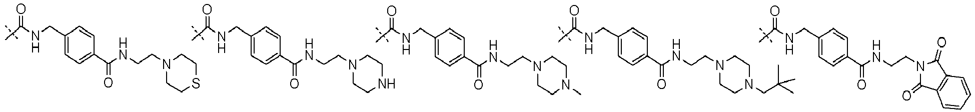 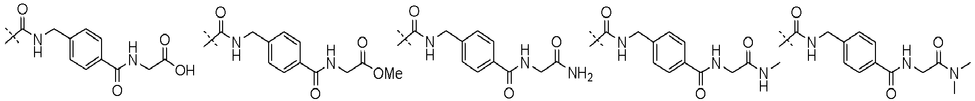 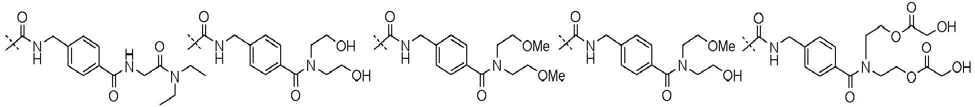 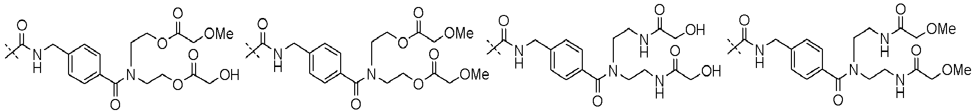 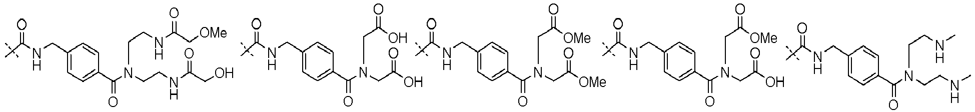 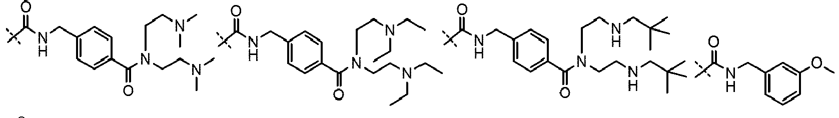 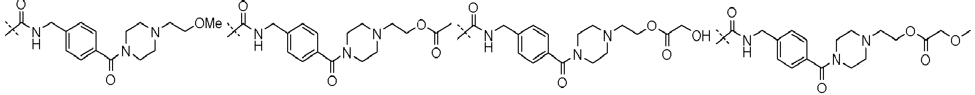 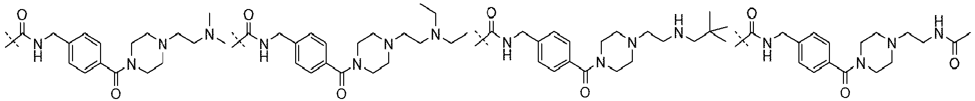 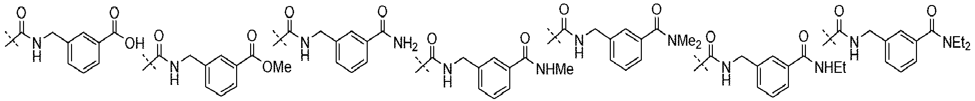 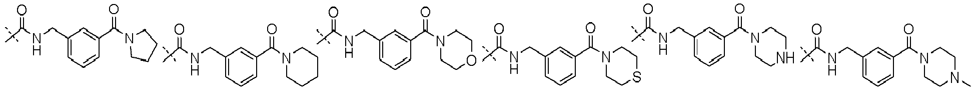 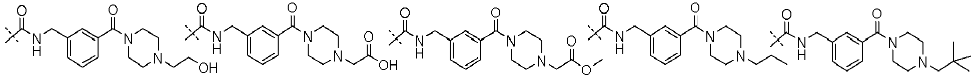 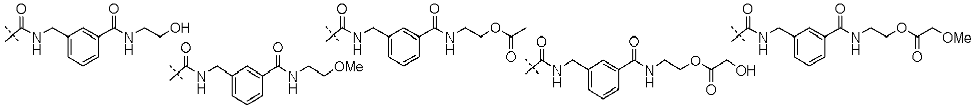 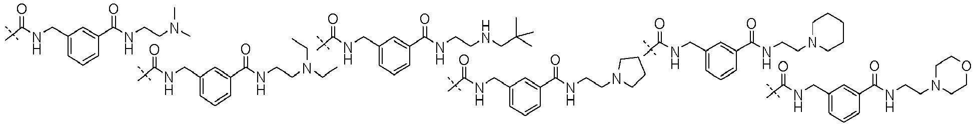 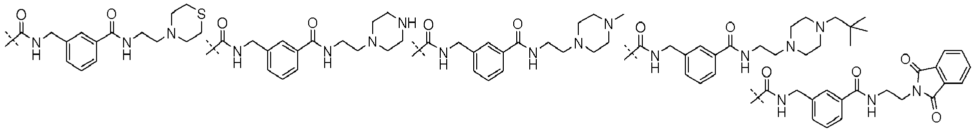 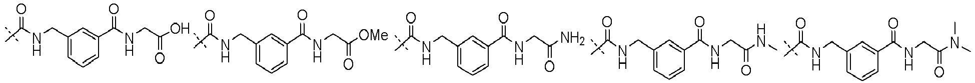 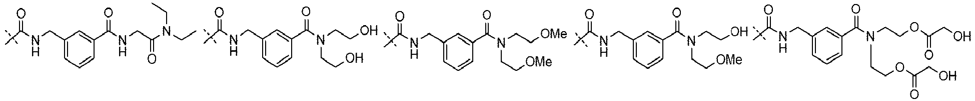 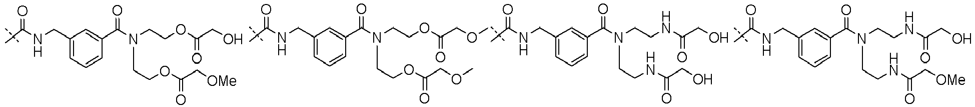 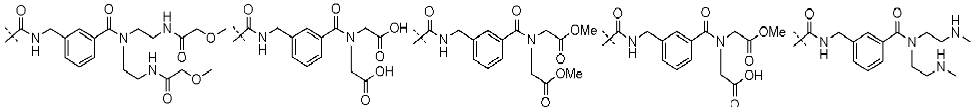 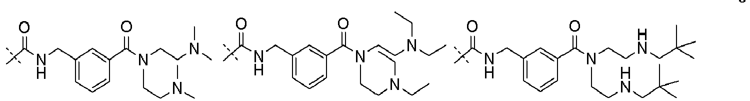 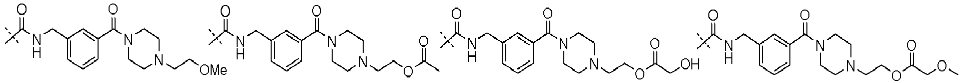 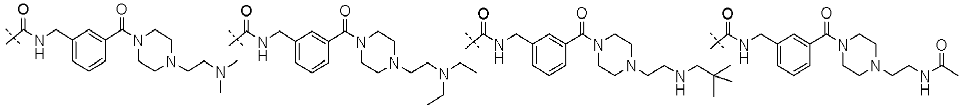 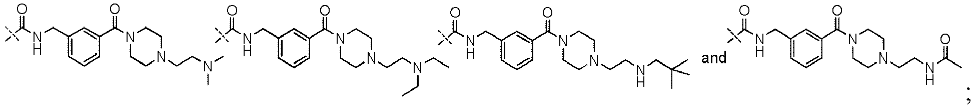 wherein Z is CH₂, O, S, NH, NCH₃, NEt, N-isopropyl, N-isopropyl, N-neoPentyl, N-CH₂CH₂OH, or N-CH₂CH₂OMe.
33. The compound of any one of the preceding clauses, wherein R₁ is 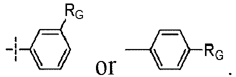 34. The compound of clause 33, wherein R_{G} is OH, OMe, OAc, NH₂, NHMe, NHAc, NMe₂, NEt₂, NHCH₂CMe₃.
35. The compound of any one of the preceding clauses, wherein R₁ is selected from the group consisting of: 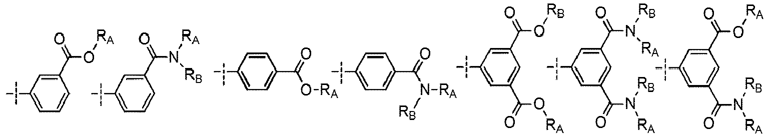 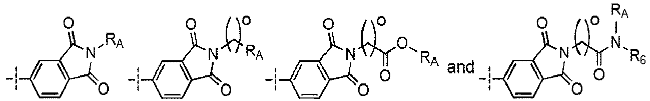 36. The compound of any one of the preceding clauses, wherein R₁ is OR_{A}, OCOCH₂OR_{A}, SR_{A}, NHR_{A}, N(R_{A})₂, or NHCOCH₂OR_{A}.
37. The compound of any one of the preceding clauses, wherein R₁ is OAc, OCOCH₂Cl, OCOCH₂CH₃, OCOCHMe₂, OCOCMe₃, OCOCH=CH₂, NHCH₂CH₂OH, NHCH₂CH₂OMe, N(CH_{Z}CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂.
38. The compound of clause 1, wherein R₁ is aryl or heteroaryl optionally substituted by one or more groups which may be the same or different selected from halogen, hydroxy, (C₁- C₆)alkyl, (C₃-C₇)cycloalkyl, SR_{A}, (CH₂)ₚOR_{A}, (CH₂)ₚNR_{A}R_{B}, (CH₂)ₚC(=O)R_{A}, (CH₂)_{P}C(=O)NR_{A}R_{B} and (CH₂)ₚC(=O)OR_{A}; wherein
p is 0, 1, 2, 3, 4, 5, 6; and
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{D} is independently halogen, hydroxy, O(C₁-C₄)alkyl, C(=O)(C₁-C₄)alkyl, C(=O)O(C₁-C₄)alkyl or 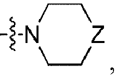 wherein Z is CH₂, O, S, NH, NCH₃, NEt, N-isopropyl, N-isopropyl, N-neoPentyl, N-CH₂CH₂OH, or N-CH₂CH₂OMe.
39. The compound of clause 1, wherein R₁ is 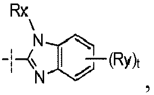 wherein Rx is H, (C₁-C₆)alkyl, or (C₃-C₇)cycloalkyl; Ry is H, (C₁-C₆)alkyl, (C₃-C₇)cycloalkyl, OR_{A}, SR_{A}, NR_{A}R_{B}, -C(=O)R_{A},-C(=O)OR_{A}, or -C(=O)NR_{A}R_{B}; and t is 1, 2, 3, or 4.
40. The compound of clause 39, wherein Rx is H or Me; and Ry is -C(=O)OR_{A} or -C(=O)NR_{A}R_{B}.
41. The compound of clause 40, wherein R₁ is 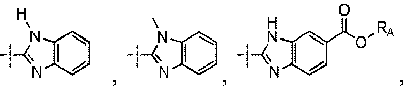 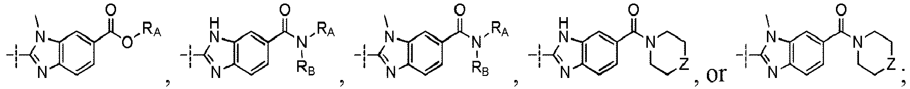 wherein Z is CH₂, O, S, NH, NCH₃, NEt, N-isopropyl, N-isopropyl, N-neoPentyl, N-CH₂CH₂OH, or N-CH₂CH₂OMe.
42. The compound of any one of the preceding clauses, wherein R_{A} and R_{B} are each independently H, Me, Et, n-Propyl, isoProyl, isoButyl, neoPentyl, cyclopentyl, cyclohexyl, CH₂CH₂OH, CH₂CH₂OMe, 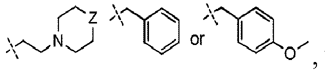 wherein Z is CH₂, O, S, NH, NCH₃, NEt, N-isopropyl, N-isopropyl, N-neoPentyl, N-CH₂CH₂OH, or N-CH₂CH₂OMe.
43. The compound of clause 42, wherein R_{A} is H, Me, Et, n-Proyl, isoProyl, n-Butyl, or isobutyl.
44. The compound of clause 42, wherein R_{A} and R_{B} are each independently H, Me, Et, isopropyl, isobutyl, cyclopentyl, or cyclohexyl.
45. The compound of any one of the preceding clauses, wherein each occurrence R_{A} and R_{B} is independently H, (C₁-C₆)alkyl, phenyl, CH₂-phenyl, (C₁-C₆)alkyl-OH, (CH₂)ₚO(CH₂)ₘOH, or (CH₂)ₚO(CH₂)ₘO(CH₂)ₘOH, (C₁-C₆)alkyl-O-(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl.
46. The compound of any one of the preceding clauses, wherein each occurrence R_{A} and R_{B} is independently H or (C₁-C₆)alkyl.
47. The compound of clause 46, wherein R_{A} and R_{B} are each independently H, Me, Et, isopropyl, isobutyl, cyclopentyl, or cyclohexyl.
48. The compound of any one of the preceding clauses, wherein R_{A} and R_{B}, together with the nitrogen atom to which they are attached, form a heterocycle selected from 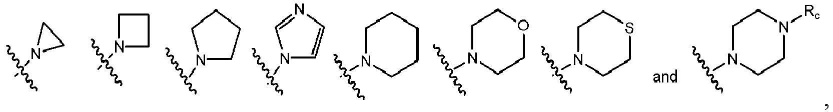 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.
49. The compound of clause 1, having a structure selected from the group consisting of the following structures: 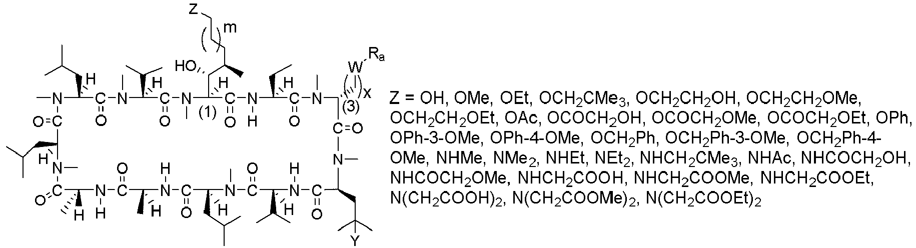 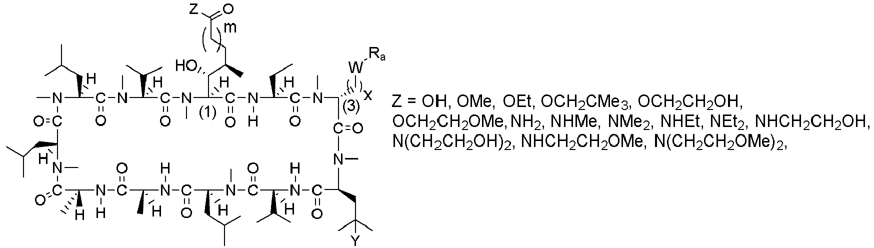 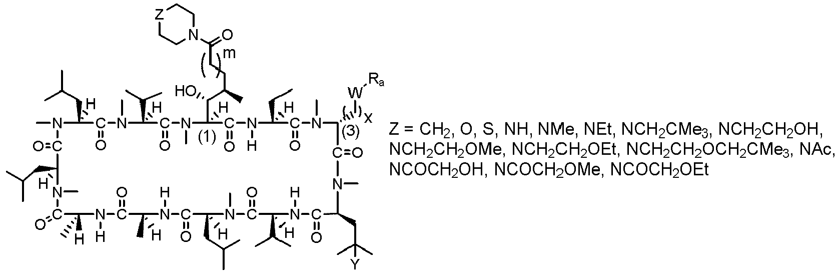 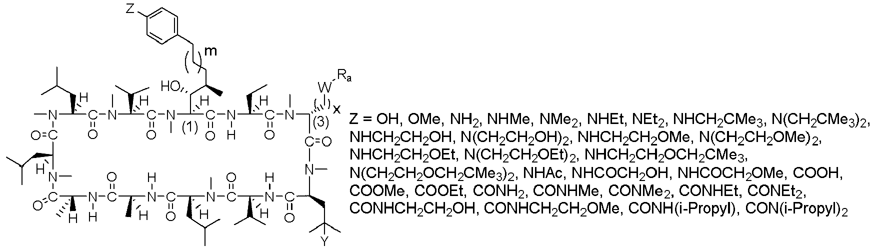 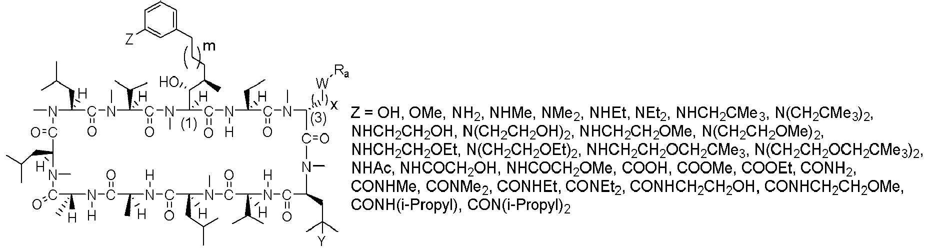 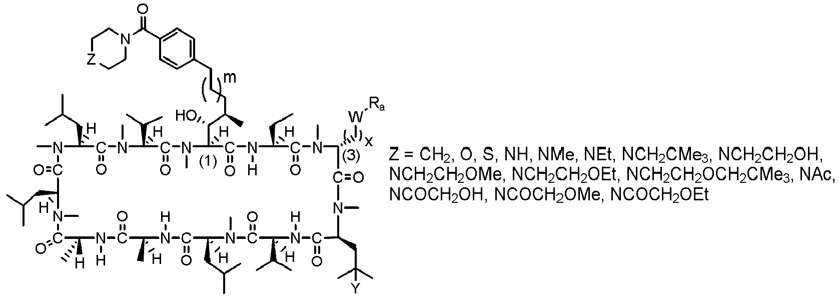 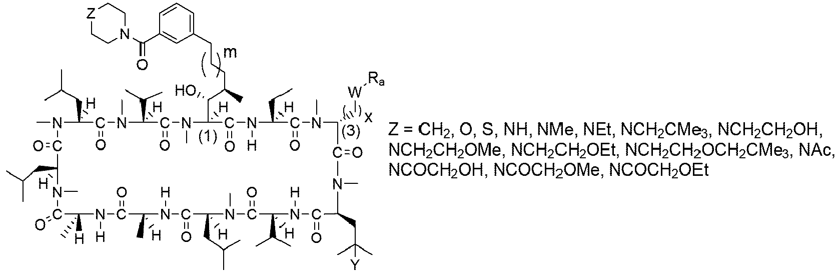 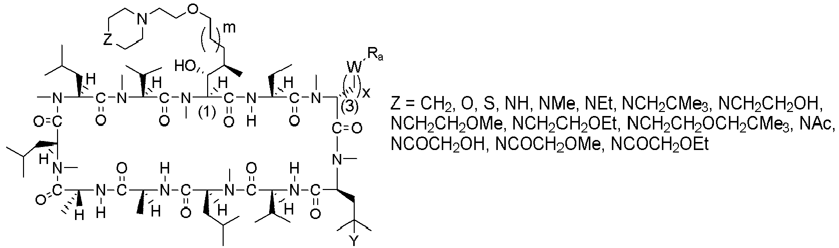 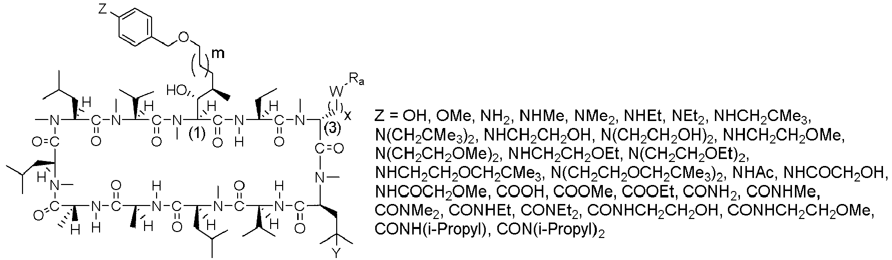 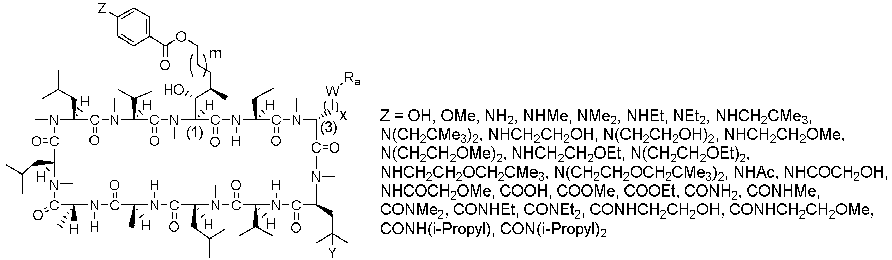 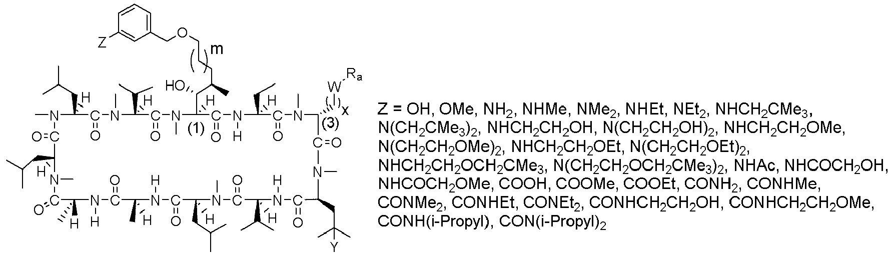 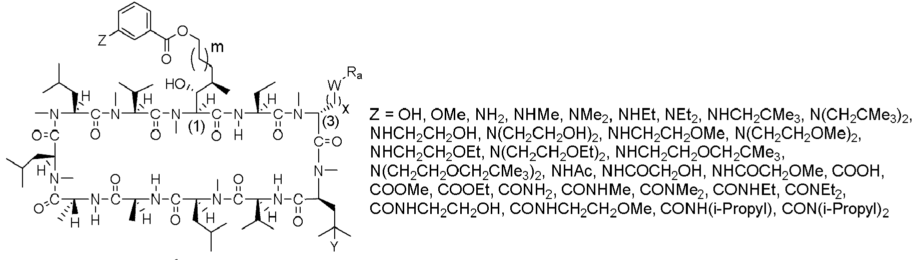 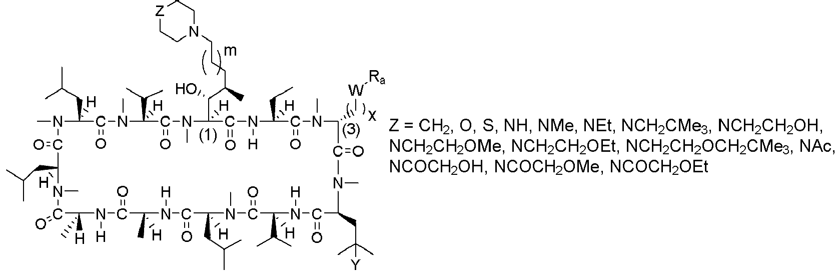 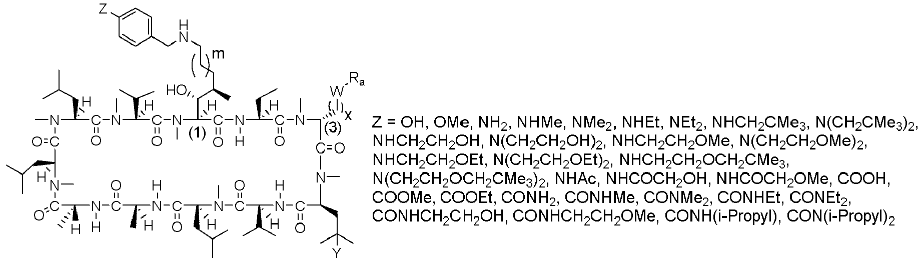 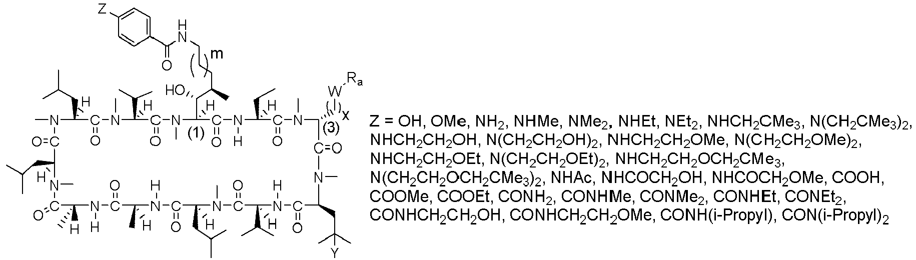 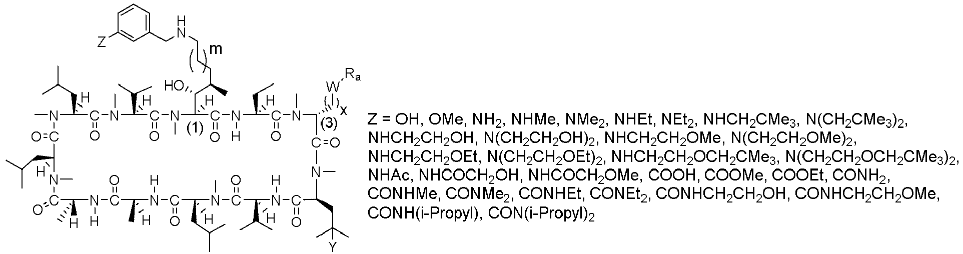 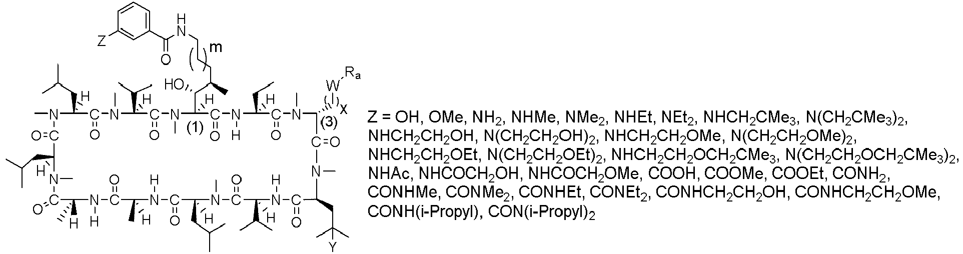 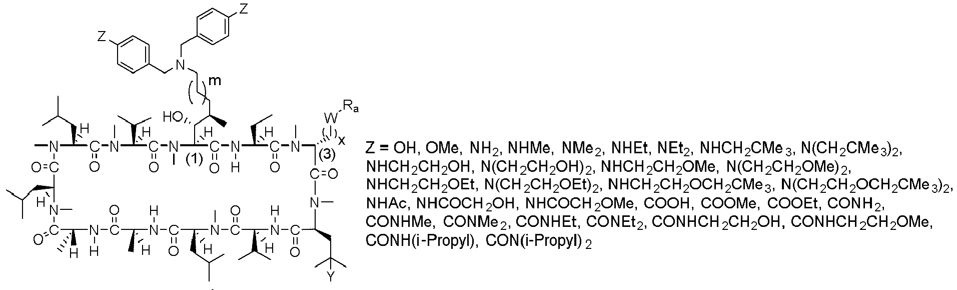 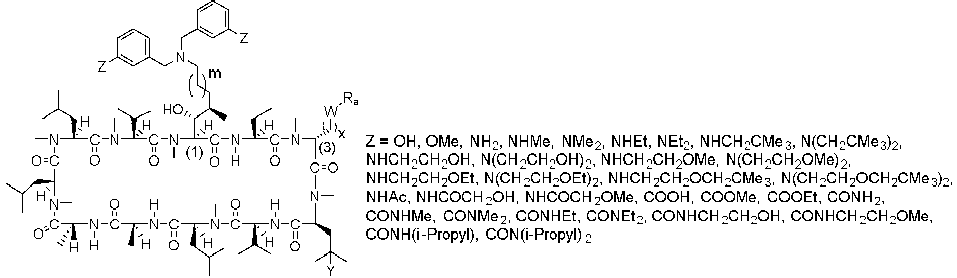 wherein x is 0 or 1; m is 2, 3, 4, 5, 6, 7, or 8; Y is H, OH or OMe; W is O or S; and each Rₐ is independently selected from the group consisting of the moieties shown in Table 1;

**Table 1**

| Rₐ | | |
|---|---|---|
| 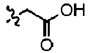 | 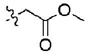 | 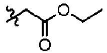 |
| 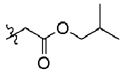 | 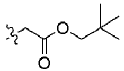 | 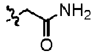 |
| 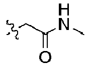 | 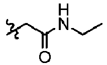 | 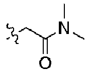 |
| 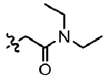 | 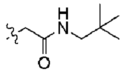 | 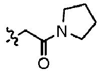 |
| 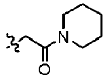 | 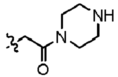 | 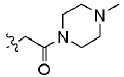 |
| 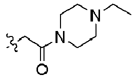 | 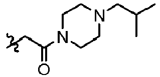 | 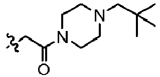 |
| 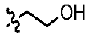 | 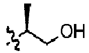 | 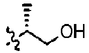 |
| 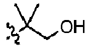 | 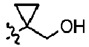 | 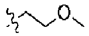 |
| 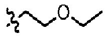 | 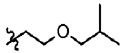 | 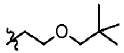 |
| 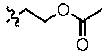 | 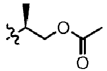 | 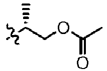 |
| 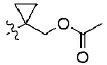 | 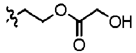 | 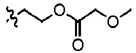 |
| 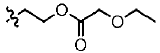 | 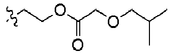 | 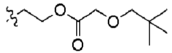 |
| 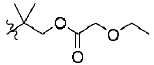 | 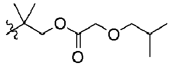 | 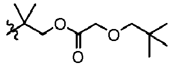 |
| 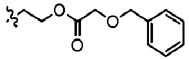 | 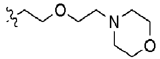 | 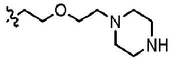 |
| 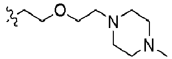 | 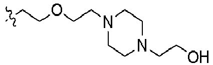 | 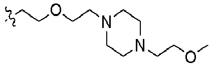 |
| 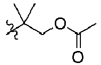 | 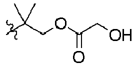 | 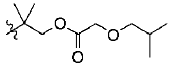 |
| 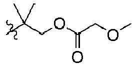 | 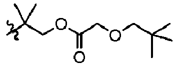 | 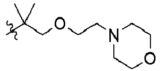 |
| 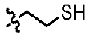 | 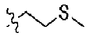 | 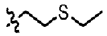 |
| 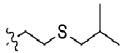 | 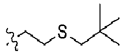 | 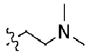 |
| 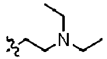 | 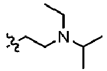 | 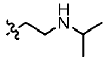 |
| 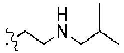 | 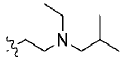 | 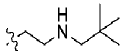 |
| 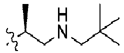 | 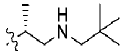 | 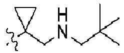 |
| 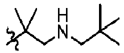 | 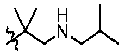 | 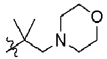 |
| 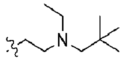 | 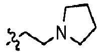 | 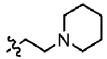 |
| 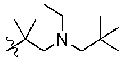 | 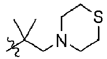 | 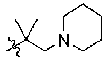 |
| 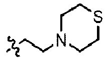 | 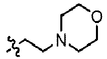 | 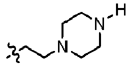 |
| 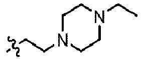 | 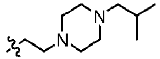 | 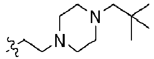 |
| 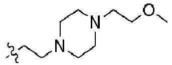 | 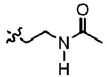 | 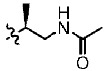 |
| 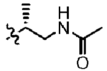 | 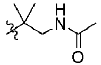 | 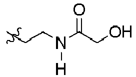 |
| 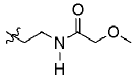 | 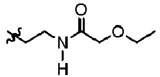 | 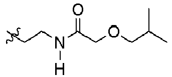 |
| 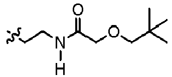 | 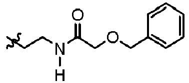 | 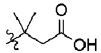 |
| 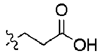 | 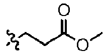 | 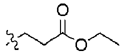 |
| 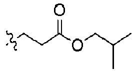 | 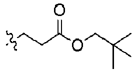 | 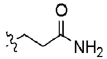 |
| 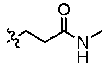 | 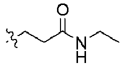 | 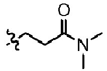 |
| 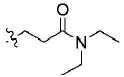 | 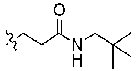 | 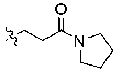 |
| 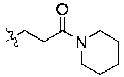 | 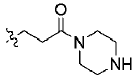 | 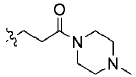 |
| 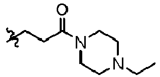 | 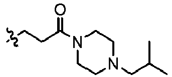 | 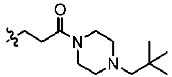 |
| 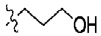 | 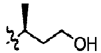 | 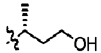 |
| 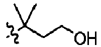 | 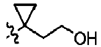 | 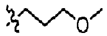 |
| 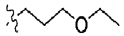 | 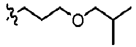 | 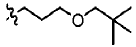 |
| 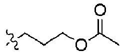 | 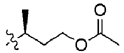 | 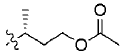 |
| 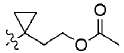 | 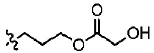 | 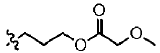 |
| 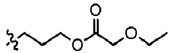 | 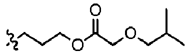 | 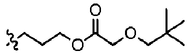 |
| 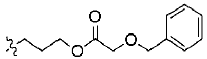 | 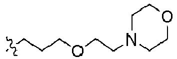 | 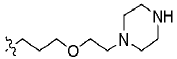 |
| 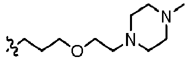 | 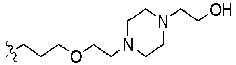 | 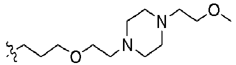 |
| 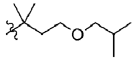 | 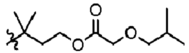 | 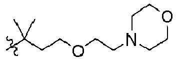 |
| 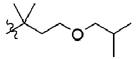 | 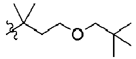 | 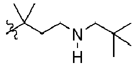 |
| 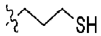 | 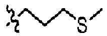 | 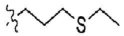 |
| 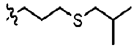 | 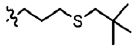 | 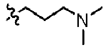 |
| 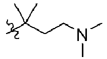 | 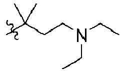 | 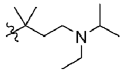 |
| 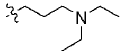 | 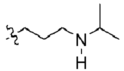 | 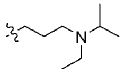 |
| 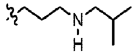 | 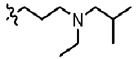 | 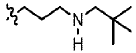 |
| 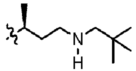 | 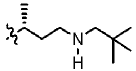 | 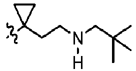 |
| 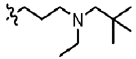 | 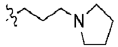 | 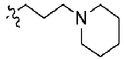 |
| 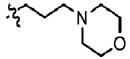 | 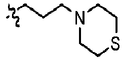 | 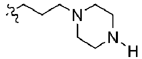 |
| 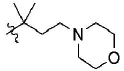 | 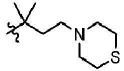 | 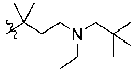 |
| 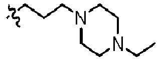 | 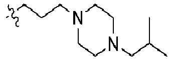 | 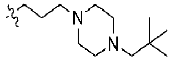 |
| 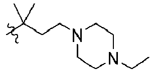 | 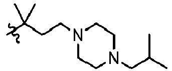 | 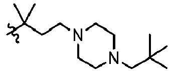 |
| 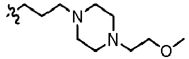 | 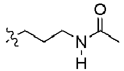 | 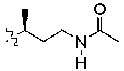 |
| 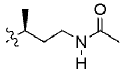 | 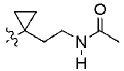 | 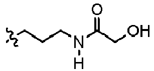 |
| 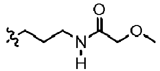 | 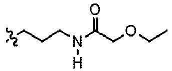 | 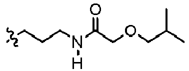 |
| 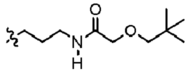 | 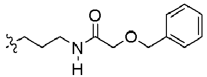 | 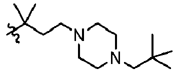 |
| 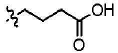 | 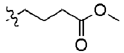 | 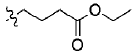 |
| 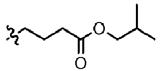 | 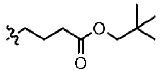 | 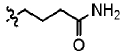 |
| 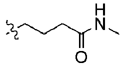 | 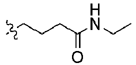 | 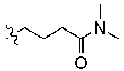 |
| 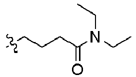 | 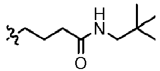 | 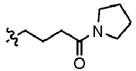 |
| 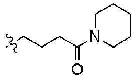 | 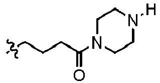 | 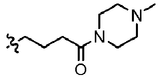 |
| 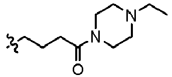 | 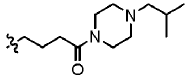 | 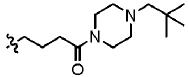 |
| 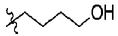 | 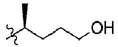 | 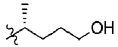 |
| 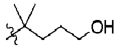 | 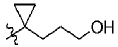 | 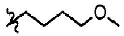 |
| 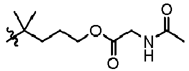 | 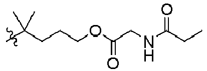 | 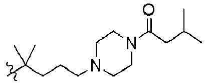 |
| 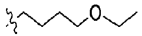 | 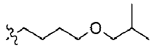 | 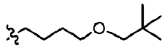 |
| 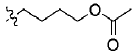 | 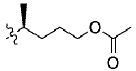 | 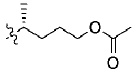 |
| 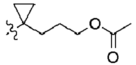 | 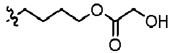 | 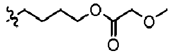 |
| 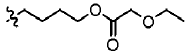 | 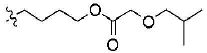 | 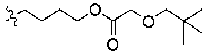 |
| 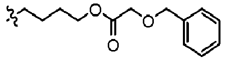 | 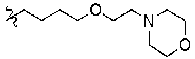 | 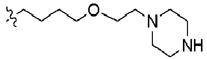 |
| 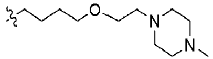 | 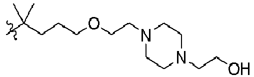 | 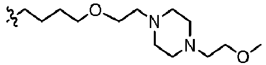 |
| 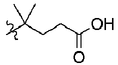 | 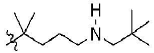 | 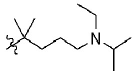 |
| 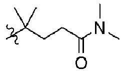 | 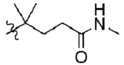 | 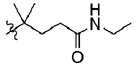 |
| 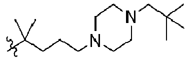 | 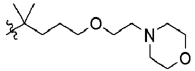 | 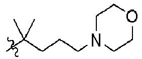 |
| 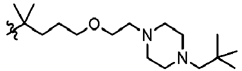 | 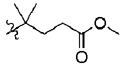 | 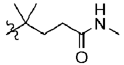 |
| 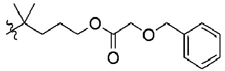 | 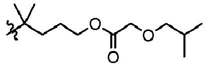 | 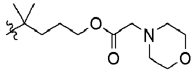 |
| 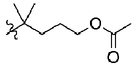 | 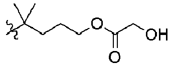 | 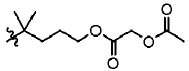 |
| 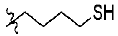 | 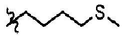 | 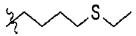 |
| 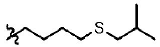 | 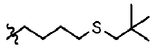 | 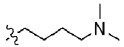 |
| 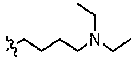 | 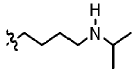 | 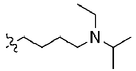 |
| 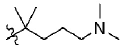 | 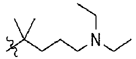 | 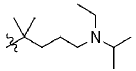 |
| 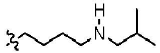 | 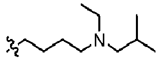 | 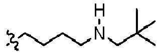 |
| 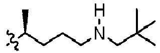 | 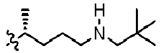 | 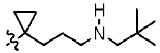 |
| 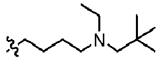 | 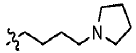 | 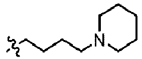 |
| 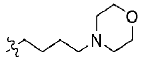 | 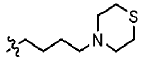 | 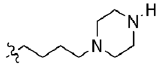 |
| 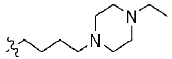 | 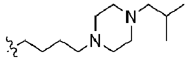 | 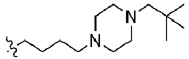 |
| 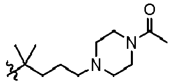 | 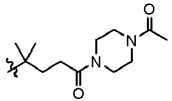 | 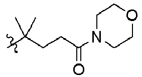 |
| 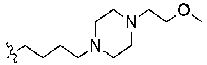 | 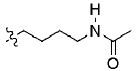 | 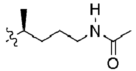 |
| 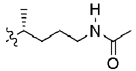 | 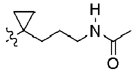 | 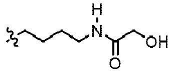 |
| 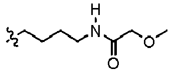 | 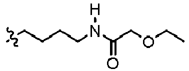 | 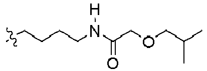 |
| 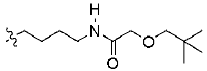 | 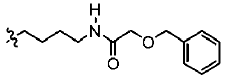 | 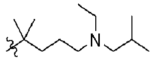 |
| 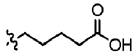 | 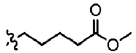 | 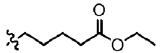 |
| 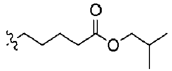 | 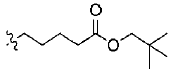 | 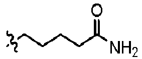 |
| 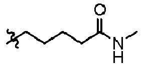 | 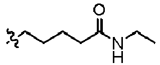 | 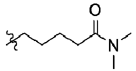 |
| 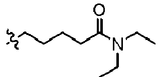 | 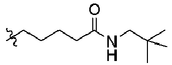 | 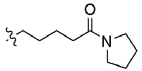 |
| 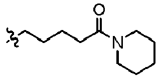 | 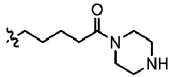 | 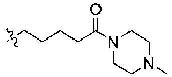 |
| 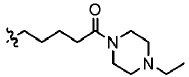 | 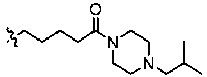 | 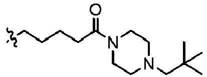 |
| 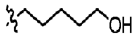 | 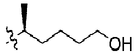 | 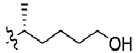 |
| 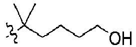 | 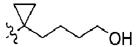 | 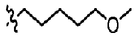 |
| 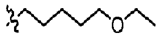 | 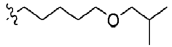 | 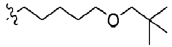 |
| 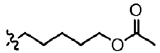 | 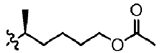 | 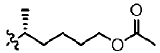 |
| 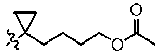 | 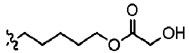 | 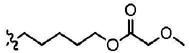 |
| 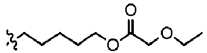 | 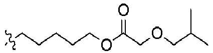 | 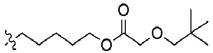 |
| 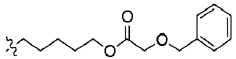 | 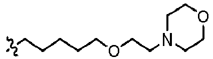 | 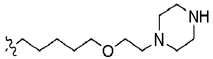 |
| 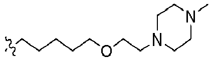 | 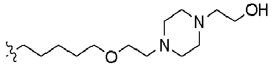 | 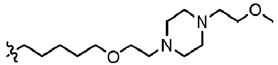 |
| 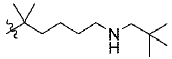 | 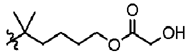 | 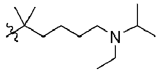 |
| 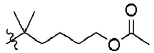 | 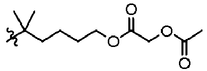 | 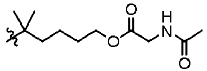 |
| 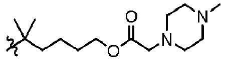 | 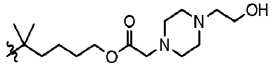 | 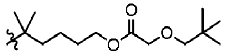 |
| 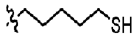 | 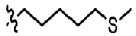 | 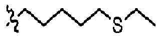 |
| 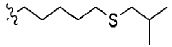 | 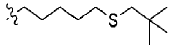 | 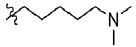 |
| 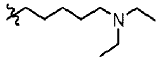 | 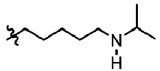 | 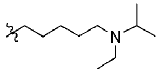 |
| 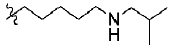 | 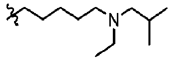 | 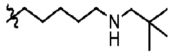 |
| 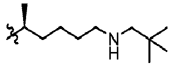 | 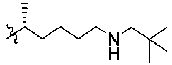 | 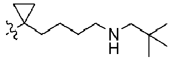 |
| 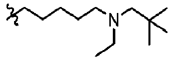 | 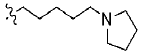 | 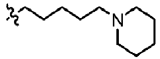 |
| 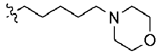 | 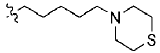 | 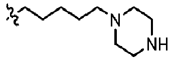 |
| 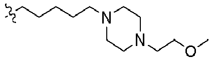 | 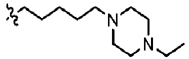 | 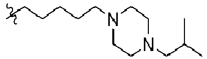 |
| 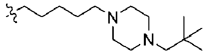 | 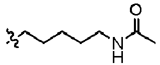 | 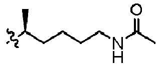 |
| 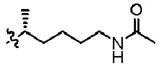 | 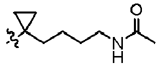 | 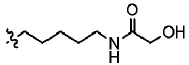 |
| 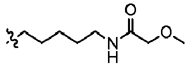 | 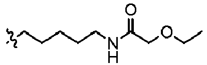 | 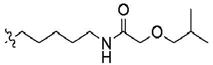 |
| 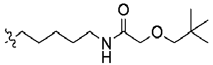 | 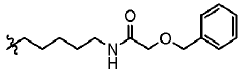 | 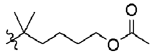 |
| 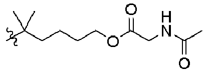 | 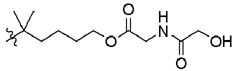 | 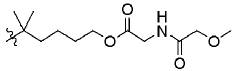 |
| 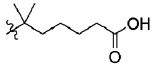 | 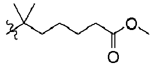 | 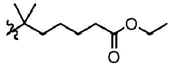 |
| 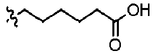 | 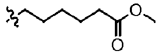 | 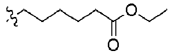 |
| 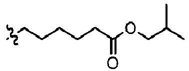 | 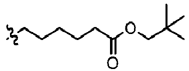 | 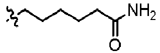 |
| 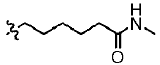 | 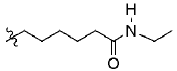 | 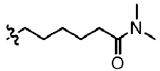 |
| 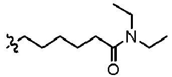 | 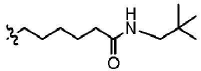 | 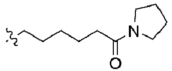 |
| 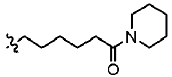 | 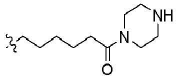 | 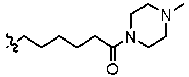 |
| 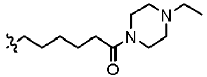 | 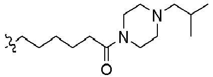 | 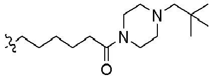 |
| 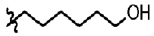 | 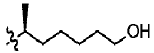 | 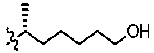 |
| 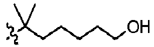 | 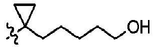 | 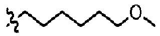 |
| 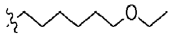 | 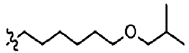 | 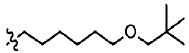 |
| 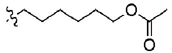 | 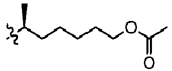 | 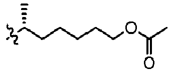 |
| 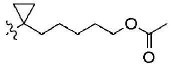 | 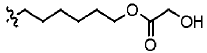 | 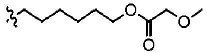 |
| 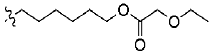 | 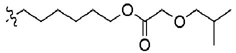 | 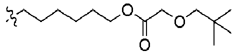 |
| 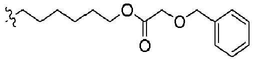 | 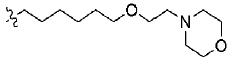 | 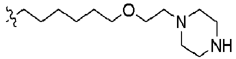 |
| 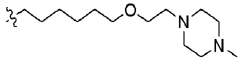 | 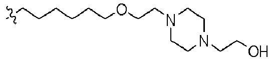 | 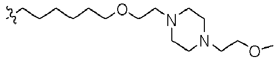 |
| 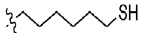 | 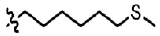 | 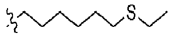 |
| 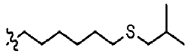 | 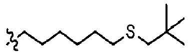 | 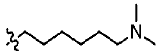 |
| 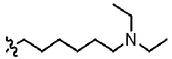 | 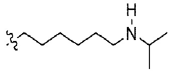 | 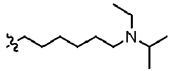 |
| 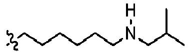 | 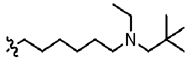 | 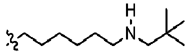 |
| 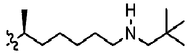 | 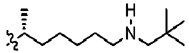 | 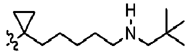 |
| 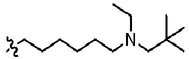 | 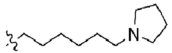 | 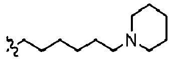 |
| 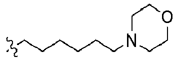 | 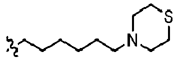 | 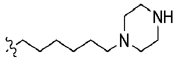 |
| 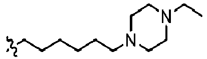 | 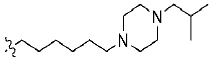 | 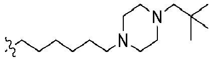 |
| 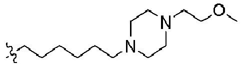 | 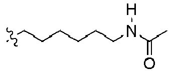 | 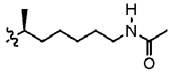 |
| 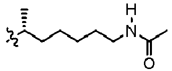 | 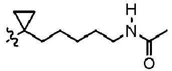 | 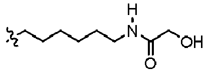 |
| 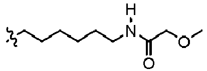 | 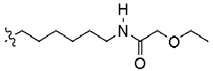 | 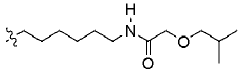 |
| 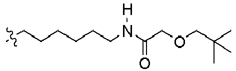 | 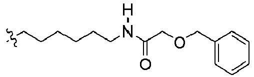 | 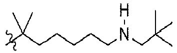 |
| 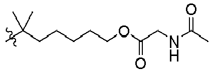 | 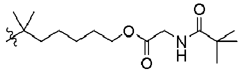 | 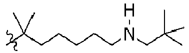 |

, or a pharmaceutically acceptable salt thereof.
50. The compound of any one clauses 1-49, wherein x is 0.
51. The compound of any one clauses 1-49, wherein x is 1.
52. A pharmaceutical composition comprising at least one compound according to any one of clauses 1-51 and a pharmaceutically-acceptable carrier or diluent.
53. A method for treating or preventing a viral infection in a mammalian species in need thereof, the method comprising administering to the mammalian species a therapeutically effective amount of at least one compound according to any one of clauses 1-51.
54. A method for treating or preventing hepatitis C virus infection in a mammalian species in need thereof, the method comprising administering to the mammalian species a therapeutically effective amount of at least one compound according to any one of clauses 1-51.
55. A method for treating or preventing hepatitis B virus infection in a mammalian species in need thereof, the method comprising administering to the mammalian species a therapeutically effective amount of at least one compound according to any one of clauses 1-51.
56. A method for treating or preventing HIV infection in a mammalian species in need thereof, the method comprising administering to the mammalian species a therapeutically effective amount of at least one compound according to any one of clauses 1-51.
57. A method for inhibiting a cyclophilin in a subject in need thereof, the method comprising administrating to said subject an effective cyclophilin-inhibiting amount of at least one compound according to any one of clauses 1-51.
58. A method for treating or preventing diseases that are mediated by cyclophilins in a mammalian species in need thereof, the method comprising administering to the mammalian species a therapeutically effective amount of at least one compound according to any one of clauses 1-51.
59. A method for treating or preventing diseases in a mammalian species in need thereof, the method comprising administering to the mammalian species a therapeutically effective amount of at least one compound according to any one of clauses 1-51, wherein the diseases are selected from inflammation, respiratory inflammation, rheumatoid arthritis, and dry eye.
60. A method for treating or preventing diseases in a mammalian species in need thereof, the method comprising administering to the mammalian species a therapeutically effective amount of at least one compound according to any one of clauses 1-51, wherein the diseases are selected from neurodegenerative diseases such as Alzheimer's disease, Parkinson's disease, Huntington's Diseases, and ALS; traumatic brain injury; stroke; ischemia-reperfusion injury in the brain, heart, kidney, and myocardial infarction.
61. A method for treating or preventing diseases in a mammalian species in need thereof, the method comprising administering to the mammalian species a therapeutically effective amount of at least one compound according to any one of clauses 1-51, wherein the diseases are selected from cardiovascular diseases, vascular stenosis, atherosclerosis, abdominal aortic aneurysms, cardiac hypertrophy, aortic rupture, pulmonary arterial hypertension, myocarditis and myocardial fibrosis, and ischaemic heart diseases.
62. A method for treating or preventing diseases or conditions in a mammalian species in need thereof, the method comprising administering to the mammalian species a therapeutically effective amount of at least one compound according to any one of clauses 1-51, wherein the diseases or conditions are selected from cancer, obesity, diabetes, muscular dystrophy, lung diseases, liver diseases, kindey diseases, and hair loss.

## Claims

**1.** A compound of formulae (II) or (III): 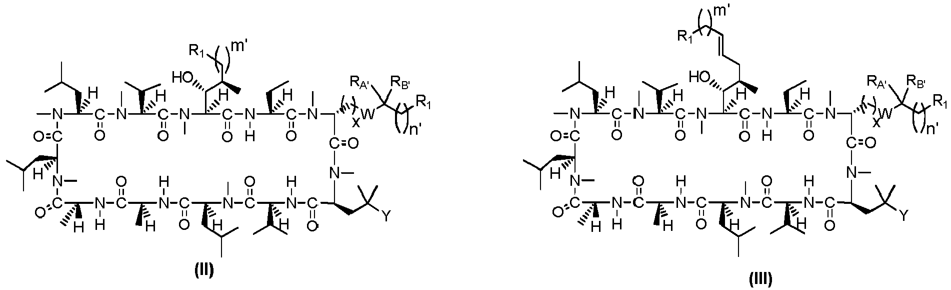 or pharmaceutically acceptable salt thereof, wherein:
x is 1;
W is O, S, or CH₂;
each occurrence of R₁ is independently H, halogen, aryl or substituted aryl, heteroaryl or substituted heteroaryl, CN, OR_{A}, SR_{A}, NR_{A}R_{B}, -NR_{A}(CH₂)_{O}OR_{B},-N((CH₂)_{O}OR_{A})((CH₂)_{O}OR_{B}) -C(=O)R_{A}, -C(=O)OR_{A}, -OC(=O)R_{A}, -OC(=O)(C₁-C₆ alkyl-R_{H}), -OC(=O)(CH₂)ₒOR_{A}, -C(=O)NR_{A}R_{B}, -NR_{A}C(=O)R_{B}, -N(C(=O)R_{A})(C(=O)R_{B}),-N(C(=O)(C₁-C₆ alkyl-R_{H}))(C(=O)(C₁-C₆ alkyl-R_{H})), -N(C(=O)(C₁-C₆ alkyl-R_{H}))₂,-NR_{A}C(=O)(CH₂)_{O}OR_{B}, -NR_{A}C(=O)(CH₂)_{O}OR_{B}, -N(C(=O)(CH₂)_{O}OR_{B})₂,-NR_{A}C(=O)(CH₂)_{O}NR_{A}R_{B}, -NR_{A}(CH₂)_{O}C(=O)OR_{B},-N((CH₂)_{O}C(=O)OR_{A})((CH₂)_{O}C(=O)OR_{B}), -NR_{A}(CH₂)_{O}C(=O)NR_{A}R_{B},-N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B},-C(=O)N((CH₂)_{O}OR_{B})₂, -N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B}, -NR_{B}-C(=NH)-NR_{A}R_{B}, O(CH₂)ₘOR_{A}, O(CH₂)ₘCOOR_{A}, O(CH₂)ₘCONR_{A}R_{B}, O(CH₂)ₘCONR_{A}(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, NR_{C}(CH₂)ₘNRc(CH₂)ₘNR_{A}R_{B}, 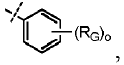 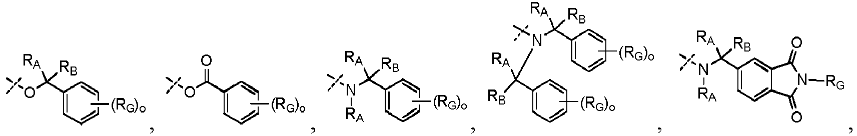 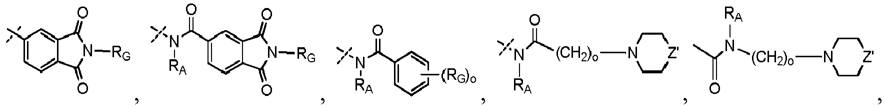 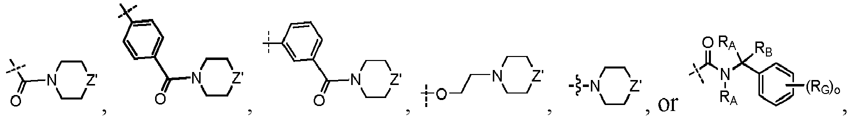 wherein said aryl or heteroaryl is optionally substituted by one or more groups which may be the same or different selected from the group consisting of halogen, hydroxy, (C₁-C₆)alkyl, (CH₂)ₚOR_{A}, (CH₂)ₚNR_{A}R_{B}, (CH₂)_{P}C(=O)NR_{A}R_{B} and (CH₂)ₚC(=O)OR_{A};
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl or 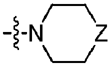 wherein Z is CH₂, O, S, NH, NCH₃, NEt, N-isopropyl, N-neoPentyl, N-CH₂CH₂OH, or N-CH₂CH₂OMe;
each occurrence of R_{G} is independently R_{A}, OR_{A}, SR_{A}, NR_{A}R_{B}, -(CH₂)ₒR_{A},-(CH₂)_{O}C(=O)OR_{A}, -(CH₂)_{O}C(=O)NR_{A}R_{B}, C(=O)OR_{A}, OC(=O)R_{A}, NR_{A}C(=O)R_{B}, NR_{A}C(=O)(CH₂)_{O}OR_{A}, C(=O)O(CH₂)_{O}OR_{A}, C(=O)OR_{B}, C(=O)NR_{A}R_{B}, C(=O)NR_{A}(CH₂)_{O}OR_{B}, C(=O)N((CH₂)_{O}OR_{A})((CH₂)_{O}OR_{B}), C(=O)N((CH₂)_{O}C(=O)OR_{A})((CH₂)_{O}C(=O)OR_{B}), C(=O)N((CH₂)_{O}NR_{A}R_{B})((CH₂)_{O}NR_{A}R_{B}), C(=O)N((CH₂)_{O}OC(=O)(CH₂)_{O}OR_{A})((CH₂)_{O}OC(=O)(CH₂)_{O}OR_{B}), C(=O)N((CH₂)_{O}NR_{A}C(=O)(CH₂)_{O}OR_{B})((CH₂)_{O}NR_{A}C(=O)(CH₂)_{O}OR_{B}), C(=O)NR_{A}(CH₂)_{O}NR_{A}R_{B}, , 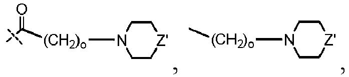 C(=O)NR_{A}(CH₂)_{O}OC(=O)R_{B}, C(=O)NR_{A}(CH₂)_{O}C(=O)OR_{B}, C(=O)NR_{A}(CH₂)_{O}C(=O)NR_{A}R_{B}, C(=O)NR_{A}(CH₂)_{O}OC(=O)(CH₂)_{O}OR_{B}, or 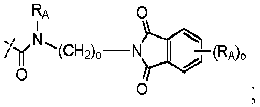
each occurrence of R_{H} is independently halogen;
each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNR_{A}C(=O)R_{B}, or N(CH₂)ₒOC(=O)(CH₂)ₒOR_{A};
each occurrence of o is independently 0, 1, 2, 3, 4, 5, or 6;
each occurrence of m is independently an integer of 1, 2, 3, 4 or 5;
p is 0, 1, 2, 3, 4 and 5;
Y is H or OR₅; wherein R₅ is H or methyl;
m' and n' are each independently 0, 1, 2, 3, 4, 5, 6, 7, 8, 9, 10, 11, or 12;
each occurrence of R_{A'} and R_{B'} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl or benzyl optionally substituted with from one to five groups which may be the same or different selected from halogen, -O(C₁-C₆)alkyl, -C(=O)O(C₁-C₆)alkyl, amino, alkylamino and dialkylamino;
or R_{A'} and R_{B'}, together with the carbon atom to which they are attached, form a saturated or unsaturated cycloalkyl ring containing from three to seven ring atoms, and may be optionally substituted by from one to four groups which may be the same or different selected from the group consisting of alkyl, phenyl and benzyl.

**2.** The compound of claim 1, having structure of Formulae (IV) or (V): 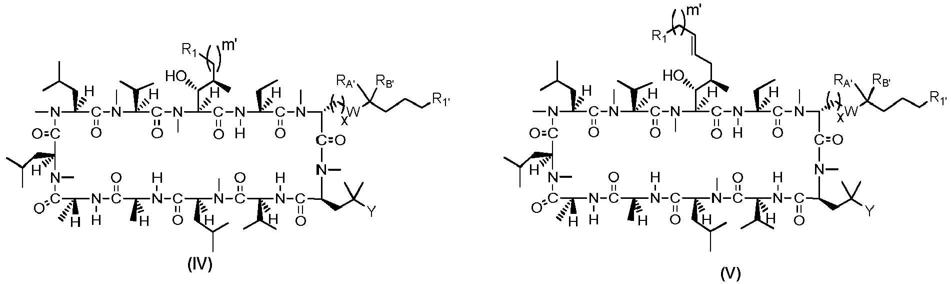 ; wherein each occurrence of R_{1'}, is independently H, halogen, aryl or substituted aryl, heteroaryl or substituted heteroaryl, OR_{A}, SR_{A}, NR_{A}R_{B}, -NR_{A}(CH₂)_{O}OR_{B},-N((CH₂)_{O}OR_{A})((CH₂)_{O}OR_{B}) -C(=O)R_{A}, -C(=O)OR_{A}, -OC(=O)R_{A}, -OC(=O)(C₁-C₆ alkyl-R_{H}), -OC(=O)(CH₂)_{O}OR_{A}, -C(=O)NR_{A}R_{B}, -NR_{A}C(=O)R_{B}, -N(C(=O)R_{A})(C(=O)R_{B}),-N(C(=O)(C₁-C₆ alkyl-R_{H}))(C(=O)(C₁-C₆ alkyl-R_{H})), -N(C(=O)(C₁-C₆ alkyl-R_{H}))₂,-NR_{A}C(=O)(CH₂)_{O}OR_{B}, -NR_{A}C(=O)(CH₂)_{O}OR_{B}, -N(C(=O)(CH₂)_{O}OR_{B})₂,-NR_{A}C(=O)(CH₂)_{O}NR_{A}R_{B}, -NR_{A}(CH₂)_{O}C(=O)OR_{B},-N((CH₂)_{O}C(=O)OR_{A})((CH₂)_{O}C(=O)OR_{B}), -NR_{A}(CH₂)_{O}C(=O)NR_{A}R_{B},-N((CH₂)_{O}C(=O)NR_{A}R_{B})((CH₂)_{O}C(=O)NR_{A}R_{B}), -NR_{A}(CH₂)_{O}C(=O)NR_{A}(CH₂)_{O}OR_{B},-C(=O)N((CH₂)_{O}OR_{B})₂,-N((CH₂)_{O}C(=O)NR_{A}(CH₂)_{O}OR_{B})((CH₂)_{O}C(=O)NR_{A}(CH₂)_{O}OR_{B}),-C(=O)NR_{A}(CH₂)_{O}OR_{B}, -C(=O)NR_{A}(CH₂)_{O}OR_{B}, -C(=O)NR_{A}(CH₂)_{O}NR_{A}R_{B}, -N=CR_{A}-NR_{A}R_{B}, -NR_{B}-C(=NH)-NR_{A}R_{B}, O(CH₂)ₘOR_{A}, O(CH₂)ₘCOOP_{A}, O(CH₂)ₘCONR_{A}R_{B}, O(CH₂)ₘCONR_{A}(CH₂)ₘOR_{A}, O(CH₂)ₘO(CH₂)ₘOP_{A}, O(CH₂)ₘNR_{A}R_{B}, O(CH₂)ₘO(CH₂)ₘNR_{A}R_{B}, NR_{C}(CH₂)ₘNR_{A}R_{B}, NR_{C}(CH₂)ₘNR_{C}(CH₂)ₘNR_{A}R_{B}, 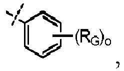 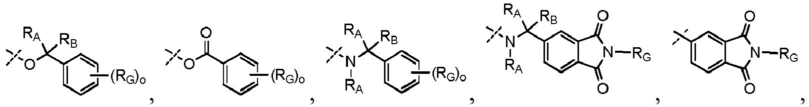 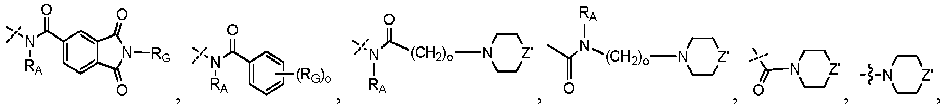 or 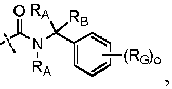 wherein said aryl or heteroaryl is optionally substituted by one or more groups which may be the same or different selected from the group consisting of halogen, hydroxy, (C₁-C₆)alkyl, (CH₂)ₚOR_{A}, (CH₂)ₚNR_{A}R_{B}, (CH₂)_{P}C(=O)NR_{A}R_{B} and (CH₂)ₚC(=O)OR_{A}.

**3.** The compound of claim 2, wherein R₁is OR_{A}, OCOCH₂OR_{A}, SR_{A}, NHR_{A}, N(R_{A})₂, or NHCOCH₂OR_{A}, optionally R_{1'} is OAc, OCOCH₂Cl, OCOCH₂CH₃, OCOCHMe₂, OCOCMe₃, OCOCH=CH₂, NHCH₂CH₂OH, NHCH₂CH₂OMe, N(CH₂CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂.

**4.** The compound of claim 1, wherein R_{A'} and R_{B'} are each independently H, Me, Et, n-Propyl, isoProyl, isoButyl, neoPentyl, cyclopentyl, cyclohexyl, CH₂CH₂OH, CH₂CH₂OMe, 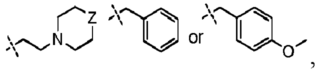 wherein Z is CH₂, O, S, NH, NCH₃, NEt, N-isopropyl, N-isopropyl, N-neoPentyl, N-CH₂CH₂OH, or N-CH₂CH₂OMe.

**5.** The compound of claim 1, wherein R₁ is -NR_{A}C(=O)R_{B} and R_{1'} is OR_{A}, optionally R₁ is -NHC(=O)R_{B} and R_{1'} is OH.

**6.** The compound of claim 1, having structure of Formula (VI): 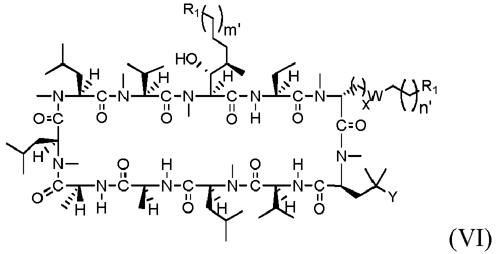 wherein
W is CH₂, O or S;
Y is H or OR₅; wherein R₅ is H or methyl;
m' and n' are each independently 0, 1, 2, 3, 4, 5, 6, 7, 8, 9, or 10; and
each occurrence of R₁ is independently H, halogen, aryl or substituted aryl, heteroaryl or substituted heteroaryl, OR_{A}, SR_{A}, NR_{A}R_{B}, -C(=O)R_{A}, -C(=O)OR_{A}, -C(=O)NR_{A}R_{B},-NR_{A}C(=O)R_{B}, -NR_{A}C(=O)(CH₂)_{O}R_{B}, -NR_{A}C(=O)(CH₂)_{O}OR_{B}, -NR_{A}C(=O)(CH₂)_{O}NR_{A}R_{B}, or -C(=O)NR_{A}(CH₂)_{O}R_{B}.

**7.** The compound of any one of the preceding claims, wherein R₁ is selected from the group consisting of (i) H, OR_{A}, SR_{A}, NR_{A}R_{B}, -C(=O)R_{A}, -C(=O)OR_{A}, -C(=O)NR_{A}R_{B},-NR_{A}C(=O)R_{B}, -NR_{A}C(=O)(CH₂)_{O}R_{B}, -NR_{A}C(=O)(CH₂)_{O}OR_{B}, -NR_{A}C(=O)(CH₂)_{O}NR_{A}R_{B},-C(=O)NR_{A}(CH₂)_{O}OR_{B},
-C(=O)NR_{A}(CH₂)_{O}OR_{B}, and -C(=O)NR_{A}(CH₂)_{O}NR_{A}R_{B};
(ii) O(CH₂)ₘOR_{A}, O(CH₂)ₘO(CH₂)ₘOR_{A}, O(CH₂)ₘNR_{A}R_{B}, O(CH₂)ₘO(CH₂)ₘNR_{A}R_{B}, NR_{C}(CH₂)ₘNR_{A}R_{B}, and NR_{C}(CH₂)ₘNRc(CH₂)ₘNR_{A}R_{B};
(iii) OR_{A}, SR_{A}, NR_{A}R_{B}, -C(=O)R_{A}, -C(=O)OR_{A}, and -C(=O)NR_{A}R_{B};
(iv) OH, OMe, OEt, O-isopropyl, O-isoButyl, O-neoPentyl, O-cyclopentyl, O-cyclohexyl, SH, SMe, S-isopropyl, S-isoButyl, S-neoPentyl, S-cyclopentyl, S-cyclohexyl, 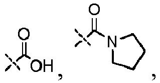 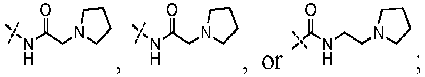 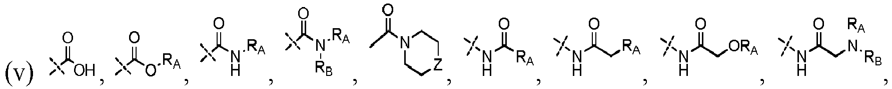 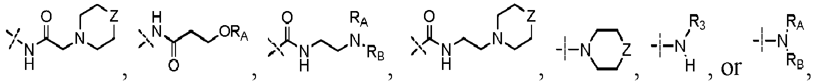 wherein Z is CH₂, O, S, NH, NCH₃, NEt, N-isopropyl, N-isopropyl, N-neoPentyl, N-CH₂CH₂OH, or N-CH₂CH₂OMe;
(vi) H, OH, OMe, OEt, O-isoProyl, O-isoButyl, O-neoPentyl, O-cyclopentyl, O-cyclohexyl, OCH₂CH₂OH, OCH₂CH₂OCH₃, OCH₂COOH, OCH₂COOCH₃, OCH₂CONH₂, OCH₂CONHMe, OCH₂CONMe₂, OCH₂CONHCH₂CH₂OH, OCH₂CONHCH₂CH₂OMe, OAc, OOCCH₂Cl, OOCCH₂OR_{A}, OOCCH₂CH₃, OOCCHMe₂, OOCCMe₃, and OC(=O)CCH=CH₂;
(vii) 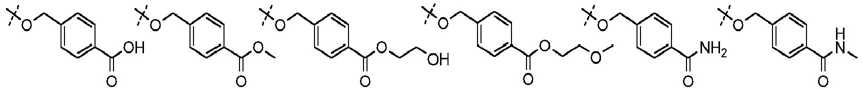 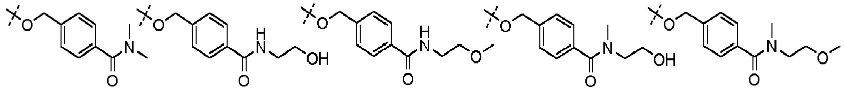 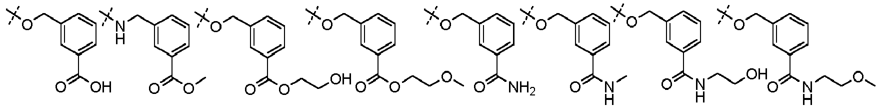 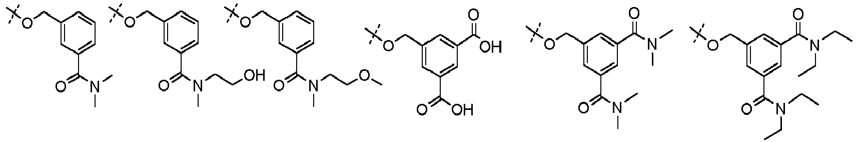 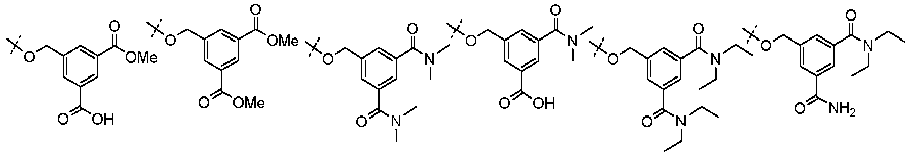 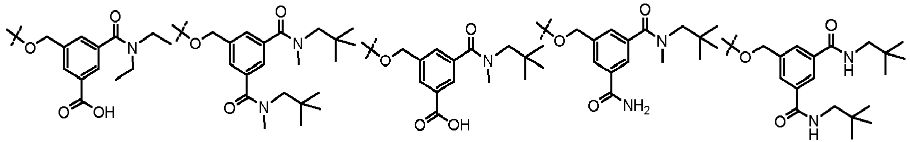 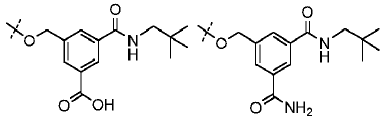 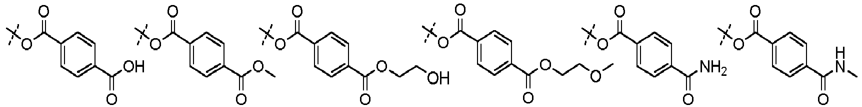 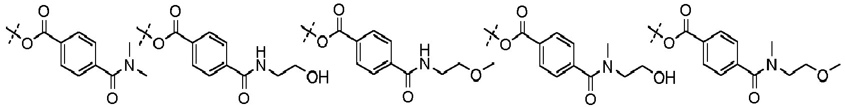 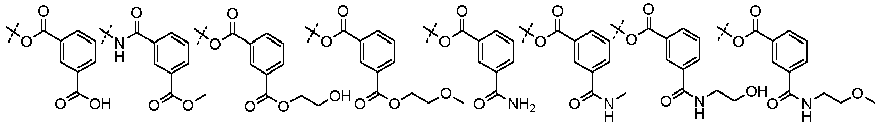 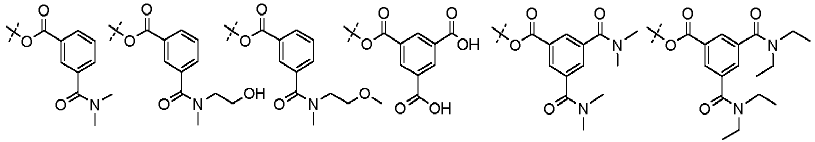 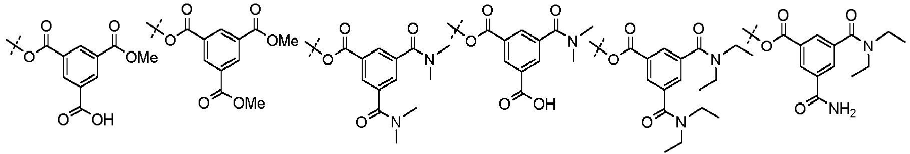 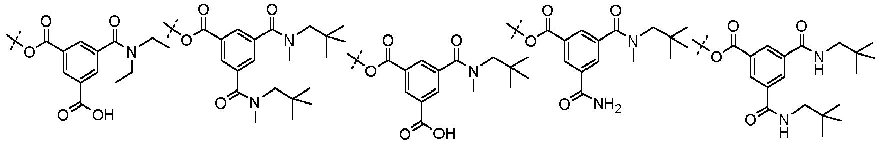 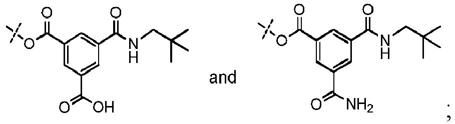
(viii) SH, SMe, SEt, S-isoProyl, S-isoButyl, S-neoPentyl, O-cyclopentyl, or S-cyclohexyl;
(ix) NH₂, NHCH₃, NHCH₂CH₃, NHCH₂CHOH, NHCH₂CH₂OMe, NMe₂, NEt₂, NHCH₂CHMe₂, NHCH₂CMe₃, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂OR_{A}, NHCOCH₂CH₃, NHCOCHMe₂, NHCOCMe₃, NHCOCH=CH₂, N(COCH₂Cl)₂, N(COCH₂OR_{A})₂, N(COCH₂CH₃)₂, N(COCHMe₂)₂, N(COCMe₃)₂, and N(COCH=CH₂)₂;
(x) is 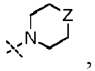 wherein Z is CH₂, O, S, NH, NMe, NEt, N-isopropyl, N-neoPentyl, N-CH₂CH₂OH, NCH₂CH₂OCH₃, NCH₂CH₂OCH₃, NCH₂COOH, NCH₂COOMe, N-CH₂CONH₂, NCH₂CONHMe, or NCH₂CONMe₂;
(xi) 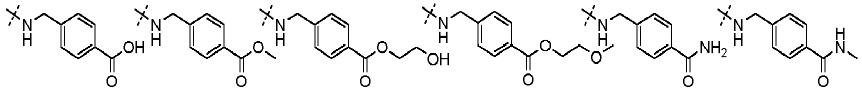 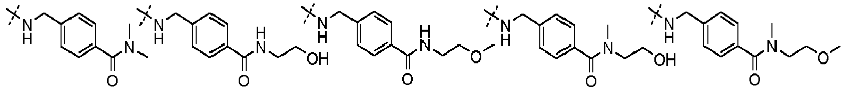 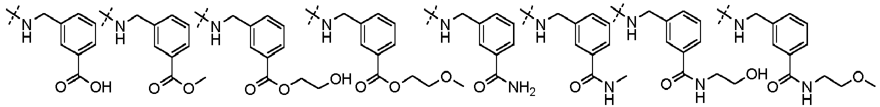 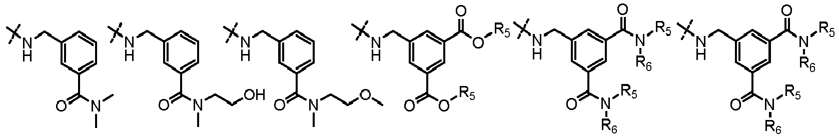 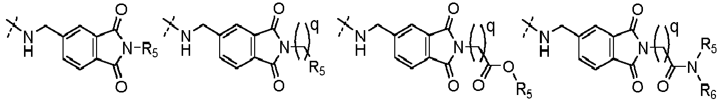 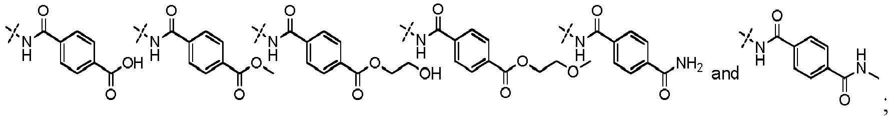
(xii) 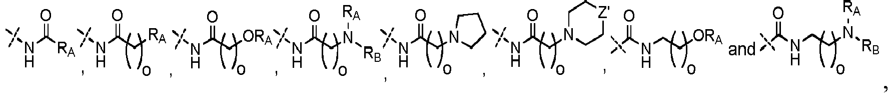 wherein each occurrence Z' is independently CH₂, O, S, NR_{A}, N(CH₂)ₒOR_{A}, N(CH₂)ₒNR_{A}R_{B}, N(CH₂)ₒCOOR_{A}, N(CH₂)ₒOC(=O)R_{A}, N(CH₂)ₒCONR_{A}R_{B}, N(CH₂)ₒNR_{A}C(=O)R_{B}, or N(CH₂)ₒOC(=O)(CH₂)ₒOR_{A};
(xii) -COOH, -COOMe, -COOEt, -CONH₂, -CONHMe, -CONMe₂, -CONHEt,-CONEt₂, -CONHCH₂CH₂OH, -CONHCH₂CH₂OMe, -CON(CH₂CH₂OH)₂,-CON(CH₂CH₂OMe)₂, or -CONMe₂;
(xiii) 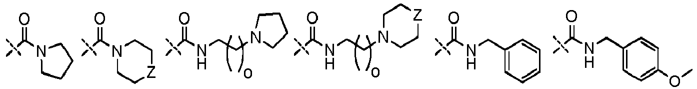 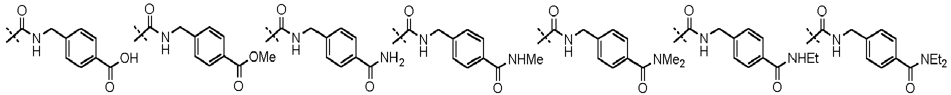 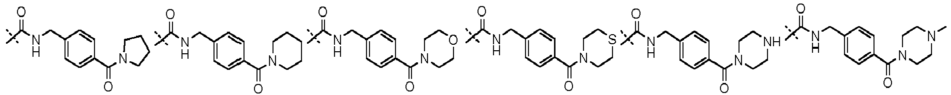 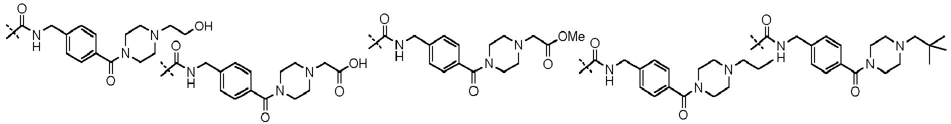 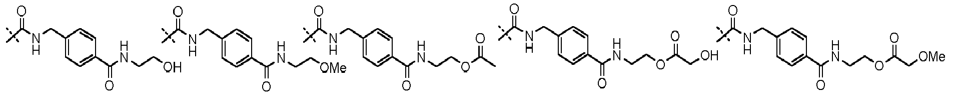 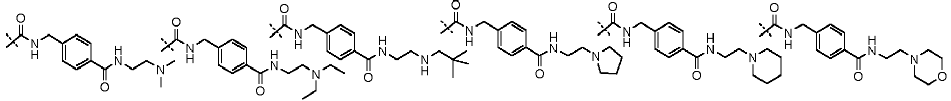 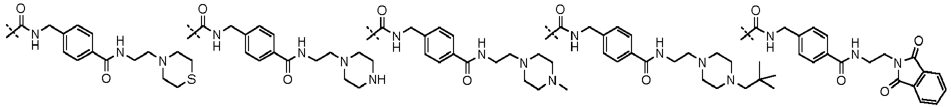 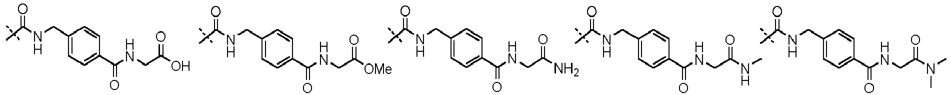 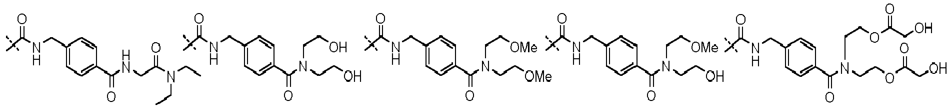 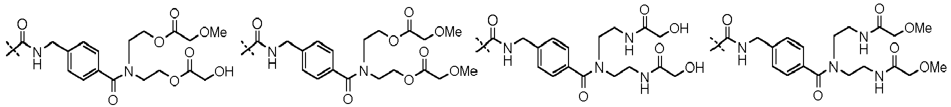 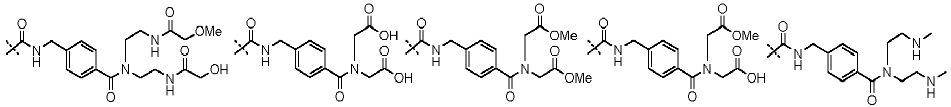 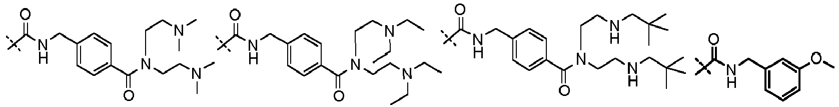 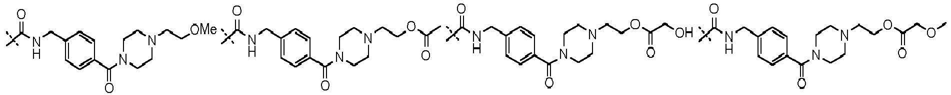 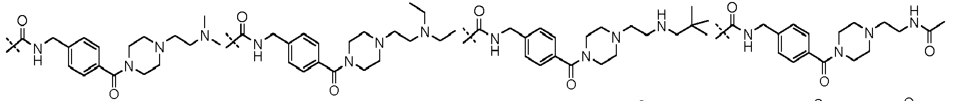 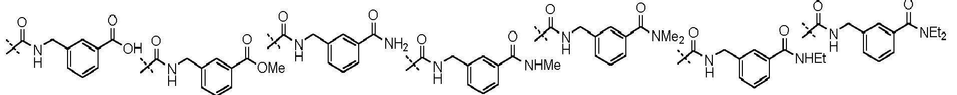 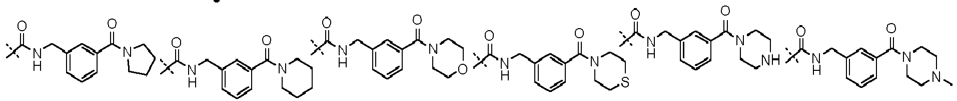 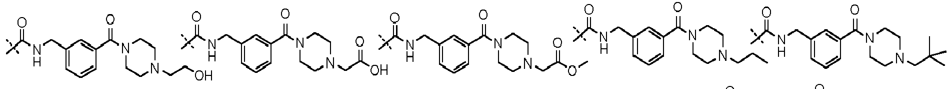 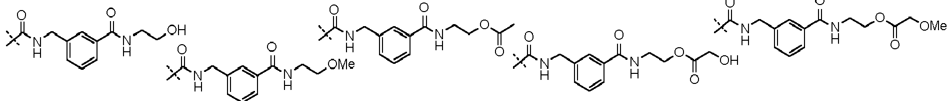 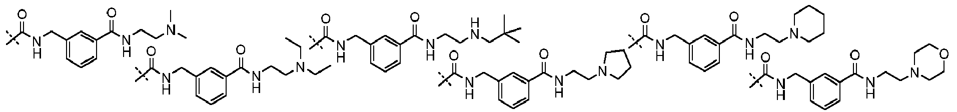 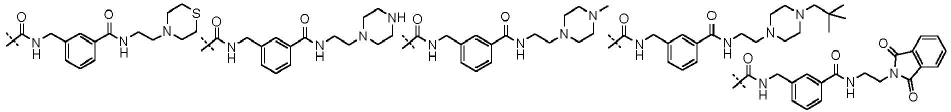 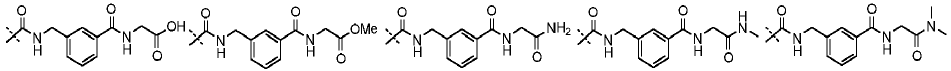 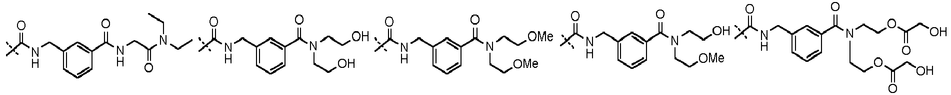 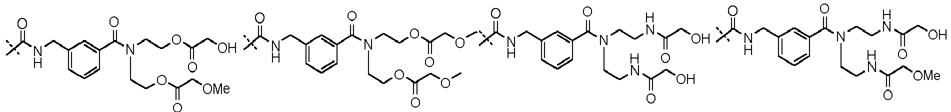 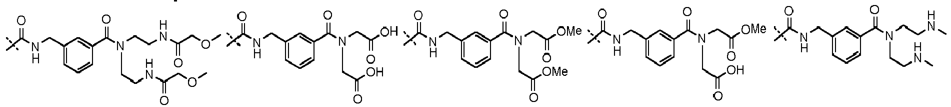 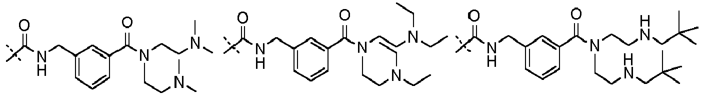 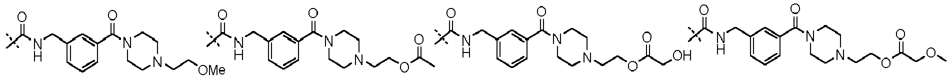 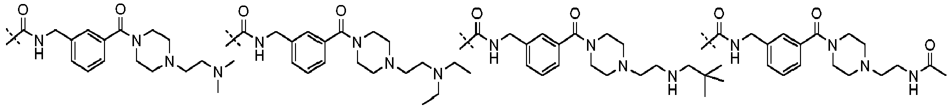 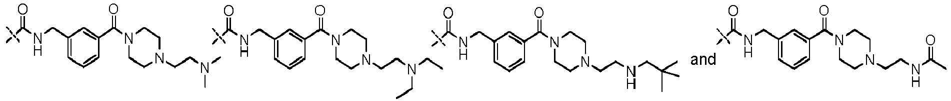 ; wherein Z is CH₂, O, S, NH, NCH₃, NEt, N-isopropyl, N-isopropyl, N-neoPentyl, N-CH₂CH₂OH, or N-CH₂CH₂OMe;
(xiv) 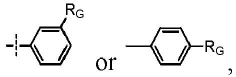 optionally wherein R_{G} is OH, OMe, OAc, NH₂, NHMe, NHAc, NMe₂, NEt₂, NHCH₂CMe₃; 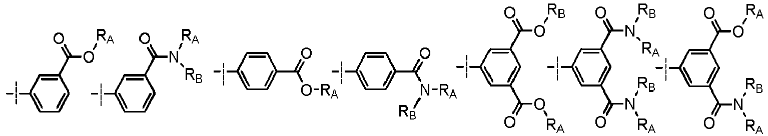
(xv) 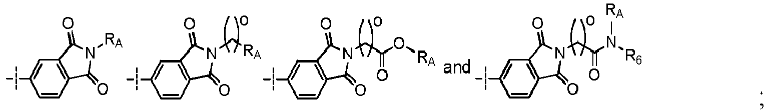
(xvi) OR_{A}, OCOCH₂OR_{A}, SR_{A}, NHR_{A}, N(R_{A})₂, or NHCOCH₂OR_{A}; or
(xvii) OAc, OCOCH₂Cl, OCOCH₂CH₃, OCOCHMe₂, OCOCMe₃, OCOCH=CH₂, NHCH₂CH₂OH, NHCH₂CH₂OMe, N(CH₂CH₂OH)₂, N(CH₂CH₂OMe)₂, NHCH₂CHMe₂, NHCH₂CMe₂, NHAc, NHCH₂COOH, NHCH₂COOCH₃, NMeCH₂COOH, NMeCH₂COOCH₃, NHCH₂CONH₂, NHCH₂CONHMe, NHCH₂CONMe₂, NHCH₂CONHCH₂CH₂OH, NHCH₂CONHCH₂CH₂OMe, NMeCH₂CONH₂, NMeCH₂CONHMe, NMeCH₂CONMe₂, N(CH₂COOH)₂, N(CH₂CONH₂)₂, N(CH₂CONHMe)₂, N(CH₂CONMe₂)₂, N(CH₂CONHCH₂CH₂OH)₂, N(CH₂CONHCH₂CH₂OMe)₂, NHCOCH₂Cl, NHCOCH₂CH₃, NHCOCHMe₂, NHCOCMe₃, NHCOCH=CH₂, N(COCH₂Cl)₂, N(COCH₂CH₃)₂, N(COCHMe₂)₂, N(COCMe₃)₂, or N(COCH=CH₂)₂

**8.** The compound of claim 1, wherein R₁ is aryl or heteroaryl optionally substituted by one or more groups which may be the same or different selected from halogen, hydroxy, (C₁-C₆)alkyl, (CH₂)ₚOR_{A}, (CH₂)ₚNR_{A}R_{B}, (CH₂)_{P}C(=O)NR_{A}R_{B} and (CH₂)ₚC(=O)OR_{A}; wherein
p is 0, 1, 2, 3, 4 and 5; and
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{D} is independently halogen, hydroxy, O(C₁-C₄)alkyl, C(=O)(C₁-C₄)alkyl, C(=O)O(C₁-C₄)alkyl or 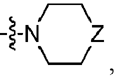 wherein Z is CH₂, O, S, NH, NCH₃, NEt, N-isopropyl, N-neoPentyl, N-CH₂CH₂OH, or N-CH₂CH₂OMe.

**9.** The compound of any one of the preceding claims, wherein R_{A} and R_{B} are each independently H, Me, Et, n-Propyl, isoProyl, isoButyl, neoPentyl, cyclopentyl, cyclohexyl, CH₂CH₂OH, CH₂CH₂OMe, 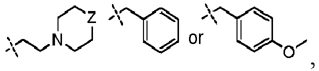 wherein Z is CH₂, O, S, NH, NCH₃, NEt, N-isopropyl, N-isopropyl, N-neoPentyl, N-CH₂CH₂OH, or N-CH₂CH₂OMe.

**10.** The compound of claim 9, wherein R_{A} is H, Me, Et, n-Proyl, isoProyl, n-Butyl, or isobutyl; or
wherein R_{A} and R_{B} are each independently H, Me, Et, isopropyl, isobutyl, cyclopentyl, or cyclohexyl.

**11.** The compound of any one of the preceding claims, wherein each occurrence R_{A} and R_{B}:
(i) is independently H, (C₁-C₆)alkyl, phenyl, CH₂-phenyl, (C₁-C₆)alkyl-OH, (CH₂)ₚO(CH₂)ₘOH, or (CH₂)ₚO(CH₂)ₘO(CH₂)ₘOH, (C₁-C₆)alkyl-O-(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl;
(ii) is independently H or (C₁-C₆)alkyl, optionally wherein R_{A} and R_{B} are each independently H, Me, Et, isopropyl, isobutyl, cyclopentyl, or cyclohexyl; or
(iii) together with the nitrogen atom to which they are attached, form a heterocycle selected from 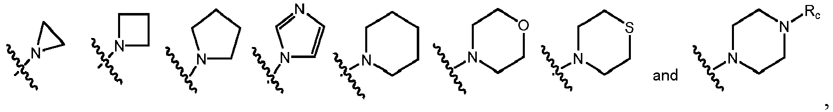 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.

**12.** The compound of claim 1, having a structure selected from the group consisting of the following structures: 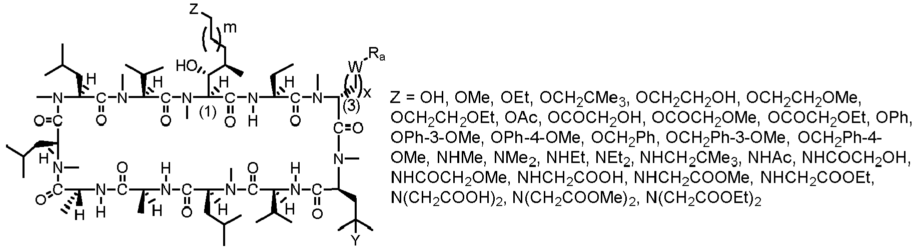 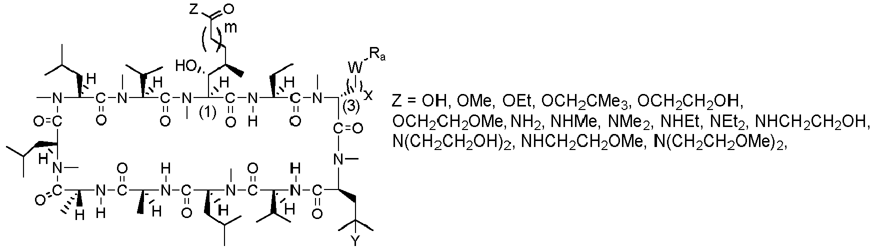 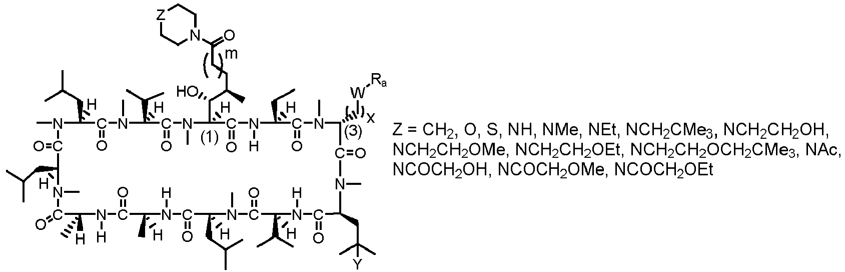 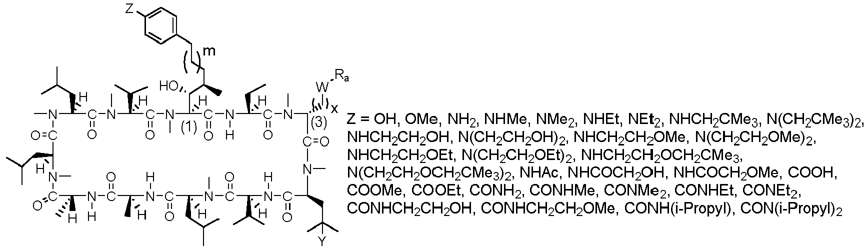 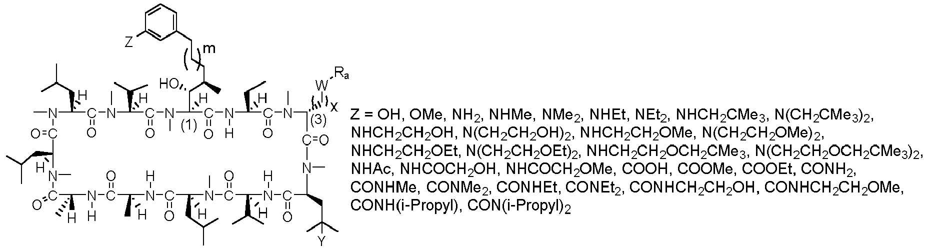 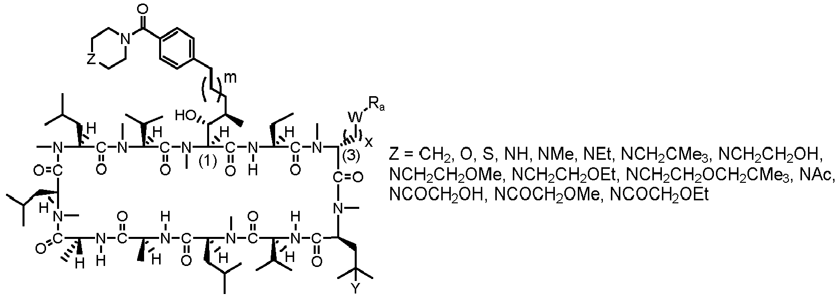 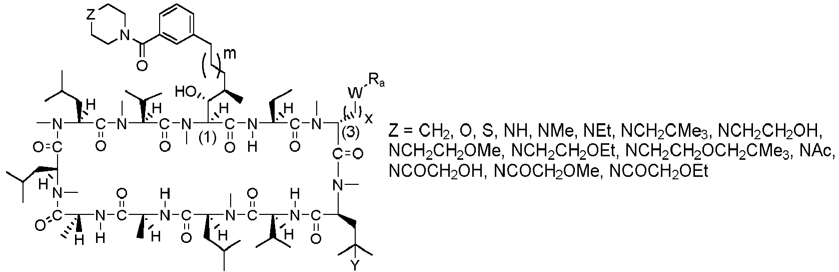 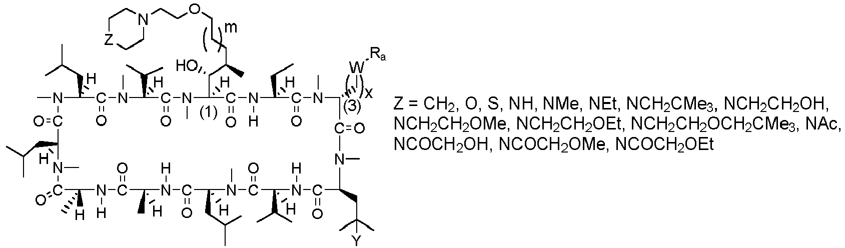 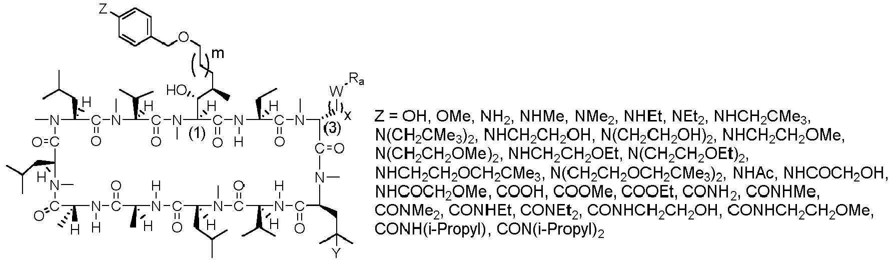 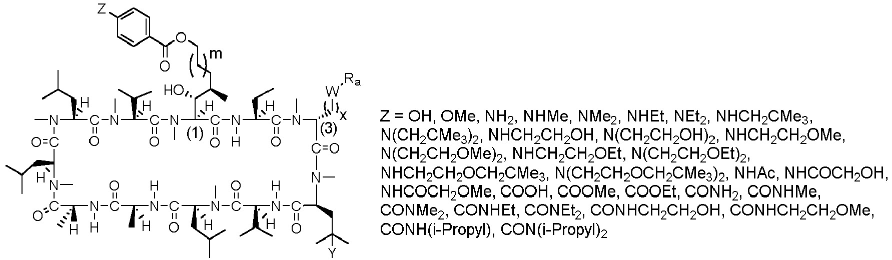 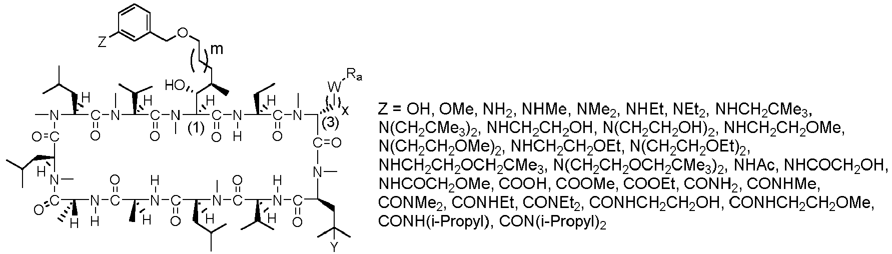 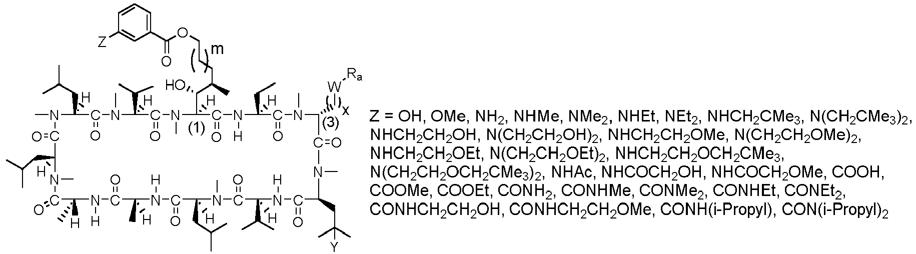 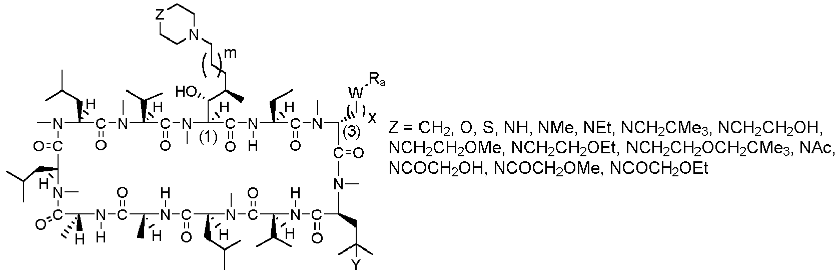 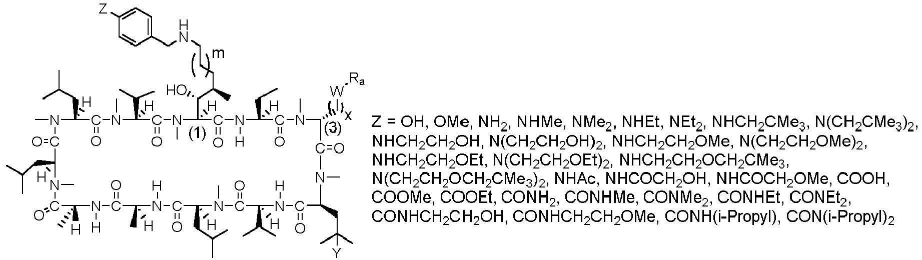 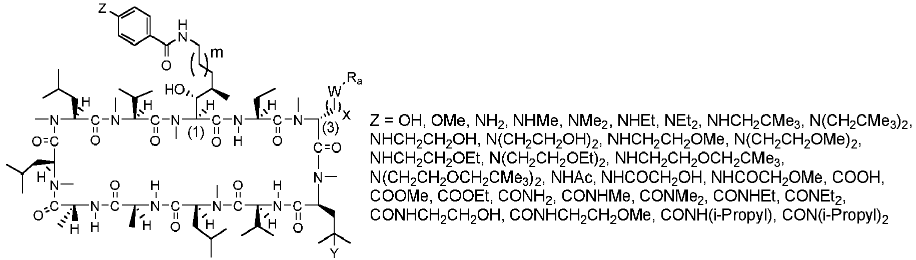 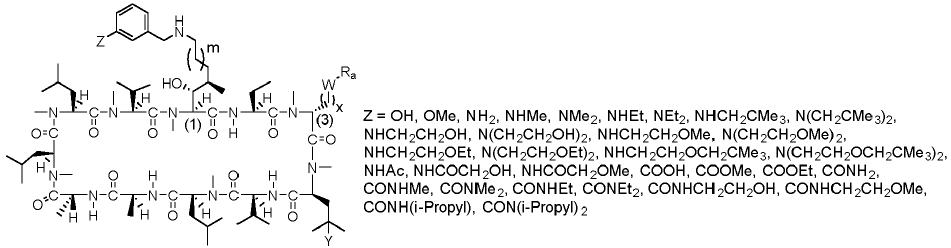 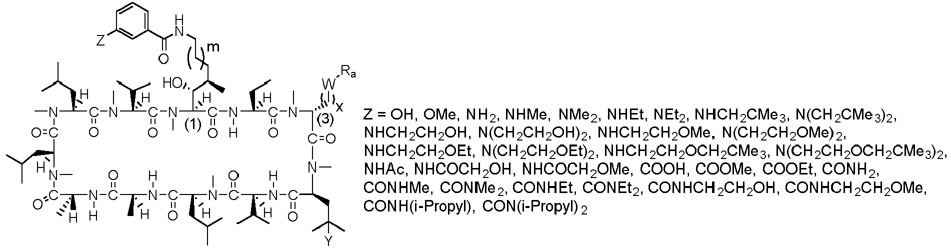 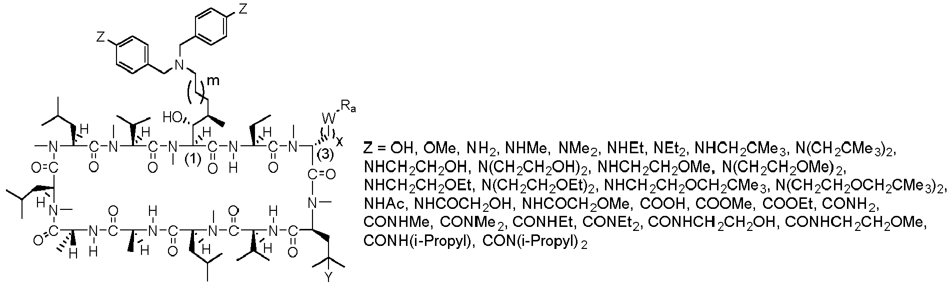 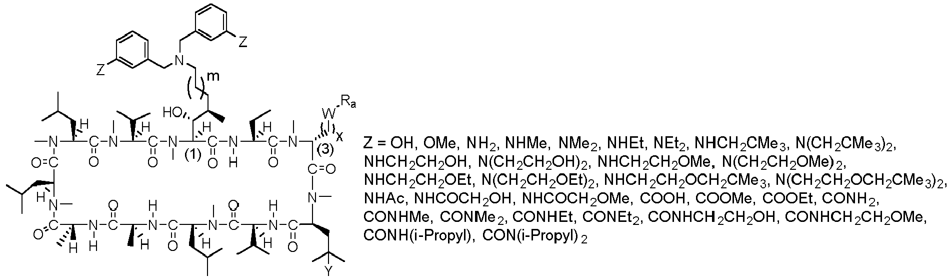 wherein x is 1; m is 2, 3, 4, 5, 6, 7, or 8; Y is H, OH or OMe; W is O or S; and each Rₐ is independently selected from the group consisting of the moieties shown in Table 1;
**Table 1**
| Rₐ | | |
|---|---|---|
| 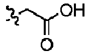 | 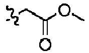 | 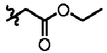 |
| 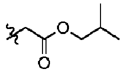 | 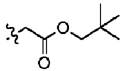 | 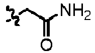 |
| 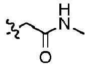 | 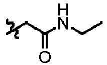 | 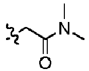 |
| 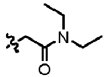 | 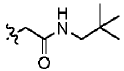 | 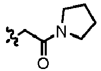 |
| 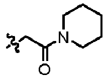 | 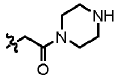 | 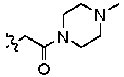 |
| 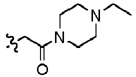 | 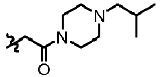 | 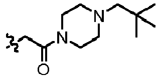 |
| 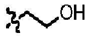 | 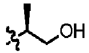 | 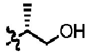 |
| 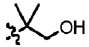 | 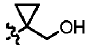 | 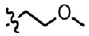 |
| 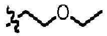 | 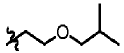 | 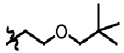 |
| 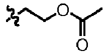 | 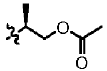 | 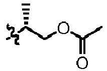 |
| 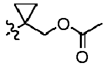 | 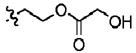 | 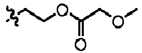 |
| 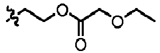 | 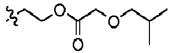 | 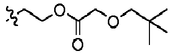 |
| 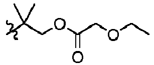 | 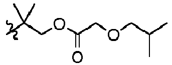 | 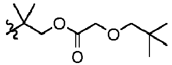 |
| 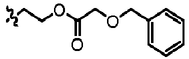 | 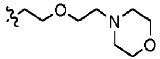 | 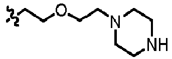 |
| 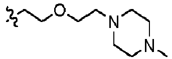 | 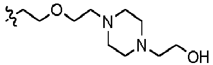 | 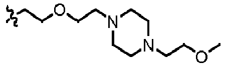 |
| 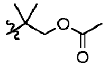 | 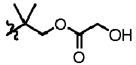 | 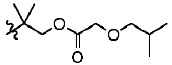 |
| 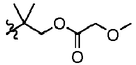 | 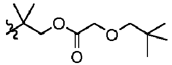 | 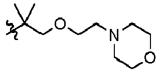 |
| 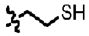 | 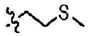 | 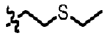 |
| 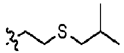 | 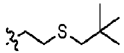 | 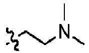 |
| 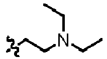 | 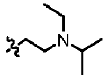 | 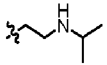 |
| 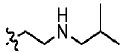 | 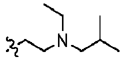 | 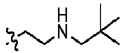 |
| 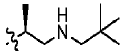 | 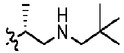 | 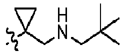 |
| 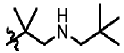 | 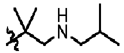 | 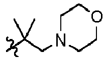 |
| 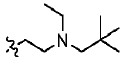 | 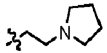 | 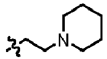 |
| 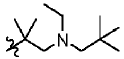 | 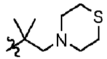 | 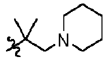 |
| 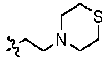 | 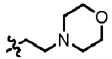 | 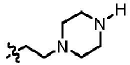 |
| 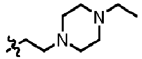 | 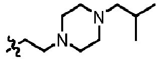 | 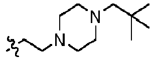 |
| 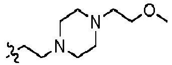 | 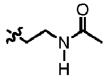 | 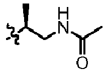 |
| 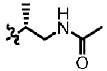 | 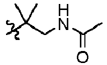 | 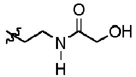 |
| 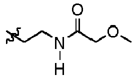 | 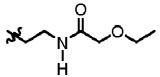 | 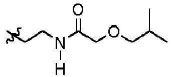 |
| 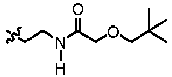 | 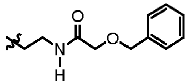 | 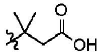 |
| 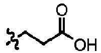 | 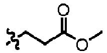 | 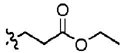 |
| 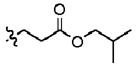 | 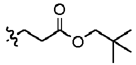 | 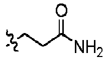 |
| 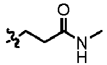 | 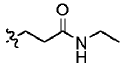 | 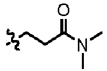 |
| 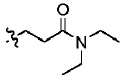 | 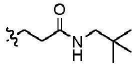 | 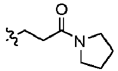 |
| 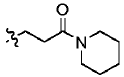 | 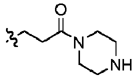 | 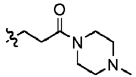 |
| 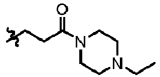 | 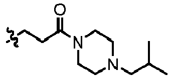 | 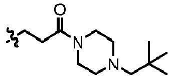 |
| 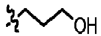 | 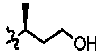 | 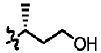 |
| 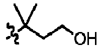 | 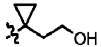 | 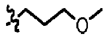 |
| 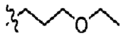 | 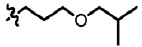 | 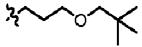 |
| 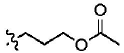 | 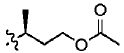 | 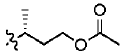 |
| 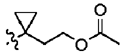 | 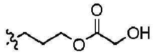 | 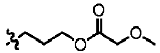 |
| 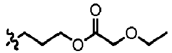 | 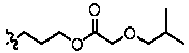 | 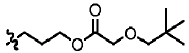 |
| 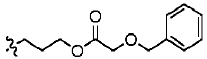 | 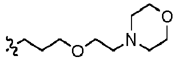 | 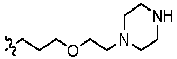 |
| 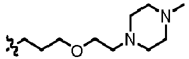 | 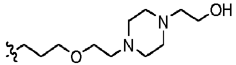 | 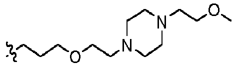 |
| 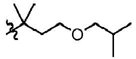 | 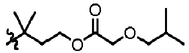 | 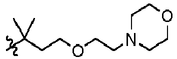 |
| 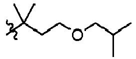 | 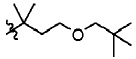 | 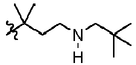 |
| 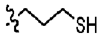 | 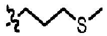 | 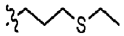 |
| 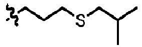 | 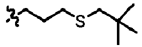 | 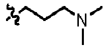 |
| 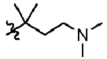 | 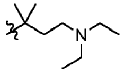 | 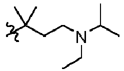 |
| 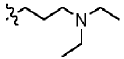 | 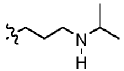 | 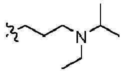 |
| 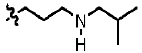 | 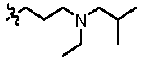 | 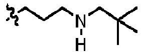 |
| 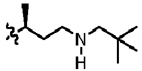 | 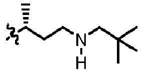 | 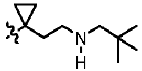 |
| 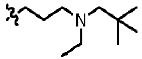 | 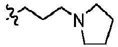 | 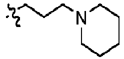 |
| 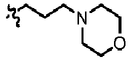 | 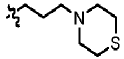 | 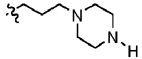 |
| 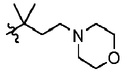 | 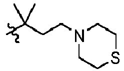 | 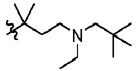 |
| 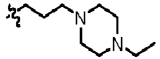 | 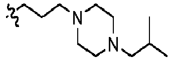 | 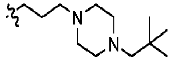 |
| 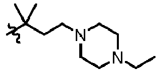 | 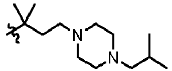 | 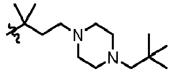 |
| 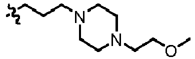 | 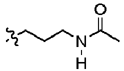 | 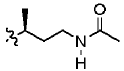 |
| 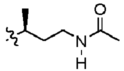 | 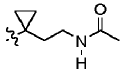 | 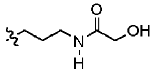 |
| 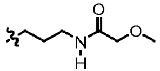 | 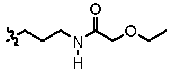 | 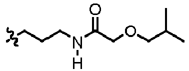 |
| 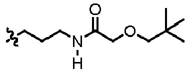 | 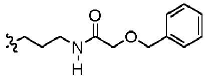 | 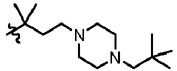 |
| 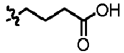 | 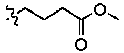 | 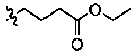 |
| 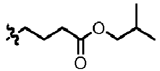 | 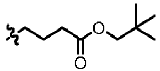 | 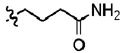 |
| 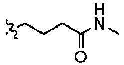 | 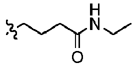 | 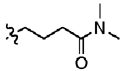 |
| 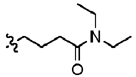 | 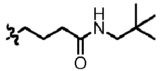 | 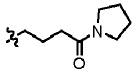 |
| 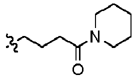 | 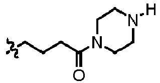 | 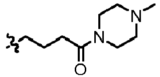 |
| 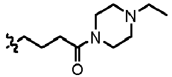 | 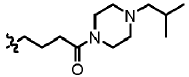 | 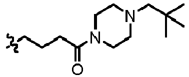 |
| 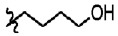 | 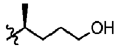 | 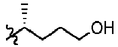 |
| 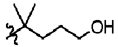 | 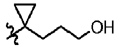 | 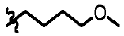 |
| 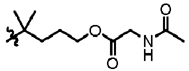 | 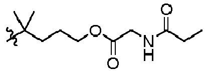 | 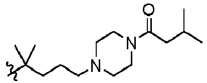 |
| 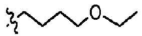 | 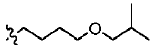 | 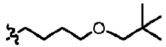 |
| 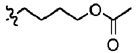 | 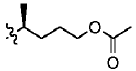 | 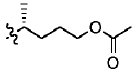 |
| 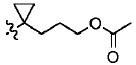 | 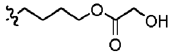 | 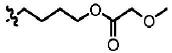 |
| 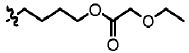 | 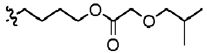 | 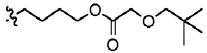 |
| 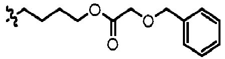 | 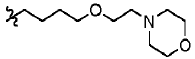 | 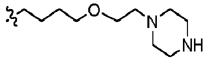 |
| 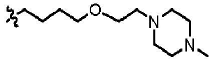 | 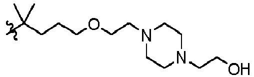 | 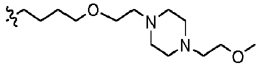 |
| 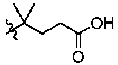 | 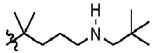 | 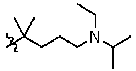 |
| 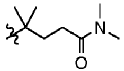 | 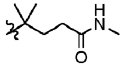 | 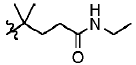 |
| 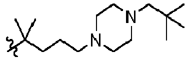 | 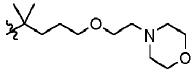 | 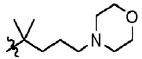 |
| 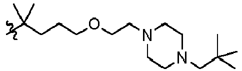 | 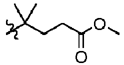 | 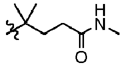 |
| 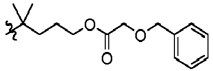 | 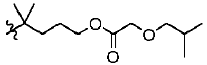 | 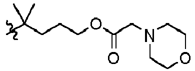 |
| 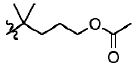 | 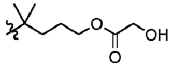 | 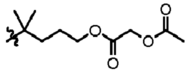 |
| 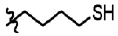 | 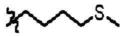 | 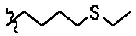 |
| 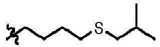 | 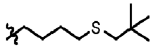 | 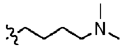 |
| 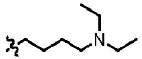 | 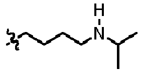 | 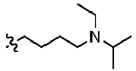 |
| 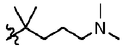 | 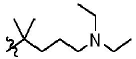 | 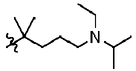 |
| 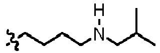 | 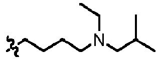 | 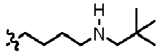 |
| 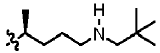 | 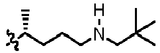 | 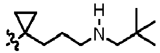 |
| 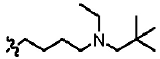 | 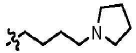 | 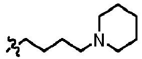 |
| 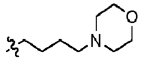 | 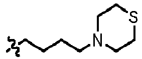 | 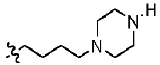 |
| 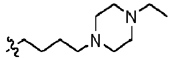 | 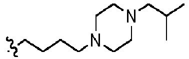 | 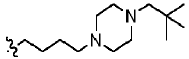 |
| 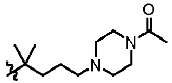 | 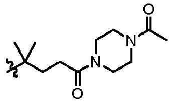 | 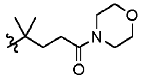 |
| 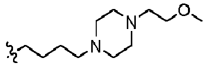 | 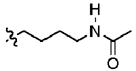 | 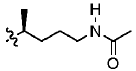 |
| 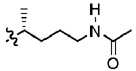 | 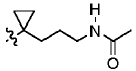 | 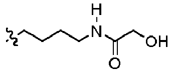 |
| 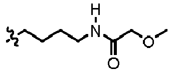 | 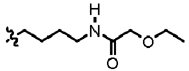 | 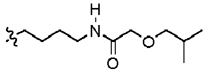 |
| 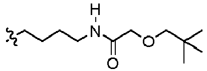 | 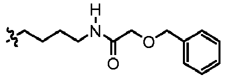 | 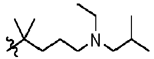 |
| 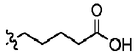 | 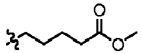 | 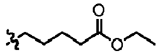 |
| 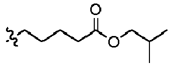 | 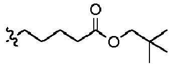 | 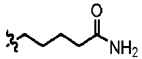 |
| 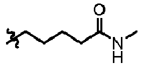 | 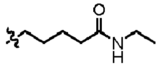 | 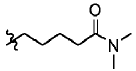 |
| 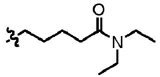 | 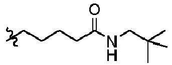 | 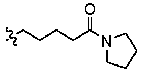 |
| 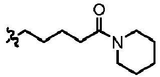 | 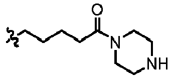 | 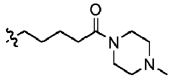 |
| 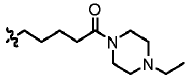 | 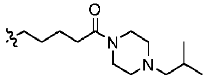 | 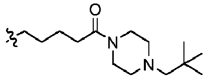 |
| 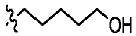 | 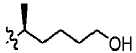 | 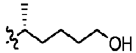 |
| 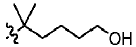 | 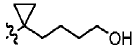 | 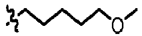 |
| 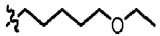 | 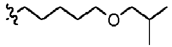 | 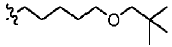 |
| 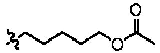 | 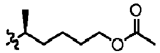 | 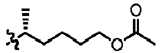 |
| 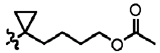 | 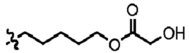 | 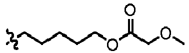 |
| 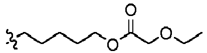 | 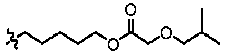 | 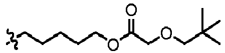 |
| 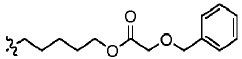 | 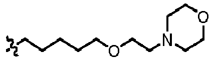 | 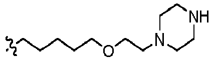 |
| 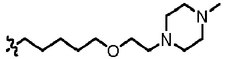 | 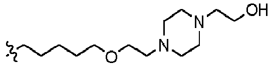 | 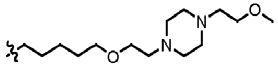 |
| 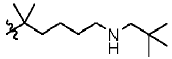 | 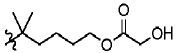 | 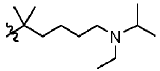 |
| 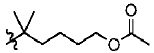 | 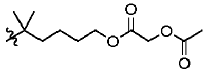 | 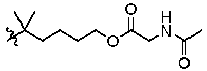 |
| 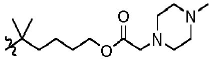 | 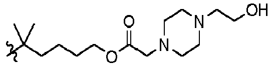 | 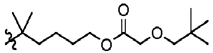 |
| 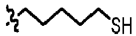 | 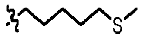 | 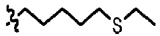 |
| 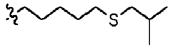 | 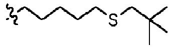 | 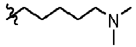 |
| 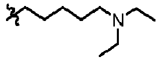 | 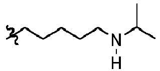 | 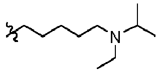 |
| 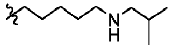 | 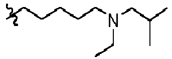 | 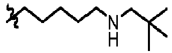 |
| 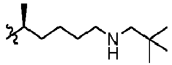 | 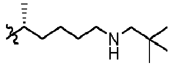 | 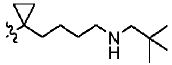 |
| 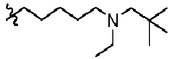 | 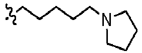 | 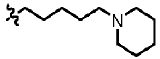 |
| 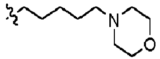 | 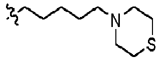 | 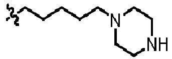 |
| 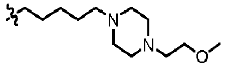 | 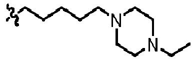 | 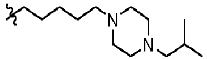 |
| 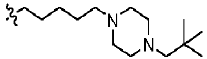 | 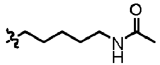 | 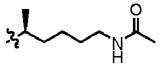 |
| 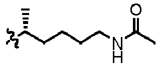 | 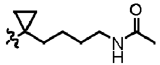 | 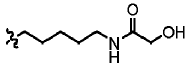 |
| 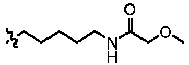 | 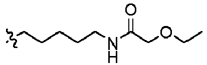 | 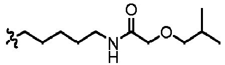 |
| 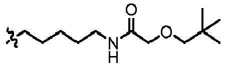 | 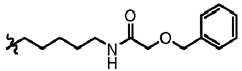 | 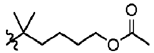 |
| 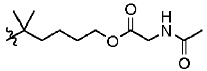 | 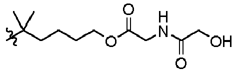 | 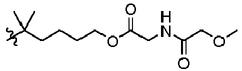 |
| 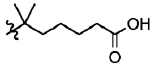 | 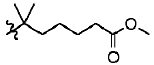 | 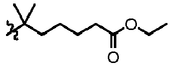 |
| 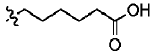 | 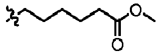 | 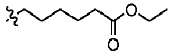 |
| 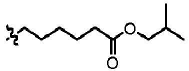 | 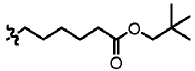 | 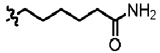 |
| 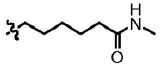 | 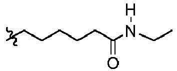 | 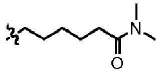 |
| 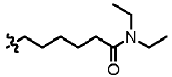 | 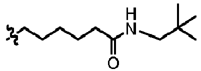 | 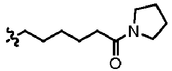 |
| 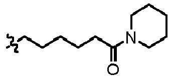 | 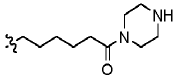 | 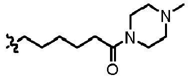 |
| 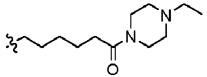 | 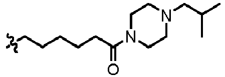 | 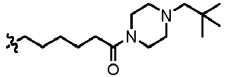 |
| 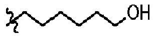 | 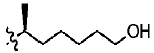 | 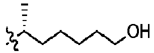 |
| 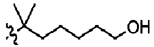 | 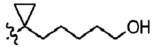 | 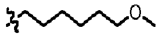 |
| 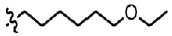 | 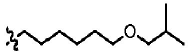 | 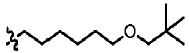 |
| 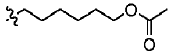 | 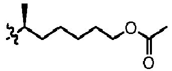 | 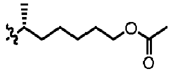 |
| 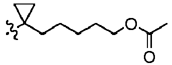 | 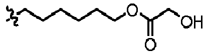 | 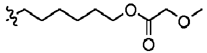 |
| 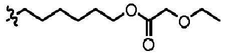 | 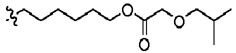 | 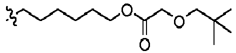 |
| 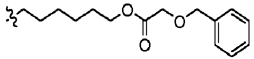 | 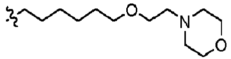 | 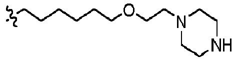 |
| 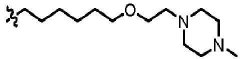 | 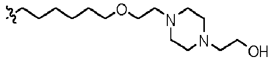 | 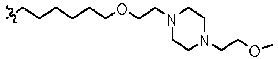 |
| 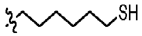 | 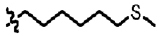 | 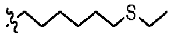 |
| 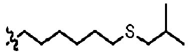 | 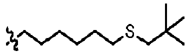 | 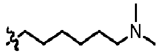 |
| 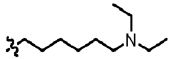 | 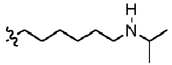 | 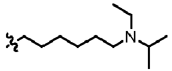 |
| 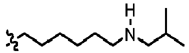 | 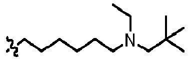 | 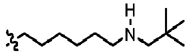 |
| 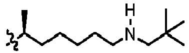 | 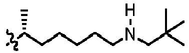 | 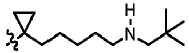 |
| 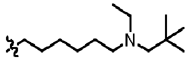 | 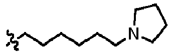 | 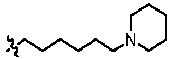 |
| 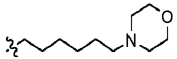 | 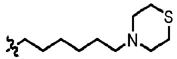 | 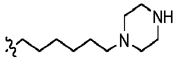 |
| 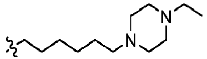 | 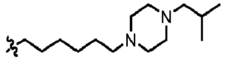 | 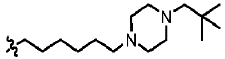 |
| 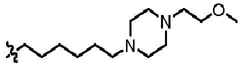 | 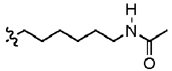 | 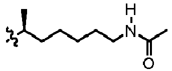 |
| 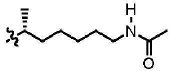 | 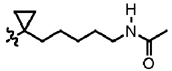 | 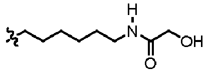 |
| 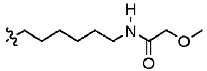 | 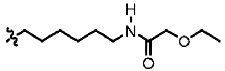 | 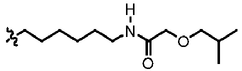 |
| 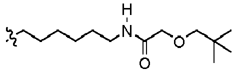 | 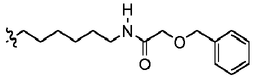 | 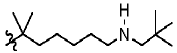 |
| 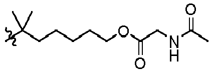 | 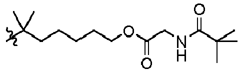 | 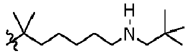 |
, or a pharmaceutically acceptable salt thereof.

**13.** The compound of claim 1, having the structure of 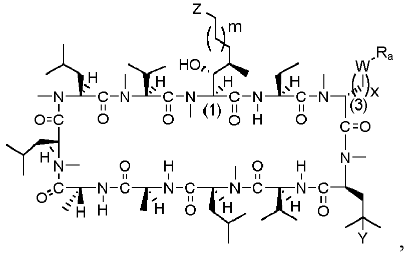 wherein
Z is OH, OMe, OEt, OCH₂CMe₃, OCH₂CH₂OH, OCH₂CH₂OMe, OCH₂CH₂OEt, OAc, OCOCH₂OH, OCOCH₂OMe, OCOCH₂OEt, OPh, OPh-3-OMe, OPh-4-OMe, OCH₂Ph, OCH₂Ph-3-OMe, OCH₂Ph-4-OMe, NHMe, NMe₂, NHEt, NEt₂, NHCH₂CMe₃, NHAc, NHCOCH₂OH, NHCOCH₂OMe, NHCH₂COOH, NHCH₂COOMe, NHCH₂COOEt, N(CH₂COOH)₂, N(CH₂COOMe)₂, or N(CH₂COOEt)₂;
x is 1; m is 2, 3, 4, 5, 6, 7, or 8;
Y is H, OH or OMe;
W is O or S; and
Rₐ is 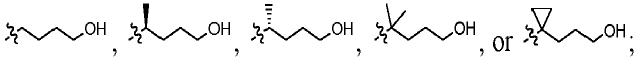 or a pharmaceutically acceptable salt thereof.

**14.** The compound of claim 1, wherein W is S; x = 1; and 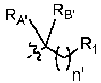 is 4-hydroxybutyl.

**15.** The compound of claim 1, wherein the compound is

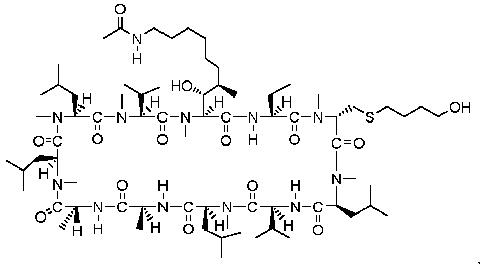

**16.** The compound of claim 1, wherein the compound is 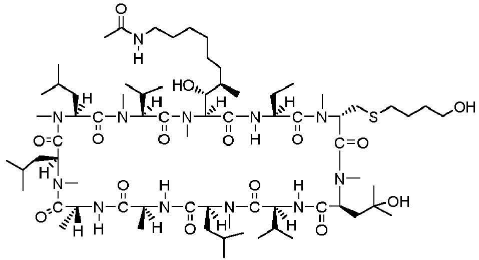 or

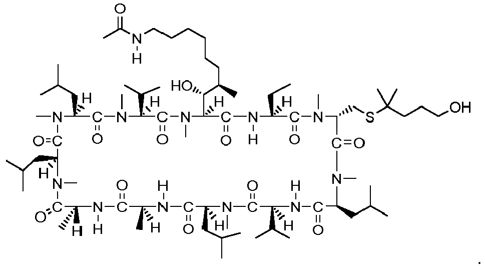

**16.** A pharmaceutical composition comprising at least one compound according to any one of the preceding claims and a pharmaceutically-acceptable carrier or diluent.

**17.** A compound of any one of claims 1-15 for use in treating or preventing a disease in a mammalian species in need thereof, wherein the disease is selected from the group consisting of a viral infection, inflammation, respiratory inflammation, rheumatoid arthritis, dry eye, a neurodegenerative diseases, traumatic brain injury, stroke, ischemia-reperfusion injury in the brain, heart, or kidney, myocardial infarction, cardiovascular diseases, vascular stenosis, atherosclerosis, abdominal aortic aneurysms, cardiac hypertrophy, aortic rupture, pulmonary arterial hypertension, myocarditis and myocardial fibrosis, ischaemic heart disease, cancer, obesity, diabetes, muscular dystrophy, lung disease, liver disease, kidney disease, and hair loss.

**18.** The compound of claim 17, wherein the viral infection is a hepatitis B virus infection, a hepatitis C virus infection, or a HIV infection; or
wherein the neurodegenerative disease is Alzheimer's disease, Parkinson's disease, Huntington's Diseases, or ALS.
